(19) Europäisches Patentamt
European Patent Office
Office européen des brevets

(11) Publication number: **0 332 322 A2**

(12) # EUROPEAN PATENT APPLICATION

(21) Application number: 89301799.6

(51) Int. Cl.[4]: G06F 15/40

(22) Date of filing: 23.02.89

(30) Priority: 26.02.88 US 161188
03.10.88 US 252881

(43) Date of publication of application:
13.09.89 Bulletin 89/37

(84) Designated Contracting States:
AT BE CH DE ES FR GB GR IT LI LU NL SE

(71) Applicant: ELSEVIER SCIENCE PUBLISHING CO., INC.
655 Avenue of the Americas
New York, N.Y. 10010(US)

(72) Inventor: Bodick, Neil
129 Cuthbert Street
Philadelphia Pennsylvania 19106(US)
Inventor: Marquis, Andre L.
501 Dorset Road
Devon Pennsylvania 19333(US)

(74) Representative: Kearney, Kevin David Nicholas et al
KILBURN & STRODE 30 John Street
London, WC1N 2DD(GB)

(54) **Data creating, storage and processing system.**

(57) Apparatus for the creation, editing and utilising a knowledge base which contains textual information, quantitative information and pictorial images. A computer system comprised of text and image input devices, a display, a mass storage unit for the knowledge base and a programmed processor. When used as an expert authoring system, for example, in medical applications, features which are characteristic of respective conditions in a disease are established by the author and values are assigned thereto, thus creating a knowledge base of features having associated values. This created information is used by the author to evaluate what he observes in a patient or histological sample. The "valuing" of these previously established features within the parameters for values that also were established previously form a case record that also includes one or more pictorial images of valued features. The retrievability of case records and editing of valued features insures internal consistency of the knowledge base.

FIG. 1

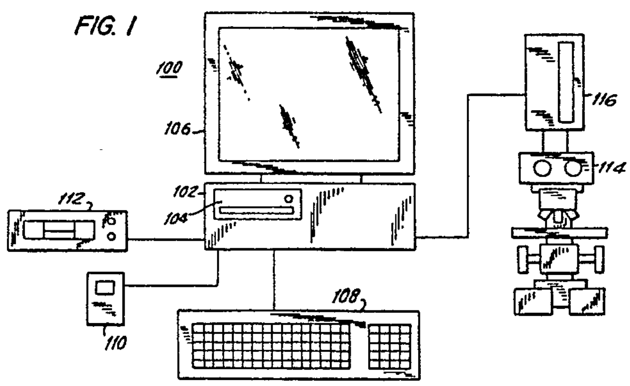


EP 0 332 322 A2

# DATA CREATING, STORAGE AND PROCESSING SYSTEM

## Field of the Invention

This invention relates to the field of computerised data processing and, more particularly, to a data creating, storage and processing system which operates with both pictorial image and textual information data. Moreover, the invention provides an interactive system to create, edit and utilise a knowledge base that finds particular application in medical diagnosis, and especially pathology and radiology diagnosis.

## Background of the Invention

Electronic information storage systems having data bases which can be accessed by computer are well known. Some examples of accessible data bases are the Lexis legal research system, a data base containing legal information, and the Dialog information retrieval system, which provides access to numerous scientific and business data bases. These and several other data bases have succeeded in offering the user a vast amount of textual information which is rapidly accessible and relatively inexpensive to retrieve. However, the ability to search electronically for desirable information in these data bases often requires extensive training and skill.

With some notably few exceptions, such as the experimental patent search and retrieval system of the United States Patent and Trademark Office, most data processing systems do not store and thus are unable to display pictorial or other photographic/visual information.
One of the reasons for this is that such image information generally requires a great deal of computer memory to store it. Although computer memory has been a decreasing cost in computer systems recently, it is still an important factor in system design and application and the amount of storage required for images is very large. Also, as a consequence of the large amount of memory space required to store images, the time required to access, process and display image information can be lengthy, so lengthy as to be unacceptable.

In addition to their failure to provide pictorial image display, most, if not all data processing systems that are available commercially provide limited data access tools for the user to retrieve information. This has influenced the use of these systems because there has been a general inability to assure internal consistency of the information represented therein. For example, consistency of data is extremely important for applications wherein that data represents observations by a human author in a subject under investigation. Since such observations are inherently subjective to some degree -- the characterization of what one individual observes will not necessarily be the same as the characterizations observed by another, and what one perceives today may differ from his perceptions tomorrow --it is important that the user of the data processing system have the ability to verify his observations and conclusions over a period of time, and to correct or modify those observations and conclusions for the sake of consistency. Consequently, the users of such systems and data bases in scientific disciplines may not have sufficient confidence in the reliability of the data therein or the dependability of observations, conclusions, characterizations or deductions that may be included in the data base.

A useful field of scientific endeavor in which a text and pictorial data processing system finds ready application is medical diagnosis. A physician, even a specialist, often needs assistance in diagnosing a medical condition or disease based upon his clinical observations of a patient. Of course, reference to various textbooks, learned journals and other sources of expert information are quite useful and, of course, have been relied upon, but the wealth of such information now available is substantially limited by the lack of any useful resource to access it. In general, state-of-the-art electronic libraries, although proposed for medical research, have not been enthusiastically embraced by many practicing physicians. It is believed that this is due, in part, to the high cost of providing a data processing system with a centralized, accessible data base, in part to the need for special training in using computerized systems for data retrieval and in part to the lack of existing reliable data bases. Nevertheless, the primary reason for limited use of such electronic libraries by physicians appears to be attributed to the lack of confidence in the reliability of medical data that may be stored therein, such as diagnoses based upon clinical or histological observations, characterizations and conclusions. There is little assurance that such data is internally consistent or authenticated or even verifiable; and it is difficult to build an internally consistent data set that is created with the authenticity needed for diagnoses.

The field of pathology offers an excellent opportunity for a data creating, storage and processing system to create and use a data base which would assist the pathologist in identifying characteristics associated with various diseases and in formulating his diagnosis. An internally consistent data base, that is, a knowledge base, created by an expert from case histories of numerous patients would provide a pathologist with reference materials for comparison with his present observations, thereby facilitating his diagnosis. Preferably, the data processing system should operate with both textual and pictorial information derived from clinical features, histological designations and features observed from cytology specimens, thereby providing the best evidence of cases which resulted in specific diagnoses by the expert. The knowledge base should be dynamic in the sense that during its creation, the data processing system is operable to add thereto newly discovered characteristics observed in patients and to delete therefrom other characteristics which prove to be of lesser diagnostic importance, thereby improving, over time, the utility of the data base in aiding a pathologist in identifying maladies and diseases. Additionally, the data processing system should be interactive not only to permit use thereof by the pathologist but also to provide the expert who is creating the knowledge base with the retrieval tools needed to observe and compare previous observations with present analyses so as to maintain consistency of the information in the knowledge base. Since histological and cytological characterizations are, to some extent, subjective, it is useful for the expert to compare a feature which he observes in one patient or sample to the same feature which he observed in a previous patient or sample. For example, it may be important to characterize the shape of a specimen cell; and what might presently be observed as an oval or round cell might previously have been characterized as an irregular cell.

It is believed, however, that at the present time no interactive system is available with the appropriate access tools for use by an expert to craft a knowledge base which that expert, as well as a jury of experts, can scrutinize for consistency and authenticity, and for subsequent access and use in the diagnosis of disease. More broadly stated, it is believed there are no presently available interactive systems for providing a knowledge base for use as a computerized aid to the cognitive process of diagnosis. Nor is there known to be available an electronic system for use by an expert to create a specific knowledge base of information which, when accessed by a user, serves as an aid in cognitive analysis of observed characteristics.

A knowledge base, as that term is used herein, differs from the classic concept of a data base in that the information represented by the knowledge base may be crafted to assure its consistency, and is based upon the recognized "knowledge" of the individual(s) who creates it.

## Objects of the Invention

Therefore, it is an object of this invention to provide apparatus for crafting a knowledge base which contains textual, quantitative and pictorial information that is internally consistent and which can be accessed rapidly.

It is a further object of this invention to provide a data processing system which creates, stores and processes textual, quantitative and pictorial information and which retrieves information corresponding to a particular request by a user.

It is another object of this invention to provide a computerized system which stores and processes textual and quantitative information on and pictures of various diseases and which a pathologist or other physician would use with a high degree of confidence.

Still another object of this invention is to provide a computer system which creates particular overlay images and links those overlay images to pictorial images to direct the user to important features present in electronically displayed pictures.

Another object of this invention is to provide a system that creates, retrieves and displays pictorial images helpful in diagnosing a disease, wherein relevant portions of the images are highlighted by symbols and annotated with text.

A further object of this invention is to provide a system for creating a knowledge base which, when accessed, combines retrieved alphanumeric and pictorial information into individual, separately viewable video displays.

It is an additional object of this invention to provide a computerized pathology data processing system which presents both textual information and related visual imagery for simultaneous display.

A further object of this invention is to provide a system for storing linked alphanumeric and visual information and which organizes the stored information into easily accessible categories.

A still further object of this invention is to provide a system which allows the user to select the manner and format used to organize alphanumeric information and visual images.

Another object of this invention is to provide a system for use by an expert pathologist to create and structure a computerized pathology knowledge base containing both text and visual images in a way which the expert believes will provide the most logical access to the information therein.

An additional object of this invention is to provide a system by which a user may modify or change the contents and organization of a stored knowledge base with a minimum number of computer commands.

Still another object of this invention is to provide a system for crafting a knowledge base comprised of alphanumeric and visual information which can be accessed and used to permit easy comparison between stored data and new data.

Yet another object of this invention is to provide a system for training a user in a particular field or domain represented by a knowledge base by providing a structured and pre-planned examination of relevant data from within the knowledge base.

A further object of this invention is to provide a system which uses artificial intelligence to help assist the user in accessing and retrieving data from the knowledge base.

Still another object of this invention is to provide a computerized system which assists a physician to reach a diagnosis using a computerized pathology knowledge base that contains both text and visual images.

Yet a further object of this invention is to provide a system for creating an electronically accessible knowledge base formed of case records of diseases, wherein each case is formed of features observed in a disease and each feature is either selected from a store of pre-established features and assigned one of plural preset values available for that feature, or is newly created by an author/user.

An additional object of this invention is to provide a system for editing a knowledge base to add or delete features from substantially all stored case records with a minimum of computer commands.

Another object of this invention is to provide a system for linking the several features and values included in a case record and for retrieving a case record on the basis of selecting for display a feature included in that case record, whereby consistency in evaluating a feature in different case records is improved.

A still further object of this invention is to provide a system for generating a video image data of a subject, such as a microscope specimen, linking that image to a feature and storing the image in a case record for subsequent retrieval and display to demonstrate to a user the previously observed feature.

Yet an additional object of this invention is to provide a system for searching a knowledge base for all case records which contain one or more features selected by a user for retrieval and display of those case records, and particularly pictorial information included in those case records.

It is an additional object of this invention to provide a system for displaying the distribution of cases having different values of selected feature(s) to indicate to the user the relative numbers of cases containing such different values and thereby apprising the user of the behavior of this feature in a particular diagnosis.

Another object of this invention is to provide a system for characterizing features as discrete or continuous, and for selecting values from a store of preset values or enabling an author/user to freely select or measure the value.

Broadly stated, this invention is directed to a data creating, storage and processing system comprising: image pick-up means for imaging one or more features observed in a sample of a disease and for producing pictorial image data representative thereof for display and for storage; display means for displaying pictorial images of one or more features observed in a disease and for displaying case record data; storage means for storing case record data, each case record including a case record identification, feature data selected for that case, value data selected for respective features in that case, and pictorial image data representing pictorial images of features of a disease associated with that case; manually operable input means including: case identifying means for generating case identifying data to identify a case record written into and retrievable from said system, feature selecting means operable by a user to select from a feature bank those feature data which the user observes in one or more diseases represented by a case, and value assigning means operable by said user to select from a value bank value data which the user observes in the selected feature; and processing means for linking feature data, value data and pictorial image data to case records, for retrieving case records, and for displaying pictorial images associated with retrieved case records.

In accordance with one application of this invention, a medical/pathology knowledge base containing textual and pictorial information on various diseases is created and stored in computer-readable form. In one embodiment, the knowledge base includes user-selectable designations of diagnostic features which the author of the knowledge base (i. e. an "expert" author) determines are characteristic of respective conditions to be diagnosed. These features preferably comprise a case record of a patient diagnosed with a particular disease; and several case records are stored and retrieved for subsequent display of the features therein and of one or more pictorial images of those features. The knowledge base is used with a high speed computer system whose memory preferably includes a compact disc laser recorder/playback system. The laser disc (known as a "WORM") provides a very large memory suitable for storing pictorial images with relatively rapid access thereto. These pictorial images are linked to case records which may be stored in other storage devices, such as magnetic disc, whereby the retrieval of a case record accesses the pictorial images linked thereto and the retrieval of a pictorial image accesses the linked case record, thereby displaying the pictorial image and features of that case record.

Preferably, the knowledge base is organized and codified by a recognized expert in the field, for example, in pathology. The invention provides the expert with a highly flexible format and data access tools which allows the expert to structure the data in almost any manner he believes will provide the easiest and most rapid access thereto, thereby helping users of the knowledge base to reach correct diagnoses. Creation and editing the knowledge base can be made with only relatively few computer commands.

The present invention also provides the user/pathologist with numerous ways to access and use the knowledge base. Information on specific diseases may be requested, such as a request for retrieval of all cases having specified features or a request for all cases in which a particular disease was diagnosed. Side-by-side presentation of pictorial images and display of text information relating to different diseases or features assist evaluation and diagnosis.

The preferred embodiment of this invention includes a programmable processor which is in data communication with an input means, a display and an accessible storage means, the processor being programmed to respond to the input means to establish user-selectable designations of diagnostic features which the author determines are characteristic of respective conditions to be diagnosed and to establish user-selectable values of those features. Pictorial image data produced by an image input means is linked by the processor with one or more features and is stored in the storage means. Preferably, the image data is stored on the aforementioned WORM device, while the feature data is stored in another storage device, such as a magnetic disk.

In one aspect of this invention, the processor is programmed to cause the display to provide prompts for guiding the author in selecting respective ones of established features and to assign an established value to a selected feature which the author observes in a pictorial image. The processor is further programmed to enable the author to create new features (i. e. features which had not been established and stored previously) and to assign new values to those newly created features. One characteristic of this aspect is that the knowledge base created by the processor is comprised of case records, each case including feature, value and (in most but not necessarily all cases) pictorial image data; and the addition of a new feature to one case results in the addition of that feature to all cases, this newly added feature awaiting assignment of a value in such other cases. Likewise, the deletion of a feature from one case (as opposed to merely the assignment thereto of a "0" value) results in the deletion of that feature from all cases.

In accordance with another aspect of this invention, the linking of features and pictorial image data in a case record cooperates with the programming of the processor to retrieve all case records having the feature(s) selected by the author, from which case records the author may display text and pictorial information contained within a desired one of those retrieved case records.

In accordance with yet another aspect of this invention, the processor is programmed to respond to the input means to generate overlay data which, in turn, is displayed as an overlay superimposed over a pictorial image for the general purpose of identifying with greater particularity a particular feature which is included in the displayed pictorial image. The processor is programmed to link the overlay data with the pictorial image data and to store that overlay data such that subsequent retrieval of this pictorial image data will be accompanied by the retrieval of its associated overlay.

As yet another aspect of this invention, the processor is programmed to retrieve all case records having the particular feature(s) selected by the author; with the expectation that the values of such feature(s) normally will vary from one case to another. The processor is programmed to determine the distribution relative to each other of such cases having different respective values for the feature(s) and to display such distribution. Hence, the user of this invention is provided with an indication, such as a graph-type display, of the number of cases in which a particular feature exhibits a first value, the number of cases in which that feature exhibits a second value, and so on. For example, if the feature in question is age of a patient, a display is provided of the number of cases in which the patient is less than 20 years old, in which the patient is between 20 and 30 years of age, in which the patient is between 30 and 40 years of age, etc. These cases may be, for example, of those patients having non-inflammatory cells of a particular size and shape and of particular pleomorphic size and in which non-inflammatory cells exhibit a certain type of chromatin pattern, etc. The processor is additionally programmed to search for case records having those particular features which are entered by the user, in accordance with typical boolean combinations of those features (e. g. all cases having each and every one of the entered features or all cases having at least one of those entered features).

In accordance with yet another aspect of this invention, each case record may be linked with several pictorial images all of which may be retrieved from the storage means when the case record is accessed. As one characteristic of this aspect, all of the linked pictorial images are displayed concurrently, in relatively reduced size, and the processor is programmed to permit the user to select one of those reduced size images (as by operating the input means) for display in relatively magnified size. Thus, pictorial displays of different features contained in the case record, together with attendant overlays, may be selected, as desired, by the user.

As yet another aspect of this invention, the features which may be included in a case record may be characterized as exhibiting discrete or continuous values. The processor is further programmed to permit editing of already established features or newly created features; whereupon when a feature is designated by the author as having a discrete value, preset value data that has already been provided in the knowledge base is displayed for assignment by the author to the feature or, alternatively, the author may operate the input means to enter into the knowledge base a new discrete value. Alternatively, if the feature is designated by the author as having a continuous value, prompts are displayed for the author to select a range for the continuous value which may be assigned to that feature.

In accordance with a still further aspect of this invention, the processor is additionally programmed to measure a value for a feature. For example, the feature may be the length of a cell or the distance between substances observed in a cell; or the feature may be the area of a cell or cell nucleus or diseased portion of tissue; or the feature may be the density of certain material in a designated portion of a cell or cell tissue. Such length, area, density is measured by the processor.

In accordance with yet another aspect of this invention, to simplify the storage and display of features, the processor is programmed to establish author-selectable categories of features, each category being comprised of a unique set of features. Operation of the input means is used to establish such categories and to link an established category to those features which the author determines should be included therein. Hence, for review or display purposes, the author need not access each and every feature from the knowledge base, particularly if several features are not of interest to the author. Rather, the author merely need retrieve the far lesser number of categories from the knowledge base and then may select the particular category from which features are to be reviewed or displayed. The processor is additionally programmed to add or delete categories from the knowledge base, thereby adding or deleting those categories from the individual case records stored in the knowledge base.

## Brief Description of the Drawings

The following detailed description, given by way of example, and not intended to limit the present invention solely to the described embodiments, will best be understood in conjunction with the accompanying drawings in which:

FIG. 1 is a block diagram of one embodiment of the system of the present invention; and

FIGS. 2-30 are representations of various displays, or computer screens and accompanying flow charts which describe the manner in which the screens are produced and the knowledge base is crafted.

## Detailed Description of Preferred Embodiment

The present invention finds ready application in the field of pathology. Consequently, it is described in that environment. However, it will be apparent that the presentation of pictorial images in conjunction with textual data which relate to those images and assist in the evaluation of them is valuable in any area where the appearance of an object under study/examination is of critical importance. These areas may include other disciplines of medicine, such as dermatology and ophthalmology, as well as such non-medical areas as engineering, art history and biology, to name but a few examples. It will, therefore, be appreciated that this invention finds general utility in the cognitive process of diagnosis.

Although the equipment and computer components used in this invention are conventional and known in the art, it is nonetheless useful to describe the hardware, thereby placing the invention in its operational environment.

Referring now to FIG. 1, the present invention is comprised of a computerized authoring system 100 formed of a processor 102 which cooperates with a magnetic storage device 104, a monitor 106, a keyboard 108, a manually movable cursor controller 110 and a video camera 116. Processor 102 preferably is a microcomputer such as a Sun model 3/110 supermicrocomputer manufactured by Sun Microsystems operating under the Unix 4.2/5 operating system software and which uses an I.E.E.E. 696 bus with 1 megabyte of random access memory. Those of ordinary skill in the art, familiar with the Unix-controlled Sun microcomputer will recognize such advantageous operating features as layering of programs and displays, background operation of programs, and use of a manual cursor controller (conventionally known as a "mouse"), among others. Processor 102 is provided with graphics computer circuit cards which can capture, digitize, display and process a 512 x 512 x 8 bit image.

To capture images, a microscope 114, such as a Leitz 12, is fitted with a trinocular adapter for video interface with video camera 116, such as an Ikegami RGB camera. Preferably, the video signals produced by camera 116 are digitized either by a suitable analog-to-digital converter included in or coupled to the camera, or by a converter provided in processor 102. Video images are displayed on monitor 106 which is a high resolution RGB monitor adapted to display 1100 x 900 pixels.

The system also includes a video disk recorder/player 112, such as an optical disk Write Once Reproduce Many (WORM) device. The very large storage capacity of this device (e. g. on the order of about 1000 M bytes), coupled with its rapid access time, make the optical disk an ideal medium for the storage of images derived from video camera 116. Roughly 2000 images each of 512 x 480 x 8 bits can be stored on a single optical disk. It will be recognized that a library of prepared optical disks enables the illustrated apparatus to be used in diagnosis, analysis or "problem-solving" in a particular field or discipline (i. e. in a particular "domain") for which the disks have been prepared.

Although other computers and a different selection of peripheral equipment may be used in the present invention, such equipment should be selected with an awareness that high processing speeds and large memory capacity are important considerations.

The system shown in FIG. 1 relies upon image compression techniques to enhance its video image storage capability. This results in an effective increase in the number of images that may be stored on optical storage device 112. The storage and manipulation of visual images, either color or black-and-white, is known in the art. Such images are typically processed to form a pattern of pixels, with each pixel representing the color or black and white value at a particular location. For example, in the present invention, images are divided in rectangular grids measuring 512 pixels in length and 480 pixels in width, with a total of approximately 246,000 pixels being used to represent the image. In a typical black and white image system, each of these pixels is assigned a value of from 0 to 255, with 0 representing black and 255 representing white (or vice versa). In terms of the amount of memory required to store such an image, each pixel requires 8 bits to represent its darkness value. This results in a memory requirement of approximately 220 K bytes per image. In a typical color system, the image is formed of the three primary colors, red, green and blue, with each pixel in the image being assigned an 8 bit value for each of the three colors. This method of digitizing a color image requires approximately 775 K bytes to store a single color image. Although the cost of memory has decreased markedly, even with optical storage devices such as the laser compact disc recorder/player, which has both a very large storage capacity as well as rapid access to stored information, storage of color images in the conventional manner would require too much time both to manipulate the images and to access them.

The present invention employs a technique for digitizing and storing visual images which reduces the storage requirement of a single color image from 775 K bytes to 220 K bytes yet retains most of the information provided in the original image and provides a highly acceptable video image for display on monitor 106. Although the number of bits required for each original image is still large, a useful number, roughly 2000 images, can be stored on a single WORM device, and the processing and presentation of a single image may be performed in less than two seconds, which is an acceptable time delay.

Color video camera 116, which is mounted on microscope 114, supplies video signals to processor 102 comprising three separate images, a red image, a blue image and a green image, where each pixel in each image is represented by an 8-bit value designating its respective red, green and blue value. Once a color "plane" (image) has been supplied tc processor 102, a data reduction, or compression, process is carried out. For the purpose of the presently described embodiment of this invention, namely a knowledge base system for pathology, it has been determined that adequate color accuracy will be achieved if only 6 shades of red, 7 shades of green and 6 shades of blue, each shade being of increasing color intensity, are provided. Of course, the distribution of color shades may be altered or modified as desired.

As the data reduction process operates in substantially the same way on each of the three color images or "planes" which comprise a single visual image, only the red "plane" processing will be discussed. The red plane is acquired as a 512 X 480 pattern of pixels, with each pixel having a value of 0 to 255, thus providing 256 shades of red. However, only 6 shades of red need be stored. Instead of each pixel having a value representing one of 256 shades of red, each pixel of the red plane image is provided with a value representative of only one of 6 shades of red. Each of these shades represents the maximum shade of the color within a given range of the original 256 shades. The original 256 shades are divided into six ranges with each range encompassing 43 original shades (6 x 43 = 258). For six equal ranges, the original 256 shades are divided into (0-42), (43-85), (86-128), (129-171), (172-214) and (215-257), and these ranges are represented simply as the values 42, 85, 128, 171, 214 and 257. For example, if the original pixel had a "shade" value of 70, it would be assigned a value of 85, which represents the closest shade of red within the range (43 - 85) into which the original shade fell. Similarly, if the original pixel had a shade value of 51, it would be assigned a value of 42. Hence, these values are selected to divide the entire range of shades in a color plane into 6 evenly distributed ranges.

A potential negative consequence of this compression process is that resolution and contrast may be reduced because some visual information is lost when a range reduction process of this type occurs. To minimize this problem, an error averaging process is used. Each time a pixel's color value is reduced or increased to a value in the relevant range, the resulting net error is divided among the pixel's immediate neighbors, thus lightening or darkening their shade of color prior to their being processed. In terms of the array of pixels which comprises the image, the pixel to the right of the pixel being processed, as well as the pixels vertically below it receive a portion of the total error. For example, assume a given pixel has a red value of 74. As 74 is closer to 85 than 42, this pixel receives a value of 85. However, this introduces an error of 11 shades of red. As the final value of red assigned to this pixel is too large, the four neighbors of this pixel, designated as $N_1$-$N_4$, have their values reduced in the following manner: 7/16 of the total correction is applied to $N_1$ (the pixel to the right), 3/16 to $N_2$ (the pixel below and to the left), 5/16 to $N_3$ (the pixel below), and 1/16 to $N_4$ (the pixel below and to the right). This is described more completely in "An Adaptive Algorithm for Spatial Greyscale" by Robert W. Floyd and Louis Steinberg. Other methods of distributing the collective errors could be used. In this case, the approximate result would be, that $N_1$'s shade would be reduced by 5, $N_2$'s shade by 2, $N_3$'s by 4 and $N_4$'s by 0. As can be appreciated, at the borders of the image, although the calculation of the corrections remains the same, corrections which would apply to pixels beyond the border of the image are discarded.

This method of reducing the number of color shades while maintaining resolution and contrast is applied to the green and blue color planes in the same manner is performed on the red plane. The final processing step combines the three color values into one 8 bit value per pixel. This is done by using a so-called "lookup" table, which resides in the system hardware, to assign a number from 0 to 255 which represents each particular combination of the 6 red, 7 green, and 6 blue shades. Consequently, the requirement for storing essentially three separate color images, each requiring 220 K bytes of memory, is reduced to storing only one image of 220 K bytes, without any serious deterioration of the image contrast or resolution.

Referring again to FIG. 1, processor 102 is in data communication with each of monitor 106, keyboard 108, cursor controller 110 (referred to herein by its conventionally understood designation of "mouse"), optical storage device 112 and video camera 116. The program provided for processor 102, described in greater detail below, enables the illustrated apparatus to function as a computerized authoring system by which a user, also referred to as an "author" or "expert", operates keyboard 108, mouse 110 and video camera 116 to create a knowledge base adapted to be stored and retrieved from magnetic disk storage 104 and from optical disk storage 112. In this regard, the magnetic disk storage preferably is formed as a so-called hard disk drive, several models of which are manufactured and commercially available from various sources. As will become apparent, the knowledge base is comprised of both textual and pictorial information; and it is convenient to store the textual information on hard disk 104 while storing the pictorial information on optical disk 112. Nevertheless, the pictorial information is sufficiently linked to the textual information as to be easily and readily read from the optical disk to display on monitor 106 particular characteristics which are identified by the textual information read from hard disk 104.

In the domain of pathology, for which the illustrated system is quite useful, the knowledge base created by the author who uses this system preferably is comprised of case records which document various diseases that the author has observed in patients. Of course, the purpose of this knowledge base is to assist in subsequent diagnoses by pathologists or other physicians based upon a comparison of the characteristics presently being observed with those characteristics included in the knowledge base. Accordingly, while the illustrated system is described herein as a computerized authoring system, it will be appreciated that this system is not limited solely to the function of "authoring", that is, the function of creating the knowledge base. Indeed, and as will become apparent from the ensuing discussion, system 100 operates readily as a reference system for the particular domain of case records which have been created, thus supporting the cognitive process of diagnosis, and also as a training system for the education and training of users in that domain.

In the interest of simplification, system 100 is described in the environment of pathology. When used as an authoring system, case records are created as a compilation of designations selected by the author of those diagnostic features which the author determines are characteristic of respective conditions, or diseases, to be diagnosed. These are referred to herein as "features" and each case record is formed of several features. It is expected that substantially all case records will include some of the same types of features, such as the identification of the case, the name of the patient, the eventual diagnosis, the identification of various biological cell analyses (the cytology number), date of cytology, the age and sex of the patient, as well as various other features which are relevant to the disease in question. It is convenient to classify various related features as "categories", wherein each category is comprised of a unique set of features. As examples, for the domain of cytology, the category of "patient descriptor" may include the features of age, sex and exposure to asbestos. The feature of "age" may be divided into ranges of "values", such as the ranges 0-20, 21-30, 31-40, 41-50, etc. The feature of sex may be assigned one of two values: male or female. The feature of asbestos exposure may be assigned the value of present, absent, questionable, unknown or other. As another example, the category of "current sites with positive cytology" may include the feature of "positive cytology, right pleura", with the possible values of present, absent, questionable, unknown and other. Another feature included in this category is "positive cytology, left pleura", with the possible values: present, absent, questionable, unknown and other. Another feature included in this category is "positive cytology, peritoneum", with the possible values: present, absent, questionable, unknown and other. Finally, another feature included in this category is "positive cytology, pericardium", with the values: present, absent, questionable, unknown and other. The various categories of features and possible values for these features for the domain of cytology is set out in Appendix A.

Prior to the formulation of even the first case record, the author, or expert, creates a glossary of categories, features included in each category, and the possible values to be assigned to each feature. This glossary, or dictionary (or sometimes described in the drawings as a thesaurus), is prepared on the basis of the expert's familiarity with the knowledge base domain in which he is an expert. It is expected that each case record subsequently formulated by the expert will include some, if not all, of the categories contained in the dictionary, and it is further expected that some of the features within a category simply will not be present in the case being documented. However, by first creating the dictionary of categories, features and values, the author is provided with a "template" of substantially all of the diagnostic features which should be addressed in documenting a case and in diagnosing a disease. From experience, it has been found that this template permits a case record to be created in about 15-20 minutes.

It will be recognized that some of the values assigned to a particular feature may be simply numerical values (e. g. age, number of cells in various groups, percentage of cells reacting with keratin, etc.); other values may be best described as "textual" (such as the value, or description, of cell shape -- which may be oval, round, irregular or variable -- or cytoplasm density --which may be variable, dense, delicate or pale -- or the chromatin pattern of a cell nucleus -- which may be clear, pale, even, finely granular, coarsely granular, hyperchromatic, irregular or variable -- etc.); and still other features may have values which must be measured (such as pleomorphic size of a cell, density of cell cytoplasm, or cell area). These values may be thought of as being discrete (a text description constitutes the value) or continuous (a numerical value), or calculated from measured parameters. This last type of value is described herein as a "scripted" value because the measurement thereof is determined on the basis of a "script" of specified procedures and algorithms. Once created by the expert, the dictionary contains substantially all of the features which the expert determines are most useful or influential in diagnosing diseases, together with substantially all of the values which may be assigned to each feature. For those cases wherein the preestablished features are not sufficient to characterize an observed condition, the expert operates the system, as will be described, to create a new feature and assign a value thereto. Advantageously, when a new feature is created, it is added to the dictionary and thus is included in the store of features (i. e. the feature bank) and is available to the expert for selection in characterizing observed conditions in future cases and for editing existing cases. For example, in observing cases of malignant mesthelioma, the expert might not have observed the halo inside the cell border of Pap-stained cytology specimens. Hence, the feature "inside halo" might not have been created and, thus, will not exist in the data base dictionary. If, during observations of other cases of malignant mesthelioma, the expert now finds "inside halo" in Pap-stained cytology specimens, this feature may be added to the data base dictionary and the value that may be assigned thereto may be "absent", "occasional", "frequent" or "other". This newly-added feature will be added to previously created case records, but no value will be assigned thereto. However, since a value must be assigned to make a case record complete, even if it is "other" (e. g. not applicable to a particular case), the expert has the opportunity to edit those case records which he created previously so as to assign an appropriate value to this new feature.

Likewise, the feature of pleomorphism for cell orientation might have been observed by the expert in early cases resulting in the diagnosis of malignant mesthelioma. However, as the expert observes additional cases of malignant mesthelioma, he might find that this feature is relatively unimportant and has little, if any, bearing on the ultimate diagnosis. The expert may delete this feature of pleomorphism for cell orientation from the knowledge base dictionary, thereby deleting this feature and its value from all case records. Thus, features may be added to or deleted from the knowledge base, resulting in the addition or deletion of those features from previously created case records. It will be understood that each case record contains all of the features created for the dictionary, even if the value of a particular feature in a given case is "not applicable".

Notwithstanding the characterization of a feature as being discrete or continuous, the feature also may be visually observable (although some, such as age, sex and several other clinical features will not). For example, most of the features included in the category "general cell morphology" are visual features. Examples of these include the size and shape of the cell and of the nucleus, as well as pleomorphic size and shape. Similarly, the features included in the category of Pap-cytoplasmic morphology and the features included in the category of cell block histochemical stains, as examples, also generally are visual features. Such visual features in a specimen being examined are displayed on monitor 106 after being imaged onto video camera 116 by microscope 114. As is known by those of ordinary skill in the art, the operating system used with processor 102 is such that display 106 may be controlled as to provide textual information in one portion of the display and pictorial information in another. Thus, while the expert is viewing a specimen, the various features which characterize that specimen may be selected from the knowledge base dictionary and the appropriate preestablished values may be assigned thereto. In the event that the dictionary does not contain an appropriate feature characterization, the expert may operate keyboard 108 to create a new feature, assign an appropriate value to that feature and enter that newly created feature into the knowledge base dictionary. Thus, various characteristics that are observed in the displayed image are suitably identified by designating those characteristics as particular features retrieved from the knowledge base and by assigning proper values from the knowledge base to those designated features.

A graphics application program is included in the overall software of processor 102. This graphics application program is adapted to display various signals, referred to as "overlays", which the expert may select and position on display 106 so as to particularly point out or highlight particular features or other characteristics of a displayed pictorial image. This graphics application program is similar to a commercially available program designed for Apple and MacIntosh microcomputers and known as MacPaint". Like the "MacPaint" graphics application system, mouse 110 is controlled to superimpose a cursor over a desired overlay symbol, select that symbol for use, and then reposition the cursor with the overlay symbol "attached" thereto to any desired location on the display screen. The final, desired position of the overlay symbol is identified by, for example, its position on the display screen, that is, its pixel location, and this location data together with overlay identification data are stored as part of the case record. When the pictorial image for which this overlay had been created subsequently is read from optical disk 112 and displayed on monitor 106, the overlay data linked to this image is read from magnetic disk 104 and is superimposed onto the displayed pictorial image. Thus, overlays may be created and positioned to illustrate particular features or other characteristics during subsequent displays of that image.

As mentioned above, optical disk drive 112 is known as a WORM drive and, thus, a pictorial image derived from video camera 116 and displayed on monitor 106 may be written onto the optical disk. Typically, this pictorial image is included in a case record and identification data is recorded in the case record to identify the location on the optical disk of that pictorial image. Hence, the pictorial images are linked to the case record to permit quick and easy retrieval and display.

As may be expected, two or more pictorial images may be linked to a particular case record. One type of feature may best be illustrated in one pictorial image, whereas a different feature might be present in a different image. Usually, the different images associated with the same case record are sufficiently distinct as to be quickly recognized by the expert. Because of this ready distinction among the images, only a small amount of data is needed to provide a rough or approximate display of each image. Accordingly, each case record stored on magnetic disk 104 and to which one or more pictorial images stored on optical disk 112 are linked includes image data which, when read from the magnetic disk, results in the display of the aforementioned rough or approximate images. These approximate images are displayed in relatively reduced size and are referred to herein as "icons", each icon corresponding to a pictorial image stored on optical disk 112. A desired icon may be selected, as by operating mouse 110 to position the cursor over the desired icon, causing processor 102 to read the corresponding pictorial image from optical disk 112 for display on monitor 106.

During his creation of a case record, the author is expected to identify those features which, in his opinion, are best illustrated in a displayed pictorial image. This may be achieved simply by linking the selected features to the displayed image, for example, by identifying in the case record the particular image (or images) in which the feature is illustrated. A feature may be linked to an image by designating a selected feature as a "visual" feature while a pictorial image is displayed. It is assumed that by selecting such a designated feature at the time that a pictorial image is displayed, the expert observes that feature in that image.

Of course, certain categories of features are not visual features. As an example, those features included in the category of "clinical features" typically are not visual. Examples of clinical features include the age and sex of a patient, whether the patient has been exposed to asbestos, whether the patient previously had a tumor and other selected diseases, and whether the patient has particular clinical symptoms (e. g. shortness of breath, pain, ascities or bowel obstruction). Nevertheless, such clinical features are selected and assigned values in the same manner as visual features are selected and valued.

The manner in which system 100 is used interactively by an expert to craft a knowledge base of case records comprised of features now will be described in conjunction with the various display screens presented on monitor 106. The manner in which those screens are generated by processor 102 and the manner in which the processor responds to the operation of keyboard 108 and to mouse 110 by the expert also will be described. Referring first to FIG. 2, there is illustrated a basic display screen 200, referred to herein as the "home" screen. The operating and applications programs of processor 102 cause monitor 106 to display this home screen which is comprised generally of a case window 202 and a feature window 220. Those of ordinary skill in the art will understand what is meant by reference to "windows" in the environment of a computer.

Case window 202 displays a case list 204 which identifies the case records stored in the data base. It is appreciated that only a limited number of case identifications may be displayed in case window 202; and this case list may be scrolled to display additional identifications. A suitable scroll "button" may be displayed (not shown) and activated by operating mouse 110 to position the cursor on this scroll button, whereafter the user simply operates one of the selector switches provided on the mouse. Such a scrolling feature and scroll button are conventional and further description thereof need not be provided. It should be noted here that the operating and applications systems cause monitor 106 to display discrete areas which are referred to as "buttons". When used with mouse 110, a button is activated simply by superimposing the cursor on a desired button and then activating a selector switch on the mouse. Alternatively, monitor 106 may be provided with a touch-sensitive screen which senses a user's finger in contact with the location of a displayed button, whereupon that button is activated. As a further alternative, keyboard 108 may be operated to position the cursor at a desired button and to activate that button. As yet another alternative, various keys on keyboard 108 may be associated with respective buttons such that the actuation of a key correspondingly activates that button. As yet another alternative, system 100 may be provided with a light pen which the user may juxtapose opposite a desired button and thereby actuate that button. It will be appreciated that other input devices may be used in cooperation with the various display screens to be described for the purpose of entering data and interacting with the displayed information. For simplification, mouse 110 and keyboard 108 are described as such input devices.

A case 206 selected by the expert and included in displayed case list 204 is highlighted to identify that it has been selected. In the absence of any deliberate selection by the expert, a so-called default case identification, such as the first case identified in case list 204, may be highlighted and, thus, selected.

Case window 202 also displays a list diagnosis button 208, a new case button 210, an edit case button 212, and a delete (or cut) case button 214. Actuation of a respective button results in the operation or function so identified. For example, actuation of the list diagnosis button 208 results in a display on monitor 106 of a list of various diagnoses that have been entered into the knowledge base. Diagnoses are listed in a hierarchy, and the selection by the user of any diagnosis included in the displayed list results in a list of next lower level diagnoses, the selection of any one of which results in yet another list of a still lower level of diagnoses, and so on. These sequential levels of diagnoses indicate the dependence of a given diagnosis on the preceding higher levels of diagnoses. As shown in FIG. 18, these diagnoses lists are superimposed onto home screen 200 which remains as the "background".

New case button 210, when actuated, is used by the expert to create a new case. Data fields, referred to as "text input items", associated with basic case features are displayed and require the entry of particular values. For example, text input items requiring the entry of the case identification number and the particular diagnosis of this case are displayed, as described with reference to FIG. 19. Thus, the basis of the case record is created. Upon creation of a new case, the case identification number (or other suitable identification, such as a case name) is added to the case list and, typically, appears as the last item in that list.

Actuation of the edit case button 212 retrieves from the knowledge base both the textual information and visual image information contained in the record of the particular case which is highlighted. (In a list of items, the one item which is highlighted is the "selected" item. Since the user has the choice of selecting any item in the list, the list is referred to as a choice of items, or simply a "choice".) Thus, actuation of edit case button 212 retrieves the case record of selected case 206. As a result, monitor 106 displays the icons of those pictorial images contained within the case record, as well as a more complete (i. e. magnified) pictorial image of the first icon, shown in FIG. 20. The icon of the displayed pictorial image is indicated, such as by highlighting the border of that icon; and a listing of those overlays which have been established for this pictorial image is provided. Additionally, a default category of features is indicated, as in selected category window 222 (also referred to as the current category message) of feature window 220 (to be described), and those features of the case (i. e. the case features) included in the indicated category are listed in feature list 224 (or feature choice). If the expert previously had annotated the case record with a text description, such as a description of why this case has been selected and what it represents, the annotation also is displayed.

Actuating edit case button 212 establishes an edit case mode of operation, whereby the expert may create, alter, modify or add to the information contained in the case record. For example, if a new feature had been added to the dictionary, the value of this feature in the case record will appear as "not reported", and now may be assigned. This new feature may be illustrated in one or more pictorial images linked to this case and the expert may create and position overlays in those pictorial images. Thus, the edit case mode of operation, described more particularly in conjunction with FIGS. 21-23, permits refinement and the addition of further observations of features contained in that case and enables the expert to be consistent in his characterization of a feature from one case to another. Changes in the conditions of a patient and additional diagnoses of the patient's disease or diseases likewise may be documented in the edit case mode. Of course, if a new case record had just been created, as by the aforementioned actuation of new case button 210, the edit case function is used by the expert to value the features of that case; i. e. to document the case.

Actuation of the delete case button 214 simply deletes the record of the case which has been selected from the knowledge base. That is, the record of selected case 206 is effectively erased, as described in FIG. 24. The delete case mode may be useful if the expert discovers subsequent to the creation of the case record that his diagnosis was erroneous. Also, if the initial reasons relied upon by the expert to create that case no longer are valid, the delete case mode may be used to refine the overall knowledge base.

Home screen 200 also displays a feature window 220 which includes a selected category window 222 (also known as the current category message), a feature list (or choice) display 224 and various buttons 228-242. As mentioned above, for ease of classification and utilization of the various features included in the data base, unique sets of features are classified into respective categories. Examples of such categories include clinical features, Pap overview, cytoplasmic morphology, nuclear morphology, cell background, cell morphology, cell group features, immunoperoxidase and microscopic features. Preferably, an initial category, designated a default category, is displayed as the current category message 222, and the features included in the displayed category are listed in feature choice 224. The category indicated in current category message 222 may be changed, as by actuating the list categories button 228, described below, and those features included within the newly-selected category are displayed in feature choice 224.

Any one of the features identified in feature choice 224 may be selected for further processing, as will be described. The selected feature is highlighted, as represented by selected feature 226. In one embodiment, when a new feature list 224 is displayed, the first feature in that list is highlighted and is designated the selected feature. If desired, any other default feature may be designated the selected feature. Although the feature list may be displayed without a default feature, it is preferred, to avoid errors, to designate one of those displayed features as the selected feature even if the expert has not intentionally done so.

The buttons included in feature window 220 include a list categories button 228, a new feature button 230, a new category button 232, an edit feature button 234, an edit category button 236, a view feature button 238, a cut feature button 240 and a cut category button 242. These buttons may be actuated in the manner described above with reference to buttons 208-214 to establish operating modes or functions represented by those buttons. In one embodiment, the actuation of list categories button 228 superimposes a window, or frame, in which all of the categories included in the dictionary of the data base are listed. This is shown more particularly in FIG. 3. Selection of any one of the listed categories is indicated in current category message 222; and the features included in the selected category are displayed in feature choice 224.

The actuation of new feature button 230 results in the display of a create feature frame (discussed below with reference to FIGS. 6-9) with a text input item requesting the entry of a data field to identify the new feature which the expert wishes to create. Since this feature is created for the category indicated in message 222, the newly created feature is added to feature list 224, preferably immediately following selected feature 226, where ever that selected feature may be.

The actuation of new category button 232 results in the display of a new category window (described below with reference to FIG. 4) having text input items which request the entry of data in particular data fields by which the new category may be identified and described.

It will be appreciated that newly created features and categories are added to the knowledge base dictionary and to the case records included in the knowledge base. Typically, the creation of a new category is accompanied by the creation of new features included in that category and is used by the expert to provide a basis for documenting a case and also to further refine the various case records included in the knowledge base.

The actuation of edit feature button 234 causes monitor 106 to display an edit feature frame (described more particularly with reference to FIGS. 10-12) which identifies the selected feature 226, identifies its type (i. e. whether it is a discrete, continuous or scripted feature) and identifies whether it is a visual feature. The list of possible values for this feature, which is retrieved from the knowledge base, may be modified by adding or deleting values, as will be described.

The actuation of edit category button 236 causes monitor 106 to display an edit category frame which identifies the then current category indicated in message 222. The name of this category may be modified, for example, to provide a more accurate description of the features contained therein. Also, an annotation describing this category may be revised as desired. Any changes to the name or annotation of the category are stored in the knowledge base and, thus, are written into each case record. The edit category function is more fully described with reference to FIG. 5.

The actuation of view feature button 238 causes monitor 106 to display a feature profile frame (described more particularly with reference to FIG. 13). The purpose of the feature profile frame and, thus, one of the functions of the view feature mode of operation is to permit the expert to search for cases included in the data base in which the feature being viewed (i. e. selected feature 226) is present. It is expected that a typical feature will have different values in different cases. The feature profile frame not only identifies those cases having the selected feature but also provides an indication of the distribution relative to each other of those cases having different values of this feature. For example, a histogram illustration may be displayed. The view feature mode of operation also enables the expert to establish search criteria, such as to search for those cases having not only the selected feature 226 but other features, thereby enabling the expert to determine the relative importance or influence of the selected feature in conjunction with other characteristics. The search criteria also may be based on a selected diagnosis, whereby the knowledge base is searched for case records having both the selected diagnosis and the selected feature. A list of those cases which have been found by this search is displayed, and the case record of any of the displayed cases may be retrieved and displayed on monitor 106.

As a preferred aspect of this invention, if the selected feature for which the feature profile has been produced is a visual feature, it may be displayed. As described with reference to FIG. 14, those pictorial images included in the retrieved cases may be read from optical disk 112 and displayed on monitor 106. The expert thus may observe the visual characteristics of the selected feature 226, particularly in the environment established by the search criteria which he has established.

The actuation of cut feature button 240 effectively deletes the selected feature 226 from the knowledge base. Since each case record includes a value of this feature, even if that value is "not applicable", it is recognized that the deletion of selected feature 226 may result in the loss of significant information from the case records in the knowledge base. Consequently, as shown in FIG. 15, a warning message is provided on monitor 106 requiring the user either to confirm or withdraw this cut feature operation. Upon confirmation, this feature is deleted from the knowledge base dictionary and from all of the case records included therein. However, if the user withdraws this selection of the cut feature operation, monitor 106 merely returns to display home screen 200, thereby permitting the user to initiate any desired operation, as represented by the actuation of one of buttons 208-214, or one of buttons 228-242, or diagnosis button 244.

If cut category button 242 is actuated, an operation similar to that described above with respect to the actuation of cut feature button 240 is carried out. In the cut category operation, a confirmatory message is displayed on monitor 106, as illustrated in FIG. 16. The purpose of the cut category operation is to delete from the knowledge base the current category indicated in message 222. However, this category may include one or more features whose values have already been assigned. Since the cut category operation would delete those features from the knowledge base, processor 102 is programmed to preclude the cut category operation from being carried out in the event that the current category selected for deletion contains one or more features which themselves have not yet been deleted from the dictionary. If the current category does not include such features, the user may confirm that the current category should be deleted; and upon such confirmation the knowledge base is updated by deleting the current category from the dictionary.

Home screen 200 is provided with a diagnosis button 244, the actuation of which causes monitor 106 to display a so-called diagnosis tree. This tree is created by the expert and is used to verify the accuracy by which diagnoses are made on the basis of observed features and diagnoses which are precedent. An example of an existing diagnosis tree is illustrated in FIG. 26, and FIGS. 27-29 illustrate the manner in which the diagnosis tree may be expanded or edited. Since each case record includes a diagnosis, the diagnosis tree is a valuable tool in recognizing how one diagnosis leads to another.

A brief and simple example of the utility of this diagnosis tree is as follows: Let it be assumed that, in the population of patients in which the knowledge base is interested, all of those patients have been diagnosed as having tumors. However, the tumor may be benign or malignant. In the diagnosis tree, these two diagnoses may be thought of as nodes of the tree, and both are on the same level and may be thought of as "siblings". For those patients having benign tumors, the next lower level of diagnoses is based upon the observation that such diagnoses appear only in patients who have benign tumors. This next lower level of diagnoses may be thought of as "children" of the preceding upper level. Similarly, the diagnosis "malignant tumor" will have dependent therefrom one or more "children" whose diagnoses are found only in patients having malignant tumors. The ability to edit the diagnosis tree permits refinements based upon experience and a greater population of cases and, of course, the expert's observations of those cases.

The flow charts of the various operations and routines carried out by processor 102 are self-explanatory. A working understanding and familiarity with the operating system used in the Sun microcomputer, and particularly the ability of that operating system to utilize windows and frames, to add layers or levels of routines with previously executed routines continuing as "background" is assumed. Likewise, the ability of the Sun microcomputer to function with its operating system in conjunction with mouse 110, and the capabilities and functions of that mouse, also is assumed. To familiarize the reader with the illustrated flow charts, the following describes the flow chart shown in FIG. 2A.

The flow chart shown in FIG. 2A is adapted to create the information illustrated in feature window 220 and to branch to an appropriate routine or function depending upon which of the illustrated buttons is actuated. To create the information in feature window 220, the processor first retrieves from the knowledge base a list of all of the categories therein, as indicated at 201 in FIG. 2A, and as shown at 203, the processor creates the current category message illustrated as message 222 in FIG. 2. The processor then advances to instruction 205 which selects the "default" category in the category list and displays this default category as the current category in message 222. Typically, the default category simply is the first category in the category list of the knowledge base.

The processor then advances to instruction 207 and retrieves from the knowledge base the list of features included in the category depicted as the current category. These features are listed as feature list 224, any one of which may be selected, as mentioned above. After listing these features, as at 209, the processor advances to instruction 211 and selects the first feature in this list as selected feature 226. It is appreciated that, if desired, any other feature in list 224 may be selected.

The processor then creates buttons 228-242, as represented by sequential instructions 213-227. The sequence in which these buttons are created and the order in which they are displayed in feature window 220 may be varied as desired. Once feature window 220 is created and displayed, the processor simply "waits", as represented by instruction 229, until action is taken by the user.

The state of waiting for the user to actuate a button is represented by the various inquiries depicted in FIG. 2A. Depending upon which button is actuated by mouse 110, the corresponding function, or routine, represented by that button is carried out. Thus, if list categories button 228 is actuated, as indicated by an affirmative answer to inquiry 231, the processor branches to a list category routine illustrated by the flow chart of FIG. 3A. If create category button 232 is actuated, as represented by an affirmative answer to inquiry 239, the processor advances to the new category routine represented by the flow chart of FIG. 4A. If edit category button 236 is actuated, as represented by an affirmative answer to inquiry 247, the processor advances to the edit category routine illustrated in FIG. 5A. If create feature button 230 is actuated, as represented by an affirmative answer to inquiry 235, the processor advances to the new feature routine illustrated by the flow chart of FIG. 6A.

Digressing for a moment to the flow chart of FIG. 6A, the new feature operation comprises the function by which the expert may establish the appropriate values for the feature which he is creating. As mentioned above, a feature may be visual or non-visual, and may be characterized as a discrete feature whose value is represented by text (in many instances, simply a single word), a continuous feature whose value is represented by a numeral within a user-defined range of numbers or a "scripted" feature whose value (such as length, area or density) is measured directly from the displayed feature. FIG. 6 illustrates the "new feature" screen displayed on monitor 106, and it is seen that buttons are provided to enable the expert to select the feature type (visual or non-visual) and value type (discrete, continuous or scripted). Depending upon which of the value type buttons is actuated, the processor advances, as illustrated in the flow chart of FIG. 6A, to a discrete valued feature creating routine (whose display screen is illustrated in FIG. 7), to a continuous valued feature creating routine (whose display screen is illustrated in FIG. 8) or to a scripted valued feature creating routine (whose display screen is illustrated in FIG. 9).

Returning to the flow chart of FIG. 2A, if edit feature button 234 is actuated, as represented by an affirmative answer to inquiry 243, the processor advances to the edit feature routine shown in the flow chart of FIG. 10A. Digressing again, and with reference to the flow chart of FIG. 10A, since a feature may be a discrete valued feature, a continuous valued feature or a scripted valued feature, different routines are provided to edit each of these value types. Advantageously, once the expert has selected a particular value type when creating a new feature, he is not permitted to change that type during an editing operation. As can be seen from the flow chart of FIG. 10A, if a feature selected by the expert for editing is a discrete value type, the processor advances to the edit discrete feature routine depicted by the flow chart of FIG. 10B. Similarly, if the feature selected for editing is a continuous value type, the flow chart of FIG. 10A advances to the edit continuous value routine depicted in FIG. 11A. Finally, if the feature selected for editing is a scripted value type, the processor advances from the flow chart of FIG. 10A to that depicted in FIG. 12A.

Since the editing of a feature results in a modification in the feature values present in the knowledge base, it is possible that some cases which include the feature being edited will be significantly affected. For example, if a discrete value of a feature is changed, a case having the originally valued feature (i. e. a feature having the value which now is being changed) will be affected. Processor 102 takes this into account, as represented by the flow chart of FIG. 10C, such that the case record stored in the knowledge base is modified to indicate that this now-edited feature has not yet been reported. That is, since the assigned value of this feature in the case record has been changed, the expert is given the opportunity, when reviewing this case, to select another value for this now-edited feature.

Returning to FIG. 2A, if view feature button 238 is actuated, as represented by an affirmative answer to inquiry 259, the processor advances to the view feature routine illustrated in FIG. 13A. This function enables the expert to observe how this particular feature behaves in, or influences, different diagnoses. Not only is the expert provided with statistical information regarding behavior, as shown by the display screen of FIG. 13, but this view feature function permits the expert to observe that particular feature in different cases. For example, for the feature Pap nuclear border shape, the display screen shown in FIG. 13 indicates to the expert the number of cases in which the nuclear border shape is smooth, the number of cases in which this shape is irregular, the number of cases in which this shape is variable and the number of cases in which this shape has been designated as "other". As will be apparent from the flow chart of FIG. 13A, the expert may observe the pictorial image of a smooth nuclear border in those cases having same. Likewise, the expert may observe the pictorial image of an irregular nuclear border as it appears in different cases, or he may observe the pictorial image of a variable nuclear border in other cases. The flow chart of FIG. 14A, which is reached from the flow chart of FIG. 13A, depicts how this viewing of an image is carried out, and FIG. 14 illustrates a display screen of what is viewed by the expert.

Returning again to FIG. 2A, if cut feature button 240 is actuated, as represented by an affirmative answer to inquiry 251, the processor advances to the cut feature routine illustrated in FIG. 15A. Finally, if cut category button 242 is actuated, as represented by an affirmative answer to inquiry 255, the processor advances to the flow chart of FIG. 16A.

It is appreciated that, depending upon the particular function to which the processor has advanced, as selected by the expert, the specific operations that may be carried out for that function, as illustrated in the aforementioned flow charts, may be executed.

While FIG. 2A illustrates the flow chart by which feature window 220 is created, a similar flow chart is illustrated in FIG. 17 to depict the manner in which case window 202 is created and utilized by the expert. Turning briefly to FIG. 17, the case window 202 illustrated in the home screen of FIG. 2 is created as follows: the processor advances to instruction 1701, whereby the hierarchy of diagnoses stored in the knowledge base is retrieved, and as represented by instructions 1703 and 1705, a default diagnosis, such as a predetermined one of the diagnoses in the highest level of the hierarchy, is selected and displayed as current diagnosis message 260 (FIG. 2). The hierarchy of diagnoses has been described above and is best illustrated in the diagnoses tree shown in FIG. 26.

The processor retrieves from the knowledge base all of the case records in which a current diagnosis (i. e. current diagnosis 260) is present, as represented by instruction 1707. The processor then advances to instruction 1709 which lists these cases in case list 204. Thereafter, the processor selects a default case in this list, as represented by instruction 1711, this selected case being depicted in FIG. 2 as selected case 206. Then, the processor advances to instructions 1713, 1715, 1717 and 1719 by which list diagnosis button 208, new case button 210, edit case button 212 and delete case button 214 are created. The processor now awaits the actuation of one of these buttons.

If button 208 is actuated, as represented by an affirmative answer to inquiry 1723, the processor advances to the list diagnosis function represented by the flow chart shown in FIG. 18A. This function effectively enables the expert to select a desired diagnosis within the diagnoses tree. The selected diagnosis is identified as current diagnosis 260.

If new case button 210 is actuated, as represented by an affirmative answer to inquiry 1727, the processor advances to the new case function illustrated by the flow chart of FIG. 19A. As is apparent therein, this function is used to create a case record having a particular diagnosis which the expert finds is exemplified by this case.

If edit case button 212 is actuated, as represented by an affirmative answer to inquiry 1731, the processor advances to the edit case function illustrated by the flow charts of FIGS. 20A and 20B. This function is used by the expert in assigning values to the features observed in this case. The observation generally is one of pictorial images; and it is the edit case function which provides the facility to link overlays to clearly illustrate particular features in the case. One or more (usually several) pictorial images are selected and linked to the case; and those images which best depict a particular feature are linked with overlays. The expert may link more than one overlay to a particular pictorial image; and overlays illustrating different features may be linked to the same image.

The edit case function is used by the expert not only to create a new case record but to modify an existing case record. This tool is used advantageously by the expert to assure consistency in his observations, characterizations of features and conclusions. Hence, differences in the value of a feature that might arise because of subjective observations are effectively obviated. What had been observed by the expert some time ago as "few" atypical cells and is now characterized as "moderate" atypical cells may be modified such that the expert's observations remain consistent. What is "few" today might not have been "few" previously.

As shown by the flow charts of FIGS. 20A and 20B, slightly different "tools" are used to edit discrete features, continuous features, and scripted features in a case record. These tools are manipulated by the processor under user control, as depicted by the flow charts of FIGS. 21A and B, 22A and 23A.

Finally, if delete case button 214 is actuated, as represented by an affirmative answer to inquiry 1735, the processor advances to the function illustrated by the flow chart of FIG. 24A.

The various functions carried out by the processor, as described above, now will be described in greater detail in conjunction with the flow charts associated with the display screens of FIGS. 3-16 and 18-30. Since the flow charts of FIGS. 2A and 17 have been described previously, further discussion thereof would be duplicative and, therefore, is not repeated. The following description is set forth in association with the particular function carried out by the respective flow chart.

## List Category

FIG. 3A illustrates the routine which is carried out by the processor in response to the actuation of list category button 228 to obtain the list of categories which have been created by the expert and to identify a selected one of those categories. Commencing with instruction 301, the list of categories stored in the knowledge base is retrieved and, as represented by instruction 303, the list is displayed as menu 246 (FIG. 3). Of those displayed categories, one may be selected. Inquiry 305 is made to determine if the user selects a category from menu 246. It is appreciated that a category may be selected simply by positioning the cursor over a desired category (which then is highlighted on the display) and then by actuating a suitable button on mouse 110.

If inquiry 305 is answered in the negative, the processor advances to and remains at wait state 307. However, if one of the categories listed in menu 246 is selected, the processor advances to instruction 309 whereat the selected category is designated the "current category". This category remains highlighted, as shown at 248, and is written into the "current category" message 222 of feature window 220. Then, instruction 311 is carried out to retrieve from the knowledge base the list of features included in selected category 248 which, of course, is displayed in current category message 222. The retrieved list of features is displayed in feature list 224, as indicated by instruction 313, and a predetermined one of the listed features is highlighted as selected feature 226. As represented by instruction 315, the first feature included in feature list 224 is highlighted as the selected feature 226, although any other feature may be selected. Once the features included in current category 222 is listed as feature list 224 and a selected one of these features is highlighted, as at 226, category menu 246 may be erased, as represented by instruction 317. The processor now returns to its wait state 307.

## Create a New Category

If new category button 232 is actuated when "home screen" 200 is displayed, the processor advances to carry out the flow chart shown in FIG. 4A. Instruction 401 is executed to create category frame 250, shown in FIG. 4, and within frame 250 a category name text input item 252 and a category annotation text editor 254 are created, as represented by instructions 403 and 405. The processor also creates enter button 256 and cancel button 258, all of these being included in the category frame. Then, wait state 411 is assumed until a user-executed function is initiated.

Inquiry 413 is made to determine if the user has selected category name text input 252 and, if so, if the user has inputted a new category name, as by operating keyboard 108. If so, the name selected by the user is created and displayed, and the processor then returns to wait state 411. If not, the processor merely remains at the wait state.

Inquiry 415 is made to determine if category annotation text editor 254 has been selected, as by positioning the cursor over this displayed field. If so, any text which the user desires to enter, as by operating keyboard 108, is displayed. After entering an annotation, which may helpful in subsequent determinations of the merits of the created category, the processor returns to wait state 411.

Inquiry 417 also is made to determine if the user actuates enter button 256. If so, inquiry 419 is made to determine if a unique name has been entered in category name text input 252. If this inquiry has been answered in the negative, the processor advances to instruction 421 which causes a suitable display advising the user of the failure to enter a category name. One example of such a display is as follows: "You must enter a unique category name or cancel this operation." The processor then returns to wait state 411, awaiting operator-initiated action.

If inquiry 419 is answered in the affirmative, the processor advances to instruction 423 whereat the new category name, which had been entered into category name text input 252, is written to the knowledge base. This new category then is inserted into the category list immediately following the category identified as the "current category" which, it is recalled, also is displayed as the current category message 222.

The processor then advances to instruction 427 which stores in the knowledge base the annotation entered in category annotation text editor 254, this annotation being linked with the new category name which now is stored in the knowledge base. Then, once the category name and annotation have been stored, category frame 250 is erased, as represented by instruction 429. Wait state 411 then is assumed.

If, at any time during the create new category routine cancel button 258 is actuated by the user, inquiry 431 is answered in the affirmative and category frame 250 is erased. Thus, the routine by which a new category is created may be interrupted and terminated at any time, merely by actuating cancel button 258.

## Editing a Category

If edit category button 236 is actuated while "home screen" 200 is displayed, the processor advances to carry out the routine illustrated by the flow chart of FIG. 5A. It will be appreciated that the flow chart of FIG. 5A is quite similar to that described above in conjunction with FIG. 4A. The difference here is that edit category frame 250 (which is quite similar to category frame 250) is created inclusive of the identification in category name text input 252 of a previously created category, and also inclusive in annotation text editor 254 of a previously created annotation. Category name text input 252 displays the current category which appears as the current category message 222, and annotation text editor 254 displays the annotation which has been associated with that previously established category. These differences in instructions between the edit category and create new category routines are represented by instructions 501, 503 and 505 in FIG. 5A.

The remaining instructions shown in FIG. 5A are quite similar to those shown in FIG. 4A, with the following additional difference: If the user changes the name of the category displayed in category name text input 252, once enter button 256 is actuated, the changed name is displayed as the current category message 222, and this changed name replaces the previously created category name which had been stored in the knowledge base.

Thus, a new category, together with its annotation, may be created and stored in the knowledge base, as represented by the routine shown in FIG. 4A, or a previously created category may be modified, as represented by the flow chart shown in FIG. 5A. Since the create new category and edit category routines are quite similar, further discussion of the edit category routine need not be provided.

## Create a New Feature

Let it be assumed that, while "home screen" 200 is displayed, the user actuates new feature button 230. As a result, the processor branches to the flow chart shown in FIG. 6A.

As represented by this routine, new feature frame 600 is created, and included within this frame are feature name text input 602 and feature annotation text editor 604, as represented by instructions 601, 603 and 606, respectively. The processor also creates a set feature type message, as represented by instruction 607, under which are displayed visual button 606, as created by instruction 609, and non-visual button 608, as created by instruction 611.

The processor also creates a set value type message, as represented by instruction 613; and displayed beneath this message are a discrete button 610, a continuous button 612 and a scripted button 614, as created by instructions 615, 617 and 619, respectively. In addition, the processor advances to instruction 621, to create cancel button 616, and to instruction 623 to create enter button 618.

Following the creation and display of these fields, the processor assumes and remains at its wait state 625 until operator-initiated action is provided.

While in its wait state, inquiry 631 is made to determine if visual button 606 has been actuated. If so, the display of the visual button is brightened and, concurrently, the display of the non-visual button is dimmed.

If inquiry 631 is answered in the negative inquiry 635 is made to determine if the user has actuated non-visual button 608. If so, the display of this button is brightened and the display of the visual button is dimmed. If neither the visual button nor the non-visual button is actuated, the processor remains at its wait state.

Preferably, before any of the feature type or value type buttons are actuated, the user first will enter the name of the feature that he is creating. Hence, inquiry 627 is made to determine if the feature name text input 602 has been selected and, if so, the name of the feature as created by the user, is displayed. The processor then returns to its wait state. Also, inquiry 629 is made to determine if feature annotation text editor 604 has been selected and if text has been entered therein.

While in the wait state, the processor also inquires, at 639, if discrete button 610 has been selected by the user. If so, the display of the discrete button is brightened and, concurrently, the display of continuous button 612 and scripted button 614 is dimmed, as represented by instruction 641. The processor then advances to instruction 643, at which any prior value data that may have been created previously for the feature whose name is identified in feature name text input 602 is erased; and the processor then branches to the routine represented by the flow chart in FIG. 7A. As will be described below, the flow chart of FIG. 7A includes a wait state 711 to which the processor eventually advances.

While in wait state 625, inquiry 645 is made to determine if the user has actuated continuous button 612. If so, the processor advances to instruction 647 whereat the display of the continuous button is brightened, and, concurrently, the display of discrete button 610 and scripted button 614 is dimmed. Then, instruction 649 is carried out to erase any existing value data that may have been entered previously for the feature whose name is identified in feature name text input 602, whereafter the processor branches to the routine illustrated in FIG. 8A. As will be described below the routine shown in FIG. 8A includes a wait state 817 to which the processor eventually advances.

While in wait state 625, inquiry 651 also is made to determine if scripted button 614 has been actuated. If this inquiry is answered in the affirmative, the display of the scripted button is brightened and, concurrently, the display of discrete and continuous buttons 610 and 612 is dimmed, as represented by instruction 653. Then instruction 655 is carried out wherein any existing value data for the scripted feature whose name is displayed in feature name text input 602 is erased, visual button 606 is brightened and non-visual button 608 is dimmed, and the processor branches to the scripted feature routine illustrated in the flow chart of FIG. 9A. As will be described below, the routine shown in FIG. 9A includes a wait state to which the processor eventually advances.

While at wait state 625, the processor also inquires at 657, if cancel button 616 has been actuated. If so, instruction 659 is carried out whereby new feature frame 600 and any data that may have been entered into this frame are erased.

If, while the processor is in wait state 625, enter button 618 is actuated, inquiry 661 is answered in the affirmative, and the processor advances to inquire, at 663, if all the appropriate data has been entered for this feature. For example, inquiry is made if the feature name has been entered into feature name text input 602, if any annotation has been entered into feature annotation text editor 604, if the feature has been designated as a visual or a non-visual feature, and if the feature has been selected as being of a discrete, continuous or scripted type. If one or more of these parameters has not been entered, inquiry 663 is answered in the negative and the processor advances to instruction 665. This instruction causes the following message to be displayed: "You must enter a unique feature name and completely specify feature parameters, or cancel this operation." The processor then assumes wait state 625. However, if inquiry 663 is answered in the affirmative, the processor advances to instruction 667, by which the feature data which has been entered into feature frame 600 is collected and written to the dictionary included in the knowledge base. In addition, the name of this feature is added to the feature list, preferably immediately following the currently selected feature which, it is recalled, is indicated by highlighted feature 226. Then, this feature is added to the record of each case included in the knowledge base (a feature included in a case is referred to as a case feature") and is assigned the value "not reported". Hence, the newly created feature, although added to each case, awaits the assignment thereto of a value.

After the newly created feature has been added to the knowledge base, included in feature list 224, and added to each case record as a "case feature", the processor advances to instruction 659 by which new feature frame 600 and any value date that had been entered into this frame while creating the feature are erased. Thereafter, the processor returns to wait state 625.

## Creating a Discrete Feature

The flow chart of FIG. 6A represents the manner in which a new feature is created. It is recalled from the foregoing description that if discrete button 610 is actuated, the processor branches to the flow chart shown in FIG. 7A to permit the creation of one or more values for this discrete feature. Turning to FIG. 7A, this flow chart begins with instruction 701 by which discrete value frame 702 (shown in FIG. 7) is created. Then, the processor advances to instruction 703 by which value list 704 is created. At this time, it is assumed that the discrete feature is newly created and, thus, values have not yet been assigned. Hence, value list 704 is "empty".

Then, as represented by instruction 705, value name text input 706 is created, and also add value button 708 and delete value button 710 are created, as represented by instructions 707 and 709, respectively. The processor then advances to and remains at wait state 711.

It is recalled that a discrete feature has a value represented by text which, in many instances, simply consists of a single word. Thus, to associate one or more values with this feature (typically, several available values are assigned to a feature), value name text input 706 is selected, as by positioning the cursor thereover, and an appropriate value name is entered, as by operating keyboard 108. Inquiry 713 determines if text (i. e. a name) has been entered into value name text input 706.

If add value button 708 is actuated, inquiry 715 is answered in the affirmative, and then inquiry is made, at 717, to determine if text has been entered into value name text input 706. If not, the processor merely returns to wait state 711. However, if inquiry 717 is answered in the affirmative, inquiry 719 is made to determine if the text entered into value name text input 706 differs from the existing value text for this feature (i. e. for the feature indicated in feature name text input 602). If not, the processor returns to its wait state.

If inquiry 719 is answered in the affirmative, the text which has been entered as the value name text input is added to value list 704, preferably immediately following the currently active value indicated by the highlighted item 712 in the value list. Then, the text which had been entered into value name text input 706 is cleared, thereby conditioning this routine for the entry of additional values for the feature identified in feature name text input 602. It is appreciated that if a value name, or text, had not been entered into value name text input 706, value list 704 would not be updated, as aforedescribed.

When a discrete value is to be created for a feature having preexisting discrete values, value list 704 indicates those preexisting values, and a predetermined one of those values is highlighted, as at 712, to indicate a selected value. The selected value may be changed, as by positioning the cursor over a different value, thus highlighting that newly selected value. If the newly selected value is to be deleted, delete value button 710 is actuated. Inquiry 723 determines when this button is actuated; and the processor thereafter advances to instruction 725 which deletes from value list 704 the selected, or highlighted value 712.

Thus, it is seen that new discrete values for the feature identified in feature name text input 602 may be added or deleted, as desired. After value list 704 is updated, enter button 618 may be actuated, whereupon inquiry 661 of the flow chart shown in FIG. 6A is answered in the affirmative, and the remainder of this routine is carried out. Alternatively, cancel button 616 may be actuated, whereupon inquiry 657 (FIG. 6A) is answered in the affirmative, and any changes that may have been made in value list 704 are not written to the knowledge base.

Creating a Continuous Feature

In the event that continuous button 612 is actuated during the create feature routine, as discussed above in conjunction with the flow chart of FIG. 6A, the processor branches to the flow chart illustrated in FIG. 8A. As shown in this flow chart, continuous value frame 802 (FIG. 8) is created, as represented by instruction 801, together with the following text inputs: upper limit text input 804, lower limit text input 806 and units text input 808, all as represented by instructions 803, 805 and 807.

Thereafter, instruction 809 creates segment button 810 and instruction 811 creates reset button 812. The illustrated routine continues to instruction 813 which creates a slider 816 that is used to establish a range of numerical values any one of which may be assigned to the feature identified in feature name text input 602. Thereafter, instruction 815 is carried out to create current segment list 818 which, as will be described, displays particular segments created by the user and included in the range established by slider 816. The processor then advances to wait state 817.

After continuous value frame 802 and its included elements are created, the processor waits until action is taken by the user. In this regard, inquiry 819 determines if the user enters text, such as a number, in upper limit text input 804. If so, inquiry 821 is carried out to determine if a lower limit has been entered, that is, if a number has been entered into lower limit text input 806. If not, the number entered in upper limit text input 804 is displayed as the upper limit 814 of slider 816, as represented by instruction 823, and the routine advances to wait state 817. However, if inquiry 821 is answered in the affirmative, inquiry 825 is made to determine if the number entered in upper limit text input 804 is greater than the number entered in lower limit text input 806. If this inquiry is answered in the affirmative, the routine advances to instruction 823, discussed about. However, if inquiry 825 is answered in the negative, an error message is displayed, such as: "Limits are inconsistently defined. Please check." The processor then assumes its wait state.

If a number is entered into lower limit text input 806, inquiry 829 is answered in the affirmative, whereafter the processor inquires, at 831, if a number has been entered into upper limit text input 804. If this inquiry 831 is answered in the negative, instruction 835 is carried out, whereby the number entered into lower limit text input 806 is displayed as the lower limit of slider 816. Thereafter, the processor advances to the wait state. However, if inquiry 831 is answered in the affirmative, the processor inquires, at 833, if the number entered in lower limit text input 806 is less than the number entered in upper limit text input 804. If so, instruction 835, discussed above, is carried out. However, if the number entered in lower limit text input 806 is not less than the number entered in upper limit text input 804, instruction 837 is carried out to cause the display of an error message. One example of a suitable error message is as follows: "Limits are inconsistently defined. Please check."

Inquiry also is made, at 839, if text is entered in units text input 808. For example, if this continuous feature is age, the text entered in units text input 808 may be "years". If the feature is cell number, the text entered in units text input 808 may be "cells". As a further example, if the feature is CEA, percentage of cells reacting, the text entered in units text input 808 may be "percent". Of course, to enter text in units text input 808, this field is selected, as by positioning the cursor thereover, and keyboard 108 is operated to enter the desired text therein.

Once upper and lower limits have been established, ranges may be selected within these limits to define corresponding ranges of values. For example, if the created continuous feature is age having a lower limit of 0 and an upper limit of 70, the following ranges may be established: 0-20, 21-30, 31-40, 41-50, 51-60 and 61-70. In the preferred embodiment of this invention, these ranges are established by suitable operation of slider 816, as by advancing end limit 814 to establish these segments. Such segmenting of the overall range (0-70) of slider 816 is created by operating segment button 810. Inquiry 849 determines if segment button 810 has been actuated. If so, the processor advances to inquire, at 851, if upper and lower limits have been established and if the slider has been set to a desired segment within this overall range.

If inquiry 851 is answered in the affirmative, instruction 853 is carried out to establish a segment within the range defined by the upper and lower limits. Each segment so established is displayed in the current segment list 818. However, if upper and lower limits have not been established, inquiry 851 is answered in the negative and instruction 855 is carried out to cause an error message to be displayed. One example of a suitable error message may be as follows: "Please set upper and lower limits and slider."

As just mentioned, the overall range defined by the upper and lower limits may be segmented by use of slider 816 in conjunction with segment button 810. To operate the slider so as to establish a segment therein, the slider first is actuated, as by positioning the cursor thereover. Inquiry 841 determines if the slider has been so operated, and if this inquiry is answered in the affirmative, inquiry 843 determines if upper and lower limits have been set. If so, the particular position of end limit 814 of slider 816 establishes a segmented value, and this value is displayed by arrows adjacent the slider, as represented by instruction 845. However, if upper and/or lower limits are not established, inquiry 843 is answered in the negative, and instruction 847 causes a suitable error message to be displayed.

If, while attempting to segment the overall range established for slider 816, the user actuates reset button 812, inquiry 857 is answered in the affirmative. Then, the displayed arrows adjacent slider 816, that is, the segmented values, as well as the current segment list 818 are cleared, as represented by instruction 859. The processor then resumes its wait state 817.

Thus, it is seen that, as slider 816 is operated to establish segments, each segment so established is displayed adjacent the slider and also is entered into and displayed in current segment list 818. In this manner, the continuous values of the feature identified in feature name text input 602 are established and displayed.

## Creating a Scripted Value

It is recalled from the discussion of the flow chart shown in FIG. 6A that if scripted button 614 is actuated when creating a new feature, the processor branches to the flow chart shown in FIG. 9A, whereby scripted values may be established by the user. The flow chart shown in FIG. 9A is quite similar to that discussed above in conjunction with FIG. 8A, and it is seen that scripted value frame 902 is quite similar to continuous value frame 802. Both frames include upper and lower limit text inputs, units text input, a slider, segment and reset buttons and a current segment list. Scripted value frame 902 differs from continuous value frame 802 in that frame 902 also includes a length button 920, an area button 922 and a density button 924. These buttons, each of which may be selected by the user, enable the value of the feature to be established as length or area or density. For convenience, and to avoid duplicative description, further description of the flow chart of FIG. 9A from instruction 901 to instruction 959 is not provided because these elements of the flow chart are substantially identical to aforedescribed elements 801-859.

If one of the length, area or density buttons is actuated, the processor senses such actuation by an affirmative response from inquiry 965, inquiry 961 or inquiry 969, respectively. Depending upon which button has been actuated, the display of that button is brightened while, concurrently, the display of the two remaining buttons is dimmed. This indicates that the particular value is a measure of length or a measure of area or a measure of density, respectively.

## Editing a Feature

If edit feature button 234 is actuated, various parameters associated with a selected feature, that is, parameters associated with highlighted feature 226 included in feature list 224 may be changed. The manner in which these parameters can be altered is represented by the routine illustrated in the flow chart of FIG. 10A. Instruction 1001 creates edit feature frame 1000 which, it will be appreciated, is quite similar to new feature frame 600, except that previously established feature names, annotations, types and values will be displayed in edit feature frame 1000. In addition to creating this frame, the identification of selected feature 226 is loaded into and displayed in feature name text input 1002, and the parameters and values associated with this feature are retrieved from the knowledge base. Depending upon which feature included in the feature list is selected (i. e. highlighted), the name of that feature is loaded into and displayed by feature name text input 1002, as represented by instruction 1003.

In addition, feature annotation text editor 1004 is created and whatever annotation had been established previously for feature 226 is loaded into and displayed by the feature annotation text editor, as represented by instruction 1005. The processor then advances to instruction 1007 to create the feature type message, together with visual button 1006 and non-visual button 1008, as represented by instructions 1009 and 1011. If feature 226 had been identified previously as a visual feature, the display of visual button 1006 is brightened and the display of non-visual button 1008 is dimmed. Conversely, if this feature had been identified as a non-visual feature, non-visual button 1008 is brightened and visual button 1006 is dimmed, as represented by instruction 1013.

The routine continues to instruction 1015 which creates the value type message. Discrete button 1010 is created by instruction 1017, continuous button 1012 is created by instruction 1019 and scripted button 1014 is created by instruction 1021. Depending upon whether this feature had been identified during the create feature routine as a discrete, continuous or scripted feature, display of the correct value type button is brightened while the display of the other two buttons is dimmed, as represented by instruction 1023. The processor then advances to create cancel button 1016, as represented by instruction 1025, and to create update button 1018, as represented by instruction 1027.

Inquiry 1029 is made to determine if the feature to be edited, that is, if feature 226, is of the discrete type. If so, the processor branches to the edit discrete routine represented by the flow chart shown in FIG. 10B. It will be appreciated that this routine eventually returns to the wait state.

If inquiry 1029 is answered in the negative, the routine advances to inquire, at 1035, if the feature selected for editing is of the continuous type. If so, instruction 1037 is carried out, whereby the routine branches to the edit continuous feature routine shown in the flow chart of FIG. 11A.

Finally, if the feature selected for editing is of the scripted type, inquiry 1039 is answered in the affirmative; and the processor advances to instruction 1041 which branches to the edit scripted feature routine represented by the flow chart shown in FIG. 12A.

It will be appreciated from the following discussion that the edit operation carried out for discrete, continuous and scripted features is accompanied by a displayed edit discrete value frame or an edit continuous value frame or an edit scripted value frame. FIG. 10 illustrates edit discrete value frame 1020; and this frame is created if feature 226 selected for editing is a discrete feature. Likewise, if the feature selected for editing is a continuous feature, edit continuous value frame 1102 (FIG. 11) is created. Finally, if the feature selected for editing is a scripted value, edit scripted value frame 1202 (FIG. 12) is created. Notwithstanding the particular value type assigned to the feature (i. e. notwithstanding whether the feature is a discrete, continuous or scripted type), the editing operation for all of these types includes common functions. For example, text included in feature annotation text editor 1004 may be changed, as desired. Inquiry 1045 determines if the feature annotation text editor has been selected, as by placing the cursor thereover, and if changes to the annotation are made, as by operating keyboard 1008. Another common function is the change or modification in the name of the feature selected for editing. Inquiry 1043 determines whether the feature name text input has been selected and, if so, if modifications are made to the text therein, as by operating keyboard 108.

If update button 1018 is actuated, inquiry 1047 is answered in the affirmative, and the edit feature routine advances to instruction 1049 which branches to the update feature routine illustrated by the flow chart in FIG. 10C. As will be described below, any values or text which had been entered are written to the knowledge base and added to all case records, as shown by the flow chart of FIG. 10C.

If cancel button 1016 is actuated, inquiry 1051 is answered in the affirmative. Thereafter, the processor advances to instruction 1053 which deletes the edit feature frame 1000 as well as the edit discrete value frame 1020 or the edit continuous value frame 1102 or the edit scripted value frame 1202, whichever is displayed.

In the event that visual button 1006 is actuated, inquiry 1055 is answered in the affirmative and the processor advances to instruction 1057 which causes a suitable warning message to be displayed. As an example, the following message may be indicated: "Changes in this parameter will destroy the feature."

If non-visual button 1008 is actuated, inquiry 1059 is answered in the affirmative and the processor advances to instruction 1061 which causes a suitable warning message to be displayed. This message may be the same as displayed in response to instruction 1027.

In the event that discrete button 1010 or continuous button 1012 or scripted button 1014 is actuated, inquiry 1063 or inquiry 1067 or inquiry 1071 will be answered in the affirmative. A warning message substantially similar to that caused by instruction 1057 then will be displayed in response to the actuation of any one of these value type buttons.

Turning now to FIG. 10B, a description of the manner in which a discrete feature may be edited now is provided. Let it be assumed that the feature selected for editing is a discrete feature. From the flow chart of FIG. 10A, this information associated with the feature data retrieved from the knowledge base thus brightens the display of discrete button 1010. In addition, edit discrete value frame 1020 is created, as represented by instruction 1032, as is value list 1022, as represented by instruction 1034. Whatever discrete values had been selected previously by the user for this particular feature are retrieved from the knowledge base and displayed in the value list.

The processor advances to instruction 1036 whereby value name text input 1024 is created. The processor then creates add value button 1026 and delete value button 1028, as represented by instructions 1038 and 1040, respectively. Then, wait state 1042 is assumed until user-initiated action is taken (it will be seen that the wait state shown in FIG. 10B may be the same wait state 1033, shown in FIG. 10A, whereby the processor may respond to any action taken within edit feature frame 1000, including action taken within edit discrete value frame 1020).

Inquiry 1044 is made to determine if the user enters a new text value in value name text input 1024. It is recognized that this new value may be entered first by selecting the value name text input, as by positioning the cursor thereover, and then entering text by operating keyboard 108.

If add value button 1026 is actuated, inquiry 1046 is answered in the affirmative and inquiry 1048 is made to determine if text has been entered in value name text input item 1024. If not, the processor merely returns to its wait state. However, if inquiry 1048 is answered in the affirmative, inquiry 1050 is made to determine if the text entered in value name text input 1024 differs from any existing text value for the discrete feature being edited. If this inquiry is answered in the negative, the processor returns to its wait state. However, if this inquiry is answered in the affirmative, the routine advances to instruction 1052 whereby the value which had been entered by the user in value name text input 1024 is added into value list 1022, preferably at a position immediately following the currently selected value, that is, the value which is highlighted in the value list. Then, the text which had been entered into value name text input 1024 is cleared, thereby conditioning edit discrete value frame 1020 for a further editing operation.

The user may delete a value listed in value list 1022 by selecting the value to be deleted, as by positioning the cursor over that value, thereby highlighting it, and then actuating delete value button 1028. Inquiry 1024 determines when this button is actuated, whereafter the processor advances to inquiry 1056 to determine if this value to be deleted has been assigned to any of the case records included in the knowledge base. If this inquiry is answered in the negative, this value is deleted from value list 1022 and from the knowledge base. However, if inquiry 1056 is answered in the affirmative, a message is displayed requiring the user to respond either positively or negatively. A suitable message may be as follows: "Deleting this feature will destroy case information. Do you wish to continued" If the user responds positively, this value is deleted from the value list, from the knowledge base and from all case records. However, if the user responds negatively, the deletion operation is interrupted and the processor simply returns to its wait state.

Once a feature has been edited, regardless of whether the feature is a discrete, continuous or scripted feature, all changes made therein are stored in the knowledge base. The flow chart shown in FIG. 10C illustrates the manner in which the knowledge base is updated to reflect changes that had been made in the feature selected for editing (e. g. feature 226).

It is recalled that the processor branches to the update feature routine illustrated in FIG. 10C from instruction 1049 of the edit feature routine (FIG. 10A). With reference to the flow chart of FIG. 10C, inquiry first is made to determine if the edited feature is a discrete, continuous or scripted feature (inquiries 1062, 1082 and 1084, respectively). If inquiry 1062 is answered in the affirmative, thus indicating that the feature is a discrete feature, inquiry 1064 is made to determine if one or more values that have been associated with the feature being edited have been deleted during the current editing session. If not, inquiry 1066 is made to determine if one or more values had been added to this feature. If this inquiry is answered in the negative, thus indicating that no values associated with this feature had been added or deleted, the routine advances to inquiry 1070 to determine if the feature being edited has a unique name. Although it is unlikely that this inquiry will be answered in the negative during an edit operation, if such is the case, the processor advances to instruction 1076 to display a suitable warning message. One example of an appropriate message is as follows: "You must enter a unique feature name or cancel this operation." Thereafter, the processor simply remains at wait state 1078.

If inquiry 1066 is answered in the affirmative, thus indicating that values have been added to the feature being edited, the added values are written to the knowledge base and, thus, are added to this feature included in all case records. As mentioned above, a feature present in a case record is referred to as a "case feature". Instruction 1068 thus indicates that the new values are added to all case features included in the knowledge base. Then, the processor advances to inquiry 1070, mentioned above.

If values have been deleted from the feature being edited, inquiry 1064 is answered in the affirmative and the routine advances to inquiry 1072 to determine if those values which had been deleted have been assigned to any of the case records included in the knowledge base. It will be appreciated that a feature value that has been "reported" is one which has been assigned to a particular feature observed in a case. If inquiry 1072 is answered in the negative, the processor advances to inquiry 1070. However, if the deleted value had been assigned to a case record, the fact that this value now is deleted means that the feature for that case record will be assigned the value "not reported". This means that no value has yet been assigned to this feature observed in the case. It is apparent that a "not reported" feature generally means that the user must edit the case record to update his observation of this feature therein.

If inquiry 1070 is answered in the affirmative, thus indicating that an identified feature is being updated, all of the data included in edit feature frame 1000, including the name and annotation associated with this feature, the selected feature and value type and the various values (whether discrete, continuous or scripted) that the feature may have, are written to the dictionary of the knowledge base and also replace the data included in each case feature, as represented by instruction 1080. If the value included in a case feature has not been changed by this editing operation, that previously assigned value is, of course, retained. However, if the value previously assigned to a case feature has been deleted, the case record in which that case feature is included now indicates a "not reported" value for that feature.

If the feature being edited is a continuous or scripted feature, one or the other of inguiries 1082 and 1084 is answered in the affirmative, and the processor advances to inquiry 1086 to determine if either or both of the upper and lower limits previously established for this feature have been changed. If not, the processor advances to inquiry 1070, and the aforedescribed routine continues as discussed above. However, if an upper and/or lower limit has been changed, the processor advances to inquiry 1088 to determine if any value of this feature assigned in a case record now falls outside the range defined by the new limits. If not, this editing operation has no substantive affect upon the case records and the processor merely advances to inquiry 1070. But, if a reported value for any case feature now falls outside this newly established range, inquiry 1088 is answered in the affirmative and the processor advances to instruction 1090 to change the value for this case feature to indicate that it now is "not reported". For example, if the feature is age and the previously established range was 10 to 70 but the edited range now is 20 to 70, and if a case record included a value assigned to the 10-20 segment, this previously assigned value now falls outside the new range and, thus, the feature "age" in this case record is assigned the value of "not reported". It is appreciated that, during subsequent operations, it would be desirable to edit this case record.

As illustrated in FIG. 10C, after the value associated with this continuous or scripted feature has been changed to "not reported", the processor advances to inquiry 1070 and proceeds in the manner discussed above.

## Editing a Continuous Feature

It is recalled from the description of the flow chart shown in FIG. 10A that if the feature being edited is a continuous feature, the processor branches to the flow chart shown in FIG. 11A. As illustrated in FIG. 11A, continuous value frame 1102 is created, as represented by instruction 1101, and the processor advances to instructions 1103, 1105 and 1107 to create upper limit text input 1104, lower limit text input 1106 and units text input 1108, respectively. The upper and lower limit values, as well as the identification of the units, associated with this feature are retrieved from the knowledge base and displayed in the text inputs.

The routine continues with instruction 1109 to create segment button 1110 and with instruction 1111 to create reset button 1112. As was carried out with the create continuous value feature routine discussed above in conjunction with FIGS. 8 and 8A, slider 1116 is created, as represented by instruction 1113, and the previously assigned upper and lower limits are displayed. The processor advances to instruction 1115 to create segment list 1118 and to retrieve from the knowledge base the segments included in the range defined by the upper and lower limits for this feature. These individual segments are listed in segment list 1118 and, moreover, are displayed by arrows 1120 adjacent slider 1116. FIG. 11 illustrates that the particular continuous feature being edited previously was assigned with segments 0-2, 2-4 and 4-6 within an overall range from 0 to 7. These segments are listed in segment list 1118 and are graphically displayed by arrows 1120. Once continuous value frame 1102 is created, as aforementioned, the processor remains at wait state 1117 until user-initiated action is taken. As shown in FIG. 11A, this wait state is the same as wait state 1033 and, thus, the processor is conditioned to respond to the actuation of a feature type button, a value type button, update button 1018 or cancel button 1016, all as described above.

If text is entered in upper limit text input 1104, inquiry 1119 is answered in the affirmative and the processor advances to inquiry 1121 to determine if there are case records in the knowledge base with values that exceed this new upper limit. If this inquiry is answered in the negative, the new upper limit is displayed as the upper limit of slider 1116, and the processor returns to its wait state. However, if inquiry 1121 is answered in the negative, the processor advances to require user confirmation that the user wishes to continue with this edit operation even though a change in the upper limit of this particular feature will destroy preexisting case information. If the user indicates a desire to continue, the processor advances to instruction 1123. However, if the user does not want to continue, the routine advances to instruction 1127 whereby the original upper limit value is restored in upper limit text input 1104.

A similar set of instructions and inquiries is carried out if the user changes the preexisting value displayed in lower limit text input 1106. This is represented by inquiries 1129, 1131 and 1135 and instructions 1133 and 1137.

Inquiry 1139 is made to determine if the user enters rew text in units text input 1108. If so, this newly entered data is displayed.

The user may modify the segments which are represented by arrows 1120 and which are listed in current segment list 1118. The manner in which segments are created, displayed and listed has been described above in conjunction with the flow chart shown in FIG. 8A. It is appreciated that the portion of the flow chart shown in FIG. 11A comprised of elements 1141, 1143, 1145, 1147, 1149, 1151, 1153, 1155, 1157 and 1159 is quite similar to that portion formed of the corresponding elements shown in FIG. 8A. Hence, in the interest of brevity and to avoid unnecessary duplication, further description of the flow chart shown in FIG. 11A is not provided.

## Editing a Scripted Feature

It is recalled from the description of the flow chart shown in FIG. 10A that if the feature being edited is a scripted feature, the processor branches from instruction 1041 to the routine represented by the flow chart shown in FIG. 12A. It will be observed that the flow chart of FIG. 12A is quite similar to that just described in connection with FIG. 11A. Indeed, a comparison of scripted value frame 1202 of FIG. 12 with continuous value frame 1102 of FIG. 11 indicates substantial similarity, except that the scripted value frame includes a length button 1220, an area button 1222 and a density button 1224. In the interest of brevity and simplification, duplicative description of the flow chart shown in FIG. 12A is not provided. It is appreciated that the functions shown and described with reference to the flow chart of FIG. 11A are carried out by corresponding elements of the flow chart shown in FIG. 12A.

However, in editing a scripted value feature, the user has an opportunity to actuate length button 1220 or area button 1222 or density button 1224, depending upon whether the value of the feature being edited is a measure of length, area or density. If length button 1220 is actuated, inquiry 1265 (FIG. 12A) is answered in the affirmative, resulting in instruction 1267 to cause the display of a suitable warning message. One example of such a warning message is as follows: "Changes in this parameter will destroy the feature."

Likewise, if area button 1222 is actuated, inquiry 1261 is answered in the affirmative and the aforementioned warning message is displayed. Finally, if density button 1224 is actuated, inquiry 1269 is answered in the affirmative and the aforementioned warning message is displayed. It is recognized that one purpose of this warning message is to prevent the user from inadvertently changing the measure of the feature being edited from, for example, a measure of length to a measure of area which, of course, will have no rational bearing to the values that had been assigned previously to this feature.

## Viewing a Feature

Turning now to the flow chart shown in FIG. 13A, taken in conjunction with the display of FIG. 13, the manner in which a graphical display of the distribution of a particular feature 226 in various case records is produced now will be described. In response to the actuation of view feature button 238, the processor carries out the illustrated routine by executing instruction 1301 to create feature profile frame 1302, whereafter the feature which is in the process of being edited, or which has just been edited, that is, selected feature 226, is identified, as represented by instruction 1303. The processor advances to instruction 1305 to create list diagnosis button 1304. Then, instruction 1307 is carried out to create the "current search criteria" message displayed in feature profile frame 1302. Then, the "total cases" message is created, as represented by instruction 1309, and view button 1306 is created, as represented by instruction 1311.

The processor advances to instruction 1313 to create value list 1308. Instruction 1315 then is executed to create case/image list 1310. Inquiry 1317 then is made to determine if the feature whose profile will be displayed in feature profile frame 1302 is a visual feature. If so, list images button 1314 is created. If not, this button is not displayed.

Then, cancel button 1312 and edit case button 1336 are created, as represented by instruction 1321.

After the basic elements, fields and frames included in feature profile frame 1302 have been created, the processor advances to instruction 1323 to retrieve from the knowledge base a list of all case records having reported values for this feature 226. Each assigned value included in a case is listed in value list 1308, as represented by instruction 1325. For illustrative purposes, it is assumed that five separate values have been assigned, and these five values, whether the feature is discrete, continuous or scripted, are listed, as represented by the individual values 1316, 1320, 1324, 1328 and 1332. For example, if the feature is PAP cytoplasm, mesothelial population, the assigned values may be pale, delicate, dense, variable and poorly visualized. These values may be listed in value list 1308. Instruction 1325 also calls for determining the number of cases in which each value is reported and determining the total number of cases with a reported value for this feature. Thus, the number of cases reporting pale mesothelial population is indicated, as are the number of cases having, respectively, delicate, dense, variable and poorly visualized mesothelial population. As shown in FIG. 13, the number of cases having each respective value is indicated proximate that value.

The processor advances to instruction 1327 whereby a bar graph representation of the number of cases having the reported value is displayed. Continuing with the present example, bar graph 1318 represents five cases reporting pale mesothelial population. Bar graph 1322 represents five cases reporting delicate mesothelial population. Bar graph 1326 represents eleven cases reporting dense mesothelial population. Bar graph 1330 represents twenty-three cases reporting variable mesothelial population. Finally, bar graph 1334 represents thirty-five cases reporting poorly visualized mesothelial population. It is appreciated that each bar graph represents the ratio of the number of cases having the reported value to the total number of cases in which a value for this feature has been reported. In the present example, seventy-nine cases have this feature, and bar graph 1318 represents that those having pale mesothelial population constitute approximately 6% of all such cases.

The processor advances to instruction 1329 which sets the first value 1316 in value list 1308 as the selected value, whereafter instruction 1331 is carried out whereby those cases having this value of the feature are listed in case list 1310. It will be appreciated that the first-indicated value illustrated as "value 1") in value list 1308 is the default value for selection. Any other value may be selected, as by positioning the cursor thereover. In any event, those cases having the selected value are listed in case list 1310.

The processor then assumes wait state 1333.

The distributed display shown in value list 1308 represents the distribution of all cases having different values of the selected feature. This feature profile may be further narrowed to indicate the number of cases having not only the indicated values but also those having a particular diagnosis. This further selectivity is achieved by actuating list diagnosis button 1304. Inquiry 1335 determines if this button is actuated, and when answered in the affirmative, the processor advances to instruction 1337 by which a set of so-called "pull right" menus are displayed indicating the hierarchy of diagnoses currently in the knowledge base. These pull right menus are illustrated in FIG. 13 as menus 1340 and 1342. The user may select a particular diagnosis listed in menu 1340 or in menu 1342, as by positioning the cursor over the desired diagnosis, as represented by instruction 1339. The selected diagnosis is used as a search criterion to search the set of cases listed in value list 1308 to further limit the number of cases having both the selected diagnosis and a reported value of this feature.

The processor then advances to instruction 1325 which has been described above. Now, however, this instruction is carried out with the further criterion of the selected diagnosis to restrict the listing and display of cases having both that diagnosis and a reported value for this feature. Thereafter, instructions 1327, 1329 and 1331 are repeated.

As mentioned above, any value listed in value list 1308 may be selected by the user, as by positioning the cursor thereover. Inquiry 1343 determines if a different value has been selected and, if so, a list of those cases having this newly selected value and the selected diagnosis are listed in case list 1310, as represented by instruction 1331.

Let it be assumed that view image button 1306 is actuated. Inquiry 1345 is answered in the affirmative, and the routine advances to inquire, at 1347, if any cases are listed in case/image list 1310. If so, an image cannot be viewed; and instruction 1351 is carried out to display a suitable error message. One example of such an error message is as follows: "Cases are currently listed. Edit cases or list images." The processor then returns to wait state 1333.

However, if cases are not listed in case/image list 1310, instruction 1349 is carried out and a selected image is viewed. The manner in which an image is viewed is discussed below in conjunction with the flow chart shown in FIG. 14A.

If list images button 1314 is actuated, inquiry 1361 is answered in the affirmative and the processor advances to inquire, at 1363, if cases currently are listed in case/image list 1310. If cases are not listed, instruction 1367 is executed, by which a suitable error message is displayed. One such error message is as follows: "Images are currently listed."

However, if cases are listed in case/image list 1310, inquiry 1363 is answered in the affirmative and the processor advances to instruction 1365. As indicated, this instruction serves to select from the cases listed in case/image list 1310 the list of images having the value the user selected previously (represented by inquiry 1343), and those images are listed in case/image list 1310. It is appreciated that, once these images are listed, the view image operation, represented by instruction 1349, may be carried out.

If edit case button 1336 is actuated, inquiry 1353 is answered in the affirmative, and the processor advances to inquire, at 1355, if cases are listed in case/image list 1310. If not, a suitable error message is displayed, as represented by instruction 1359. One such message may be as follows: "Images are currently listed. View images or reset diagnosis." However, if cases are listed in the case/image list, inquiry 1355 is answered in the affirmative and the processor advances to instruction 1357 by which the edit case routine, described below in conjunction with the flow charts of FIGS. 20A and 20B, is carried out.

Finally, if cancel button 1312 is actuated, inquiry 1369 is answered in the affirmative and the processor advances to instruction 1371 by which feature profile frame 1302 is erased.

## View Image

Let it be assumed that during the view feature routine (FIG. 13A) list images button 1314 was actuated such that a list of images having the selected value is listed in case/image list 1310, and then view image button 1306 is actuated. As a result, the processor branches to the flow chart shown in FIG. 14A, and now described. Instruction 1401 is carried out to create view image frame 1402. The images listed in case/image list 1310 identify the case record with which each image is associated. The identification of that case as well as the identification of the image illustrating the feature which was being viewed also are obtained. Then, the first image included in case/image list 1310 together with its associated overlays is retrieved from the knowledge base.

The processor then advances to instruction 1403, whereupon the identified image is displayed in view image frame 1402. The routine continues to instruction 1405 to create image overlay list 1404 which lists the overlays associated with the displayed image. Each overlay is identified by the case feature displayed by the image plus the reported value of that feature. The first of the listed overlays is displayed, as represented by overlays 1406. If desired, any other preselected overlay included in list 1404 may be selected for display.

Then, instructions 1407, 1409 and 1411 are carried out to create the "case" message and display the identification of the case with which the displayed image is associated, to create the "image" message and display the identification of the image being displayed, and to create the "diagnosis" message and display the diagnosis associated with this case from which the displayed image has been derived. The processor then advances to wait state 1413.

If edit case button 1408 is actuated, inquiry 1415 is answered in the affirmative and the processor advances to instruction 1417 whereby the identification of the case with which the displayed image is associated is obtained, and then the processor branches to the edit case routine illustrated in the flow charts of FIGS. 20A and 20B.

If a new overlay (created in a prior operation, as described below in conjunction with FIGS. 21-23) is selected from overlay list 1404, as by positioning the cursor over a desired overlay name, inquiry 1419 is answered in the affirmative and the processor advances to instruction 1421. By this instruction, the presently displayed overlay is cleared from view image frame 1402, and the newly selected (but previously created) overlay is retrieved from the knowledge base and displayed. Overlays 1406 indicate that a selected overlay may consist of plural overlay areas which illustrate the feature under consideration. In this manner, examples of the feature observed in a case may be illustrated clearly. Finally, if cancel button 1410 is actuated, inquiry 1423 is answered in the affirmative and view image frame 1402 is cleared.

## Deleting a Feature

Let it be assumed that cut feature button 240 is actuated. As a result, the processor advances to the routine illustrated by the flow chart of FIG. 15A. Instruction 1501 is carried out to obtain the identification of the selected feature 22( displayed in feature list 224 (FIG. 15). Inquiry 1503 is made to determine if a reported value for this feature has been included in any case record in the knowledge base. If not, inquiry 1511 is made, requiring confirmation that the user wishes to proceed even though this action destroys feature information. It is appreciated that a suitable warning may be displayed requiring a positive or negative response. If the response is negative, the processor merely advances to wait state 1509. However, if the response is positive, thus indicating that the user wishes to proceed, instruction 1513 is carried out by which this feature is deleted from the dictionary included in the knowledge base and also is deleted from all case records, even though the value of this feature in each such record is "not reported".

If inquiry 1503 is answered in the affirmative, inquiry 1505 is made, requiring the user to confirm that he wishes to proceed even though this action destroys both feature and case information from the knowledge base. A suitable display, such as display 1502 shown in FIG. 15, may be produced. If the user responds negatively, the processor simply returns to its wait state. However, if the user responds positively, as by actuating yes button 1504, instruction 1507 is carried out by which this selected feature is deleted from the dictionary of the knowledge base and also is deleted from all cases included in the knowledge base, even though some of these cases include a reported value for this feature.

## Deleting a Category

If cut category button 242 is actuated, the processor advances to the cut category routine illustrated in FIG. 16A. Instruction 1601 is carried out to obtain the identification of the category displayed as current category message 222 (FIG. 16). Then, inquiry 1603 is made to determine if this category contains any features. If not, inquiry 1609 is made, requiring confirmation that the user wishes to proceed even though this action destroys the category. If the user responds positively, instruction 1611 is carried out by which this category is deleted from the dictionary and also from all cases included in the knowledge base, even though no case includes a reported value for any feature included therein because this category does not contain any features. The processor then advances to wait state 1607.

However, if the category displayed as current category message 222 does contain features, the processor advances to instruction 1605 which causes display 1602 to be produced. Actuation of confirm button 1604 is needed to verify that the user understands the need to delete existing features included in this category before the category itself may be deleted.

## List Diagnosis

Let it be assumed that list diagnosis button 208 of home screen 200 is actuated. As discussed above with respect to the flow chart shown in FIG. 17, the processor branches to the list diagnosis flow chart shown in FIG. 18A and now described.

The diagnosis hierarchy first is obtained, as represented by instruction 1801. It will be appreciated that this hierarchy represents a diagnosis "tree" discussed below. The processor then advances to instruction 1803 by which "pull right" menus are created to represent this diagnosis hierarchy. Menus 1802 and 1804 of FIG. 18 represent these pull right menus.

Once these menus are displayed, inquiry 1805 is made to determine if the user selects a diagnosis therefrom. If not, the processor merely assumes its wait state 1807. However, if inquiry 1805 is answered in the affirmative, instruction 1809 is carried out whereby the selected diagnosis is highlighted and designated the current diagnosis. This diagnosis is written into current diagnosis message 260.

The processor then advances to instruction 1811 which retrieves from the knowledge base a list of cases containing this current diagnosis. This list of cases is displayed in case list 204, as represented by instruction 1813, and a predetermined one of the cases, such as the first case in the list, is designated the selected case and highlighted. Then, the processor advances to instruction 1817 by which the pull right diagnosis menus are cleared. Thereafter, the wait state is assumed.

If desired, the user may select a further sublevel of diagnosis, and this is indicated by an affirmative answer to inquiry 1819. If a further sublevel of diagnosis is selected, a further pull right menu is displayed, and the processor repeats the remaining instructions illustrated in FIG. 18A.

## Enter New Case

Let it now be assumed that new case button 210 is actuated. As discussed above in conjunction with the flow chart shown in FIG. 17, the processor now branches to a new case routine illustrated by the flow chart of FIG. 19A.

Instruction 1901 is carried out to create enter case frame 1902 (shown in FIG. 19). Then, case ID text input 1904 is created, as represented by instruction 1903. A diagnosis message is created, as represented by instruction 1905, and now is indicated as being "not reported". Then, the processor advances to instruction 1907 to create set diagnosis button 1906. Thereafter, enter button 1908 is created, as represented by instruction 1909, and a cancel button is created, as represented by instruction 1911. The processor then assumes its wait state 1913.

If case ID text input 1904 is selected as by positioning the cursor thereover, an identification may be entered, as by operating keyboard 108. Typically, a case is identified by a multi-digit numeral. Inquiry 1915 determines if a case name, or identification, is entered.

Let it be assumed that set diagnosis button 1906 is actuated. Accordingly, inquiry 1931 is answered in the affirmative, and instruction 1933 is carried out to retrieve the diagnosis hierarchy from the knowledge base. Then, instruction 1935 is executed to create pull right menus, such as menus 1910 and 1912, to represent this diagnosis hierarchy. Inquiry then is made, as at 1937, to determine if a diagnosis is selected from one of the pull right menus. If not, the wait state is assumed. However, if inquiry 1937 is answered in the affirmative, instruction 1939 is carried out to enter into the diagnosis message the identification of this selected diagnosis. Thus, at this time, a case identification has been entered and a particular diagnosis has been selected.

If enter button 1908 now is actuated, inquiry 1917 is answered in the affirmative. Thereafter, inquiry 1919 is made to determine if the user has entered a unique name in the case ID text input 1904. If not, instruction 1921 is carried out to present a warning message to the user. This message may be as follows: "You must enter a unique case name or cancel this operation."

If a unique case identification has been entered, inquiry 1919 is answered in the affirmative and the processor advances to instruction 1923 whereby the new case whose basic data has been entered in enter case frame 1902 now is written to the knowledge base.

The routine then advances to instruction 1925, whereby current diagnosis 260 is set to the name of the diagnosis which had been entered in the diagnosis message item. Then, the newly entered case is listed as the last case in case list 204. Then, enter case frame 1902 is cleared.

If the cancel button is actuated at any time, inquiry 1941 is answered in the affirmative and enter case frame 1902 is cleared, as represented by instruction 1943.

## Edit Case

If edit case button 212 is actuated, the processor branches to the edit case routine illustrated in FIGS. 20A and 20B, as discussed previously with respect to the flow chart shown in FIG. 17. Instruction 2001 first is carried out to create edit case frame 2002 (shown in FIG. 20). Then, instruction 2003 is executed to create current image subframe 2004 and image overlay list 2006. Thereafter, image icon menu 2008 is created, as represented by instruction 2007, and feature toolbox subframe 2010 is created, as represented by instruction 2009.

The processor advances to instruction 2011 by which case feature list 2012 is created. It will be appreciated that the case feature list is analogous to the aforedescribed feature list and indicates those features which are included in a particular case, thus the. designation "case feature". In addition, and as represented by instruction 2011, set category button 2014, current category message 2016 and view feature button 2018 all are created.

Instruction 2013 is carried out by which annotation editor 2020, case button 2022, image button 2024 and update button 2026 all are created. Then, inquiry 2015 is made to determine if this selected case, that is, the case selected for editing, has been edited previously. It will be appreciated that the case selected for editing is displayed by the highlighted case 206 shown on home screen 200. If inquiry 2015 is answered in the negative, for example, if the selected case has just been newly entered, the processor advances to instruction 2017 by which the current list of features is retrieved from the knowledge base and these features are used as the set of case features 2012 for the current case. At this time, since the case has not been edited previously, these case features are blank because no value has been assigned, as yet.

The processor advances to instruction 2019 whereby the list of categories is retrieved from the knowledge base. The first category included in this list is selected and displayed as current category 2016. Thereafter, those features included in this category are selected as the case features and displayed as case feature list 2012, as represented by instruction 2021. At this time, the value of each of these displayed case features is "not reported" because no value has been assigned. That is, these features have not yet been instantiated by the user. Then, the routine advances to instruction 2023 which selects the first case feature included in case feature list 2012 as the current selected feature, whereupon the processor advances to the routine illustrated in FIG. 20B, and described below.

If inquiry 2015 is answered in the affirmative, inquiry 2025 is made to determine if the selected case contains any images. If so, instruction 2027 is carried out to retrieve the case image icons from the knowledge base and display those icons in image icon menu 2008. Then, a predetermined one of these displayed icons, such as the first icon, is selected, and the image corresponding thereto is retrieved from, for example, video disk player 112 (FIG. 1). The retrieved image is displayed in current case image frame 2004 and any overlay names which have beer associated with this image are displayed in overlay list 2006. A predetermined one of these overlays, such as the first overlay displayed in list 2006, is selected and displayed on the presently displayed image. Then, the processor advances to instruction 2031.

As shown by the flow chart of FIG. 20A, instruction 2031 also may be reached if inquiry 2025 is answered in the negative. This instruction retrieves from the knowledge base annotations associated with the selected case, and the retrieved annotations are displayed in annotation editor 2020. In addition, image button 2024 is dimmed.

The processor then advances to instruction 2033 which retrieves from the knowledge base the category list, and the first category included in that list is selected and displayed as current category 2016. Thereafter, instruction 2035 is carried out to retrieve from the knowledge base the case features included in current category 2016, and these retrieved features are displayed in case feature list 2012 together with their associated values. For those features which have not yet been reported for this case, that is, for those features which have not yet been instantiated, the indicated values are "not reported". Then, instruction 2023 is carried out, whereby the first case feature 2028 included in case feature list 2012 is selected; and the processor branches to the flow chart shown in FIG. 20B.

From the flow chart of FIG. 20B, it is seen that the processor first executes instruction 2037 to clear toolbox subframe 2010. Then, inquiries 2039, 2045 and 2049 are made, in sequence, to determine if the selected case feature 2028 is a discrete feature or is a continuous feature or is a scripted feature. If the selected case feature is a discrete feature, inquiry 2039 is answered in the affirmative and the processor branches to the routine shown in FIG. 21A to create and utilize what is referred to as the discrete feature toolbox.

If selected case feature 2028 is a continuous feature, inquiry 2045 is answered in the affirmative and the processor branches to the flow chart shown in FIG. 22A. The routine illustrated in this flow chart is referred to as the continuous toolbox routine.

However, if selected case feature 2028 is a scripted feature, inquiry 2049 is answered in the affirmative and the processor branches to the routine illustrated by the flow chart of FIG. 23B. This routine is discussed below and is referred to as the scripted toolbox routine.

Regardless of the particular toolbox routine to which the processor branches, wait state 2043 eventually is assumed until operator-initiated action is taken.

As an example of operator-initiated action, if annotation editor 2020 is selected, as by positioning the cursor thereover, inquiry 2053 is answered in the affirmative and text may be entered by operating keyboard 108.

If case button 2022 is actuated, inquiry 2055 is answered in the affirmative and the processor advances to instruction 2057. As represented in FIG. 20B, instruction 2057 serves to dim image button 2024, brighten case button 2022, clear annotation editor 2020 and load into the annotation editor the annotation stored in the knowledge base relating to the selected case, as had been prepared previously. The processor then resumes wait state 2043.

If image button 2024 is actuated, inquiry 2059 is answered in the affirmative and instruction 2061 is carried out. As represented by the flow chart of FIG. 20B, instruction 2061 serves to dim case button 2022 and to brighten image button 2024. In addition, annotation editor 2020 is cleared and whatever image annotation had been prepared previously for the image currently being displayed in current image subframe 2004 is retrieved from the knowledge base and loaded into annotation editor 2020. Then, the wait state is assumed.

If update button 2026 is actuated, inquiry 2063 is answered in the affirmative and the processor advances to inquiry 2065 to determine if a case annotation or an image annotation is being edited. If neither type of annotation is being edited, inquiry 2065 is answered in the negative, and wait state 2053 is assumed. However, if a case annotation is being edited, the processor advances to instruction 2067 whereby whatever text has been entered into the annotation editor via keyboard 108 now is written to the knowledge base as a case annotation. Alternatively, if an image annotation is being edited, the processor advances to instruction 2069 whereby the image annotation text which had been entered into the annotation editor via keyboard 108 now is written to the knowledge base and is identified as an image annotation. Then, the processor returns to its wait state.

If a new case feature is selected from case feature list 2012, as by positioning the cursor over a desired one of the case features displayed in this list, inquiry 2071 is answered in the affirmative and the processor advances to instruction 2037 to carry out that portion of the illustrated routine which has been discussed above.

If the user actuates set category button 2014, inquiry 2073 is answered in the affirmative and the list of categories stored in the knowledge base is retrieved and displayed as a menu similar to that shown in FIG. 3. Then, the routine advances to inquire, at 2077, if the user has selected a category from this menu. If so, instruction 2079 is carried out such that the category now selected by the user is identified as the current category and is written into current category message 2016. The category menu from which the current category was selected then is erased.

The processor advances to instruction 2081 to retrieve from the knowledge base the list of case features included in the new current category 2016; whereafter instruction 2083 is carried out to list in case feature list 2012 all of the case features included in current category 2016. Then, as before, a predetermined one of these listed case features, such as first case feature 2028, is highlighted, or selected. The processor then returns to instruction 2037 to execute that portion of the flow chart which has been discussed above.

If desired, the user may select a new overlay name from image overlay list 2006. This may be done by positioning the cursor over a desired overlay name. If a new overlay name is selected, inquiry 2087 is answered in the affirmative and the processor advances to instruction 2089. This instruction clears the present overlay from current case image subframe 2004, retrieves from the image base the overlay which the user has selected from overlay list 2006, and displays this displayed overlay on the image now being displayed in current case image subframe 2004. Thereafter, the processor returns to its wait state.

Let it be assumed that a new icon is selected by the user from image icon menu 2008. This may be done by positioning the cursor over a desired icon. In that event, inquiry 2091 is answered in the affirmative and the processor advances to instruction 2093, whereby the identity of the case image represented by the selected icon is retrieved, together with its associated overlays, and this image is read from vidéo disk player 112 and displayed in current image subframe 2004.

If view feature button 2018 is actuated, inquiry 2095 is answered in the affirmative and the processor advances to instruction 2097 to retrieve the identity of the selected feature. That is, the identity of case feature 2028 is retrieved; and the processor branches to the routine discussed above in conjunction with FIG. 13A. Hence, the feature which has been selected (i. e. selected case feature 2028) now may be observed.

If close case button 2030 is actuated, inquiry 2098 is answered in the affirmative and instruction 2099 is carried out to clear edit case frame 2002. However, the data which had been entered into the various subframes included within edit case frame 2002 is stored in the knowledge base.

## Discrete Feature Toolbox

Let it be assumed that the case feature selected for editing is a discrete feature; and that the processor branches to the routine illustrated in FIG. 21A, as when instruction 2041 (FIG. 20B) is executed. Turning to FIG. 21A, instruction 2101 is carried out to retrieve from the knowledge base the value of the selected case feature 2028. Inquiry 2103 is made to determine if this selected case feature is visual. If not, the processor merely advances to instruction 2121 to create the feature name message in toolbox frame 2010.

However, if the feature selected for editing is a visual feature, inquiry 2103 is answered in the affirmative and instruction 2105 is carried out. Create image button 2104 is created and then the processor inguires, at 2107, if this feature which has been selected for editing is associated with images. If so, the first image icon in image icon menu 2008 is highlighted, as represented by instruction 2109, and the image associated with this icon is read from video disk player 112 and displayed in case image subframe 2004 together with its associated overlays. Then, instruction 2111 is carried out to create confirm image button 2106, followed by instruction 2113 to create delete image button 2108, followed by instruction 2115 to create the create overlay button 2110, followed by instruction 2117 to create link overlay button 2112, and finally to instruction 2119 to create delete overlay button 2114. Then, instruction 2121 (discussed above) is reached. If inquiry 2107 is answered in the negative, an image is not displayed in subframe 2004.

After instruction 2121 is executed, the processor advances to instruction 2123 to create the "feature type: discrete" message in toolbox frame 2010. Thereafter, value buttons 2102 are created, each button enumerating a possible discrete value of the feature selected for editing. As shown in FIG. 21, one of the possible values assigned to these buttons is the "not reported" value.

Then, the routine advances to instruction 2127 to retrieve from the knowledge base the value assigned to selected feature 2028 for this particular case. This value is indicated by brightening a corresponding one of value buttons 2102. However, if this particular case feature has not been edited previously, that is, if no value has been assigned to this feature for this case, the "not reported" value is indicated.

The routine continues to instruction 2129 by which any feature annotation text associated with selected case feature 2028 that had been written previously to the knowledge base is retrieved therefrom and entered into the feature annotation subframe. Thereafter, wait state 2131 is assumed.

If one of value buttons 2102 is selected, as when the user wishes to assign a value to this feature being edited, or if a previously assigned value is to be changed, inquiry 2133 is answered in the affirmative. Then, the value which is selected by the user is written to the knowledge base as the value assigned to this case feature. Thereafter, the processor returns to its wait state.

Let it be assumed that create image button 2104 is actuated. Inquiry 2137 is answered in the affirmative and the processor advances to instruction 2139. An image that may be derived from, for example, microscope 114 and video camera 116 (FIG. 1) is displayed in case image subframe 2004.

As an alternative, let it be assumed that confirm image button 2106 is actuated. Accordingly, inquiry 2141 is answered in the affirmative and instruction 2143 is executed. As represented by the flow chart of FIG. 21A, this instruction links the image displayed in case image subframe 2004 with selected case feature 2028 (that is, the feature being edited); and this image is written to the knowledge base. For example, if disk drive 112 is a WORM drive, the image may be recorded by this disk drive. In addition, an image icon representing this particular image is written to the knowledge base and displayed in image icon menu 2008.

If delete image button 2108 is actuated, inquiry 2145 is answered in the affirmative and the processor advances to inquire, at 2147, if any overlays are associated with the image currently being displayed in case image subframe 2004. If inquiry 2147 is answered in the negative, the image which then is displayed in case image subframe 2004 is deleted from the knowledge base, and also is cleared from the case image subframe, and its corresponding image icon is erased from the knowledge base and cleared from image icon menu 2008.

However, if overlays are associated with the image then being displayed, inquiry 2147 is answered in the affirmative and the processor advances to instruction 2151 by which a suitable warning message is displayed. An example of such a message is as follows: "Images with existing overlays may not be deleted. Delete overlays prior to deleting the image."

If create overlay button 2110 is actuated, inquiry 2153 is answered in the affirmative and the processor advances to instruction 2155 by which the routine branches to a paintbox routine, discussed below in conjunction with FIG. 21B.

If link overlay button 2112 is actuated, inquiry 2157 is answered in the affirmative and the processor advances to inquire, at 2159, if the image then being displayed in case image subframe 2004 previously had been confirmed. If so, inquiry 2161 is made to determine if the user had assigned a value to this case feature being edited. If not, instruction 2165 is carried out to provide a warning message. A typical warning message may be as follows: "Set value prior to confirming overlay."

However, if a value had been assigned to this feature being edited, inquiry 2161 is answered in the affirmative and the processor advances to instruction 2163. By this instruction, an overlay which is displayed in case image frame 2004 (and created in the manner discussed below in conjunction with FIG. 21B) is associated with the case feature being edited, and is written to the knowledge base. In addition, this overlay now is listed in overlay list 2006.

If the image displayed in case image subframe 2004 had not been confirmed previously, inquiry 2159 is answered in the negative and instruction 2167 is executed to cause a suitable warning message to be displayed. A typical message may be as follows: "Confirm image prior to confirming overlay."

## Paintbox

Assuming create overlay button 2110 had been actuated, instruction 2155 is executed and the processor branches to the flow chart shown in FIG. 21B. Turning to this flow chart, paintbox frame 2116 together with the selectable items displayed therein are created. As illustrated in FIG. 21, four separate arrows pointing in four different directions, a circle, a square and a line segment are created. A predetermined one of these selectable items, such as the first arrow displayed in paintbox frame 2116, is selected. Any other one of these selectable items may be selected as a default item, as may be desired. In addition, any overlay that may be displayed in case image subframe 2004 is cleared therefrom. Thus, previously existing overlays that may be residual from a preceding edit operation are cleared. This is represented by instruction 2130. Thereafter, the processor advances to its wait state 2132.

If the user positions the cursor over any desired position of an image displayed in case image subframe 2004, inquiry 2134 is answered in the affirmative. The processor then advances to inquire, at 2136, if the user actuates a particular switch, or pushbutton, disposed on mouse 110. In one hardware embodiment described herein, inquiry is made as to whether the right-most button on the mouse has been actuated. If so, the routine advances to inquiries 2138, 2144, 2150, 2156, 2162, 2169 and 2173 to determine which of the seven selectable items 2118 included in paintbox subframe 2116 had been selected (i. e. presently is highlighted). If one of the arrows is the selected item, the processor then inquires, at 2140, 2146, 2152 or 2158 if the right-most button on the mouse is released by the user. If so, the selected arrow then appears superimposed over the image then displayed in case image subframe 2004 at the position of the cursor. Thus, if one of the arrows is the selected item, that arrow is displayed as an overlay on the displayed image. This is represented by instructions 2142, 2148, 2154 and 2160.

If the circle or the square displayed in paintbox frame 2116 constitutes the selected overlay item, inquiry 2162 or inquiry 2169 is answered in the affirmative. Then, the circle or square, whichever constitutes the selected item, is displayed in case image subframe 2004 at the cursor location; and as the cursor is moved, the size of the circle or square increases. This is represented by instructions 2164 and 2170, respectively. Thereafter, upon release by the user of the right-most button on mouse 110, as indicated by an affirmative answer to inquiries 2166 or 2171, the circle or square, whose size has been "scaled" by instructions 2164 or 2170, remains as the overlay for the displayed image, as represented by instructions 2168 or 2172.

If the selected overlay item is the line displayed in paintbox frame 2116, inquiry 2173 is answered in the affirmative; and the processor advances to instruction 2174 to position a line in case image subframe 2004 under the cursor. As the cursor is moved, the line is extended. Then, if the right-most button on mouse 110 is released, inquiry 2175 is answered in the affirmative and the line drawn by instruction 2174 appears as the overlay on the displayed image.

The foregoing has described how an overlay is created at a desired position on an image displayed in case image subframe 2004. If the cursor is disposed within this subframe, but the right-most button on mouse 110 is not actuated, inquiry 2136 is answered in the negative. Inquiry 2177 then determines if another button disposed on mouse 110 is actuated. In the hardware embodiment described herein, the routine inquires if the middle button on the mouse is actuated. If so, whatever overlay is disposed beneath the cursor in case image subframe 2004 is erased. If the overlay is constituted by the line, then this line is erased as the cursor moves thereover. Hence, the line may be shortened or cleared, as desired. This erasure of the overlay continues until the middle button on mouse 110 is released. On that occurrence, inquiry 2179 is answered in the affirmative; and the processor simply returns to its wait state.

## Continuous Feature Toolbox

FIG. 21A, described above, illustrates the routine carried out by the processor to create toolbox frame 2010 for a discrete feature. A similar routine is carried out to create the toolbox frame for a continuous feature. This continuous toolbox routine is represented by the flow chart illustrated in FIG. 22A. It will be appreciated that most of the inquiries and instructions set out in the routine illustrated in FIG. 22A are substantially the same as the instructions and inquiries which have been discussed above in conjunction with the routine illustrated in FIG. 21A. Accordingly, to avoid duplication of description, and in the interest of brevity, further discussion of such substantially similar items is not provided.

However, the following differences between the displays of FIGS. 22 and 21 and those portions of the routines shown in FIGS. 22A and 21A which are associated with these different displays now are described. It is observed that toolbox frame 2010 in FIG. 22 is provided with upper and lower limits for the continuous feature, as well as a set value text input item 2202. In the flow chart shown in FIG. 22A, the upper and lower límits are retrieved from the knowledge base and displayed in toolbox frame 2010 by instruction 2225. In addition, instruction 2227 of FIG. 22A creates set value text input item 2202, retrieves from the knowledge base the value which had been assigned to case feature 2028 in a previous operation and displays this retrieved value as the "set value". Of course, if a value has not been assigned to case feature 2028, the "set value" will be displayed as "not reported".

If the user inputs a value, such as a desired numerical value, into set value text input item 2202, as by positioning the cursor over this item and operating keyboard 108, inquiry 2233 is answered in the affirmative. The processor then advances to inquire, at 2235, if the number entered as the "set value" is within the range defined by the upper and lower limits that had been assigned to this case feature. If so, the routine advances to instruction 2237 to write this inputted numerical value to the knowledge base. However, if the input value is outside the range defined by the upper and lower limits, instruction 2239 is carried out to display a warning message to the user. A typical message may be as follows: "Enter a number between the upper and lower limits." In addition, the number which had been entered by the user as the "set value" is cleared. Then, the processor returns to its wait state awaiting user-initiated action. Since such action has been described above in conjunction with the discrete toolbox routine of FIG. 21A and the paintbox routine of FIG. 21B, further, duplicative description thereof is not provided herein.

## Scripted Feature Toolbox

If, during an edit case operation (described above) the case feature selected for editing is a scripted feature, the processor branches from instruction 2051 (FIG. 20B) to the routine represented by the flow chart illustrated in FIG. 23B. In this routine, the selected case feature, such as case feature 2028 (FIG. 23A) is retrieved from the knowledge base with its assigned value, in accordance with instruction 2301. The processor then causes create image button 2306 to be displayed, as represented by instruction 2303; and inquires, at 2305, if the selected case feature is associated with any images. If not, the routine merely advances to instruction 2309. However, if images are associated with selected case feature 2028, instruction 2307 is carried out to highlight a preselected image icon, such as the first image icon, in image icon menu 2008. This highlighted image is displayed in case image subframe 2004. Then, instruction 2309 is carried out.

The processor continues to create confirm image button 2308 (instruction 2309), to create delete image button 2310 (instruction 2311), to create collect data button 2312 (instruction 2313), to create link overlay button 2314 (instruction 2315) and to create delete overlay button 2316 (instruction 2317). Thereafter, the routine executes instruction 2319 to create the feature name message displayed at the top of this toolbox frame, and to display the message "Feature type: Scripted" beneath the feature name. Then, a measurement list 2302 is created by instruction 2321.

If previous measurements for this scripted case feature have been made, these measurements are listed in measurement list 2302. For the present, let it be assumed that this list is empty.

The processor then advances to instruction 2323 to retrieve from the knowledge base the upper and lower limits that have been established for this feature (discussed above with respect to FIG. 12A). Then, instruction 2325 is carried out to retrieve from the knowledge base the value previously assigned to this scripted feature, and this value is displayed as value message 2304. If a value has not yet been assigned to this case feature, the value "not reported" is displayed. Thereafter, the routine retrieves from the knowledge base whatever text had been entered as the feature annotation, and as represented by instruction 2327, this retrieved text is displayed in the illustrated feature annotation subframe. The processor then advances to its wait state 2329.

It is seen that the scripted feature toolbox frame includes many of the same buttons as are included in the discrete feature toolbox frame shown in FIG. 21 and described above in conjunction with the flow chart of FIG. 21A. Accordingly, actuation of create image button 2306, confirm image button 2308, delete image button 2310, link overlay button 2314 or delete overlay button 2316 results in the same processor operation as was described above in conjunction with FIG. 21A. In the interest of brevity, discussion of that operation is not repeated here.

The scripted feature toolbox frame is provided with collect data button 2312, which is not included in the continuous or discrete feature toolbox frames discussed above. If the collect data button is actuated, the routine represented by the flow chart of FIG. 23B detects this, and an affirmative answer is made to inquiry 2361. The routine then advances to inquire, at 2363, if case feature 2028 is a measure of density. If so, the processor advances to instruction 2365 and branches to the collect density data routine shown in FIG. 23C.

If inquiry 2363 is answered in the negative, the routine of FIG. 23B inquires, at 2367, if case feature 2028 is a measure of linear distance. If so, the routine advances to instruction 2369 and the processor branches to the collect linear data routine shown in FIG. 23E. Finally, if inquiry 2367 is answered in the negative, the routine of FIG. 23B advances, to inquire at 2371, if case feature 2028 is a measure of area. If so, instruction 2373 is carried out and the processor branches to the collect area data routine illustrated in FIG. 23G.

## Collect Density Data

If the processor branches to the collect density data routine shown in FIG. 23C, instruction 2326 is carried out to add the term "density" to the feature type message displayed at the top of the scripted feature toolbox frame. Then, a list of image magnifications is created which, for example, is displayed as "not set", 1X, 10X, 20X and 40X. Initially, the magnification is indicated as "not set" awaiting a selection by the user.

The processor then continues to instruction 2330 to create overlay symbols 2320 in paintbox frame 2318, shown in FIG. 23A. As an example, one of these symbols, which may be selected by the user, as by positioning the cursor thereover, consists of a square. FIG. 23A also illustrates a target symbol in the paintbox frame. Selection of the target symbol initiates a target mode which, as will be described, designates an element displayed in image subframe 2004 as a target element. The density of those elements selected by the user as target elements is calculated by this collect density data routine.

One of the illustrated symbols is preselected as a default selection, and for convenience, the square is preselected as the default symbol. In addition, a "count" variable (which simply is a count of target elements) and a "square parameters" variable (which is the area of a square overlay, as determined by the selected magnification factor and the size of the overlay square, to be described) are created. Then, enter button 2324 is created, as represented by instruction 2332, and reset button 2322 is created, as represented by instruction 2334. The processor then advances to its wait state 2336.

The user now may measure the density of case feature 2028 as observed in the image displayed in case image subframe 2004. The manner in which this is carried out now is described.

The user may position the cursor at any desired location over the image displayed in case image subframe 2004. It is expected that the cursor will be positioned over the case feature whose density is to be measured. Inquiry 2338 determines if a predetermined switch or pushbutton of mouse 110, such as the left-most button, is actuated when the cursor is so positioned. If not, the routine merely resumes its wait state. However, if inquiry 2338 is answered in the affirmative, the processor inquires, at 2340, if a desired magnification ratio has been set. If not, the wait state is maintained.

If a magnification ratio has been selected and if the left-most button on mouse 110 is actuated, inquiry 2342 determines if the square symbol in paintbox frame 2318 has been selected. If so, the processor advances to inquire, at 2344, if density data has been entered previously for this case feature. If this inquiry is answered in the affirmative, instruction 2346 is executed to initialize (or reset) both the count variable and the square parameters variable to zero. Any overlay which may be present in case image subframe 2004 is cleared. Thus, a density measuring operation is enabled.

If density data had not been entered previously for this case feature, inquiry 2344 is answered in the negative and the processor merely advances to instruction 2348. This instruction superimposes the square symbol as an overlay on the image displayed in case image subframe 2004 at the position of the cursor. Then, inquiry 2350 determines if the user moves the mouse while the right-most button thereon is actuated. If not, a suitable warning message is displayed, as repreented by instruction 2366. A typical message may be as follows: "Enter data." However, if the mouse is moved while the right-most button is actuated, instruction 2352 is carried out to increase the size of the square. This sizing, or scaling, continues until the right-most button of mouse 110 is released. Such button release is indicated by an affirmative answer to inquiry 2354, whereupon the processor advances to instruction 2356 to display an overlaid square in case image subframe 2004 and to indicate this square in overlay list 2006. In addition, the actual size of the square, which is a function of the magnification ratio selected by the user and the dimension of the displayed overlaid square, is written as the variable designating the "square parameter". The processor thereafter assumes its wait state 2336.

When the user positions the cursor over the target symbol, inquiry 2342 is answered in the negative and inquiry 2358 is answered in the affirmative. Then, the routine advances to inquire, at 2360, if the right-most button included in mouse 110 has been released. If not, the processor remains at its wait state. However, if the user positions the cursor over an element displayed in the overlaid square, such as a type of cell whose density is to be calculated, and then releases the right-most mouse button, inquiry 2360 is answered in the affirmative. The user thus may identify all of the cells of this type within the overlaid square. Then, the processor advances to inquiry 2362 to determine if the size of the square (i. e. the "square parameter") is non-zero. If a square size has been entered, this inquiry is answered in the affirmative; and the routine advances to instruction 2364. This instruction increments the "count" variable and displays this count of the number of cells that have been identified in or adjacent the square displayed as an overlay in case image subframe 2004. This count is incremented each time another cell in the square is identified (or targeted). The processor then returns to its wait state.

## Collect Linear Measurement Data

If the case feature being edited is a linear measurement, the processor branches to the flow chart shown in FIG. 23E when instruction 2379 is reached in FIG. 23B. Turning to this flow chart, instruction 2369 is carried out to insert the term "linear" to the feature type message displayed at the top of the toolbox frame. If the selected case feature 2028 has been edited previously, the list of measurements which were created is retrieved from the knowledge base and displayed in measurement list 2302. In addition, the last-determined linear measurement is retrieved and displayed in value message 2304. The processor then advances to instruction 2380 to create the list of magnification ratios and display that list in paintbox frame 2318 (FIG. 23D).

The processor advances to instruction 2381 to create current value message 2377, and then instruction 2382 is carried out to create undue button 2378. Thereafter, a reset button (not shown in FIG. 23D) is created, as represented by instruction 2383. The processor then assumes its wait state 2384.

If the user actuates a predetermined button on mouse 110, such as the left-most button, when the cursor is positioned within case image subframe 2004, inquiry 2385 is answered in the affirmative;

and the processor advances to inquiry 2386 to ascertain whether a magnification ratio has been set. If not, an error message is displayed, as represented by instruction 2387. A suitable message may be as follows: "You must set the magnification prior to collecting data."

However, if inquiry 2386 is answered in the affirmative, inquiry next is made, at 2388, to determine if the user moves the mouse and, thus, the cursor, while the right-most button remains actuated. If this inquiry is answered in the affirmative, a line is drawn, or sized, between the initial cursor position and its current position, as represented by instruction 2389. Then, inquiry 2390 determines if the user releases this right-most button. If this inquiry is answered in the negative, the line continues to be drawn. However, once this button is released, the processor carries out instruction 2391 whereby the length of the sized line segment is calculated, and this linear distance measurement is displayed as current value 2377 (FIG. 23D). Furthermore, this measurement is written into measurement list 2302. Then, for all sized line segments that had been calculated, or measured, for the image displayed in case image subframe 2004, the average length thereof is determined. This average length measurement is displayed as value message 2304 and is written to the knowledge base. Furthermore, the updated measurement list, that is, the list containing the latest linear measure, is written to the knowledge base. The processor then resumes its wait state.

If undo button 2378 is actuated, inquiry 2392 is answered in the affirmative. Then, instruction 2393 is executed to delete the last linear measurement present in measurement list 2302, to delete the last line segment that had been drawn overlying the image in case image subframe 2004, and to clear the measurement displayed in current value message 2377. Thereafter, the wait state is resumed.

If the reset button (not shown) is actuated, inquiry 2394 is answered in the affirmative and the processor advances to instruction 2395. This instruction clears all of the measurements that may be listed in measurement list 2302. In addition, value message 2304 is cleared as is current value message 2377. Any overlay line segments that may be present in case image subframe 2004 also are cleared. Thereafter, the processor resumes its wait state.

## Collect Area Data

If the case feature being edited is a measure of area, the routine illustrated by the flow chart shown in FIG. 23B branches to the flow chart shown in FIG. 23G when instruction 2373 (FIG. 23B) is reached. Turning to FIG. 23G, the routine begins at instruction 23100 which adds the term "area" to the displayed feature type located at the top of the toolbox frame. If this case feature had been edited previously, the list of previously measured areas is displayed as measurement list 2302, and the previously determined value of the measured area is retrieved from the knowledge base and displayed as value message 2304. Then, the routine advances to instruction 23101 to list in paintbox frame 2318 the list of magnification ratios which may be selected.

The processor advances to instruction 23102 to create an intensity slider 2394 (FIG. 23F) which displays an upper intensity limit of 255 and a lower intensity limit of 0. Next, instruction 23103 is carried out to create an "intensity at cursor" message 2396. It will be appreciated that the "intensity" referred to herein is a measure of the brightness of a designated portion of the image displayed in case image subframe 2004. Of course, the "intensity at cursor" message indicates the brightness of that portion of the displayed image juxtaposed the cursor.

The processor advances to instruction 23104 to create undo button 2398 and a stop button (not shown). Then, instruction 23105 is carried out to create reset button 2397. Thereafter, the processor assumes its wait state 23106.

To obtain a measurement of area, the user first must define the area to be measured, and then that area must be "filled in" to distinguish it from the remainder of the image. To define the area, the user moves the cursor to "draw" a closed area on the displayed image. Inquiry 23114 determines if a predetermined switch, or button, on mouse 110 is actuated while the cursor is juxtaposed the image displayed in case image subframe 2004. If this inquiry is answered in the affirmative, the processor advances to inquire, at 23115, if the cursor is moved, while this button remains actuated. If so, a line of predetermined intensity, such as the maximum intensity (having an intensity level of 255) is drawn as the cursor is moved. It is appreciated that if the cursor moves to define a closed area, this closed area is drawn as an overlay on the displayed image. Then, the processor returns to its wait state.

As the cursor is moved within case image subframe 2004, inquiry 23117 is answered in the affirmative. Then, instruction 23118 is carried out to display the intensity of the image juxtaposed the instantaneous position of the cursor, this display being provided in intensity at cursor message 2396. It is appreciated that, as the cursor passes from brighter to dimmer regions in the displayed image, the intensity value displayed as message 2396 correspondingly varies to reflect this changing intensity.

To obtain a measurement of area, the user positions the cursor within the closed area which he has drawn. Once the cursor is so positioned, it is expected that he will actuate one particular button on mouse 110, such as the left-most button, while releasing another button of the mouse, such as the right-most button. Inquiry 23107 determines if the left-most button is actuated while the cursor is located within case image subframe 2004; and if this inquiry is answered in the affirmative, inquiry 23108 determines if the right-most button has been released. If so, the processor inquires, at 23109, if a magnification ratio has been set. If this inquiry is answered in the negative, instruction 23110 is carried out to display a warning message. A suitable message may be as follows: "You must set the magnification prior to collecting data."

However, if the magnification ratio has been set, the routine advances to inquiry 23111 to determine if slider 2394 is set. This slider is used to establish a threshold intensity level for the purpose described below. Preferably, this intensity level is set to be less than 255, the maximum intensity level. Setting the slider to establish the desired intensity level may be achieved by positioning the cursor over slider 2394 and then moving the cursor from left to right to increase the desired intensity level or, alternatively, from right to left to decrease this intensity level. Once the slider is set, inquiry 23111 is answered in the affirmative and the processor advances to instruction 23113.

Instruction 23113 causes a "recursive fill" in an area located at the cursor and extending to a boundary whose intensity level is less than that set by slider 2394. Commencing with the pixel selected by the cursor, that pixel is filled if its intensity level is below the intensity level of slider 2394. Then, adjacent pixels are similarly filled, and so on, until a pixel is reached whose intensity level is equal to or greater than the intensity level set by slider 2394. It is appreciated that if a closed area has been drawn, as represented by instruction 23116, having a boundary whose intensity level is equal to 255, the entire area within this boundary will be filled by recursive fill instruction 23113. Then, the size of the filled area is calculated and written into measurement list 2302. The average of all areas which have been similarly defined and filled on the displayed image is calculated, and this average area measurement is displayed as value message 2304. This average area measurement is written to the knowledge base. Also, updated measurement list 2302 is written to the knowledge base.

Thereafter, inquiry 23119 determines if the stop button (not shown) has been actuated. If not, the processor merely returns to its wait state 23106.

However, if the stop button has been actuated, a recursive fill operation is terminated if it is in process, as represented by instruction 23120. The routine then advances to instruction 23121, whereby the last item entered into measurement list 2302 is deleted and the last closed area which had been drawn as an overlay in case image subframe 2304 is cleared. Also, the average area measurement displayed in value message 2304 is cleared. Then, the processor returns to its wait state.

If undo button 2398 is actuated, inquiry 23122 is answered in the affirmative; and the processor then advances to instruction 23121, described above.

If reset button 2397 is actuated, inquiry 23123 is answered in the affirmative. Then, instruction 23124 is carried out to clear measurement list 2302. Also, value message 2304 is cleared and the overlays that may have been drawn, as in accordance with instruction 23116, are cleared. Thereafter, the wait state is assumed.

## Deleting a Case

If cut case button 214 of home screen 200 is actuated, the delete case routine illustrated in FIG. 24A is executed. Instruction 2401 is carried out to retrieve the identification of selected case 206 in case list 204, i. e. the case selected for deletion. Inquiry 2403 is made requiring confirmation by the user to continue with the delete case operation. A message, such as message 2402 (FIG. 24) is displayed. A typical message may be as follows: "This action destroys case information. Do you wish to proceed?" If the user responds by indicating negative action, the processor merely advances to its wait state 2407. However, if the user confirms that he wishes to proceed, instruction 2405 is carried out by which this selected case is deleted from the knowledge base.

## Selecting a "Diagnosis Tree"

To create a hierarchy of diagnoses, diagnosis button 244 of home screen 200 may be actuated. This is represented by the shaded diagnosis button illustrated in FIG. 25. As a result, the routine illustrated in FIG. 26A is carried out; and this routine results in the display illustrated in FIG. 26.

Referring to FIG. 26A, inquiry first is made, as at 2601, to ascertain if the user has actuated the diagnosis button. If not, the processor merely remains at its wait state 2621. However, if this inquiry is answered in the affirmative, the existing hierarchy of diagnoses is retrieved from the knowledge base. as represented by instruction 2603. Then, instruction 2605 is carried out to create the diagnosis tree frame 2602 and the diagnosis tree 2604, which is formed of this hierarchy of diagnoses, is displayed (FIG. 26). In addition, a vertical scroll bar 2636 and a horizontal scroll bar 2638 are created.

The processor advances to instruction 2607 to create a graphical representation of the diagnosis tree within frame 2602. As illustrated, each diagnosis is represented as a rectangle in which the name of the diagnosis is provided. Each rectangle constitutes a node from which sublevels of diagnoses may depend. A root node 2606 is created from which diagnoses 2608, 2610 and 2612 depend. These diagnoses all reside on a common level and, for convenience, are referred to herein as "siblings". An additional "sibling" diagnosis 2614 depends from root 2606 and is identified as a "not reported" diagnosis. Except for the not reported diagnosis 2614, any other diagnosis node in this level may have one or more diagnoses dependent thereon, and such dependent diagnoses are referred to as "children". Diagnosis 2608 is seen to have one child diagnosis node 2616 dependent thereon. Diagnosis 2610 is seen to have two children diagnoses 2618 and 2620 dependent thereon. All of the diagnoses included in this second lower level also are seen to be siblings. Any child diagnosis may have dependent thereon another child. such as child diagnosis 2624 which depends from child diagnosis 2620.

Any diagnosis in any level may be selected, as by positioning the cursor thereover. A selected diagnosis is highlighted, whereas all other diagnoses are displayed as relatively dimmed rectangles and are unselected.

A predetermined diagnosis node is selected as the default node. For convenience, instruction 2609 selects root node 2606 as the default node. It will be appreciated that the default node automatically is the selected node unless and until the user takes action to select a different diagnosis node.

After the root node is selected as the default node, the processor advances to instruction 2611 to form the create child button 2626 (FIG. 26). Then, instruction 2613 is carried out to create edit node button 2628. Thereafter, view node button 2630 is created, as by instruction 2615, followed by the creation of cut branch button 2632, as represented by instruction 2617. Thereafter, close button 2634 is created, as by instruction 2619. Once the diagnosis tree has been displayed and the aforementioned buttons have been created, the processor assumes its wait state 2621.

Depending upon which of buttons 2626, 2628, 2630 and 2632 is actuated, the processor branches to a corresponding routine to execute the program initiated by the actuated button. Thus, if create child button 2626 is actuated, inquiry 2623 is answered in the affirmative and the processor branches to the create child routine, illustrated by the flow chart of FIG. 27A, and represented by instruction 2625. Similarly, if edit button 2628 is actuated, inquiry 2627 is answered in the affirmative and the processor thereafter branches to the edit node routine, illustrated by the flow chart of FIG. 28A, as represented by instruction 2629. When view node button 2630 is actuated, inquiry 2631 is answered in the affirmative. The processor then branches to the view node routine represented by the flow chart shown in FIG. 30A, as indicated by instruction 2633. Finally, if cut branch button 2632 is actuated, inquiry 2635 is answered in the affirmative and the processor branches to the cut branch routine, illustrated by the flow chart shown in FIG. 29A, and represented by instruction 2637.

If close button 2634 is actuated, inquiry 2639 is answered in the affirmative. The processor then advances to inquire, at 2641, if any subframes that may have been created as a result of the actuation of buttons 2626, 2628 or 2630, as will be described below, still are displayed, or "open". If not, diagnosis tree frame 2602 is cleared, as instructed by instruction 2643. However, if a subframe is "open", instruction 2645 is carried out to display a suitable warning message. A typical message may be as follows: "All diagnosis frames must be closed prior to closing the diagnosis tree frame."

Creating a Child Diagnosis Node

Let it be assumed that create child button 2626 of FIG. 26 is actuated. The processor then branches to the create child routine now described in conjunction with the flow chart of FIG. 27A.

A child diagnosis node, by definition, depends from a preexisting diagnosis node. That preexisting diagnosis node must be the selected node in order to create a child dependent thereon. The routine illustrated in FIG. 27A begins by retrieving from the knowledge base the active diagnosis node, represented as node 2610 in FIG. 27. Then, the processor advances to instruction 2703 to display create child frame 2702 (this is one of the "subframes" mentioned above). The routine continues to instruction 2705 to create diagnosis name text input 2704; and thereafter to create prior probability text input 2706. (Elements identified by even reference numerals are illustrated in FIG. 27.) It is appreciated that diagnosis name text input 2704 is used by the user to identify the diagnosis being created; and the prior probability text input 2706 is used to indicate the probability of this diagnosis occurring in the population of cases included in the knowledge base.

The processor then advances to instruction 2709 to create diagnosis annotation editor 2708. Then, enter button 2710 is created, as by instruction 2711, and cancel button 2712 is created, as by instruction 2713. The processor then assumes its wait state 2715.

In creating a child diagnosis node, it is expected that the user will select and enter a desired name to identify this diagnosis. If so, inquiry 2719 is answered in the affirmative, and the entered name is displayed in diagnosis name text input 2704. It also is expected that the user will determine the probability of this diagnosis occurring in the interested population of case records included in the knowledge base. If this probability is entered in prior probability text input 2706, as by positioning the cursor thereover and then operating keyboard 108, inquiry 2721 is answered in the affirmative.

Furthermore, it is expected that the user will enter an annotation at diagnosis annotation editor 2708 to provide commentary on this new diagnosis. Inquiry 2717 determines when text is entered in the diagnosis annotation text editor.

Once the diagnosis name, its probability and commentary have been entered into create child frame 2702, it is anticipated that the user eventually will actuate enter button 2710. Inquiry 2723 determines when this button is actuated. Upon actuation, this inquiry is answered in the affirmative and the processor advances to inquire, at 2725, if a unique diagnosis name has been entered in the diagnosis name text input 2704. If not, instruction 2727 is carried out to cause a warning message to be displayed. A suitable warning message may be as follows: "You must enter a unique diagnosis name or cancel this operation."

If inquiry 2725 is answered in the affirmative, the routine advances to inquire, at 2729, if a number in the range between 0 and 1 has been entered by the user in the prior probability text input item 2706. If a prior probability number has not been entered, the processor advances to instruction 2739, described below.

However, if inquiry 2729 is answered in the affirmative, inquiry is made, at 2731, to ascertain if the sum of all prior probabilities for this node and sibling nodes (i. e. those diagnosis nodes on the same level as the node being created) is equal to one. If it is, the processor advances to instruction 2739. Also, any previously displayed warning messages relating to inconsistent probabilities are cleared, as indicated by instruction 2733.

If the sum of all prior probabilities for all nodes (including this node) on this level is not equal to one, the routine advances to instruction 2735 to produce an error message. A typical message may be as follows: "Inconsistently defined prior probability". Then, instruction 2739 is carried out.

Instruction 2739 writes the new diagnosis to the knowledge base, identifies this new diagnosis with the name entered in diagnosis name text input 2704, and positions this diagnosis in the hierarchy of diagnoses as the child which depends from the particular diagnosis node which is displayed as the active node. Then, the processor causes a new node in the diagnosis tree to be displayed. This new node is dependent from the selected diagnosis node, as represented by instruction 2741. The text which had been entered in diagnosis annotation editor 2708 is associated with this new diagnosis and written to the knowledge base, as represented by instruction 2743. Then, the processor advances to instruction 2745 to delete the create child frame 2702.

If, at any time, cancel button 2712 is actuated, inquiry 2747 is answered in the affirmative and instruction 2749 is carried out to clear the create child frame 2702. Thereafter, the processor assumes its wait state.

## Editing a Diagnosis Node

If edit node button 2628 is actuated when the diagnosis tree is displayed (FIG. 26), the processor branches to the edit node routine represented by the flow chart shown in FIG. 28A. A comparison of this flow chart to that shown in FIG. 27A indicates that the two are quite similar. Because of this similarity, the edit node flow chart need not be described in great detail. It should be noted, however, that an edit node frame 2802 (FIG. 28) is created in place of the create child frame 2702, shown in FIG. 27, and this too is one of the "subframes" mentioned above. Accordingly, the routine shown in FIG. 28A commences with instruction 2801 to retrieve the diagnosis tree from the knowledge base; and a desired one of the nodes displayed in this tree may be edited. A node may be selected for editing by positioning the cursor thereover. As represented in FIG. 28, the selected node, depicted as node 2610, is highlighted.

After the diagnosis tree is retrieved and displayed, edit node frame 2802 is created, as represented by instruction 2803. Then, the diagnosis name message is created and displayed, and the name of the selected diagnosis is retrieved from the knowledge base and displayed, as represented at 2804 in FIG. 28.

The routine continues to instruction 2807 to create the prior probability message and to display thereat the prior probability of selected diagnosis 2610, as retrieved from the knowledge base. This displayed prior probability is shown at 2806 in FIG. 28.

Then, the routine creates the diagnosis annotation editor 2808, as represented by instruction 2809, and displays therein the annotation retrieved from the knowledge base and associated with selected diagnosis 2610. Thereafter, enter button 2810 is created, as by instruction 2811, and cancel button 2812 is created, as by instruction 2813. Then, instruction 2815 is executed to display the "node children" message which lists those children which depend from selected node 2610. In the example shown in FIG. 28, the selected node is diagnosis node DX3, and its children are diagnosis node DX5 and diagnosis node DX8. These children diagnosis nodes are displayed in edit node frame 2802.

The user then may change the diagnosis name, adjust its prior probability and vary the commentary presented in diagnosis annotation editor 2808. Since these functions have been described above in conjunction with FIG. 27A, a discussion thereof need not be repeated here.

## Cut Branch

If cut branch button 2632 (FIG. 26) is actuated, the processor branches to the cut branch routine illustrated in FIG. 29A and now described. Instruction 2901 is executed to retrieve from the knowledge base the diagnosis tree. This diagnosis tree is displayed, as shown in FIG. 29. A desired diagnosis node should be selected for "cutting"; and it will be appreciated that the branch which extends between the selected diagnosis node and its parent in the next higher level will be cut, as indicated at 2902. Accordingly, inquiry 2903 requires confirmation by the user to continue with the cut branch routine. A suitable message requiring a response may be displayed, such as the message "Warning: Cutting diagnoses may destroy case information. Do you want to proceed?" If the user responds negatively, the processor merely advances to its wait state 2905. However, if the user confirms that he wishes to proceed, the routine advances to instruction 2907.

Instruction 2907 retrieves from the knowledge base the selected diagnosis and all information associated therewith. From FIG. 29, it is seen that the selected diagnosis is diagnosis 2620, this diagnosis depending from diagnosis 2610 and further having a child (referred to as "descendant") represented by diagnosis 2624. In addition to retrieving all information associated with selected diagnosis 2620, the name and data associated with all child nodes which depend from the selected diagnosis node also are retrieved. In the present example, all information associated with diagnosis 2624 is retrieved. All of the retrieved information is written into a store to preserve the hierarchal structure of these retrieved diagnosis nodes. That is, the hierarchal structure between diagnosis 2620 and diagnosis 2624 is stored.

Then, the processor advances to instruction 2909 to retrieve from the knowledge base a list of all cases possessing diagnoses 2620 and 2624. In each of these cases, the diagnosis which previously had been identified as diagnosis 2620 and diagnosis 2624 now is indicated as "not reported".

Then, instruction 2911 is carried out to delete from the knowledge base the selected diagnosis node and all descendants therefrom. As shown in FIG. 29, diagnoses 2620 and 2624 are deleted from the knowledge base. The routine continues to instruction 2913 to delete from the diagnosis tree the selected diagnosis 2620 and descendant, diagnosis 2624. Thereafter, the parent of selected diagnosis 2620, that is, diagnosis 2610, is designated the selected diagnosis in the illustrated diagnosis tree.

## View Node

If view node button 2630 (FIG. 26) is actuated, the processor branches to the view node routine illustrated by the flow chart of FIG. 30A. In this routine, the diagnosis tree is retrieved from the knowledge base and displayed in diagnosis tree frame 2602. Then, view node frame 3002 is created (shown in FIG. 30), as by instruction 3003. This view node frame is yet another of the aforementioned "subframes". Thereafter, the diagnosis name message is created and the name of the selected diagnosis, i. e. the name of diagnosis 2610, is retrieved from the knowledge base and displayed as the diagnosis name. The processor then advances to instruction 3007 to create the prior probability message within view node frame 3002. The probability of occurrence of selected diagnosis 2610 is retrieved from the knowledge base and displayed, as illustrated.

Instruction 3009 then is carried out to create case list 3004 and to retrieve from the knowledge base the list of cases which contain selected diagnosis 2610. This list of cases is displayed in case list 3004. Thereafter, edit case button 3006 is created, as by instruction 3011, and cancel button 3008 is created, as by instruction 3013. Then, the processor assumes its wait state 3050.

If edit case button 3006 is actuated, inquiry 3017 is answered in the affirmative and the processor advances to instruction 3019 to obtain the identification of the selected case included in case list 3004 and to branch to the edit case routine illustrated in FIG. 20A. It is appreciated that a case included in the case list may be selected merely by positioning the cursor thereover.

If cancel button 3008 is actuated, inquiry 3021 is answered in the affirmative and the processor advances to instruction 3023 whereby view node frame 3002 is cleared. Then, the processor returns to its wait state.

While the present invention has been particularly shown and described with reference to a preferred embodiment, it will be appreciated by those of ordinary skill in the art that various changes and modifications may be made without departing from the spirit and scope of the invention. For example, the instant invention is not limited solely for use with a Sun microsystem of the type described herein. It is anticipated that other data processing apparatus, or hardware, either presently commercially available or developed in the future, can be used to carry out the functions which have been described herein. It is further anticipated that the particular programs, or software, used with such hardware might exhibit routines and subroutines that differ in some respects from the routines represented by the flow charts described above. Nevertheless, the overall functions and results disclosed herein will be achieved by such hardware and software.

It also is expected that the means by which data, requests, user commands and the like may be entered into the system may differ in some respects from what has been described above. Although a keyboard and a mouse are preferred, particularly when exploiting the flexibility and ease of operation of the Sun microsystem, other input devices may be used, and some of these have been described above.

Still further, it should be recognized that the computer screens depicted in FIGS. 2-29 represent the presently contemplated best mode and preferred embodiment of the present invention. Nevertheless, other computer screen displays which furnish the user with substantially the same or similar information regarding features and case records may be adopted. It is understood that the presently described computer screens display "buttons" to exploit the flexibility of the Sun microcomputer operating and application systems. If a different data processor is used, or if the operating system is changed, or if displayed buttons no longer are applicable (for example, if a mouse is not used as the input device), the new computer screens will not display such buttons.

In developing the present invention, the separation of the various operating and data knowledge access tools into separate substantially independent sections, as represented by the feature window, the case window and the diagnosis function, has been found advantageous. Functions associated with the creation and editing features in the knowledge base, that is, the dictionary definitions and values of the respective features, affect information contained in all case records. It is helpful, therefore, to separate functions which affect the dictionary, and thus influence the entire knowledge base from functions which affect simply a particular case record. The likelihood of an author inadvertently editing or deleting a particular feature from the knowledge base (i. e. from the dictionary) thus is reduced by separating feature manipulation functions from case manipulation functions. Nonetheless, the present invention should not be construed as requiring this demarcation between the functions; and it is contemplated that, if desired, a menu capable of presenting and executing any and all functions may be presented to the user.

Although the foregoing specification has emphasized the use of the present invention in the discipline of medicine, the invention, in its broader aspects, finds ready application in a wide range of disciplines. More generally, but still not intending to limit the overall applicability thereof, this invention may be used to create a knowledge base of information and data produced by interpreting and characterizing empirical observations using the experience and knowledge of the observer. The information thus accumulated in the knowledge base may be used to reach or confirm deduced conclusions.

The present invention and knowledge base created thereby also may be used as a reference work. For example, in the medical discipline, the present invention and knowledge base may assist the training of physicians. Similarly, physicians, such as pathologists, who operate outside of their area of particular expertise may find the reference presented by the present invention a valuable support. Of course, the use of this invention as an aid in medical diagnosis is a primary objective.

It is intended that the appended claims be interpreted as including the foregoing as well as other equivalents.

## APPENDIX A

*** CLINICAL FEATURES ***

CF Patient Descriptors

CF Age - Numeric
0 - 20
21 - 30
31 - 40
41 - 50

51 - 60
61 - 70
Other
CDX F Sex - valued
Male
Female
Other

CF asbestos Exposure - valued

Present
Absent
Questionable
Unknown
Other

- CF Current Sites with Positive Cytology

CDXF Positive Cytology, Right Pleura - valued
Present
Absent
Unknown
Other

CF Positive Cytology, Peritoneum - valued

Present
Absent
Unknown
Other

CF Positive Cytology, Pericardium - valued

Present
Absent
Unknown
Other

CF Prior History of Tumor

CF Previous Tumor Occurrence - valued
Present
Absent
Unknown
Other
CF test - valued
CF test - valued
boy
girl
other
CF Previous Tumor Type - valued
Adenocarcinoma
Squamous Cell Carcinoma
Large Cell Carcinoma
Small Cell Carcinoma
Sarcoma
Lymphoma
Melanoma
Other
CF Previous Tumor Site - valued
Breast
Colon
Esophagus
Kidney
Lung
Ovary
Pancreas
Prostate
Stomach
Uterus
Other

CF Current Medical Problems

CF Congestive Heart Failure - valued
Present
Absent
Questionable
Unknown
Other
CF Myocardial Infarction - valued
Present
Absent
Questionable
Unknown
Other
CF Pulmonary Infarction - valued
Present
Absent
Questionable
Unknown
Other
CF Pulmonary Embolus - valued
Present
Absent
Questionable
Unknown
Other
CF Pneumonia - valued
Present
Absent
Questionable
Unknown
Other
CF Tuberculosis - valued
Present
Absent
Questionable
Unknown
Other
CF Liver Disease - valued
Present
Absent
Questionable

Unknown
Other
CF Renal Failure - valued
Present
Absent
Questionable
Unknown
Other
CF Pancreatitis - valued
Present
Absent
Questionable
Unknown
Other
CF Collagen Vascular Disease - valued
Present
Absent
Questionable
Unknown
Other
CF Recent Trauma - valued
Present
Absent
Questionable
Unknown
Other
CF Recent Surgery - valued
Present
Absent
Questionable
Unknown
Other
CF History of Chemotherapy - valued
Present
Absent
Questionable
Unknown
Other
CF History of Radiation Therapy - valued
Present
Absent
Questionable
Unknown
Other

CF Current Symptoms

CF Shortness of Breath - valued
Present
Absent
Questionable
Unknown
Other
CF Chest or Thoracic Pain - valued
Present
Absent
Questionable
Unknown
Other
CF Ascities - valued
Present
Absent
Questionable
Unknown
Other
CF Bowel Obstruction - valued
Present
Absent
Questionable
Unknown
Other

CF Chest Radiograph

CF Normal CXR - valued
Yes
No
Other
CF Effusion on CXR - valued
Absent
Present - Left
Present - Right
Present - Bilateral
Questionable
Other
CF Infiltrate on CXR - valued
Present
Absent
Questionable
Other
CF Pleural Thickening on CXR - valued
Present
Absent
Questionable
Other
CF Pleural Plaque on CXR - valued
Present
Absent
Questionable
Other
CF Mass on CXR - valued
Present
Absent
Questionable
Other
CF Lymphadenopathy on CXR - valued
Present
Absent
Questionable
Other

***PAP Cytology***

PAP Overview

PAP Quality of Fluid - valued
Well Preserved Fluid
Moderately Preserved Fluid
Poorly Preserved Fluid
Clotted Fluid
Other
PAP Staining Reaction - valued
Adequate
Inadequate
Other
PAP Cellularity of Specimen - valued
Low Cellularity
Moderate Cellularity
High Cellularity
Other
PAP # Atypical/Suspicious Cells/Groups - valued
Absent
Few
Moderate
Many
Other

PAP Cytoplasmic Morphology

PAP Cytoplasm, Abnormal Population - valued
Pale
Delicate
Dense
Variable
Poorly Visualized
NA
Other
PAP Cytoplasm, Mesothelial Population - valued
Pale
Delicate
Dense
Variable
Poorly Visualized
NA
Other
PAP Halo Outside Cell Border - valued
Absent
Occasional
Frequent
Other
PAP Halo Inside Cell Border - valued
Absent
Occasional
Frequent
Other
PAP Cytoplasmic Pigment - valued
Absent
Yellow
Brown
Black
Green
Other
PAP Cytoplasmic Vacuoles - valued
Absent
Occasional
Frequent
Other
PAP Number of Cytoplasmic Vacuoles - valued
Primarily Single
Primarily Multiple
NA
Other
PAP Cytoplasmic Vacuolar Size - valued
Small
Large
Variable
NA
Other
PAP Mucin Containing Vacuoles - valued
Present
Absent
Other
PAP Nuclear Deformation by Vacuoles - p/a
Present
Absent
Other
PAP Phagocytosis - p/a
Present
Absent
Other

PAP Nuclear Morphology

PAP Multinucleation - valued
Absent
Primarily Binucleate
Primarily 2 Nuclei/Cell
Other
PAP Chromatin, Abnormal Population - valued
Clear
Pale
Even
Finely Granular
Coarsely Granular
Hyperchromatic
Irregular
Variable
NA
Other
aaron - p/a
Present
Absent
Other
PAP Chromatin, Mesothelial Population - valued
Clear
Pale
Even
Finely Granular
Coarsely Granular
Hyperchromatic
Irregular

Variable
NA
Other
PAP Nuclear Border Shape - valued
Smooth
Irregular
Variable
Other
PAP Nucleoli - valued
Absent
Occasional
Frequent
Other
PAP Nucleoli per Nucleus - valued
Primarily Single
Primarily Multiple
NA
Other
PAP Nucleolus Size - valued
Small
Large
Variable
Other
PAP Nucleolus Shape - valued
Round
Irregular
Variable
Other
PAP Mitoses - p/a
Present
Absent
Other

PAP Features of Cell Background

PAP Macrophages - valued
Absent
Few
Moderate
Many
Other
PAP Lymphocytes - valued
Absent
Few
Moderate
Many
Other
PAP Polymorphonuclear Leucocytes - valued
Absent
Few
Moderate
Many
Other
PAP Reactive Mesothelial Cells - valued
Absent
Few
Moderate
Many
Other
PAP Activated Lymphocytes - valued
Absent
Few
Moderate
Many
Other

***Cell Block Features***

CB Overview

CB Quality of Fluid - valued
Well Preserved Fluid
Moderately Preserved Fluid
Poorly Preserved Fluid
Clotted Fluid
Other
CB Staining Reaction - valued
Adequate
Inadequate
Other
CB Cellularity of Specimen - valued
Low Cellularity
Moderate Cellularity
High Cellularity
Other
CB # Atypical/Suspicious Cells/Groups - valued
Absent
Few
Moderate
Many
Other

CB General Cell Morphology

CB Cell Shape, Abnormal Population - valued
Round
Oval
Cuboidal
Columnar
Polygonal
Spindle
Irregular
NA
Other
CB Cell Shape, Mesothelial Population - valued
Round
Oval
Cuboidal
Columnar
Polygonal
Spindle
Irregular
NA

Other
CB Nuclear Shape, Abnormal Population - valued
Round
Oval
Spindle
Irregular
Variable
NA
Other
CB Nuclear Shape, Mesothelial Population - valued
Round
Oval
Spindle
Irregular
Variable
NA
Other
CB Pleomorphism of Size, Single Cells - valued
Minimal
Moderate
Marked
Other
CB Pleomorphism of Shape, Single Cells - valued
Minimal
Moderate
Marked
Other
CB Pleomorphism, N:C Ratio, Single Cells - valued
Minimal
Moderate
Marked
Other
CB Pleomorphism of Nuclei, Single Cells - valued
Minimal
Moderate
Marked
Other

CB Features of Cell Groups

CB Cell Groupings Present - p/a
Present
Absent
Other
CB Shape of Cell Groups - valued
Papillary
Acinar
Flat
Variable
Other
CB Cell Number in Largest Groups - numeric
2
3 - 4
5 - 10
11 - 20
21
Other
CB Cell Number in Average Groups - numeric
2
3 - 4
5 - 10
11 - 20
21
Other
CB Pleomorphism of Cell Size in Groups - valued
Minimal
Moderate
Marked
Other
CB Pleomorphism of Cell Shape in Groups - valued
Minimal
Moderate
Marked
Other
CB Cell Orientation in Groups - valued
Regular
Partially Disoriented
Disoriented
Other
CB Similarity to Mesothelial Cells - valued
Present
Partial
Absent
Other
CB Distinct Outer Border of Cell Groups - p/a
Present
Absent
Other
CB Psamomma Bodies - p/a
Present
Absent
Other
CB Connective Tissue Cores - p/a
Present
Absent
Other

CB Histochemical Stains

CB Mucicarmine - valued
Positive
Negative
Not Done
Other
CB PAS - valued
Positive
Negative
Not Done
Other
CB PAS After Diastase - valued
Reduced Positive
REsidual Positive
Not Done
Other
CB Trichrome - valued
Positive

Negative
Not Done
Other
CB Colloidal Iron, Cells - valued
Positive
Negative
Not Done
Other
CB Colloidal Iron, Background - valued
Positive
Negative
Not Done
Other
CB Colloidal Iron After Hyal, Cells - valued
Reduced Positive
Residual Positive
Not Done
Other
CB Colloidal Iron After Hyal, Background - valued
Reduced Positive
Residual Positive
Not Done
Other

CB Immunoperoxidase

CB Keratin, Strength of Reaction - valued
Negative
+
+ +
+ + +
Not Done
Other
CB Keratin, % of Cells Reacting - numeric
0
1 - 5
6 - 10
11 - 25
26 - 50
51 - 75
76 - 95
96 - 100
Not Done
Other
CB CEA Strength of Reaction - valued
Negative
+
+ +
+ + +
Not Done
Other
CB CEA, % of Cells Reacting - numeric
0
1 - 5
6 - 10
11 - 25
26 - 50
51 - 75
76 - 95
96 - 100
Not Done
Other
CB GICA, Strength of Reaction - valued
Negative
+
+ +
+ + +
Not Done
Other
CB GICA, % of Cells Reacting - numeric
0
1 - 5
6 - 10
11 - 25
26 - 50
51 - 75
76 - 95
96 - 100
Not Done
Other
CB SSEA Strength of Reaction - valued
Negative
+
+ +
+ + +
Not Done
Other
CB SSEA, % of Cells Reacting - numeric
0
1 - 5
6 - 10
11 - 25
26 - 50
51 - 75
76 - 95
96 - 100
Not Done
Other
CB EMA Strength of Reaction - valued
Negative
+
+ +
+ + +
Not Done
Other
CB EMA, % of Cells Reacting - numeric
0
1 - 5
6 - 10
11 - 25
26 - 50
51 - 75
76 - 95
96 - 100
Not Done
Other

***Microscopic Preparation***

MP Magnification

MP 10x Objective - valued
330x to screen
Other
MP 25x Objective - valued
880x to screen
Other
MP 45x Objective - valued
1475x to screen
Other
MP 100x Oil Immersion - valued
2800x to screen
Other

MP Tissue Preparation

MP Staining - valued
Hemotoxalin and Eosin
Papanicalou
Mucicarmine
PAS
PAS After Diastase
Trichrome
Colloidal Iron
Colloidal Iron After Hyaluronidase
Keratin
CEA
CICA
SSEA
EMA
Other
MP Hemotoxalin and Eosin - Type Not Reported
MP Papanicalou - Type Not Reported
MP Mucicarmine - Type Not Reported
MP PAS - Type Not Reported
MP PAS after Diastase - Type Not Reported
MP Trichrome - Type Not Reported
MP Colloidal Iron - Type Not Reported
MP Colloidal Iron after Hyaluronidase - Type Not Reported
MP Keratin - Type Not Reported
MP CEA - Type Not Reported
MP GICA - Type Not Reported
MP SSEA - Type Not Reported
MP EMA - Type Not Reported

## Claims

1. A data creating, storage and processing system comprising:
image pick-up means for imaging one or more features observed in a sample of a disease and for producing pictorial image data representative thereof for display and for storage;
display means for displaying pictorial images of one or more features observed in a disease and for displaying case record data;
storage means for storing case record data, each case record including a case record identification, feature data selected for that case, value data selected for respective features in that case, and pictorial image data representing pictorial images of features of a disease associated with that case;
manually operably input means including: case identifying means for generating case identifying data to identify a case record written into and retrievable from said system, feature selecting means operable by a user to select from a feature bank those feature data which the user observes in one or more diseases represented by a case, and value assigning means operable by said user to select from a value bank value data which the user observes in the selected feature; and
processing means for linking feature data, value data and pictorial image data to case records, for retrieving case records, and for displaying pictorial images associated with retrieved case records.

2. The system of Claim 1, wherein said processing means includes overlay means for generating an overlay selected by said user and for supplying overlay data to said display means to display the selected overlay superimposed over a displayed pictorial image; and means for storing in said storage means overlay data representing said overlay and the position thereof relative to said displayed pictorial image.

3. The system of Claim 1 or 2 wherein said processing means includes means for supplying to said display means pictorial image data included in a case record having visual feature data selected by user operation of said feature selecting means for displaying a pictorial image of said visual feature data.

4. The system of Claim 3 wherein said processing means further includes means for causing said display means to display the number of cases stored in said storage means having said selected visual feature data and means for determining the distribution relative to each other of such cases having different respective values for that feature.

5. The system of Claim 1, 2 or 3 wherein said pictorial image data represent plural pictorial images, and wherein said processing means causes all of the pictorial images included in a case record retrieved by the user to be displayed on said display means.

6. The system of Claim 5 wherein said processing means supplies to said display means pictorial image data for displaying all of the pictorial images in relatively reduced size, and said processing means includes image selecting means responsive to operation by said user for selecting one of the displayed pictorial images to be displayed in relatively magnified size.

7. The system of any of the preceding claims wherein the value data are representative of measured values; and wherein said processing means includes means for determining the measured value for preselected features, including:

(a) manually operable curve tracing means for tracing an area of a feature displayed on said display means, and means for determining the area traced by said curve tracing means;

(b) manually operable distance measuring means for measuring the distance between points located in the pictorial image displayed by said display means; and

(c) manually operable point identifying means, and means for calculating the density of identified points.

8. The system of any of the preceding claims wherein said storage means includes optical storage means for storing said pictorial image data.

9. The system of Claim 8 wherein said processing means includes means for linking pictorial image data stored in said optical storage means with said case record.

10. A storage and data processing system, comprising:
manually operative input means to write data into and to read data from said system, and to request predetermined processing operations on said data;
image data input means including image pick-up means for generating and writing into said system image data derived from and representing preselected visual images;
display means for displaying both alphanumeric information of data written into and read from said system and pictorial images of image data written into and read from said system;
accessible storage means for storing data written by said manually operative input means and by said image data input means; and
programmable processing means in data communication with said manually operative input means, image data input means, display means and storage means and programmed to establish in response to said manually operative input means user-selectable designations of diagnostic features which the user determines are characteristic of respective conditions to be diagnosed and to establish user-selectable values of said features, said processing means also being programmed to store in said storage means image data representing pictorial displays of at least some of said features, said processing means being further programmed to cause said display means to display prompts for guiding said user to select respective ones of the established features and to assign an established value to a selected feature which the user observes in a pictorial image, and said processing means being additionally programmed to link a selected feature having an assigned value with a pictorial image for storage in and retrieval from said storage means.

11. The system of Claim 10 wherein each feature is associated with plural predetermined values, wherein said display means displays said associated values when said manually operative input means is operated by a user to select a feature so as to enable one of said values to be selected by the user and assigned to the selected feature, and wherein said manually operative input means is operated by the user to select an already established feature or to create a new feature for storage in said storage means, and to select an already established value or to create a new value for that feature for storage in said storage means.

12. The system of Claim 10 or 11 wherein said manually operative input means is operative to identify a feature as a "discrete" type or a "continuous" type; and wherein said processing means is further programmed (a) to retrieve and display a set of values associated with said feature, if said feature is identified as a "discrete" type, and to assign a selected one of those displayed values to said feature as determined by the user, and (b) to retrieve and display upper and lower value range limits associated with said feature, if said feature is identified as a "continuous" type, and to assign a selected segment in said range to said feature as determined by the user.

13. The system of Claim 10, 11 or 12 wherein said manually operative input means is operative to identify a feature as a type having a variable value and said processing means is programmed to measure that value as a function of length, area or density of that feature, said processing means being programmed: (a) to measure length between points displayed in a pictorial image and selected by user-operation of said manually operative input means, (b) to measure the area of a closed curve displayed in a pictorial image and selected by the user-operation of said manually operative input means, and (c) to measure density in response to user-operation of said manually operative input means to define an area on a pictorial display displayed by said display means in which the density of predetermined elements is to be calculated.

14. The system of any of Claims 10-13 wherein said manually operative input means includes means for generating overlay data representing predetermined types of visual overlays; and said processing means is further programmed to cause said display means to display a set of overlay images from said overlay data and to display a pictorial image of selected features and is responsive to user-operation of said manually operative input means to link an overlay image with a feature present in the displayed pictorial image and to store in said storage means overlay data representing the location in the pictorial image of the overlay image linked with the feature present in said pictorial image.

15. The system of any of Claims 10-14 wherein said manually operative input means comprises physically movable cursor positioning means for moving a cursor displayed on said display means and including at least one selector switch; wherein said display means displays information linked to features, values and image data stored in said storage means; and wherein said processing means is programmed to respond to the operation of said selector switch when the displayed cursor is located at a position whereat information linked to a stored feature, value or image data is displayed to retrieve and display that linked feature, value or visual image.

16. The system of any of Claims 10-15 wherein said image data input means comprises a video camera for producing video signals representing images picked up by the video camera; digitizing means for digitizing the video signals to produce said image data; and a microscope optically coupled to said video camera for presenting images to the video camera.

17. The system of Claim 16 wherein said processing means is in data communication with said digitizing means and is further programmed to cause said display means to display simultaneously a pictorial image derived from image data retrieved from said storage means and a pictorial image derived from image data produced by said digitizing means.

25 05 89

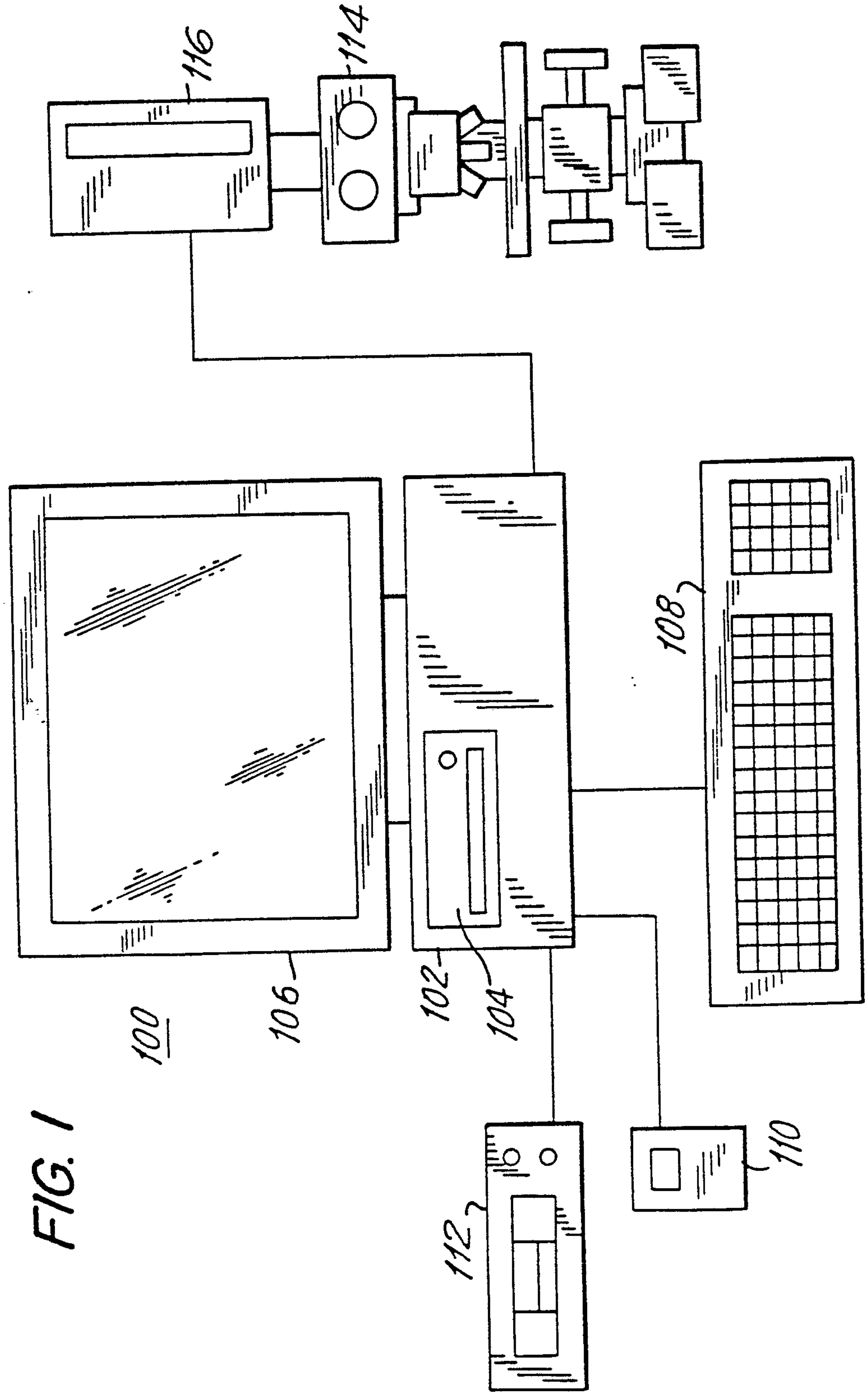


FIG. 1

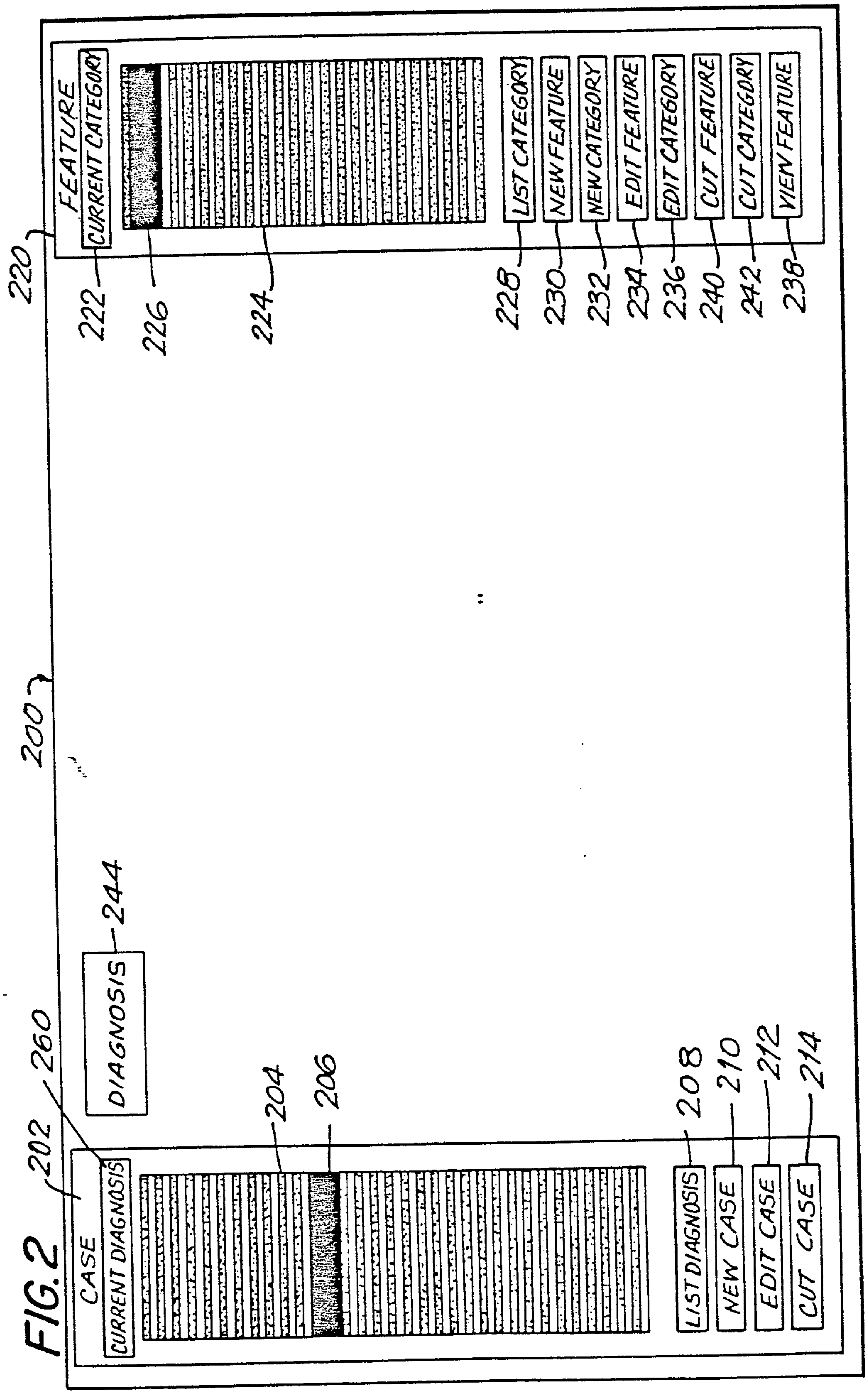
CASE
CURRENT DIAGNOSIS
DIAGNOSIS
LIST DIAGNOSIS
NEW CASE
EDIT CASE
CUT CASE
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
200
202
204
206
208
210
212
214
220
222
224
226
228
230
232
234
236
240
242
238
244
260

FIG.2

Nouvellement déposé

FIG. 2A-1

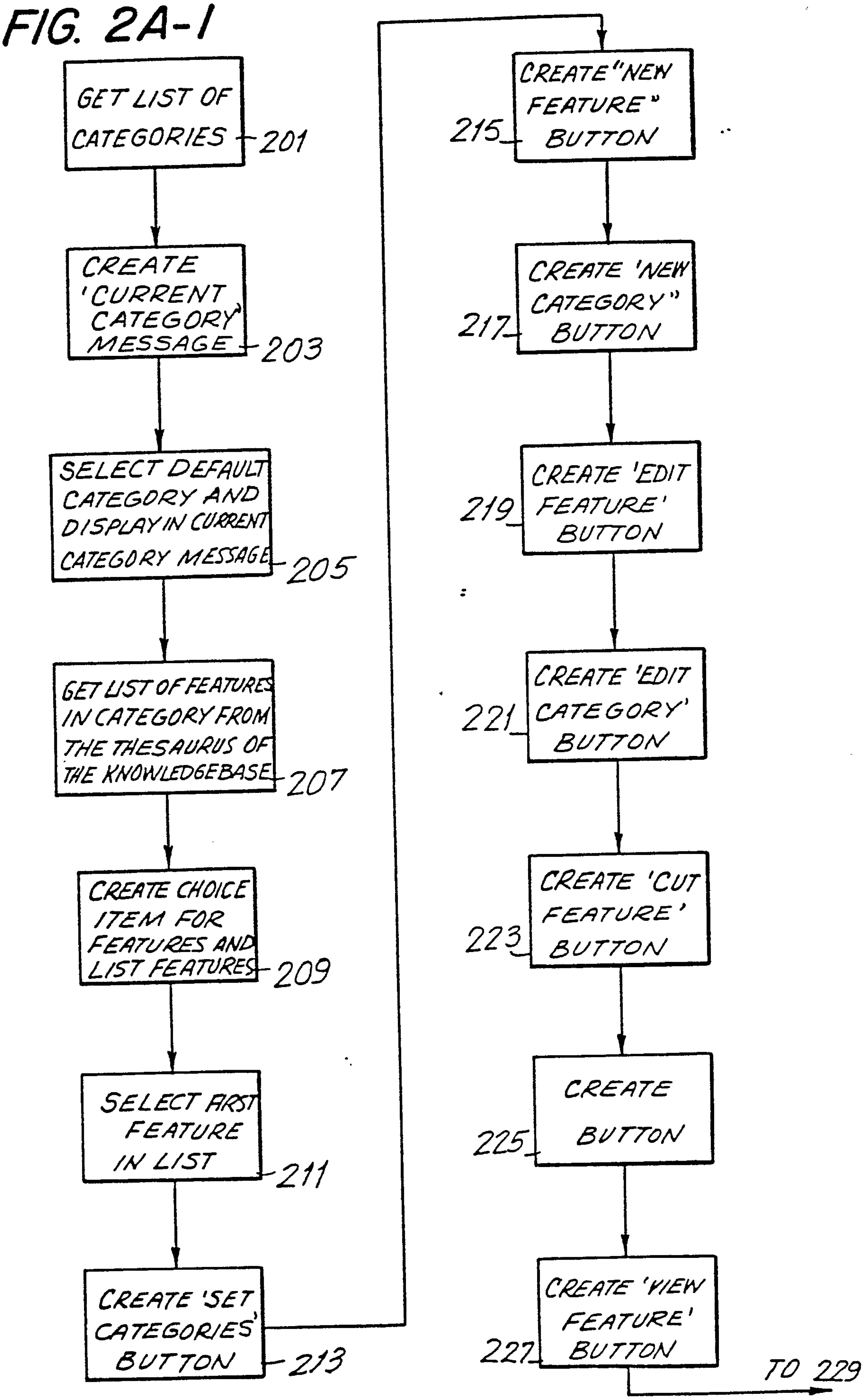

Neu eingereicht / Newly filed
Nouvellement déposé

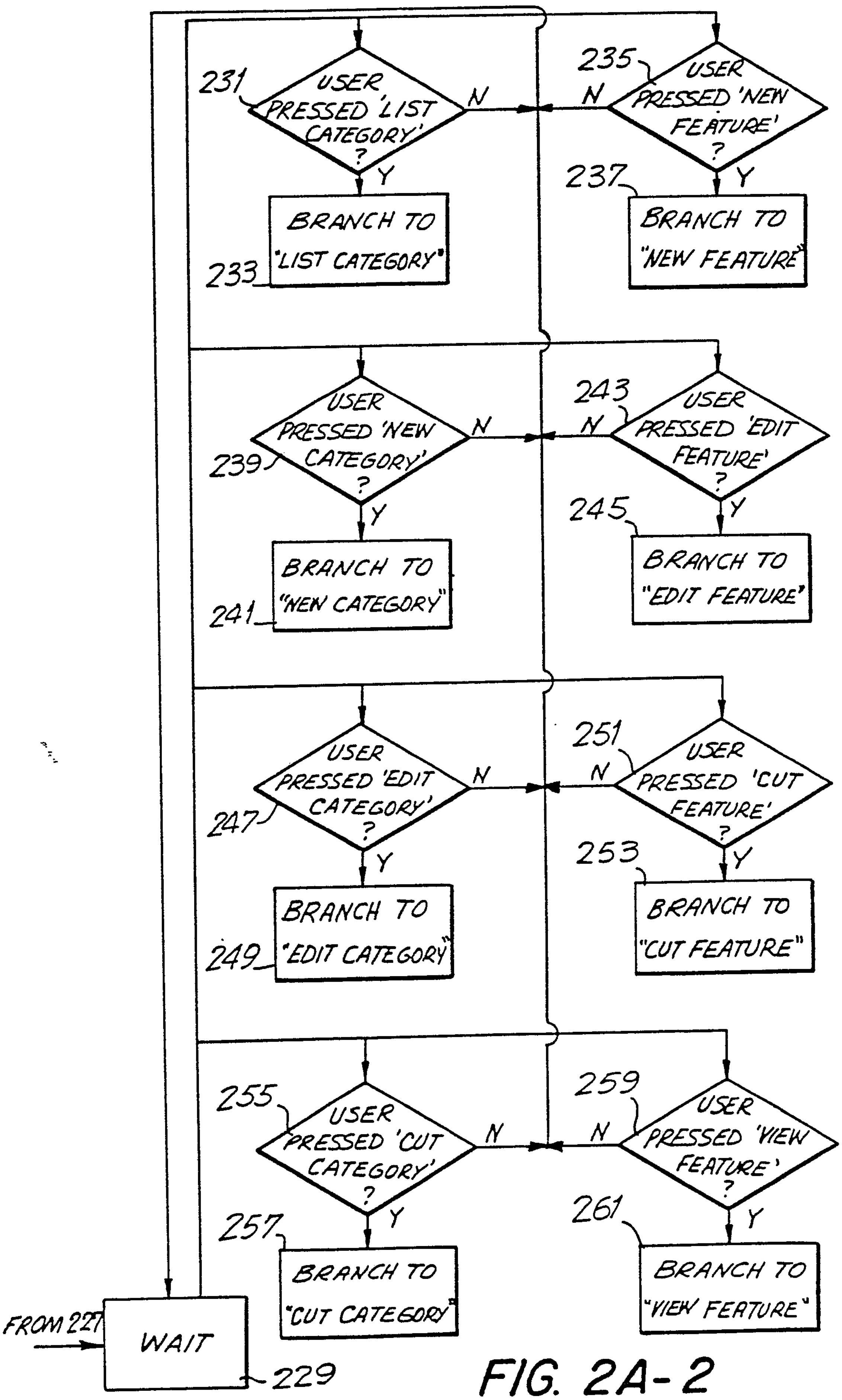


FIG. 2A-2

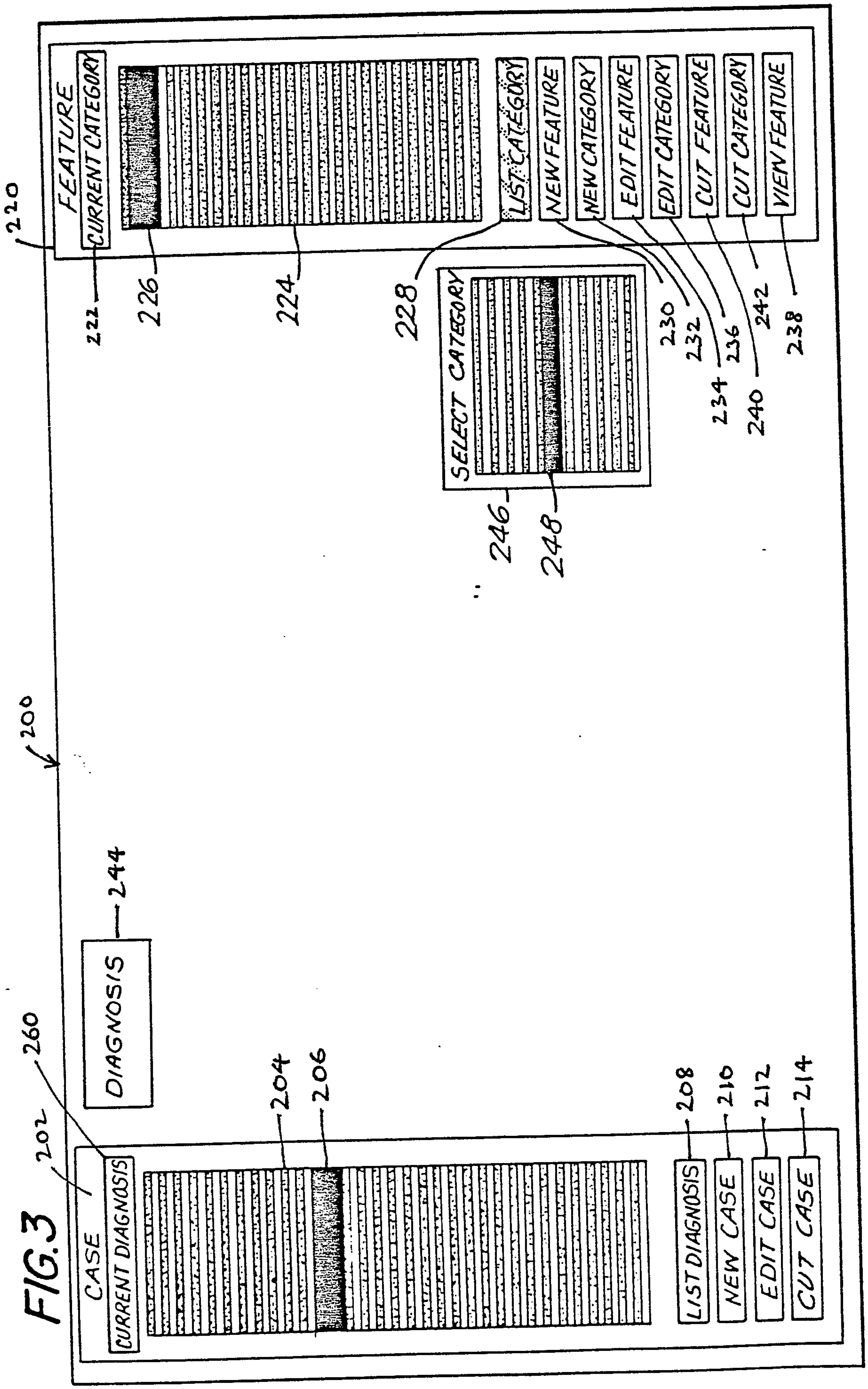
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
SELECT CATEGORY
DIAGNOSIS
CASE
CURRENT DIAGNOSIS
LIST DIAGNOSIS
NEW CASE
EDIT CASE
CUT CASE
200
202
204
206
208
210
212
214
220
221
224
226
228
230
232
234
236
238
240
242
244
246
248
260

FIG.3

List Category

Neu eingereicht / Newly filed
Nouvellement déposé

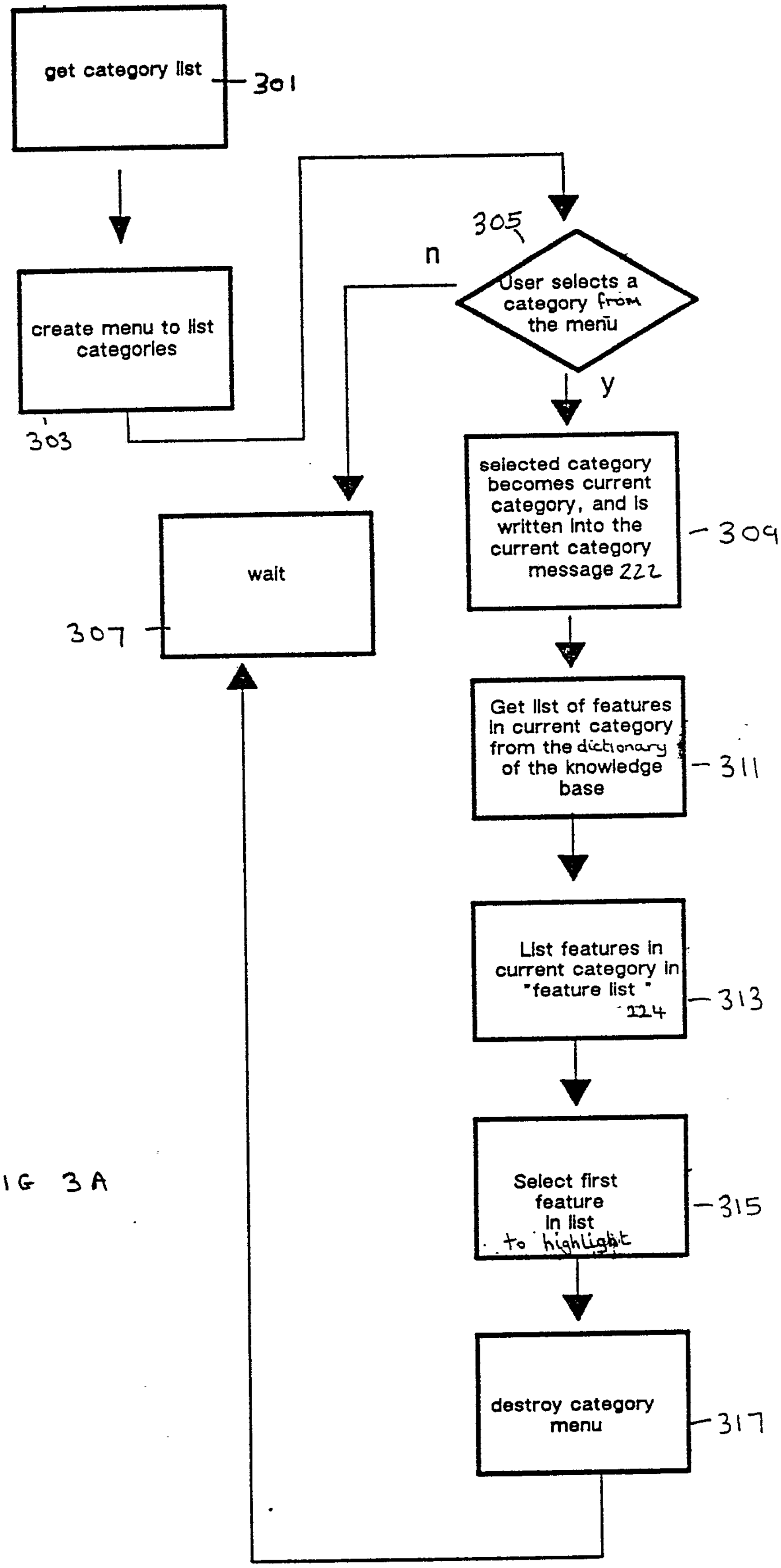


FIG 3A

Nouvellement déposé

FIG. 4

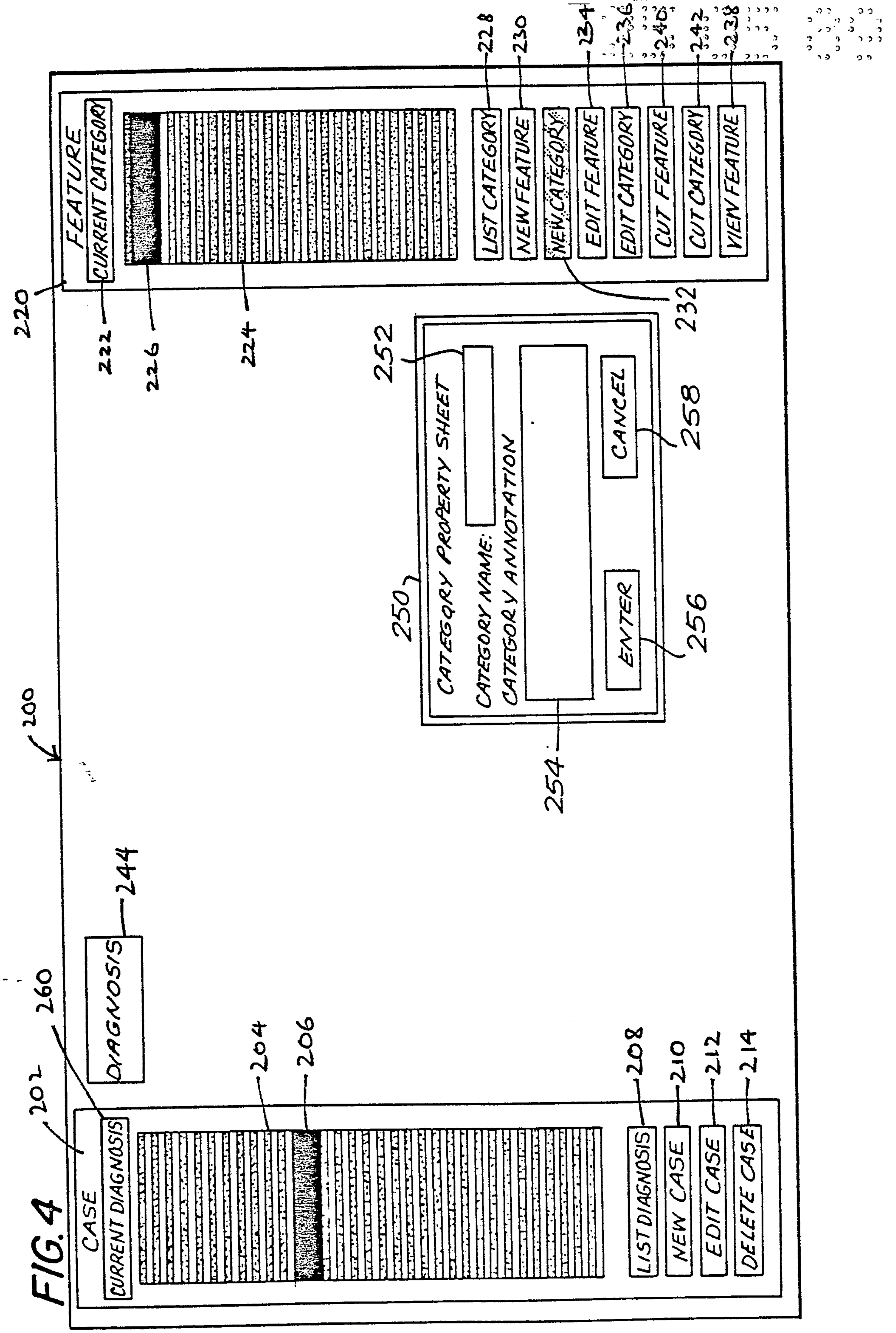

Nouvellement déposé

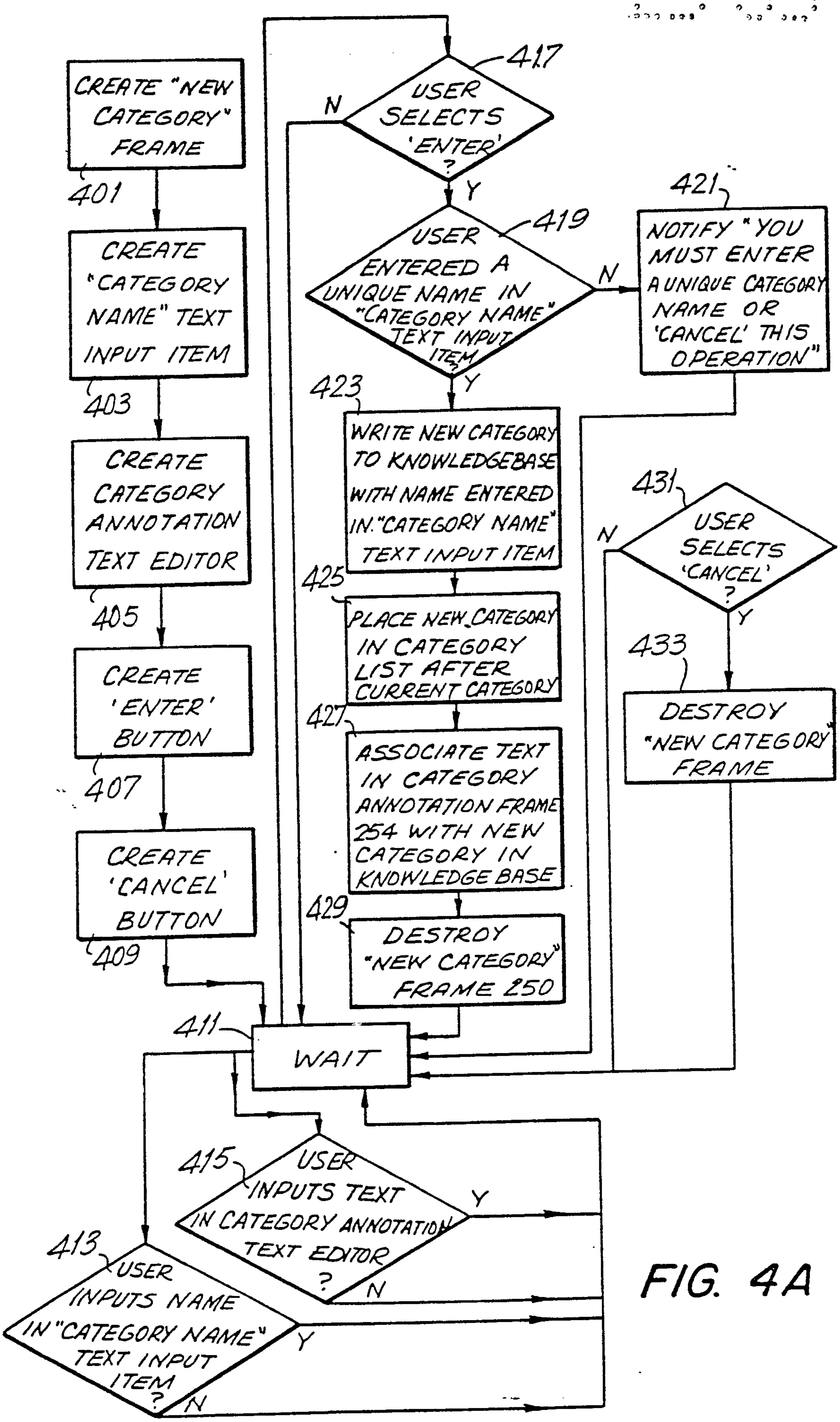


FIG. 4A

Neu eingereicht / Newly filed
Nouvellement déposé

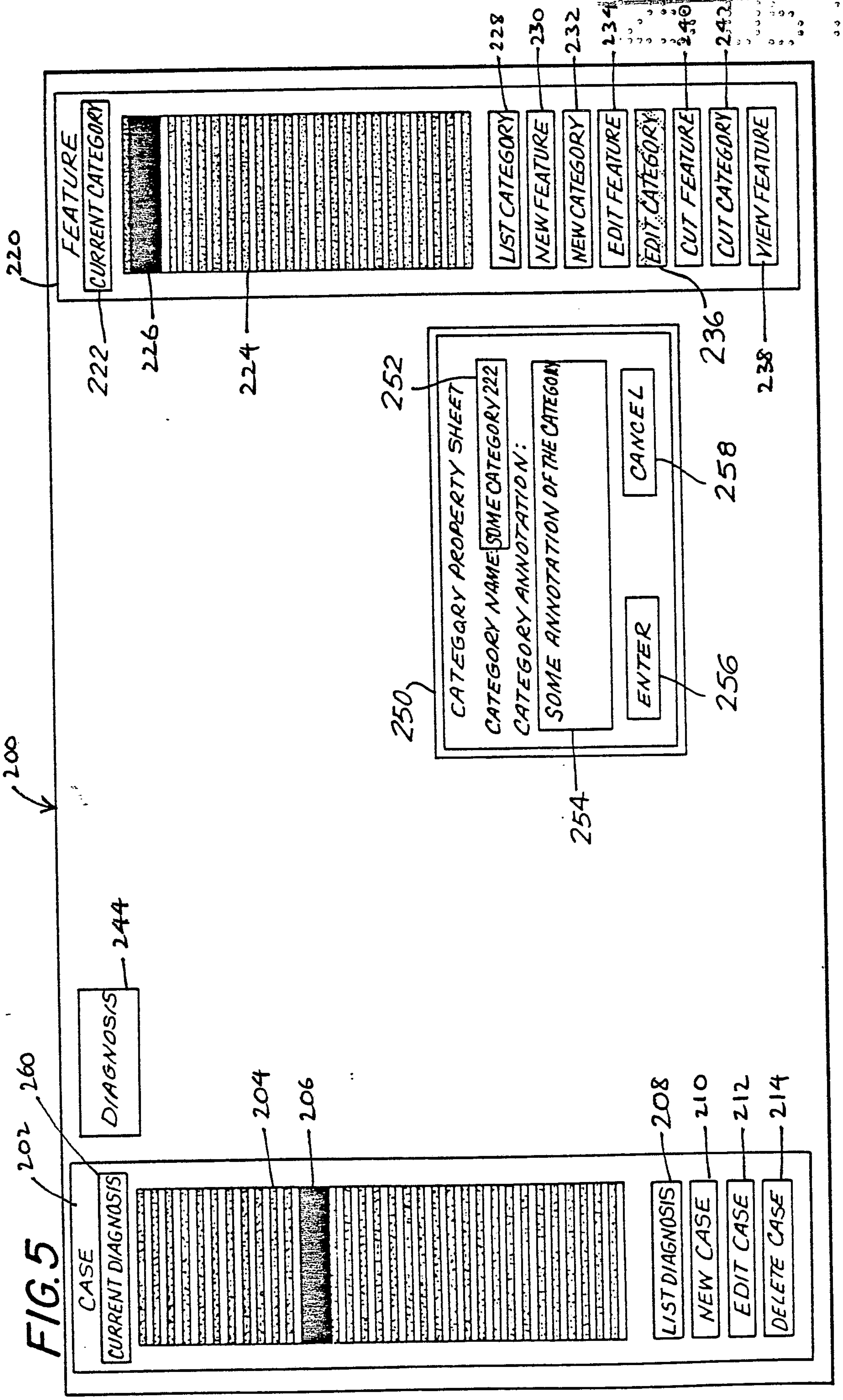


FIG.5

Neu eingereicht / Newly filed
Nouvellement déposé

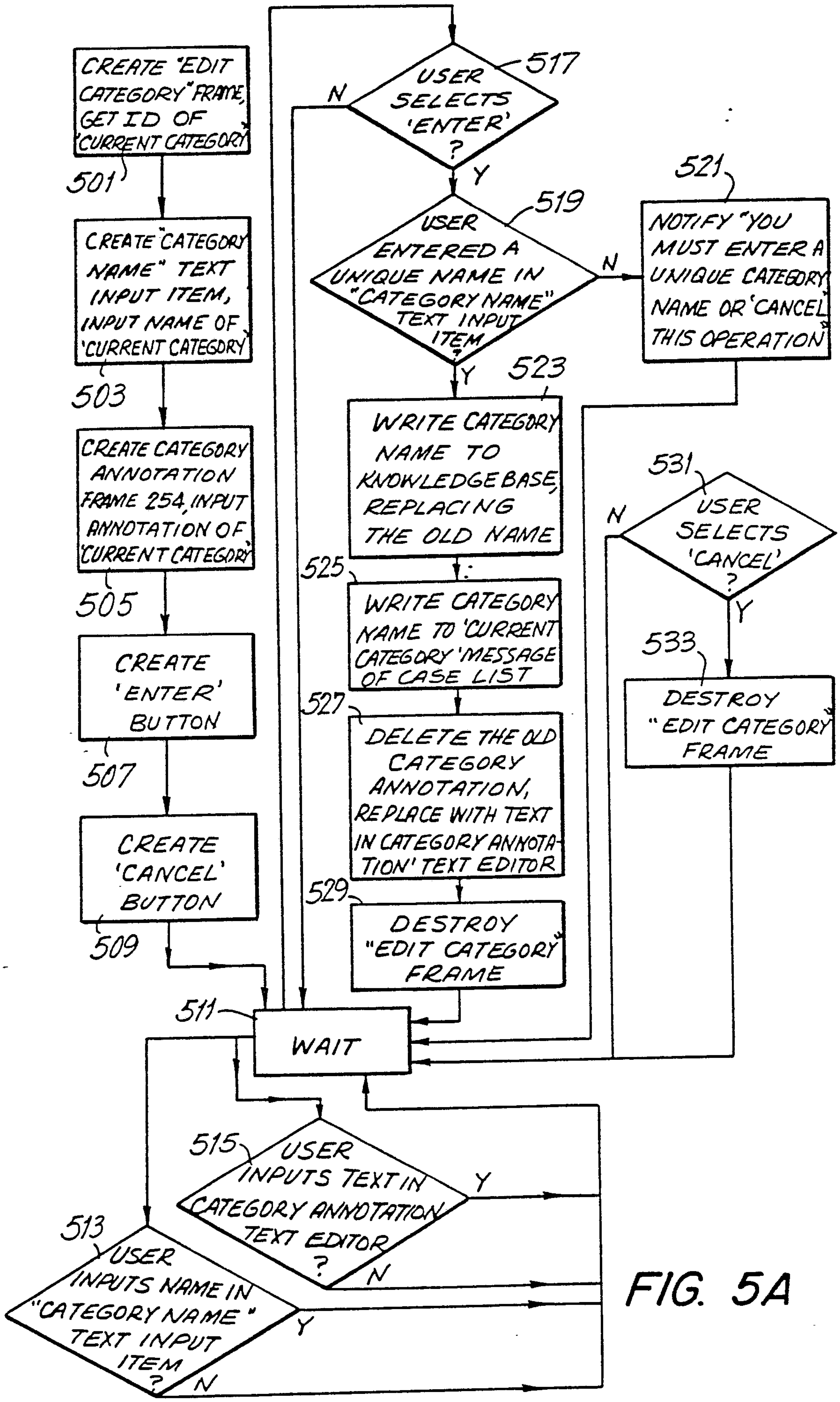


FIG. 5A

FIG.6

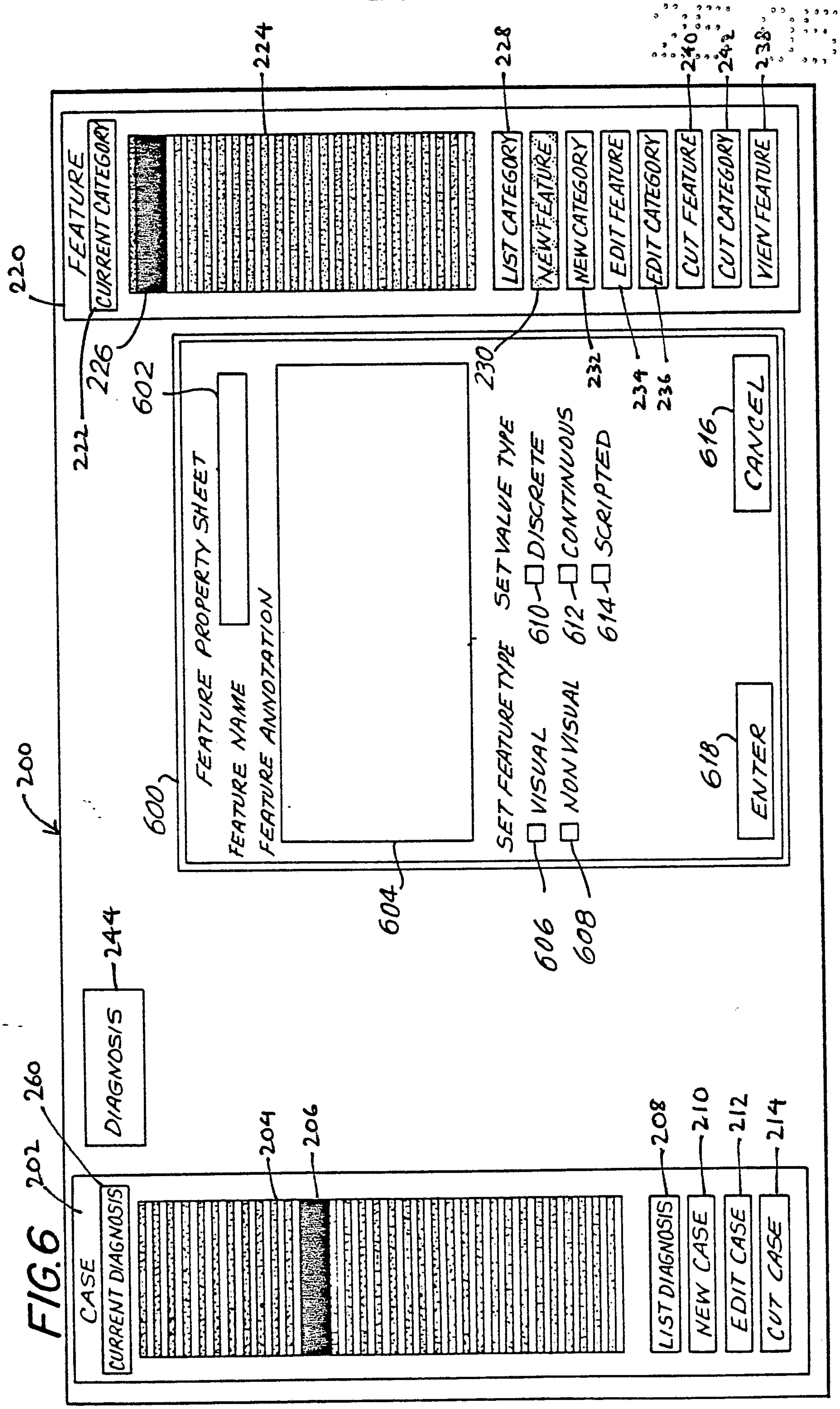
CASE
CURRENT DIAGNOSIS
DIAGNOSIS
LIST DIAGNOSIS
NEW CASE
EDIT CASE
CUT CASE
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
FEATURE PROPERTY SHEET
FEATURE NAME
FEATURE ANNOTATION
SET FEATURE TYPE
VISUAL
NON VISUAL
SET VALUE TYPE
DISCRETE
CONTINUOUS
SCRIPTED
ENTER
CANCEL
200
201
202
204
206
208
210
212
214
220
222
224
226
228
230
232
234
236
238
240
242
244
260
600
602
604
606
608
610
612
614
616
618

FIG. 6A-1

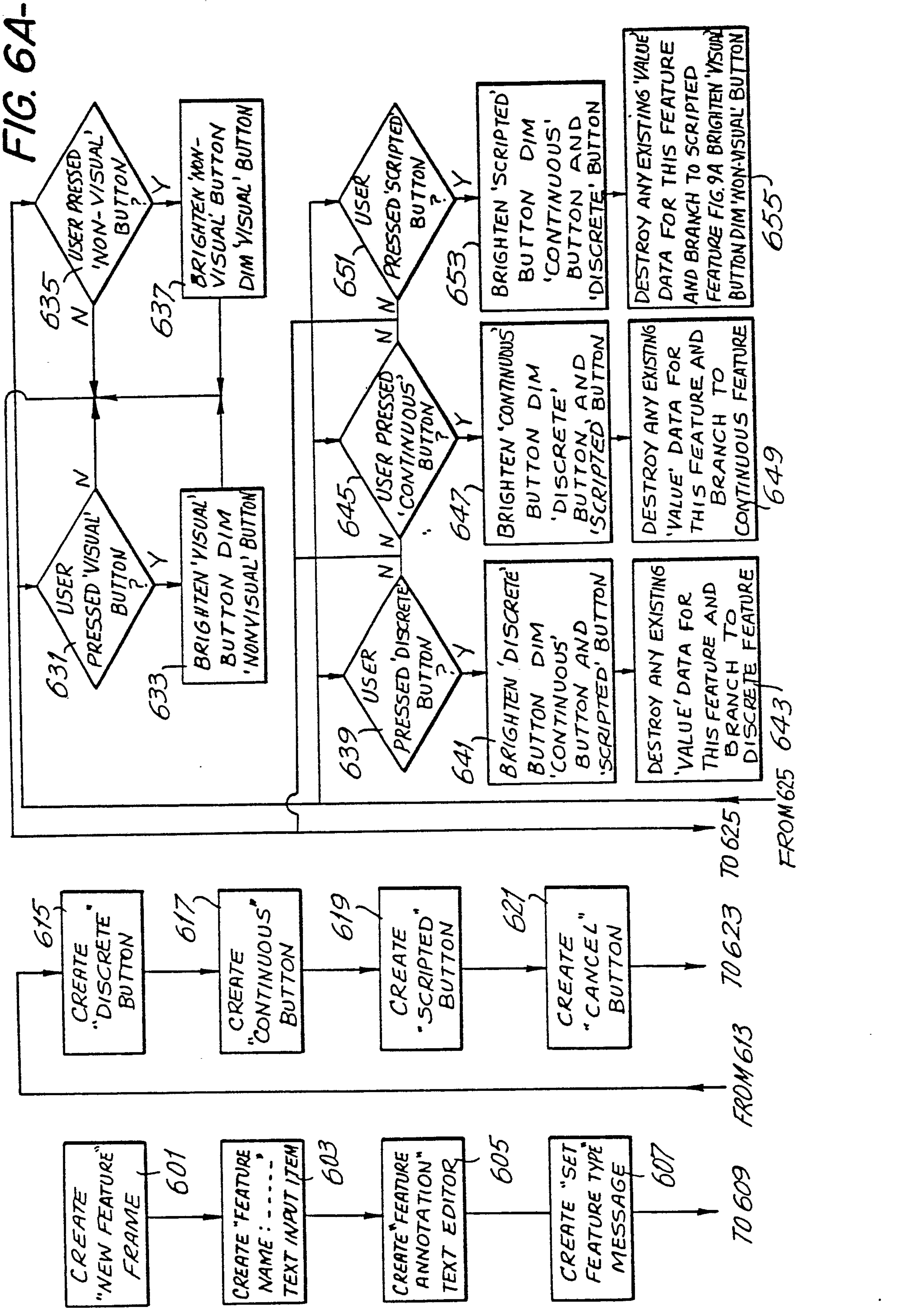
CREATE "NEW FEATURE" FRAME
601
CREATE "FEATURE NAME: ____" TEXT INPUT ITEM
603
CREATE "FEATURE ANNOTATION" TEXT EDITOR
605
CREATE "SET FEATURE TYPE" MESSAGE
607
TO 609
CREATE "DISCRETE" BUTTON
615
CREATE "CONTINUOUS" BUTTON
617
CREATE "SCRIPTED" BUTTON
619
CREATE "CANCEL" BUTTON
621
FROM 613
TO 623
USER PRESSED 'VISUAL' BUTTON ?
631
Y
N
BRIGHTEN 'VISUAL' BUTTON DIM 'NONVISUAL' BUTTON
633
USER PRESSED 'NON-VISUAL' BUTTON ?
635
BRIGHTEN 'NON-VISUAL' BUTTON DIM 'VISUAL' BUTTON
637
USER PRESSED 'DISCRETE' BUTTON ?
639
BRIGHTEN 'DISCRETE' BUTTON DIM 'CONTINUOUS' BUTTON AND 'SCRIPTED' BUTTON
641
DESTROY ANY EXISTING 'VALUE' DATA FOR THIS FEATURE AND BRANCH TO DISCRETE FEATURE
643
USER PRESSED 'CONTINUOUS' BUTTON ?
645
BRIGHTEN 'CONTINUOUS' BUTTON DIM 'DISCRETE' BUTTON, AND 'SCRIPTED' BUTTON
647
DESTROY ANY EXISTING 'VALUE' DATA FOR THIS FEATURE AND BRANCH TO CONTINUOUS FEATURE
649
USER PRESSED 'SCRIPTED' BUTTON ?
651
BRIGHTEN 'SCRIPTED' BUTTON DIM 'CONTINUOUS' BUTTON AND 'DISCRETE' BUTTON
653
DESTROY ANY EXISTING 'VALUE' DATA FOR THIS FEATURE AND BRANCH TO SCRIPTED FEATURE FIG. 9A BRIGHTEN 'VISUAL' BUTTON DIM 'NON-VISUAL' BUTTON
655
TO 625
FROM 625

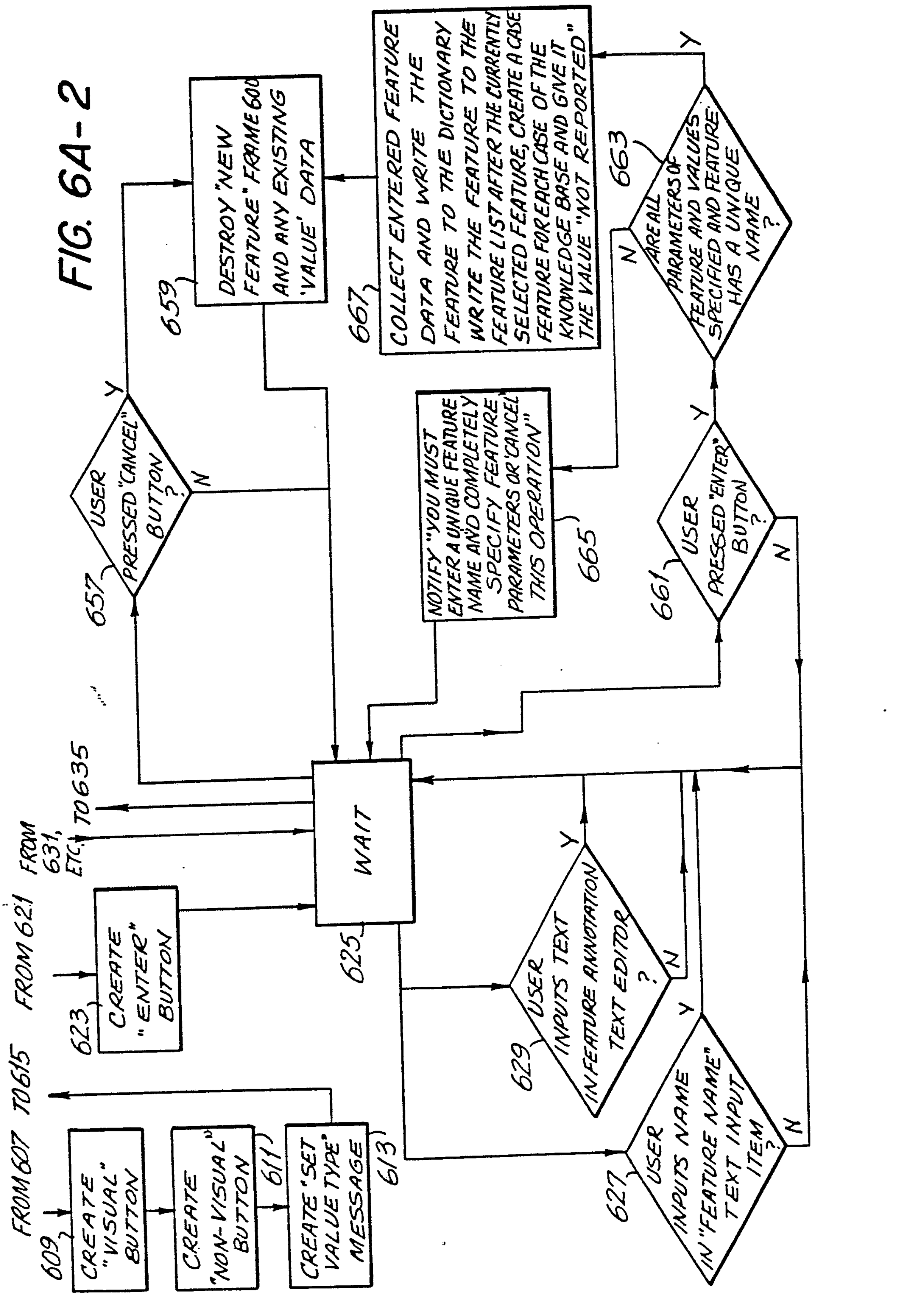
FIG. 6A-2
FROM 607
TO 615
609 CREATE "VISUAL" BUTTON
CREATE "NON-VISUAL" BUTTON
611
CREATE "SET VALUE TYPE" MESSAGE
613
FROM 621
623 CREATE "ENTER" BUTTON
FROM 631, ETC.
TO 635
625 WAIT
657 USER PRESSED "CANCEL" BUTTON ?
Y
N
659 DESTROY "NEW FEATURE" FRAME 600 AND ANY EXISTING 'VALUE' DATA
667 COLLECT ENTERED FEATURE DATA AND WRITE THE FEATURE TO THE DICTIONARY WRITE THE FEATURE TO THE FEATURE LIST AFTER THE CURRENTLY SELECTED FEATURE, CREATE A CASE FEATURE FOR EACH CASE OF THE KNOWLEDGE BASE AND GIVE IT THE VALUE "NOT REPORTED"
NOTIFY "YOU MUST ENTER A UNIQUE FEATURE NAME AND COMPLETELY SPECIFY FEATURE PARAMETERS OR 'CANCEL' THIS OPERATION"
665
661 USER PRESSED "ENTER" BUTTON ?
663 ARE ALL PARAMETERS OF FEATURE AND VALUES SPECIFIED AND FEATURE HAS A UNIQUE NAME ?
629 USER INPUTS TEXT IN FEATURE ANNOTATION TEXT EDITOR ?
627 USER INPUTS NAME IN "FEATURE NAME" TEXT INPUT ITEM ?

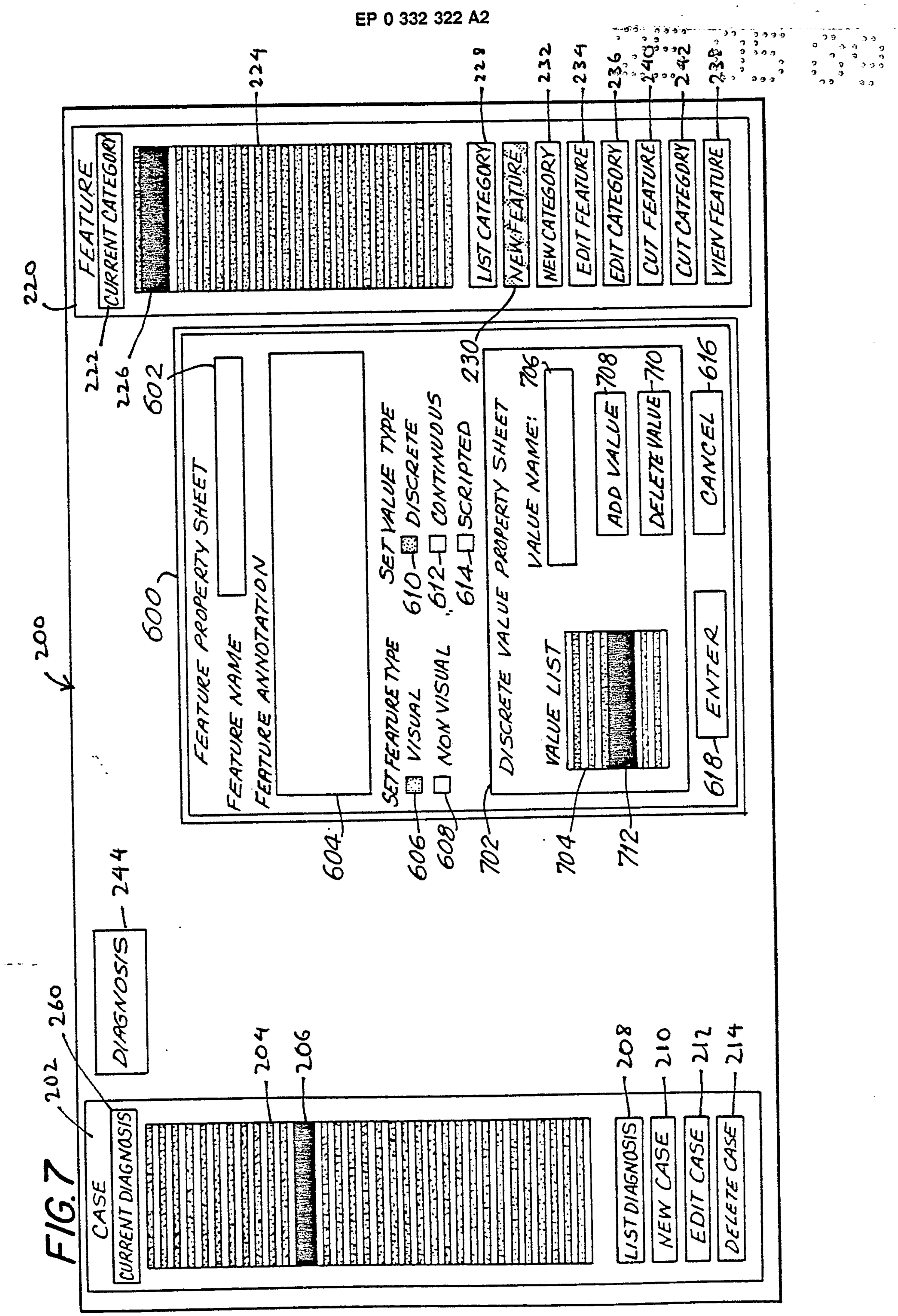
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
FEATURE PROPERTY SHEET
FEATURE NAME
FEATURE ANNOTATION
SET FEATURE TYPE
VISUAL
NON VISUAL
SET VALUE TYPE
DISCRETE
CONTINUOUS
SCRIPTED
DISCRETE VALUE PROPERTY SHEET
VALUE NAME:
VALUE LIST
ADD VALUE
DELETE VALUE
CANCEL
ENTER
DIAGNOSIS
CASE
CURRENT DIAGNOSIS
LIST DIAGNOSIS
NEW CASE
EDIT CASE
DELETE CASE
200
202
204
206
208
210
212
214
220
222
224
226
228
230
232
234
236
238
240
242
244
260
600
602
604
606
608
610
612
614
616
618
702
704
706
708
710
712

FIG. 7

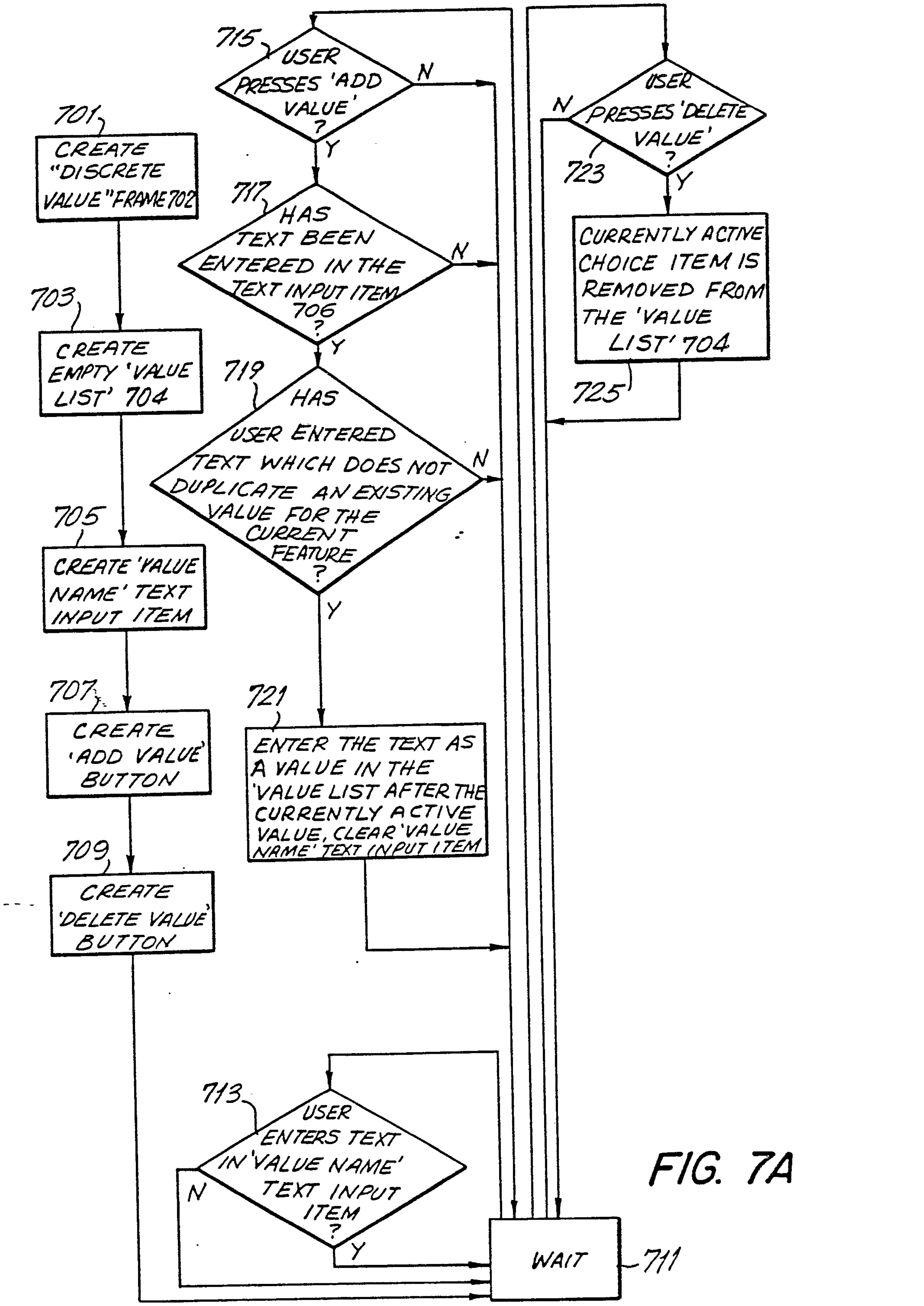
701
CREATE "DISCRETE VALUE" FRAME 702
703
CREATE EMPTY 'VALUE LIST' 704
705
CREATE 'VALUE NAME' TEXT INPUT ITEM
707
CREATE 'ADD VALUE' BUTTON
709
CREATE 'DELETE VALUE' BUTTON
715
USER PRESSES 'ADD VALUE' ?
N
Y
717
HAS TEXT BEEN ENTERED IN THE TEXT INPUT ITEM 706 ?
N
Y
719
HAS USER ENTERED TEXT WHICH DOES NOT DUPLICATE AN EXISTING VALUE FOR THE CURRENT FEATURE ?
N
Y
721
ENTER THE TEXT AS A VALUE IN THE 'VALUE LIST AFTER THE CURRENTLY ACTIVE VALUE, CLEAR 'VALUE NAME' TEXT INPUT ITEM
USER PRESSES 'DELETE VALUE' ?
N
Y
723
CURRENTLY ACTIVE CHOICE ITEM IS REMOVED FROM THE 'VALUE LIST' 704
725
713
USER ENTERS TEXT IN 'VALUE NAME' TEXT INPUT ITEM ?
N
Y
WAIT
711

FIG. 7A

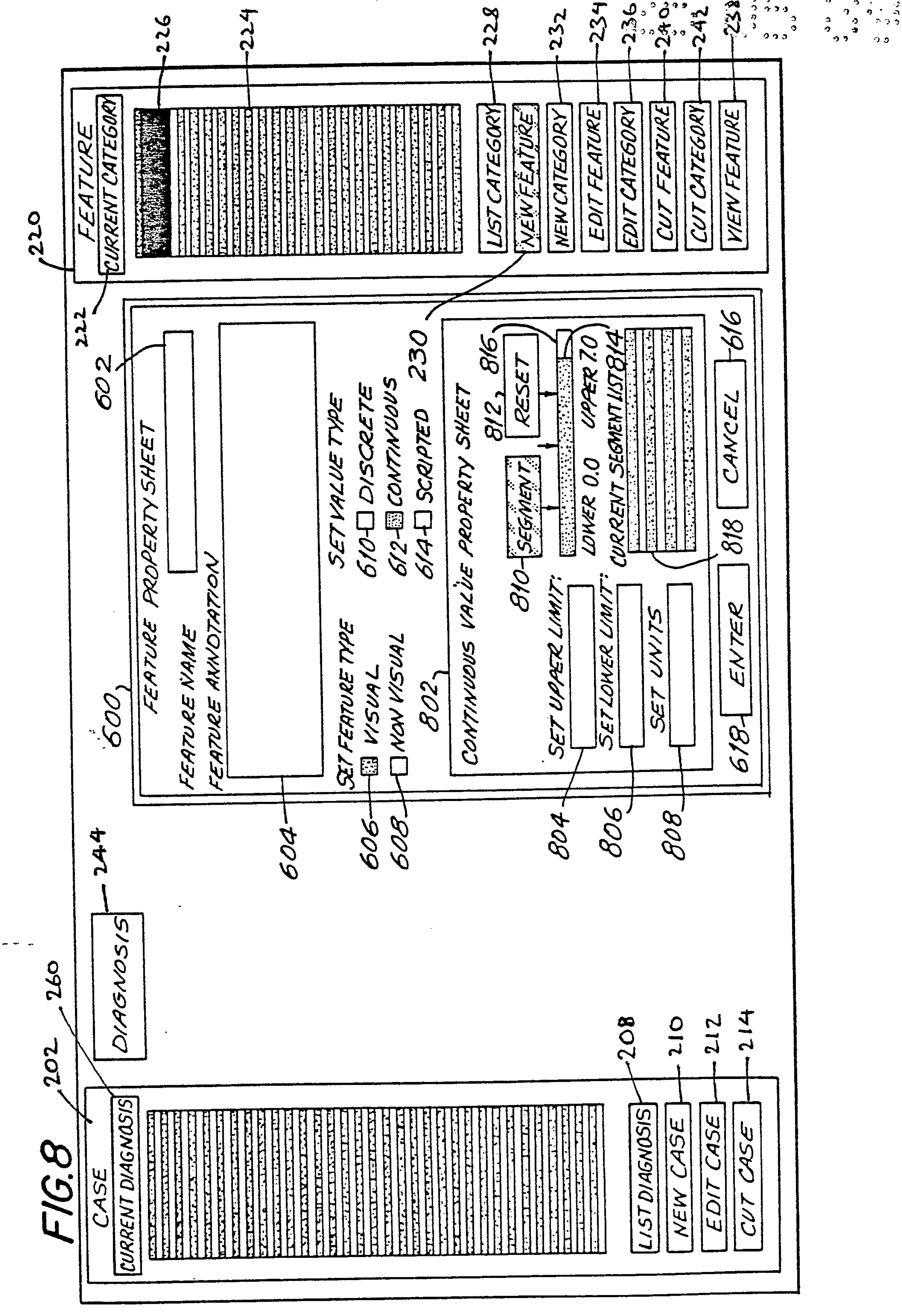
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
FEATURE PROPERTY SHEET
FEATURE NAME
FEATURE ANNOTATION
SET FEATURE TYPE
VISUAL
NON VISUAL
SET VALUE TYPE
DISCRETE
CONTINUOUS
SCRIPTED
CONTINUOUS VALUE PROPERTY SHEET
SET UPPER LIMIT:
SET LOWER LIMIT:
SET UNITS
SEGMENT
RESET
LOWER 0.0
UPPER 7.0
CURRENT SEGMENT LIST
ENTER
CANCEL
DIAGNOSIS
CASE
CURRENT DIAGNOSIS
LIST DIAGNOSIS
NEW CASE
EDIT CASE
CUT CASE
220
222
224
226
228
230
232
234
236
238
240
242
244
202
208
210
212
214
260
600
602
604
606
608
610
612
614
616
618
802
804
806
808
810
812
814
816
818

FIG.8

FIG.8A-1

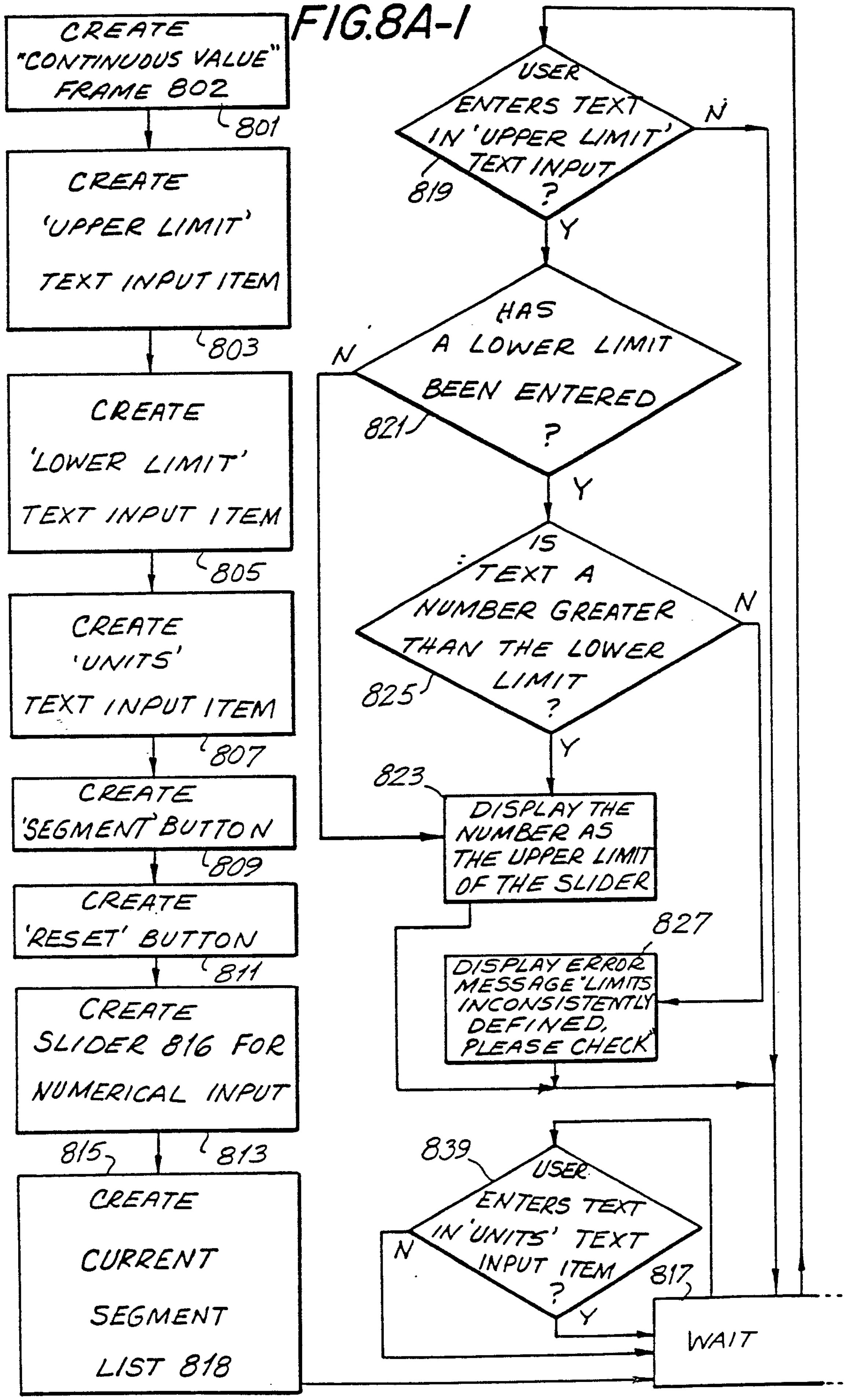
CREATE "CONTINUOUS VALUE" FRAME 802
801
CREATE 'UPPER LIMIT' TEXT INPUT ITEM
803
CREATE 'LOWER LIMIT' TEXT INPUT ITEM
805
CREATE 'UNITS' TEXT INPUT ITEM
807
CREATE 'SEGMENT' BUTTON
809
CREATE 'RESET' BUTTON
811
CREATE SLIDER 816 FOR NUMERICAL INPUT
815
813
CREATE CURRENT SEGMENT LIST 818
USER ENTERS TEXT IN 'UPPER LIMIT' TEXT INPUT ?
819
N
Y
HAS A LOWER LIMIT BEEN ENTERED ?
821
N
Y
IS TEXT A NUMBER GREATER THAN THE LOWER LIMIT ?
825
N
Y
823
DISPLAY THE NUMBER AS THE UPPER LIMIT OF THE SLIDER
827
DISPLAY ERROR MESSAGE 'LIMITS INCONSISTENTLY DEFINED, PLEASE CHECK'
839
USER ENTERS TEXT IN 'UNITS' TEXT INPUT ITEM ?
N
Y
817
WAIT

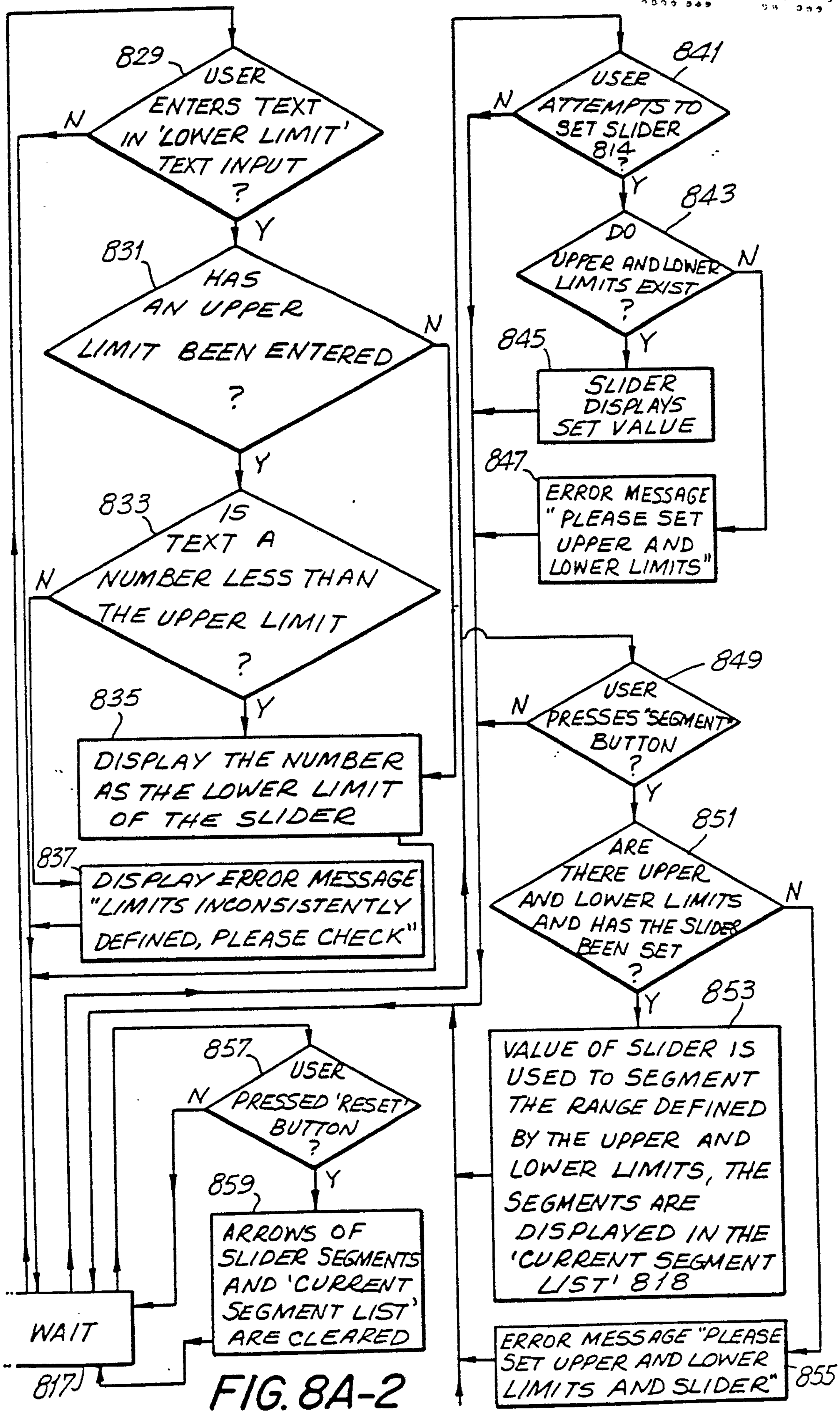
829
USER ENTERS TEXT IN 'LOWER LIMIT' TEXT INPUT ?
N
Y
831
HAS AN UPPER LIMIT BEEN ENTERED ?
N
Y
833
IS TEXT A NUMBER LESS THAN THE UPPER LIMIT ?
N
Y
835
DISPLAY THE NUMBER AS THE LOWER LIMIT OF THE SLIDER
837
DISPLAY ERROR MESSAGE "LIMITS INCONSISTENTLY DEFINED, PLEASE CHECK"
841
USER ATTEMPTS TO SET SLIDER 814 ?
N
Y
843
DO UPPER AND LOWER LIMITS EXIST ?
N
Y
845
SLIDER DISPLAYS SET VALUE
847
ERROR MESSAGE "PLEASE SET UPPER AND LOWER LIMITS"
849
USER PRESSES 'SEGMENT' BUTTON ?
N
Y
851
ARE THERE UPPER AND LOWER LIMITS AND HAS THE SLIDER BEEN SET ?
N
Y
853
VALUE OF SLIDER IS USED TO SEGMENT THE RANGE DEFINED BY THE UPPER AND LOWER LIMITS, THE SEGMENTS ARE DISPLAYED IN THE 'CURRENT SEGMENT LIST' 818
855
ERROR MESSAGE "PLEASE SET UPPER AND LOWER LIMITS AND SLIDER"
857
USER PRESSED 'RESET' BUTTON ?
N
Y
859
ARROWS OF SLIDER SEGMENTS AND 'CURRENT SEGMENT LIST' ARE CLEARED
WAIT
817

FIG. 8A-2

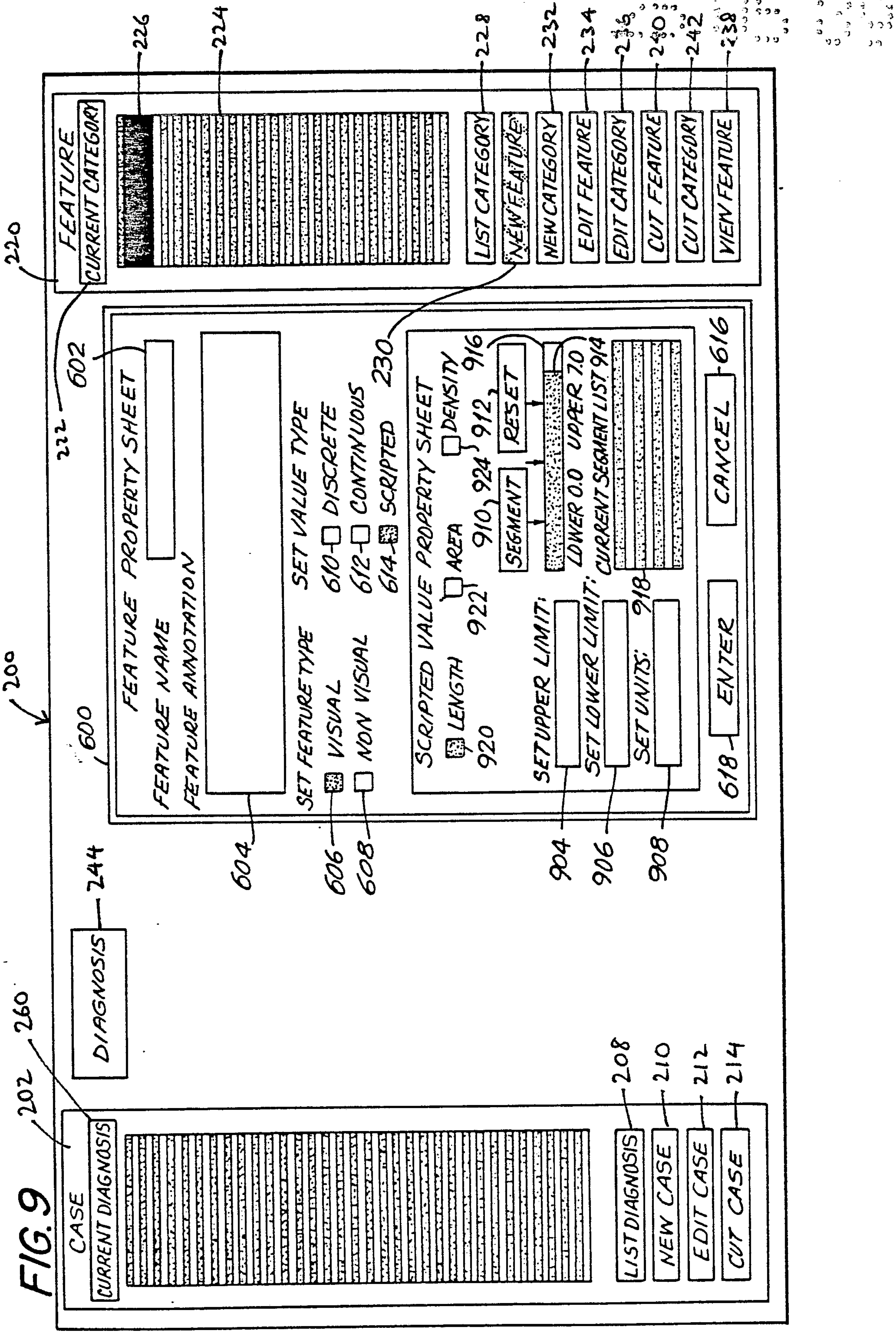
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
FEATURE PROPERTY SHEET
FEATURE NAME
FEATURE ANNOTATION
SET FEATURE TYPE
VISUAL
NON VISUAL
SET VALUE TYPE
DISCRETE
CONTINUOUS
SCRIPTED
SCRIPTED VALUE PROPERTY SHEET
LENGTH
AREA
DENSITY
SEGMENT
RESET
LOWER 0.0
UPPER 7.0
CURRENT SEGMENT LIST
SET UPPER LIMIT:
SET LOWER LIMIT:
SET UNITS:
ENTER
CANCEL
DIAGNOSIS
CASE
CURRENT DIAGNOSIS
LIST DIAGNOSIS
NEW CASE
EDIT CASE
CUT CASE
200
202
208
210
212
214
220
222
224
226
228
230
232
234
236
238
240
242
244
260
600
602
604
606
608
610
612
614
616
618
904
906
908
910
912
914
916
918
920
922
924

FIG.9

FIG.9A-1

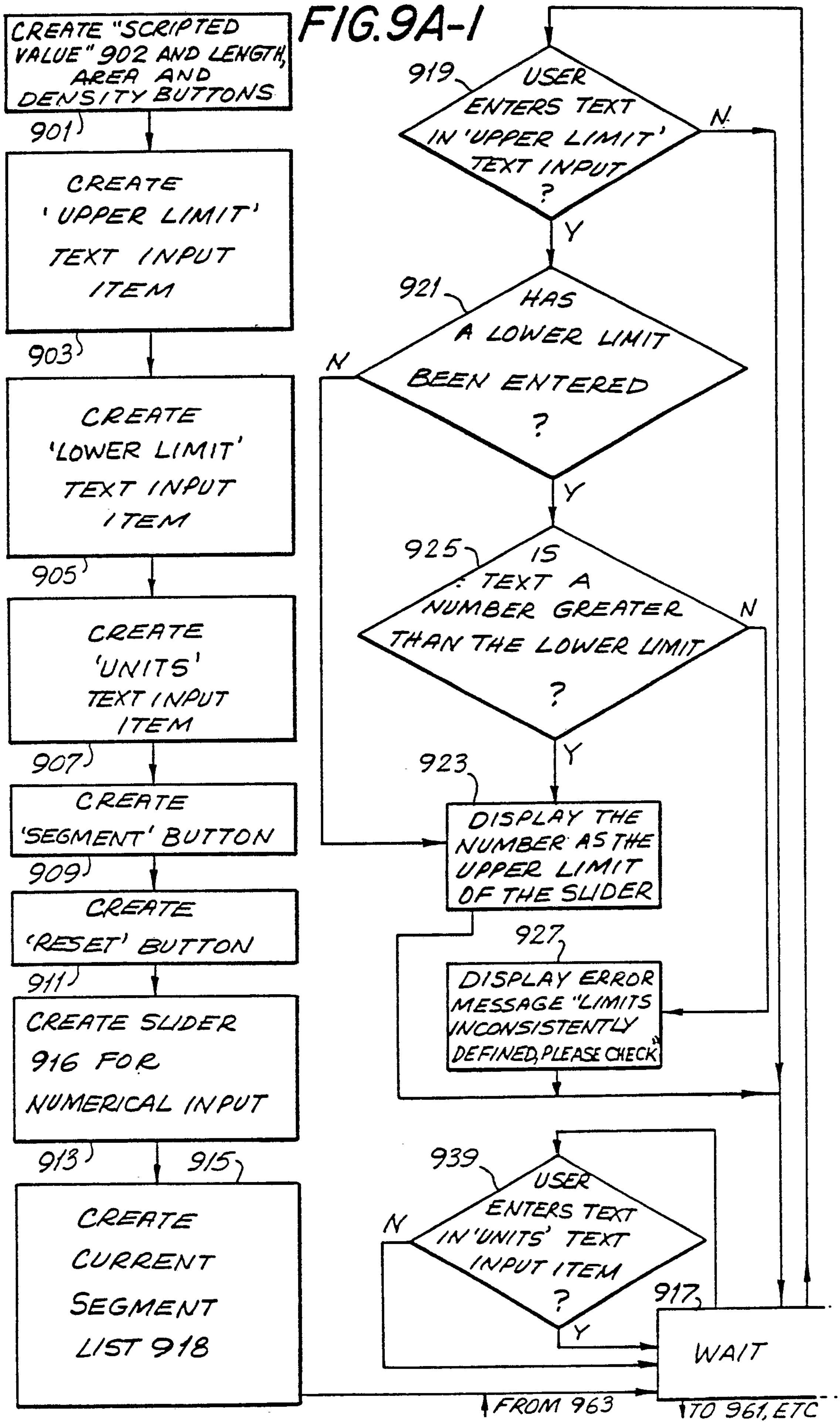
CREATE "SCRIPTED VALUE" 902 AND LENGTH, AREA AND DENSITY BUTTONS
901
CREATE 'UPPER LIMIT' TEXT INPUT ITEM
903
CREATE 'LOWER LIMIT' TEXT INPUT ITEM
905
CREATE 'UNITS' TEXT INPUT ITEM
907
CREATE 'SEGMENT' BUTTON
909
CREATE 'RESET' BUTTON
911
CREATE SLIDER 916 FOR NUMERICAL INPUT
913
915
CREATE CURRENT SEGMENT LIST 918
919
USER ENTERS TEXT IN 'UPPER LIMIT' TEXT INPUT ?
N
Y
921
HAS A LOWER LIMIT BEEN ENTERED ?
N
Y
925
IS TEXT A NUMBER GREATER THAN THE LOWER LIMIT ?
N
Y
923
DISPLAY THE NUMBER AS THE UPPER LIMIT OF THE SLIDER
927
DISPLAY ERROR MESSAGE "LIMITS INCONSISTENTLY DEFINED, PLEASE CHECK"
939
USER ENTERS TEXT IN 'UNITS' TEXT INPUT ITEM ?
N
Y
917
WAIT
FROM 963
TO 961, ETC

Nouvellement déposé

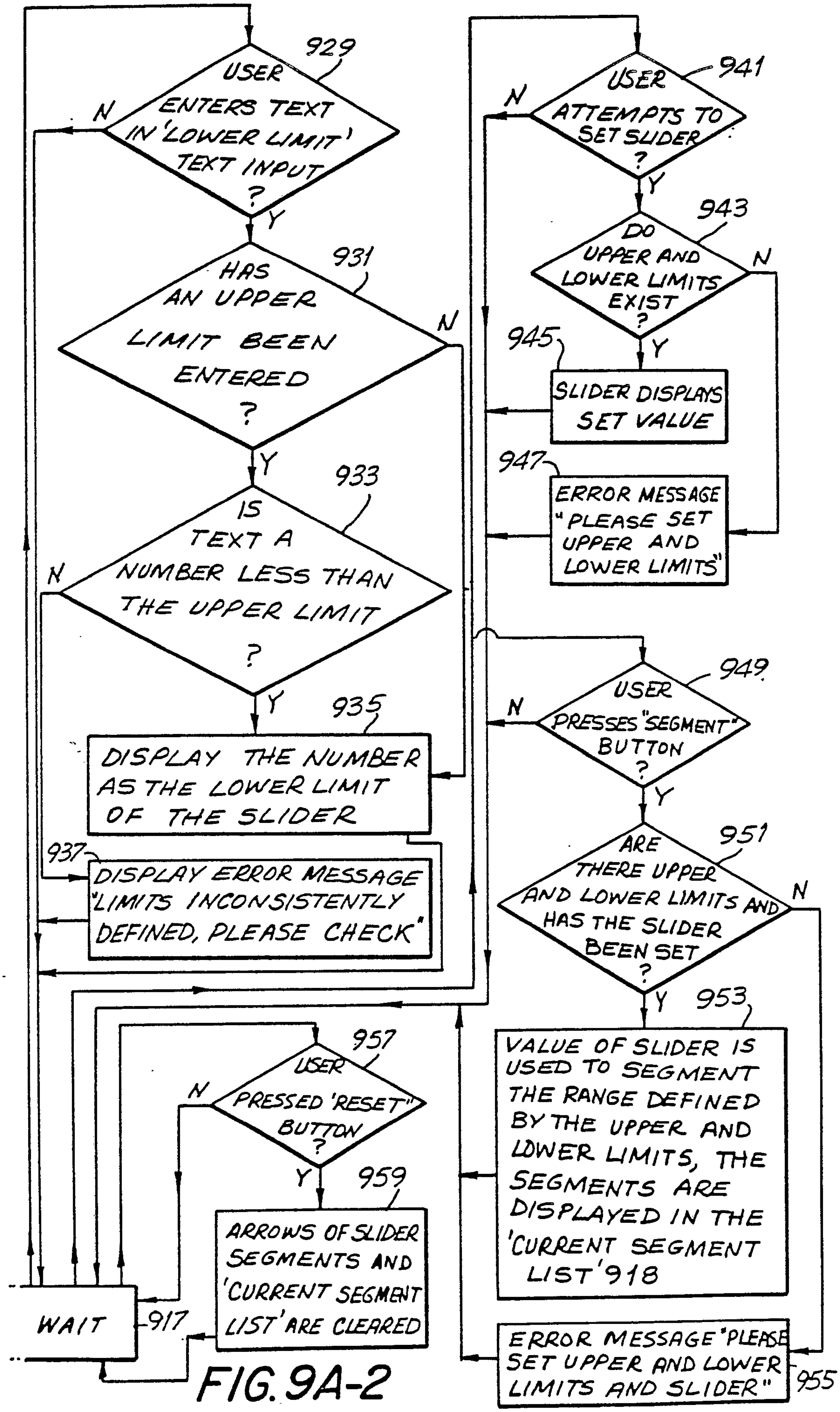


FIG. 9A-2

Nouvellement déposé

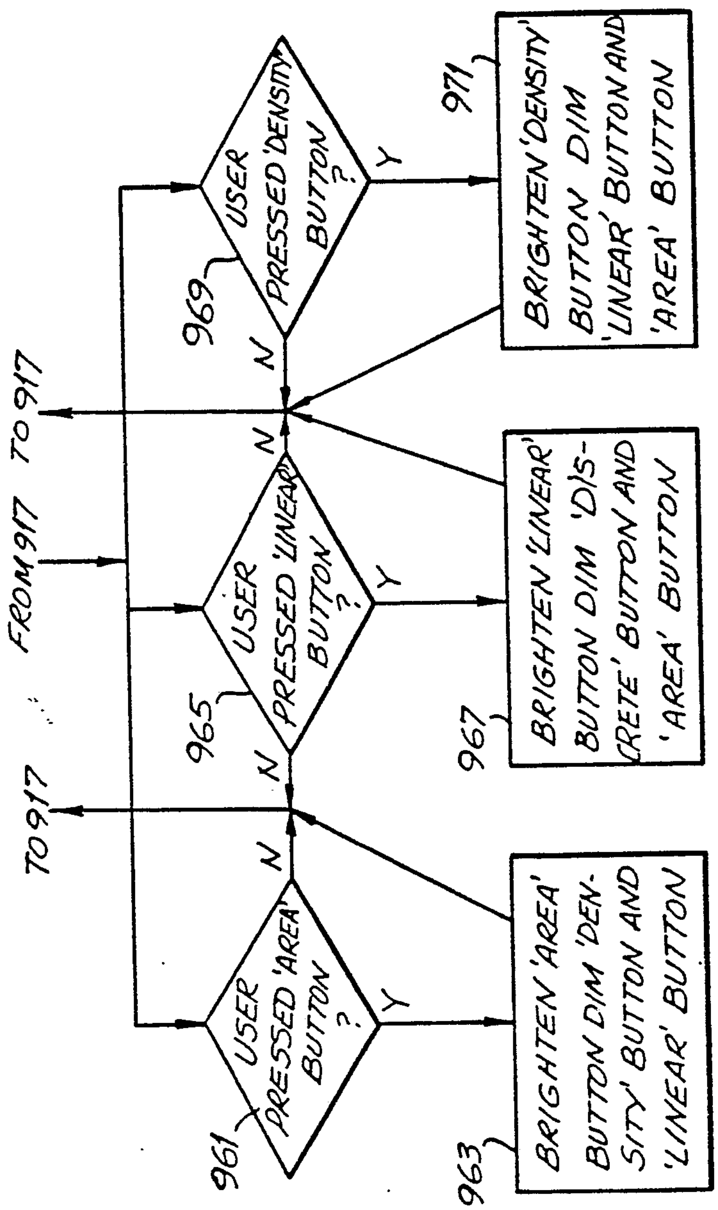


FIG.9A-3

Nouvellement déposé

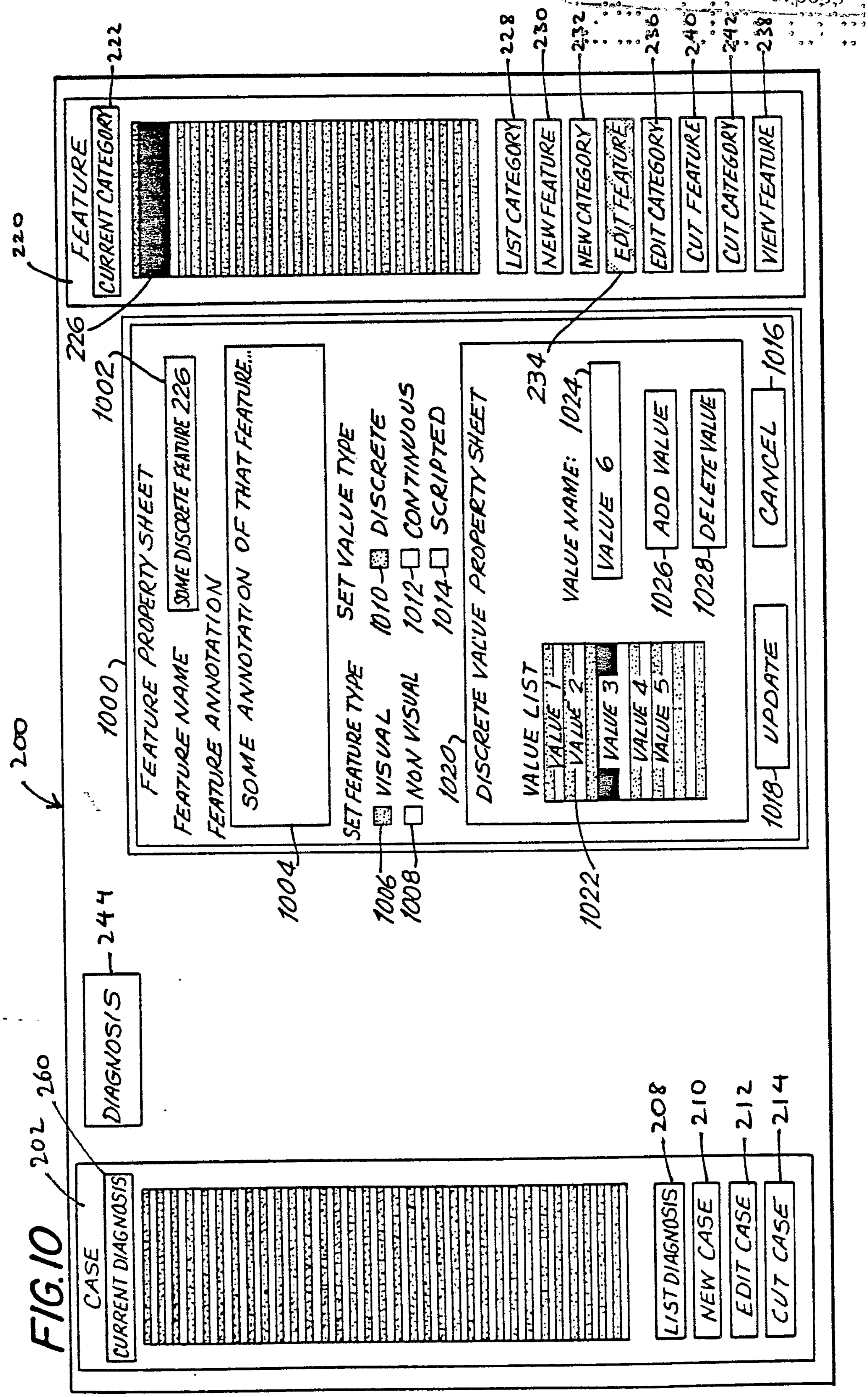


FIG.10

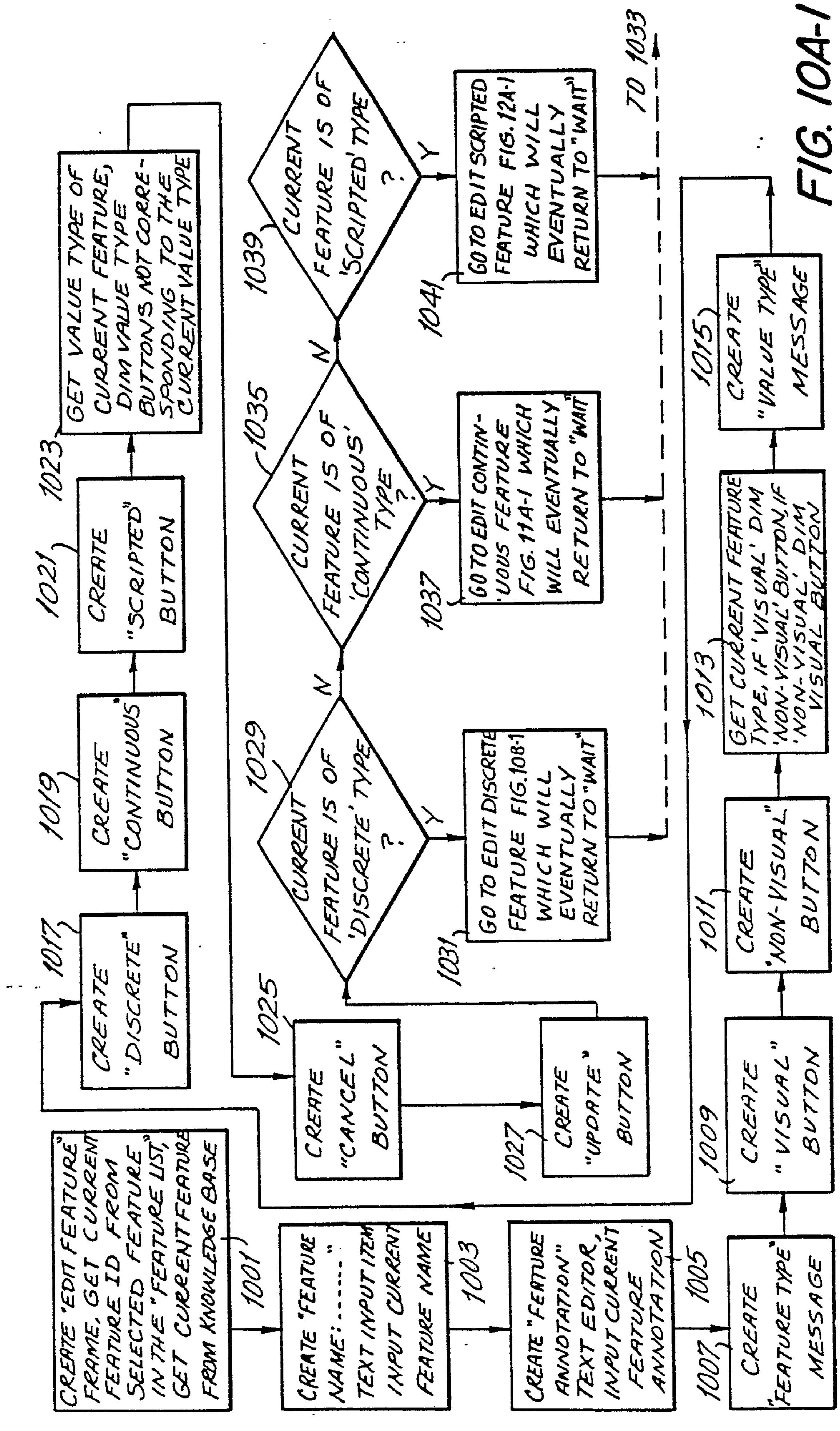

CREATE "EDIT FEATURE" FRAME, GET CURRENT FEATURE ID FROM SELECTED FEATURE IN THE "FEATURE LIST", GET CURRENT FEATURE FROM KNOWLEDGE BASE
1001
CREATE "FEATURE NAME: ......" TEXT INPUT ITEM INPUT CURRENT FEATURE NAME
1003
CREATE "FEATURE ANNOTATION" TEXT EDITOR, INPUT CURRENT FEATURE ANNOTATION
1005
CREATE "FEATURE TYPE" MESSAGE
1007
CREATE "VISUAL" BUTTON
1009
CREATE "NON-VISUAL" BUTTON
1011
GET CURRENT FEATURE TYPE, IF 'VISUAL' DIM 'NON-VISUAL' BUTTON, IF 'NON-VISUAL' DIM VISUAL BUTTON
1013
CREATE "VALUE TYPE" MESSAGE
1015
CREATE "DISCRETE" BUTTON
1017
CREATE "CONTINUOUS" BUTTON
1019
CREATE "SCRIPTED" BUTTON
1021
GET VALUE TYPE OF CURRENT FEATURE, DIM VALUE TYPE BUTTONS NOT CORRESPONDING TO THE CURRENT VALUE TYPE
1023
CREATE "CANCEL" BUTTON
1025
CREATE "UPDATE" BUTTON
1027
CURRENT FEATURE IS OF 'DISCRETE' TYPE ?
1029
Y
N
GO TO EDIT DISCRETE FEATURE FIG.10B-1 WHICH WILL EVENTUALLY RETURN TO "WAIT"
1031
CURRENT FEATURE IS OF 'CONTINUOUS' TYPE ?
1035
Y
N
GO TO EDIT CONTINUOUS FEATURE FIG.11A-1 WHICH WILL EVENTUALLY RETURN TO "WAIT"
1037
CURRENT FEATURE IS OF 'SCRIPTED' TYPE ?
1039
Y
GO TO EDIT SCRIPTED FEATURE FIG.12A-1 WHICH WILL EVENTUALLY RETURN TO "WAIT"
1041
TO 1033


FIG. 10A-1

eingereicht / Newly filed
Nouvellement déposé

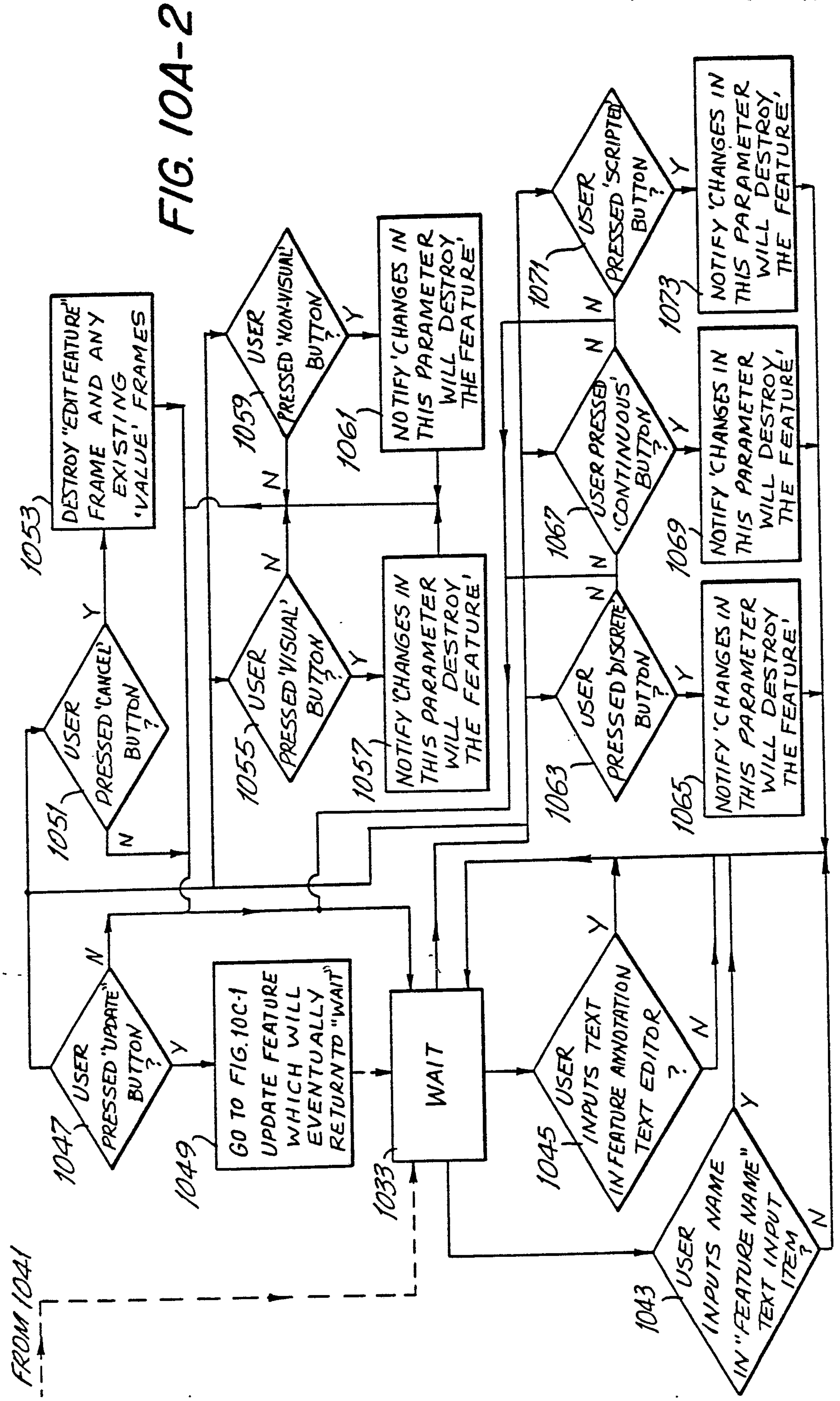


FIG. 10A-2

FIG. 10B-1

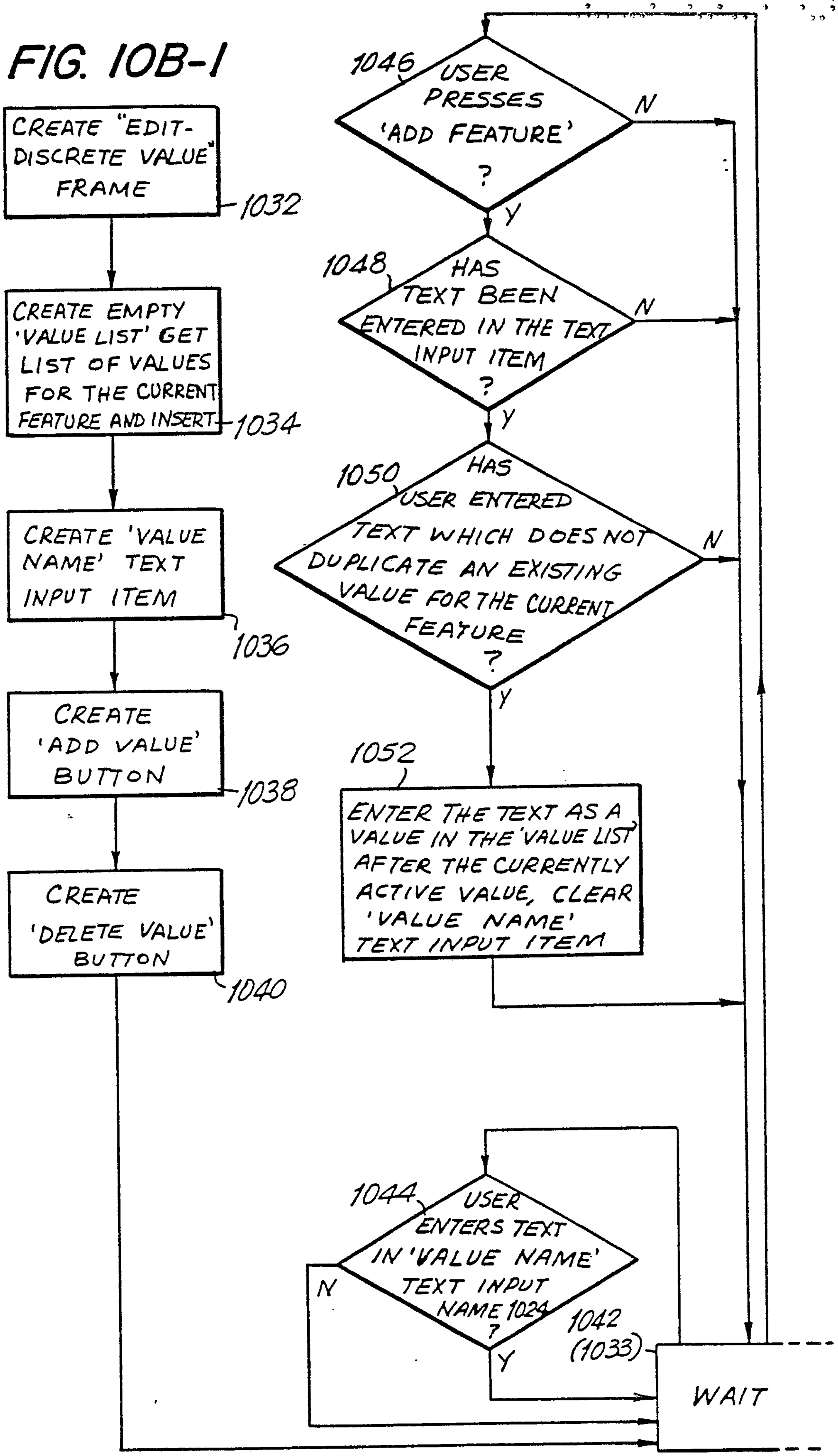
CREATE "EDIT-DISCRETE VALUE" FRAME
1032
CREATE EMPTY 'VALUE LIST' GET LIST OF VALUES FOR THE CURRENT FEATURE AND INSERT
1034
CREATE 'VALUE NAME' TEXT INPUT ITEM
1036
CREATE 'ADD VALUE' BUTTON
1038
CREATE 'DELETE VALUE' BUTTON
1040
1046
USER PRESSES 'ADD FEATURE' ?
N
Y
1048
HAS TEXT BEEN ENTERED IN THE TEXT INPUT ITEM ?
N
Y
1050
HAS USER ENTERED TEXT WHICH DOES NOT DUPLICATE AN EXISTING VALUE FOR THE CURRENT FEATURE ?
N
Y
1052
ENTER THE TEXT AS A VALUE IN THE 'VALUE LIST' AFTER THE CURRENTLY ACTIVE VALUE, CLEAR 'VALUE NAME' TEXT INPUT ITEM
1044
USER ENTERS TEXT IN 'VALUE NAME' TEXT INPUT NAME 1024 ?
N
Y
1042
(1033)
WAIT

Nouvellement déposé

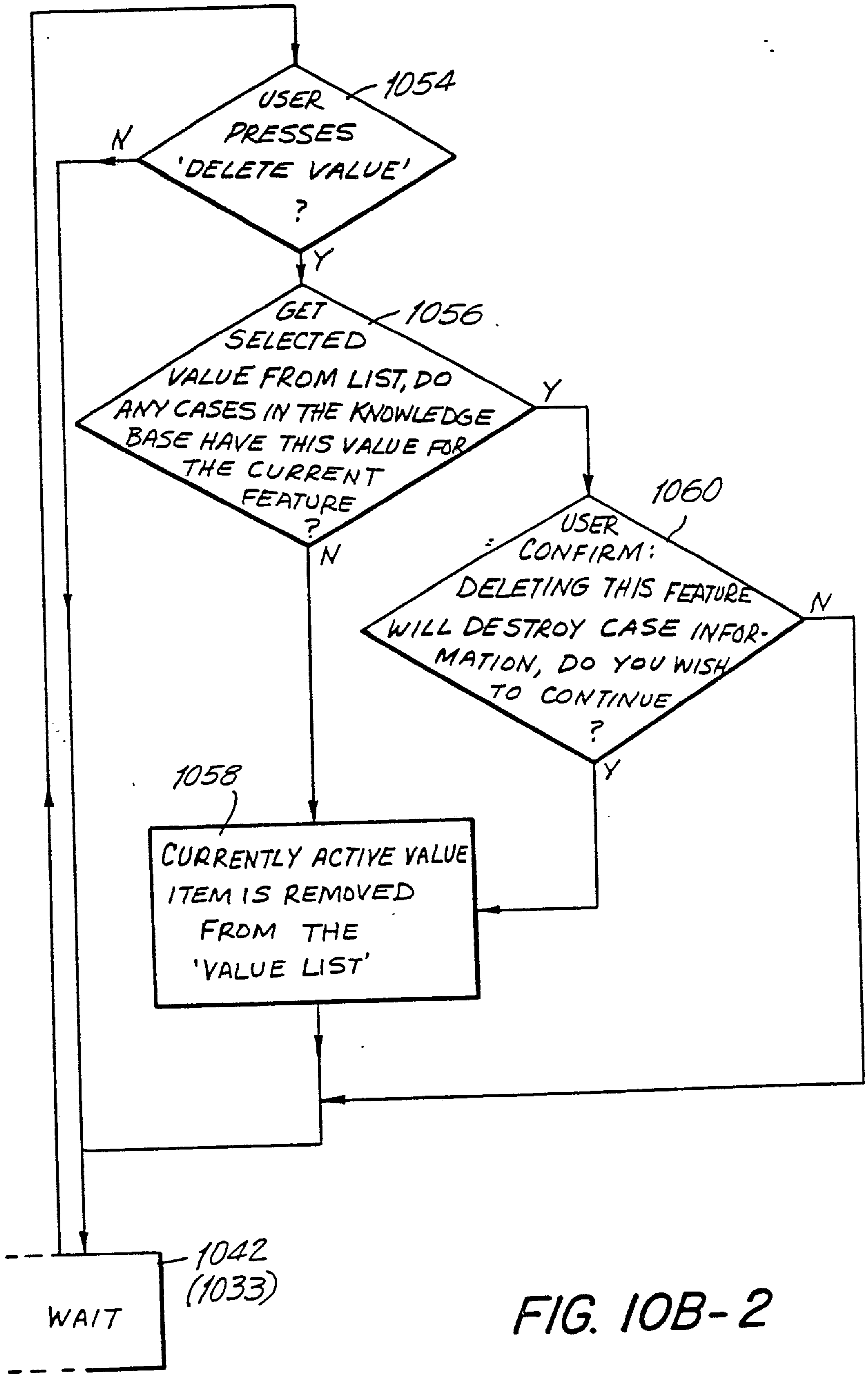


FIG. 10B-2

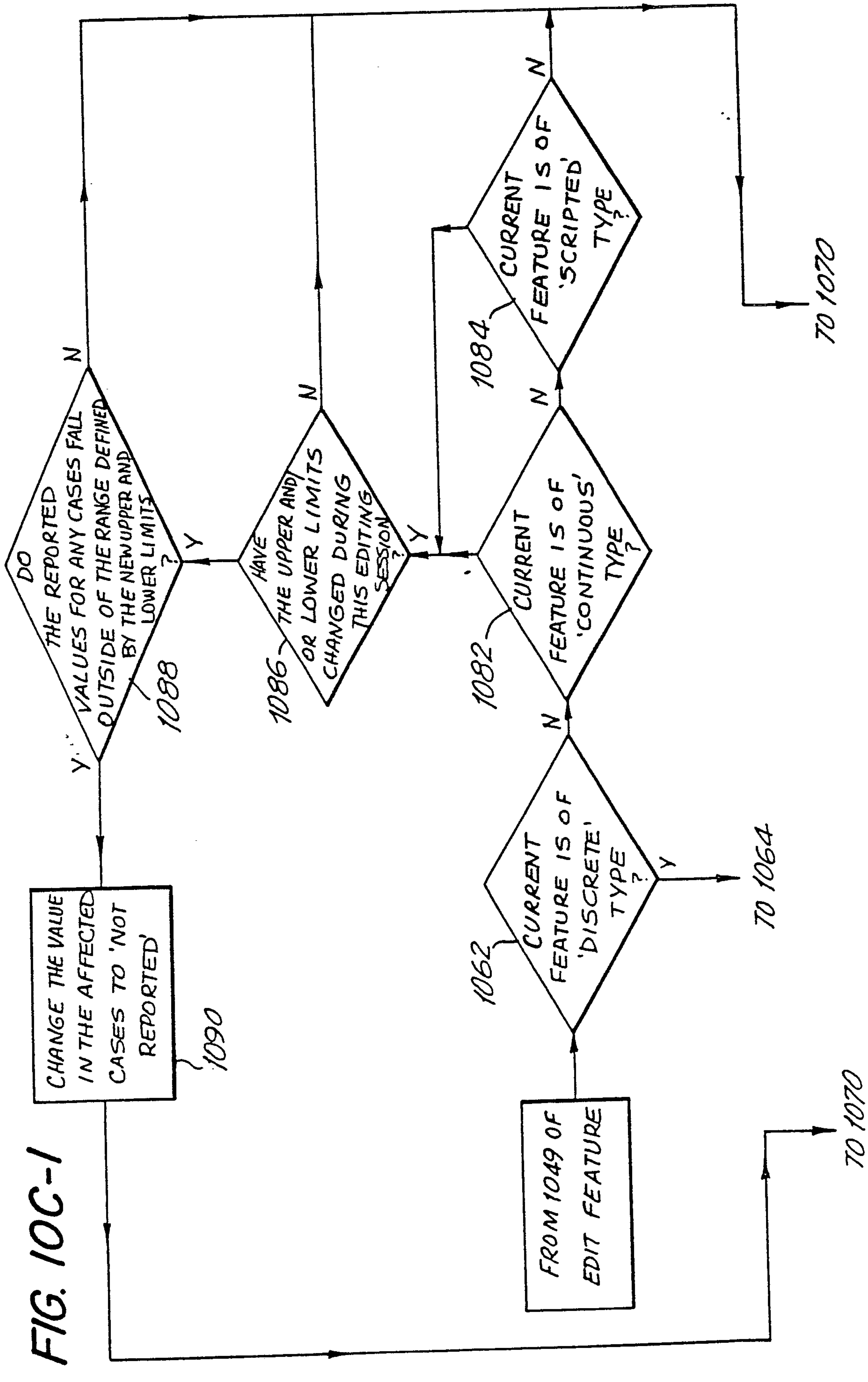
FROM 1049 OF EDIT FEATURE
1062
CURRENT FEATURE IS OF 'DISCRETE' TYPE ?
Y
TO 1064
N
1082
CURRENT FEATURE IS OF 'CONTINUOUS' TYPE ?
N
1084
CURRENT FEATURE IS OF 'SCRIPTED' TYPE ?
N
TO 1070
Y
1086
HAVE THE UPPER AND/OR LOWER LIMITS CHANGED DURING THIS EDITING SESSION ?
N
Y
1088
DO THE REPORTED VALUES FOR ANY CASES FALL OUTSIDE OF THE RANGE DEFINED BY THE NEW UPPER AND LOWER LIMITS ?
N
Y
CHANGE THE VALUE IN THE AFFECTED CASES TO 'NOT REPORTED'
1090
TO 1070

FIG. 10C-1

Nouvellement déposé

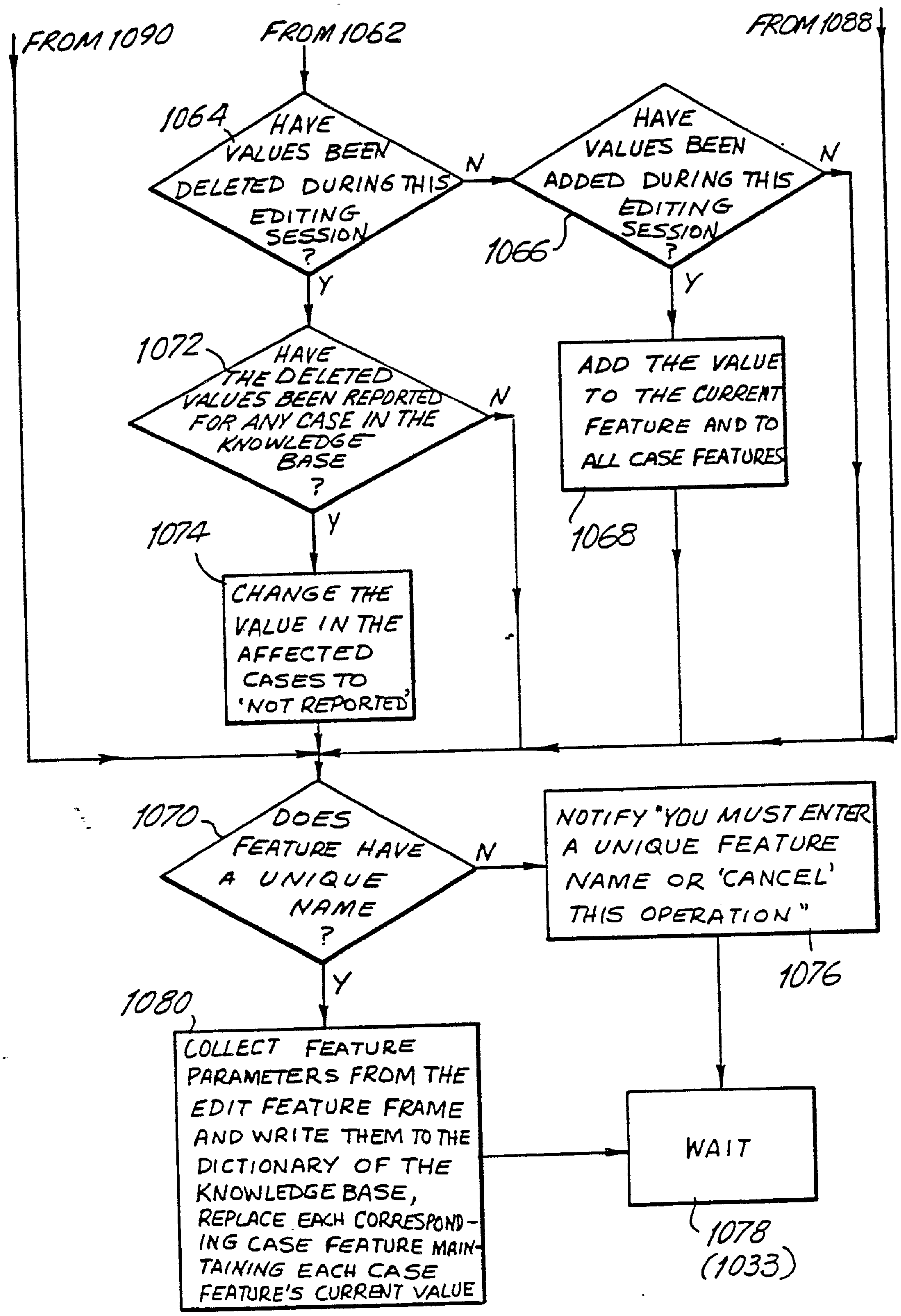


FIG. 10C-2

Nouvellement déposé

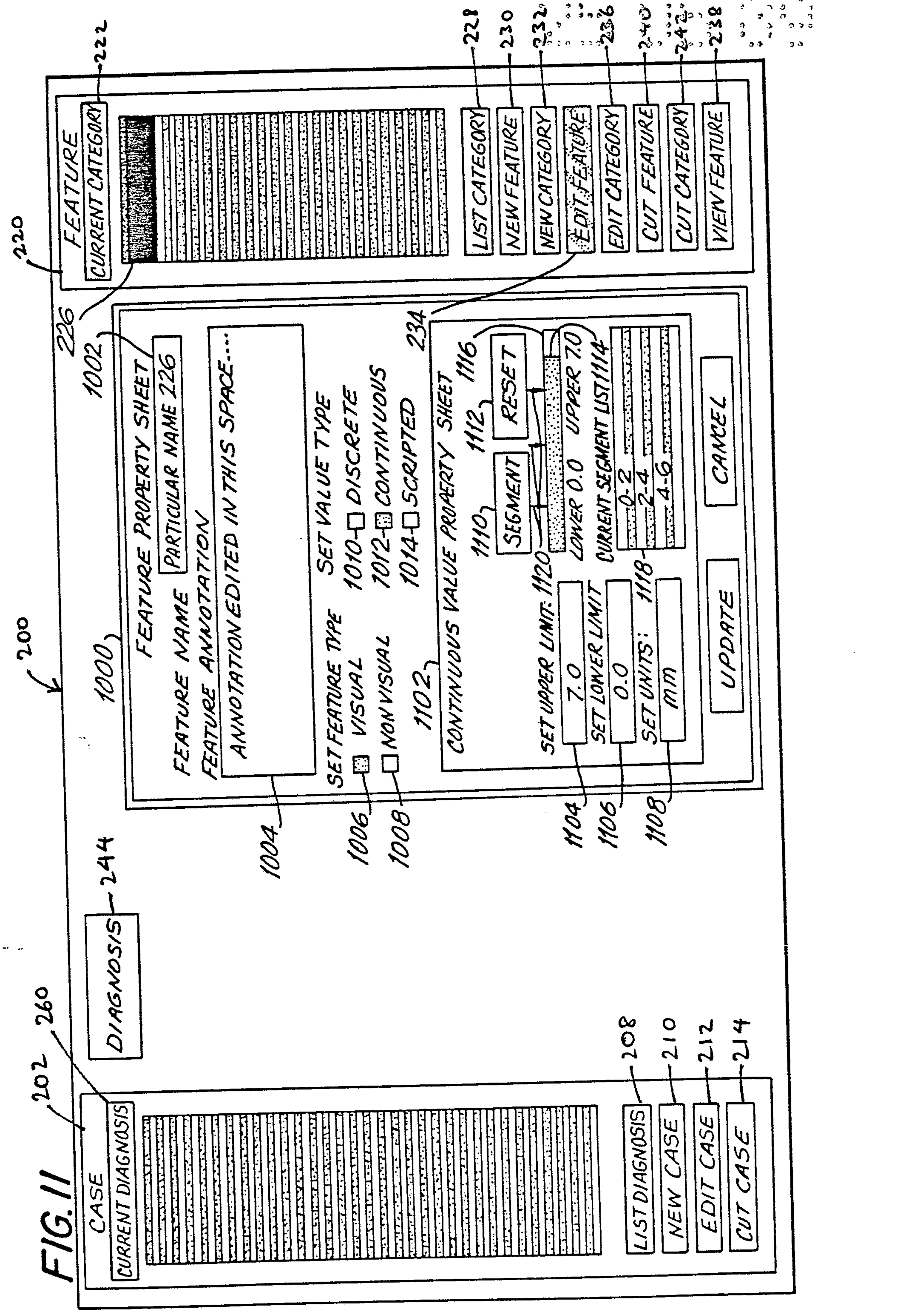


FIG. 11

Neu eingereicht / Newly filed
Nouvellement déposé

FIG.11A-1

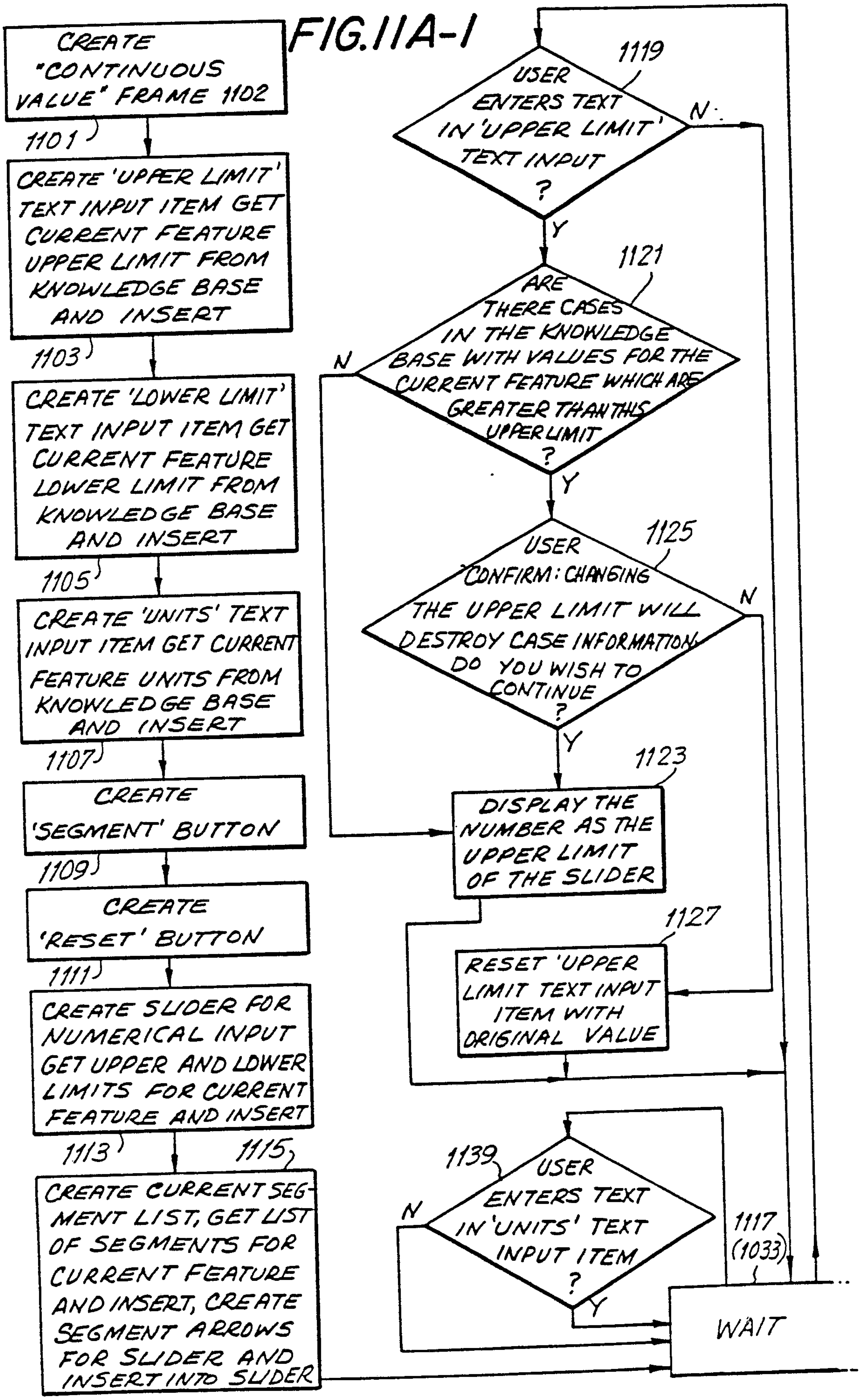

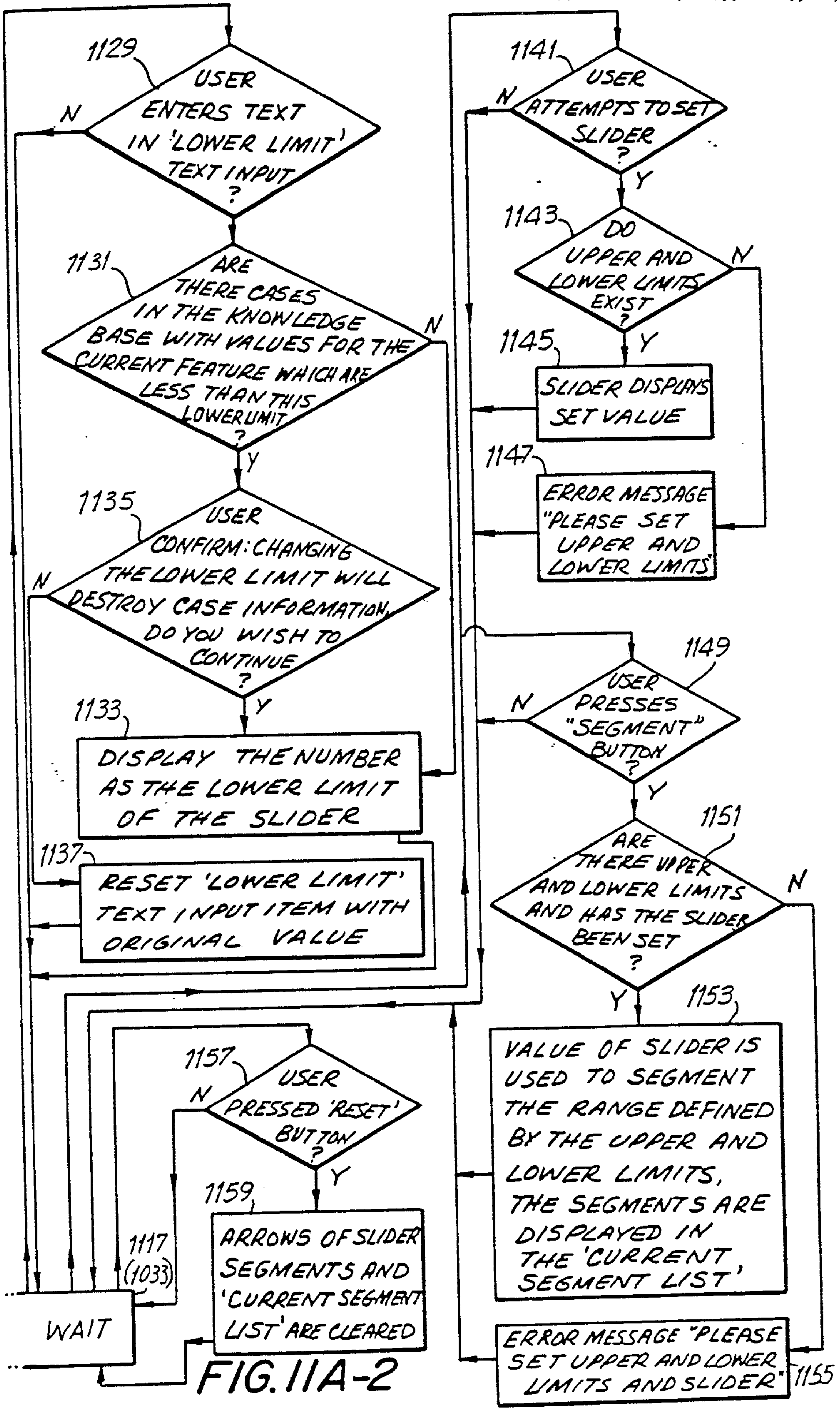
1129
USER ENTERS TEXT IN 'LOWER LIMIT' TEXT INPUT ?
N
1131
ARE THERE CASES IN THE KNOWLEDGE BASE WITH VALUES FOR THE CURRENT FEATURE WHICH ARE LESS THAN THIS LOWER LIMIT ?
N
Y
1135
USER CONFIRM: CHANGING THE LOWER LIMIT WILL DESTROY CASE INFORMATION, DO YOU WISH TO CONTINUE ?
N
Y
1133
DISPLAY THE NUMBER AS THE LOWER LIMIT OF THE SLIDER
1137
RESET 'LOWER LIMIT' TEXT INPUT ITEM WITH ORIGINAL VALUE
1141
USER ATTEMPTS TO SET SLIDER ?
N
Y
1143
DO UPPER AND LOWER LIMITS EXIST ?
N
Y
1145
SLIDER DISPLAYS SET VALUE
1147
ERROR MESSAGE "PLEASE SET UPPER AND LOWER LIMITS"
1149
USER PRESSES "SEGMENT" BUTTON ?
N
Y
1151
ARE THERE UPPER AND LOWER LIMITS AND HAS THE SLIDER BEEN SET ?
N
Y
1153
VALUE OF SLIDER IS USED TO SEGMENT THE RANGE DEFINED BY THE UPPER AND LOWER LIMITS, THE SEGMENTS ARE DISPLAYED IN THE 'CURRENT SEGMENT LIST'
1155
ERROR MESSAGE "PLEASE SET UPPER AND LOWER LIMITS AND SLIDER"
1157
USER PRESSED 'RESET' BUTTON ?
N
Y
1159
ARROWS OF SLIDER SEGMENTS AND 'CURRENT SEGMENT LIST' ARE CLEARED
1117 (1033)
WAIT

FIG.11A-2

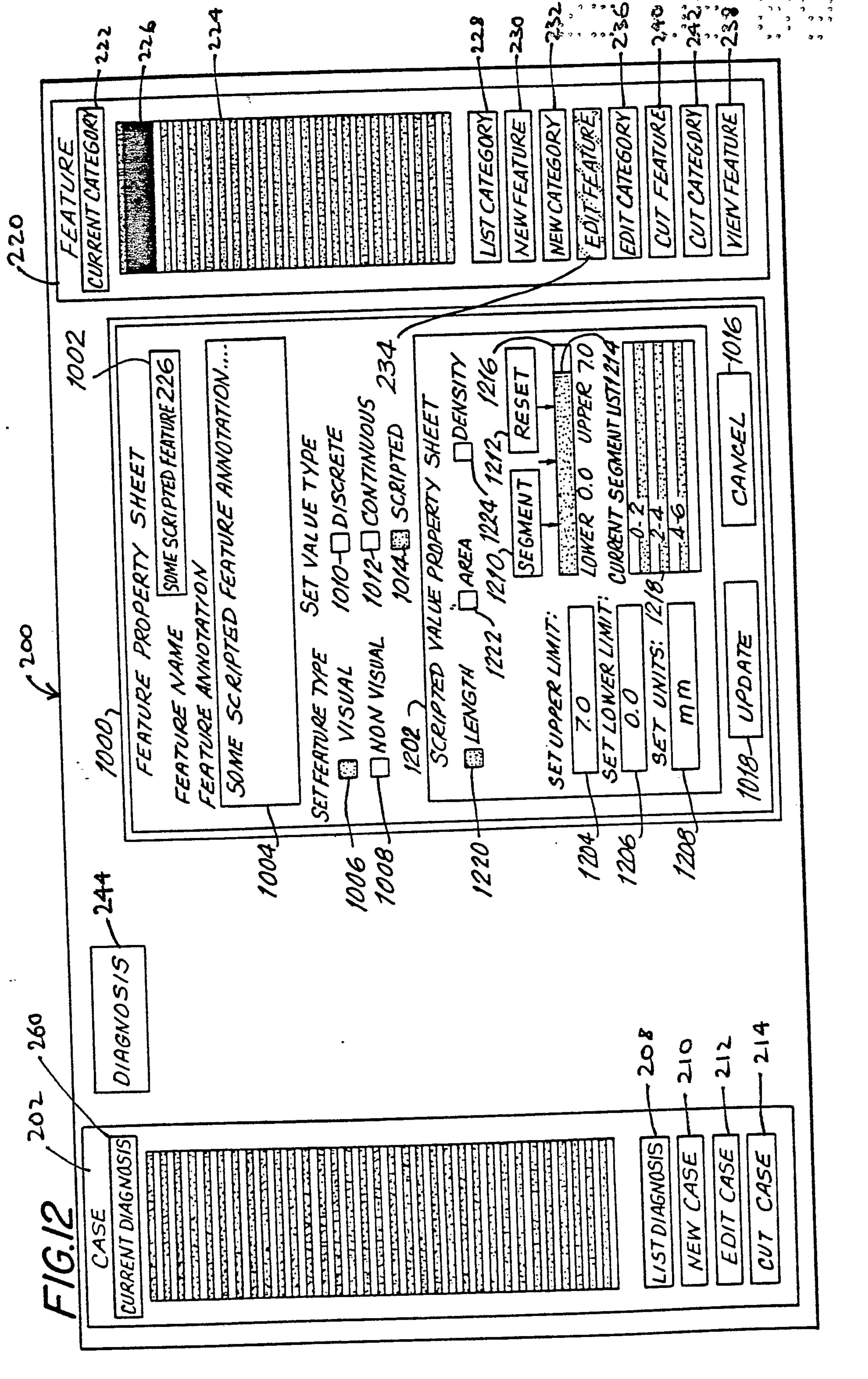
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
FEATURE PROPERTY SHEET
FEATURE NAME
SOME SCRIPTED FEATURE 226
FEATURE ANNOTATION
SOME SCRIPTED FEATURE ANNOTATION....
SET FEATURE TYPE
VISUAL
NON VISUAL
SET VALUE TYPE
DISCRETE
CONTINUOUS
SCRIPTED
SCRIPTED VALUE PROPERTY SHEET
LENGTH
AREA
DENSITY
SEGMENT
RESET
LOWER 0.0
UPPER 7.0
CURRENT SEGMENT LIST 1214
0-2
2-4
4-6
SET UPPER LIMIT:
7.0
SET LOWER LIMIT:
0.0
SET UNITS:
mm
UPDATE
CANCEL
DIAGNOSIS
CASE
CURRENT DIAGNOSIS
LIST DIAGNOSIS
NEW CASE
EDIT CASE
CUT CASE
200
202
208
210
212
214
220
222
224
226
228
230
232
234
236
238
240
242
244
260
1000
1002
1004
1006
1008
1010
1012
1014
1016
1018
1202
1204
1206
1208
1210
1212
1216
1218
1220
1222
1224

FIG.12

FIG.12A-1

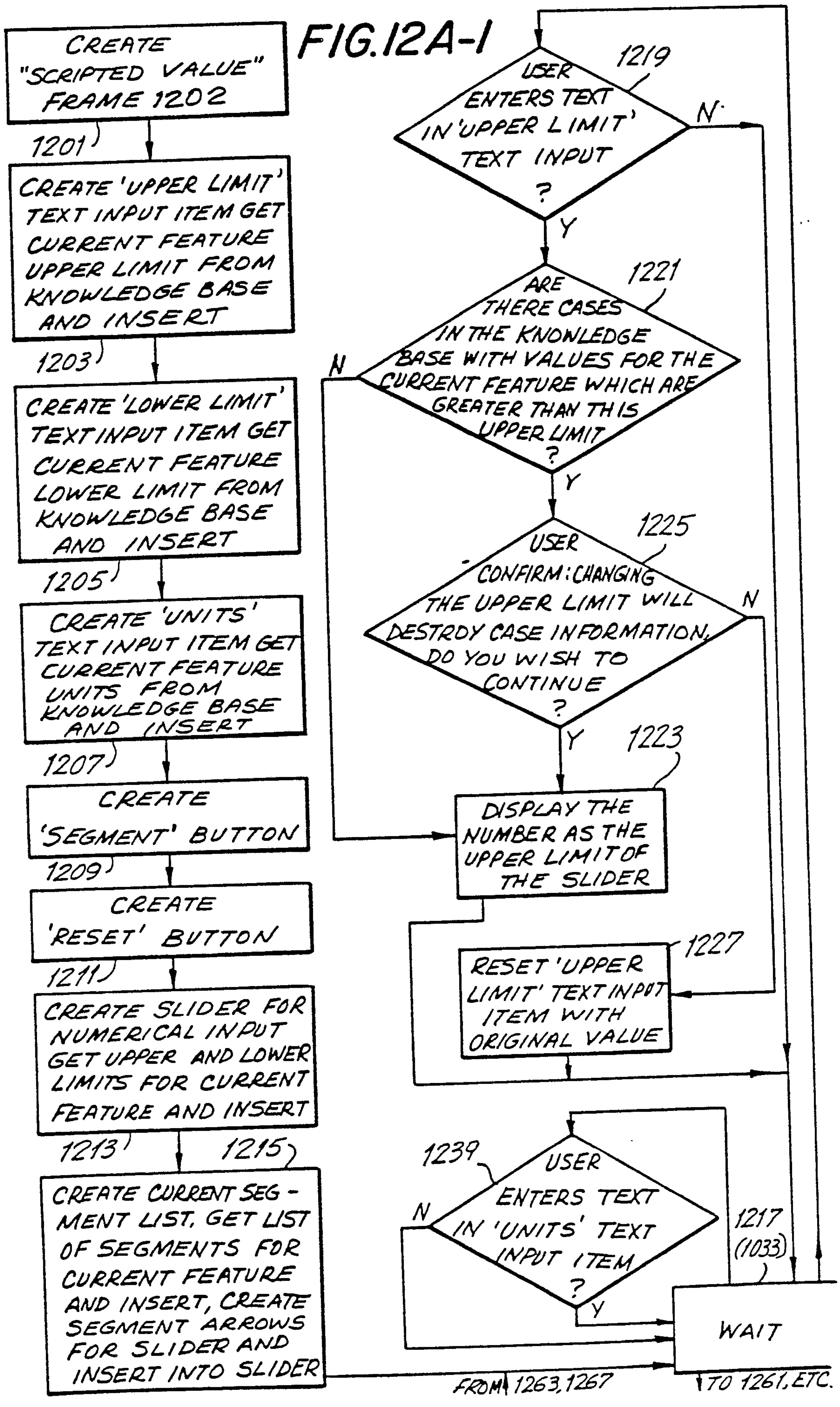
CREATE "SCRIPTED VALUE" FRAME 1202
1201
CREATE 'UPPER LIMIT' TEXT INPUT ITEM GET CURRENT FEATURE UPPER LIMIT FROM KNOWLEDGE BASE AND INSERT
1203
CREATE 'LOWER LIMIT' TEXT INPUT ITEM GET CURRENT FEATURE LOWER LIMIT FROM KNOWLEDGE BASE AND INSERT
1205
CREATE 'UNITS' TEXT INPUT ITEM GET CURRENT FEATURE UNITS FROM KNOWLEDGE BASE AND INSERT
1207
CREATE 'SEGMENT' BUTTON
1209
CREATE 'RESET' BUTTON
1211
CREATE SLIDER FOR NUMERICAL INPUT GET UPPER AND LOWER LIMITS FOR CURRENT FEATURE AND INSERT
1213
1215
CREATE CURRENT SEGMENT LIST. GET LIST OF SEGMENTS FOR CURRENT FEATURE AND INSERT, CREATE SEGMENT ARROWS FOR SLIDER AND INSERT INTO SLIDER
1219
USER ENTERS TEXT IN 'UPPER LIMIT' TEXT INPUT ?
N
Y
1221
ARE THERE CASES IN THE KNOWLEDGE BASE WITH VALUES FOR THE CURRENT FEATURE WHICH ARE GREATER THAN THIS UPPER LIMIT ?
N
Y
1225
USER CONFIRM: CHANGING THE UPPER LIMIT WILL DESTROY CASE INFORMATION, DO YOU WISH TO CONTINUE ?
N
Y
1223
DISPLAY THE NUMBER AS THE UPPER LIMIT OF THE SLIDER
1227
RESET 'UPPER LIMIT' TEXT INPUT ITEM WITH ORIGINAL VALUE
1239
USER ENTERS TEXT IN 'UNITS' TEXT INPUT ITEM ?
N
Y
1217
(1033)
WAIT
FROM 1263, 1267
TO 1261, ETC.

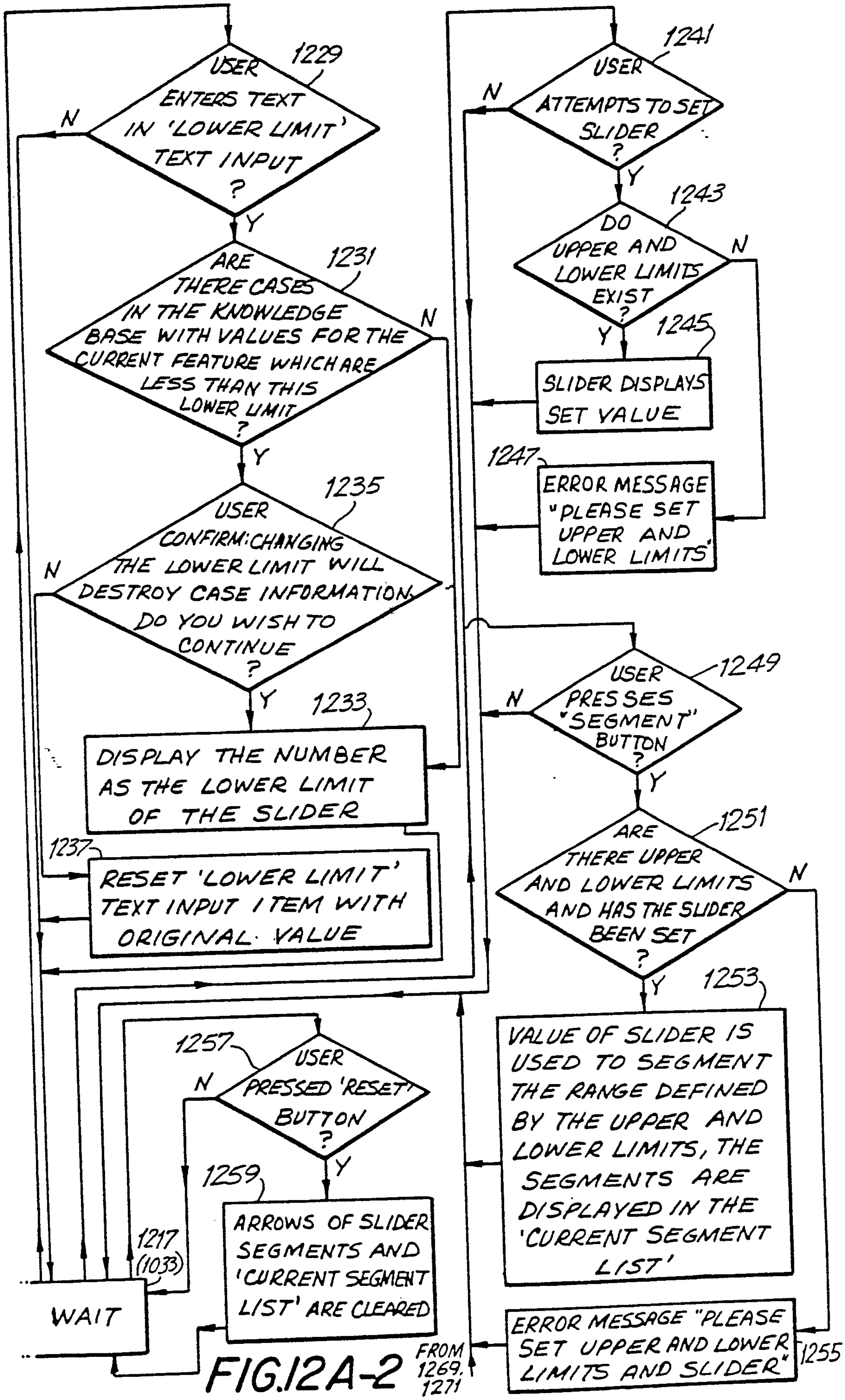
USER ENTERS TEXT IN 'LOWER LIMIT' TEXT INPUT ?
1229
N
Y
ARE THERE CASES IN THE KNOWLEDGE BASE WITH VALUES FOR THE CURRENT FEATURE WHICH ARE LESS THAN THIS LOWER LIMIT ?
1231
USER CONFIRM: CHANGING THE LOWER LIMIT WILL DESTROY CASE INFORMATION. DO YOU WISH TO CONTINUE ?
1235
DISPLAY THE NUMBER AS THE LOWER LIMIT OF THE SLIDER
1233
RESET 'LOWER LIMIT' TEXT INPUT ITEM WITH ORIGINAL VALUE
1237
USER ATTEMPTS TO SET SLIDER ?
1241
DO UPPER AND LOWER LIMITS EXIST ?
1243
SLIDER DISPLAYS SET VALUE
1245
ERROR MESSAGE "PLEASE SET UPPER AND LOWER LIMITS"
1247
USER PRESSES "SEGMENT" BUTTON ?
1249
ARE THERE UPPER AND LOWER LIMITS AND HAS THE SLIDER BEEN SET ?
1251
VALUE OF SLIDER IS USED TO SEGMENT THE RANGE DEFINED BY THE UPPER AND LOWER LIMITS, THE SEGMENTS ARE DISPLAYED IN THE 'CURRENT SEGMENT LIST'
1253
ERROR MESSAGE "PLEASE SET UPPER AND LOWER LIMITS AND SLIDER"
1255
USER PRESSED 'RESET' BUTTON ?
1257
ARROWS OF SLIDER SEGMENTS AND 'CURRENT SEGMENT LIST' ARE CLEARED
1259
WAIT
1217 (1033)
FROM 1269, 1271

FIG.12A-2

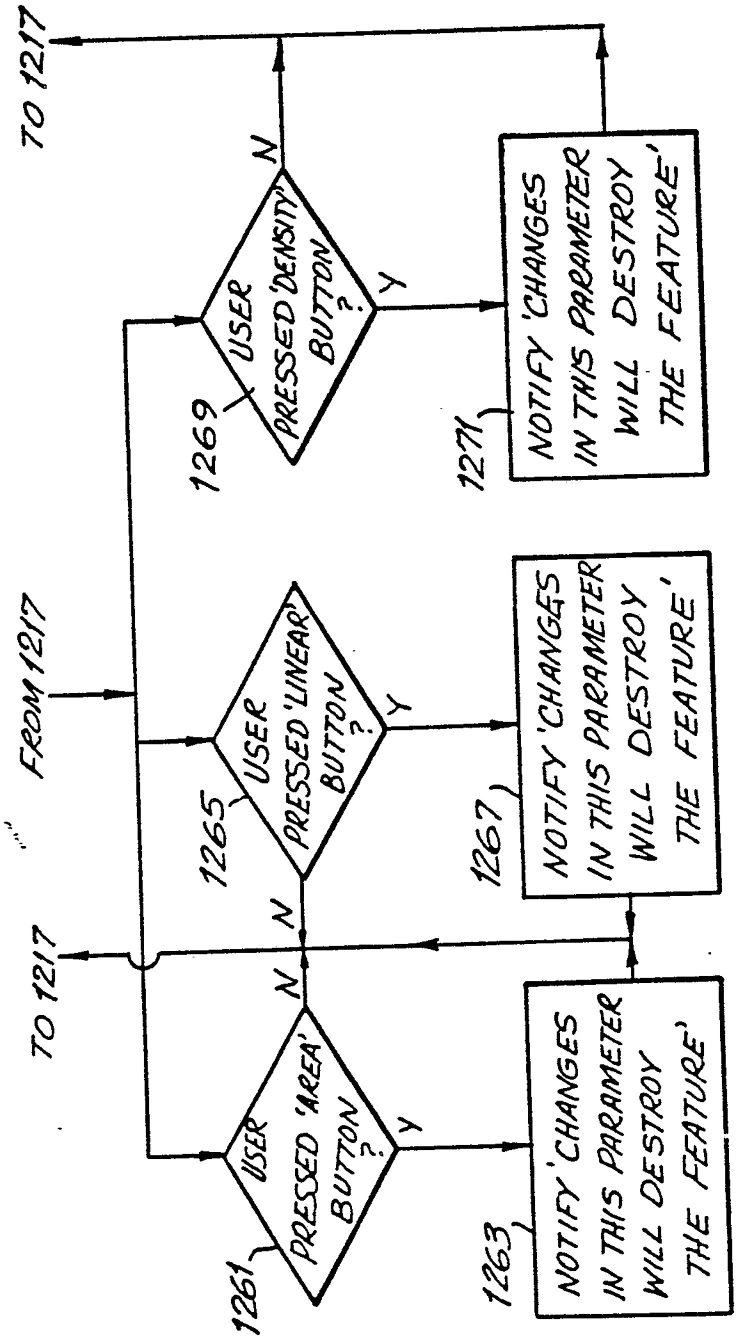
TO 1217
FROM 1217
TO 1217
1269
USER PRESSED 'DENSITY' BUTTON ?
N
Y
1271
NOTIFY 'CHANGES IN THIS PARAMETER WILL DESTROY THE FEATURE'
1265
USER PRESSED 'LINEAR' BUTTON ?
N
Y
1267
NOTIFY 'CHANGES IN THIS PARAMETER WILL DESTROY THE FEATURE'
1261
USER PRESSED 'AREA' BUTTON ?
N
Y
1263
NOTIFY 'CHANGES IN THIS PARAMETER WILL DESTROY THE FEATURE'

FIG.12A-3

Nouvellement déposé

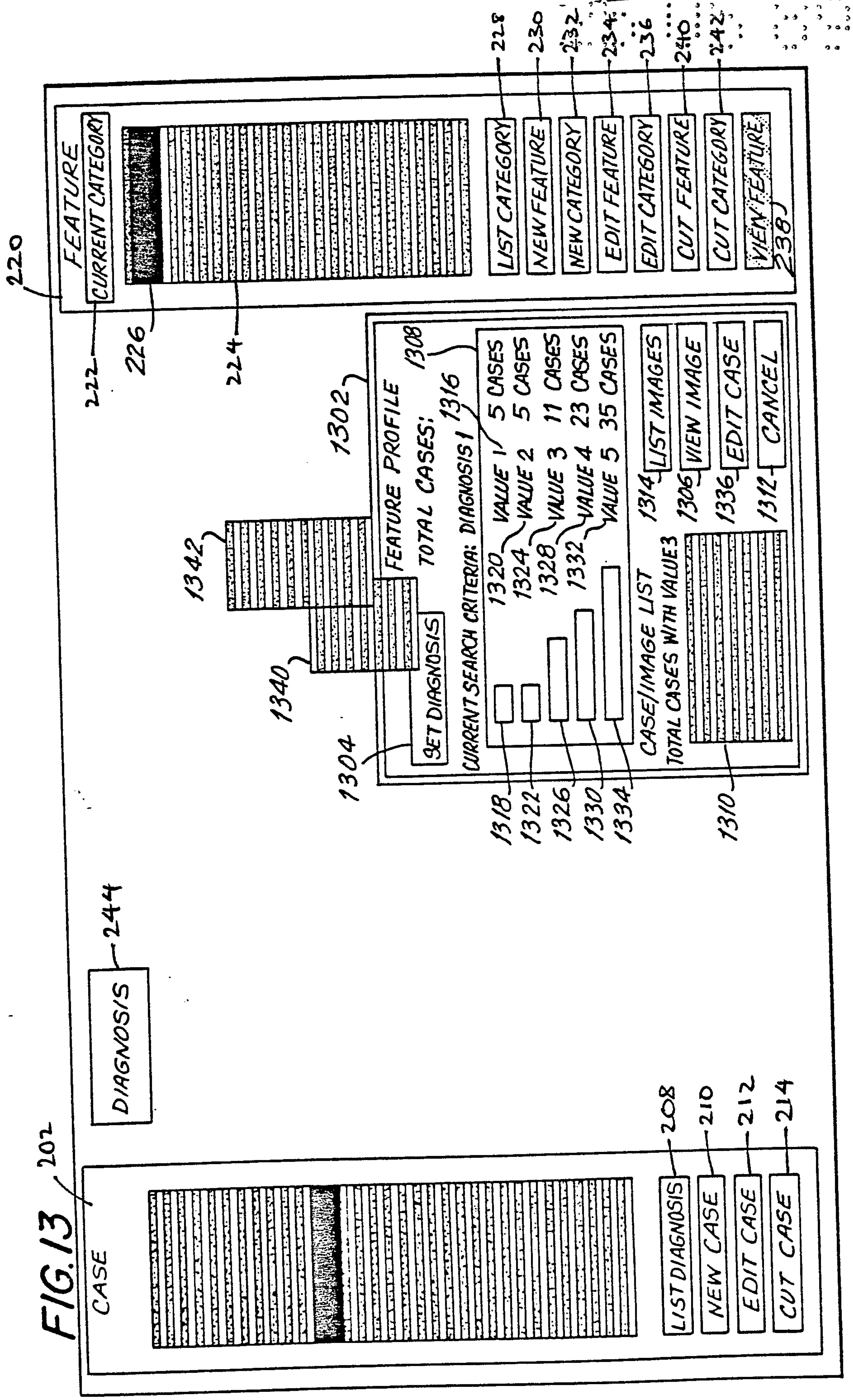


FIG.13

FIG. 13A-1

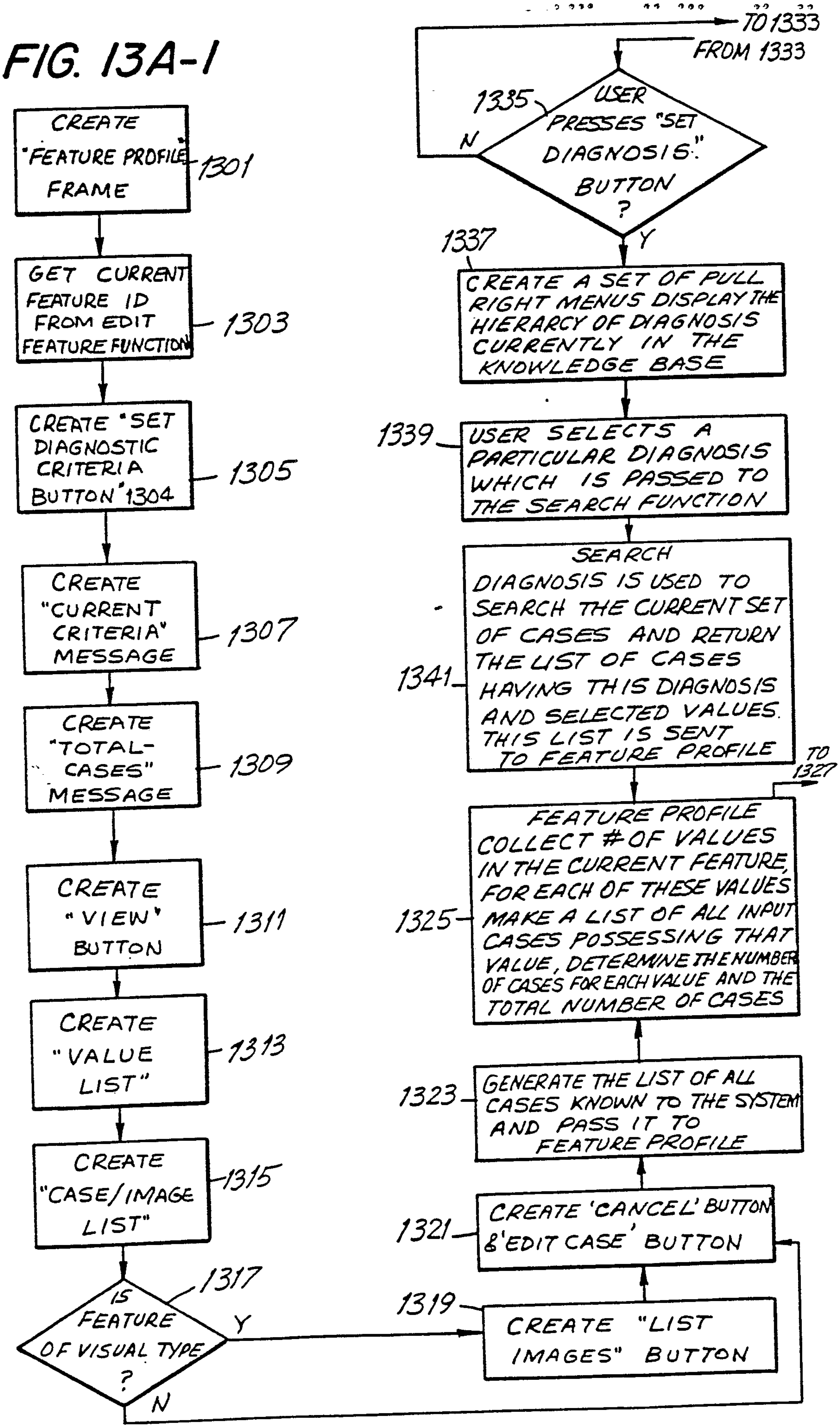
TO 1333
FROM 1333
USER PRESSES "SET DIAGNOSIS" BUTTON ?
1335
N
Y
1337
CREATE A SET OF PULL RIGHT MENUS DISPLAY THE HIERARCY OF DIAGNOSIS CURRENTLY IN THE KNOWLEDGE BASE
1339
USER SELECTS A PARTICULAR DIAGNOSIS WHICH IS PASSED TO THE SEARCH FUNCTION
SEARCH
DIAGNOSIS IS USED TO SEARCH THE CURRENT SET OF CASES AND RETURN THE LIST OF CASES HAVING THIS DIAGNOSIS AND SELECTED VALUES. THIS LIST IS SENT TO FEATURE PROFILE
1341
TO 1327
FEATURE PROFILE
COLLECT # OF VALUES IN THE CURRENT FEATURE, FOR EACH OF THESE VALUES MAKE A LIST OF ALL INPUT CASES POSSESSING THAT VALUE, DETERMINE THE NUMBER OF CASES FOR EACH VALUE AND THE TOTAL NUMBER OF CASES
1325
GENERATE THE LIST OF ALL CASES KNOWN TO THE SYSTEM AND PASS IT TO FEATURE PROFILE
1323
CREATE 'CANCEL' BUTTON & 'EDIT CASE' BUTTON
1321
CREATE "LIST IMAGES" BUTTON
1319
CREATE "FEATURE PROFILE" FRAME
1301
GET CURRENT FEATURE ID FROM EDIT FEATURE FUNCTION
1303
CREATE "SET DIAGNOSTIC CRITERIA BUTTON" 1304
1305
CREATE "CURRENT CRITERIA" MESSAGE
1307
CREATE "TOTAL-CASES" MESSAGE
1309
CREATE "VIEW" BUTTON
1311
CREATE "VALUE LIST"
1313
CREATE "CASE/IMAGE LIST"
1315
IS FEATURE OF VISUAL TYPE ?
1317
Y
N

FIG. 13A-2

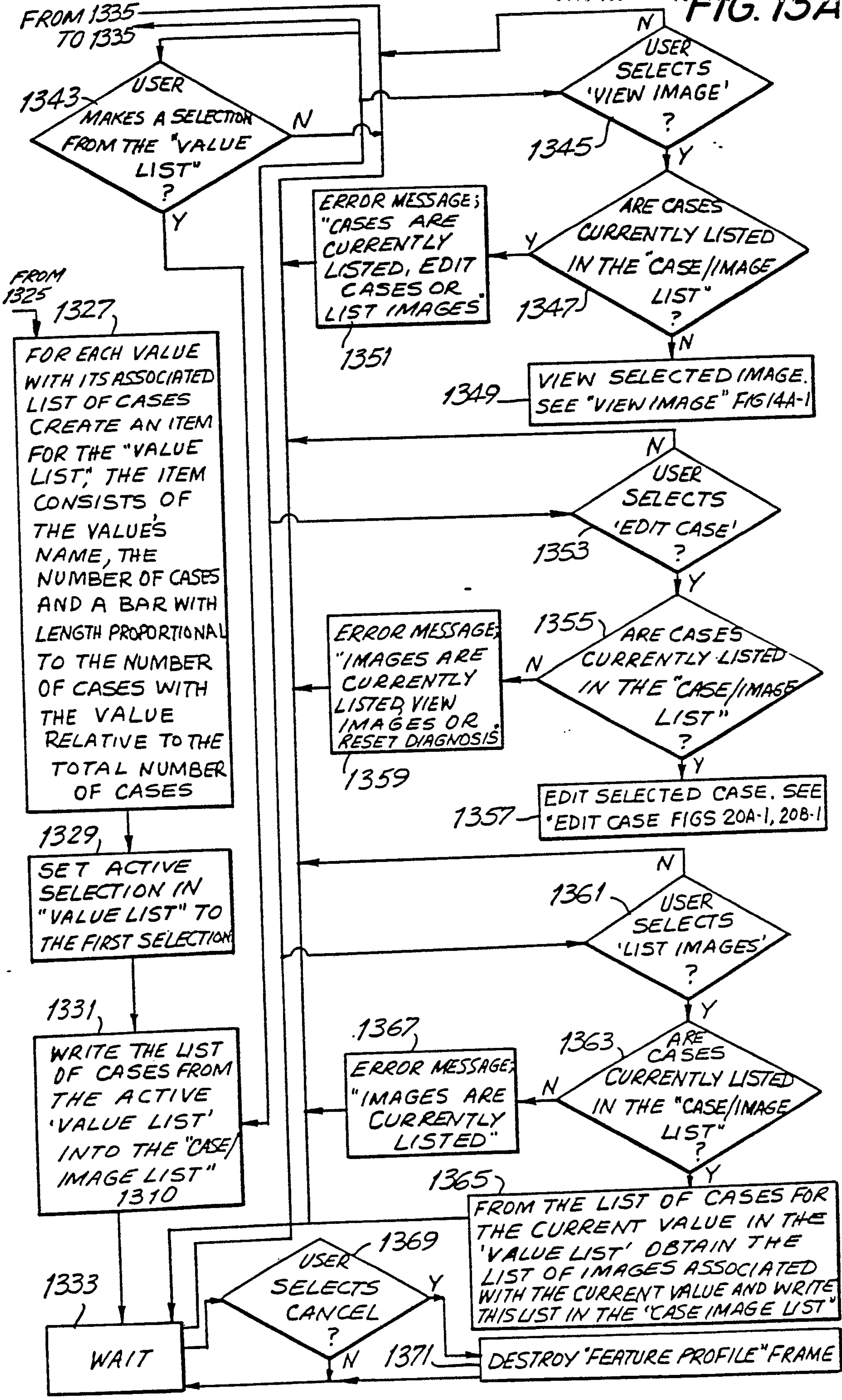
FROM 1335
TO 1335
1343
USER MAKES A SELECTION FROM THE "VALUE LIST" ?
N
Y
USER SELECTS 'VIEW IMAGE' ?
1345
ARE CASES CURRENTLY LISTED IN THE "CASE/IMAGE LIST" ?
1347
ERROR MESSAGE; "CASES ARE CURRENTLY LISTED, EDIT CASES OR LIST IMAGES"
1351
VIEW SELECTED IMAGE. SEE "VIEW IMAGE" FIG 14A-1
1349
FROM 1325
1327
FOR EACH VALUE WITH ITS ASSOCIATED LIST OF CASES CREATE AN ITEM FOR THE "VALUE LIST," THE ITEM CONSISTS OF THE VALUE'S NAME, THE NUMBER OF CASES AND A BAR WITH LENGTH PROPORTIONAL TO THE NUMBER OF CASES WITH THE VALUE RELATIVE TO THE TOTAL NUMBER OF CASES
USER SELECTS 'EDIT CASE' ?
1353
1355
ARE CASES CURRENTLY LISTED IN THE "CASE/IMAGE LIST" ?
ERROR MESSAGE; "IMAGES ARE CURRENTLY LISTED, VIEW IMAGES OR RESET DIAGNOSIS"
1359
EDIT SELECTED CASE. SEE "EDIT CASE FIGS 20A-1, 20B-1
1357
1329
SET ACTIVE SELECTION IN "VALUE LIST" TO THE FIRST SELECTION
1361
USER SELECTS 'LIST IMAGES' ?
1331
WRITE THE LIST OF CASES FROM THE ACTIVE 'VALUE LIST' INTO THE "CASE/IMAGE LIST" 1310
1367
ERROR MESSAGE; "IMAGES ARE CURRENTLY LISTED"
1363
ARE CASES CURRENTLY LISTED IN THE "CASE/IMAGE LIST" ?
1365
FROM THE LIST OF CASES FOR THE CURRENT VALUE IN THE 'VALUE LIST' OBTAIN THE LIST OF IMAGES ASSOCIATED WITH THE CURRENT VALUE AND WRITE THIS LIST IN THE 'CASE IMAGE LIST'
1333
WAIT
USER SELECTS CANCEL ?
1369
1371
DESTROY "FEATURE PROFILE" FRAME

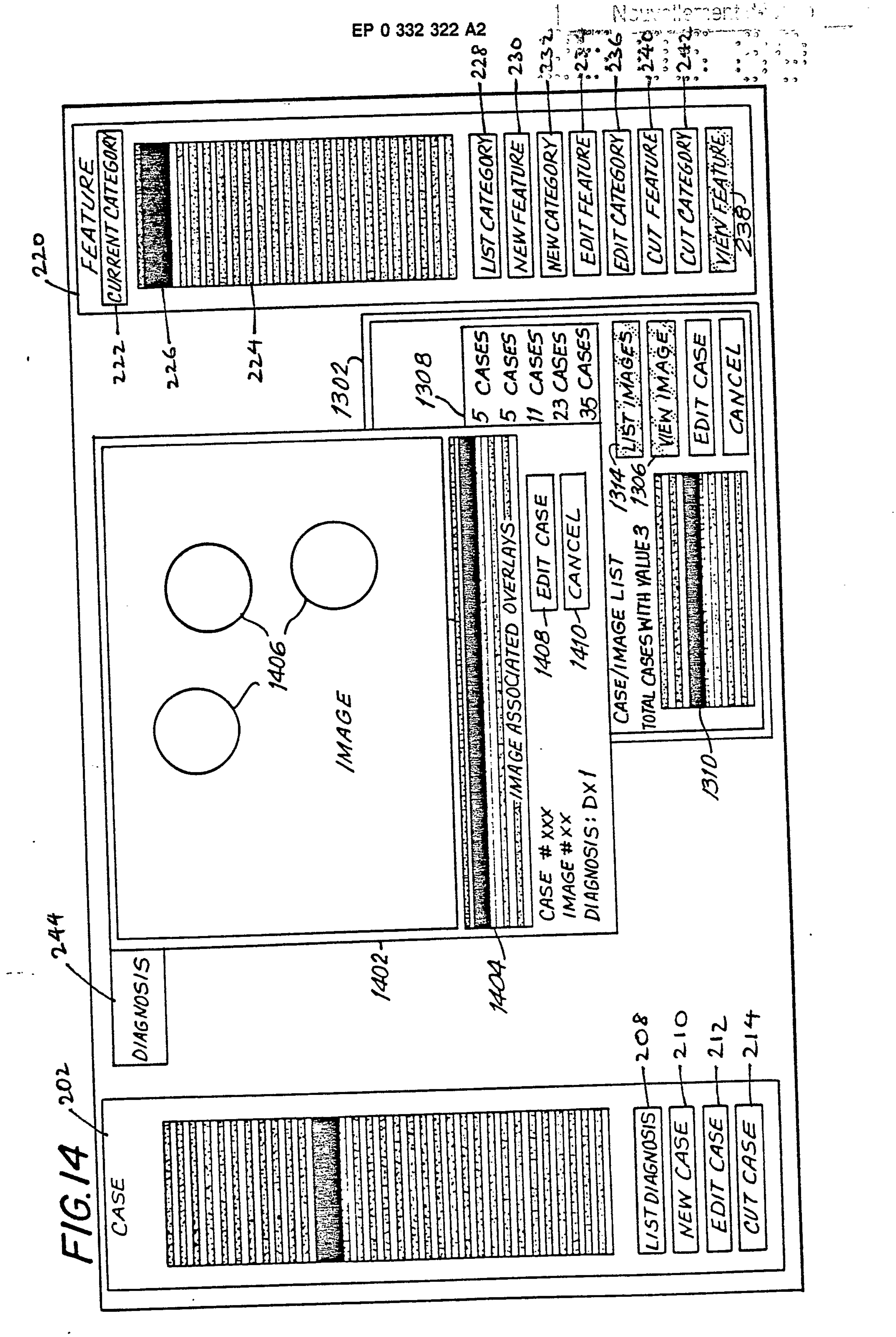

CASE
LIST DIAGNOSIS
NEW CASE
EDIT CASE
CUT CASE
DIAGNOSIS
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
IMAGE
IMAGE ASSOCIATED OVERLAYS
EDIT CASE
CANCEL
CASE # XXX
IMAGE # XX
DIAGNOSIS: DX1
CASE/IMAGE LIST
TOTAL CASES WITH VALUE 3
5 CASES
5 CASES
11 CASES
23 CASES
35 CASES
LIST IMAGES
VIEW IMAGE
EDIT CASE
CANCEL
202
208
210
212
214
220
222
224
226
228
230
232
234
236
238
240
242
244
1302
1306
1308
1310
1314
1402
1404
1406
1408
1410


FIG.14

FIG. 14A

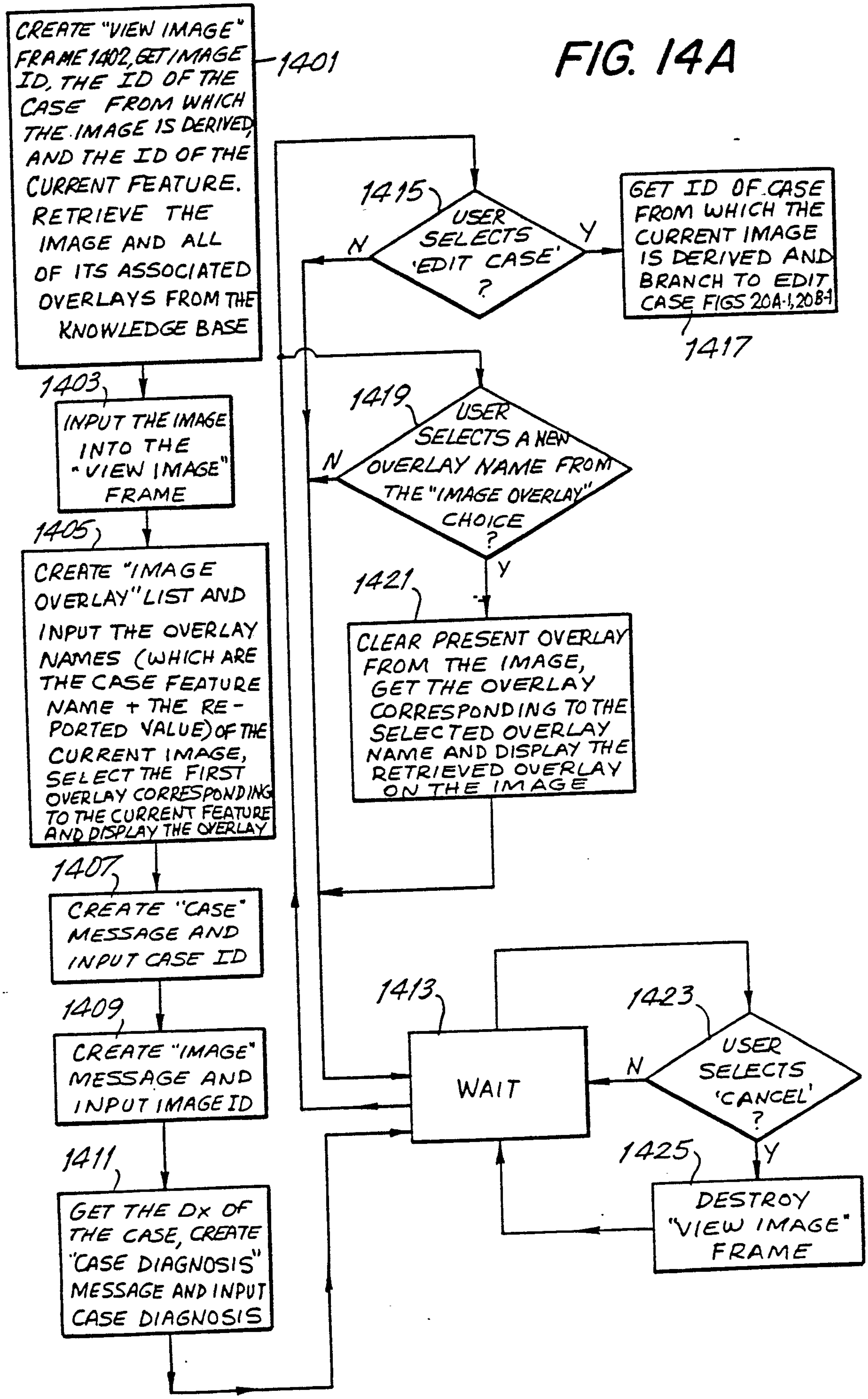
CREATE "VIEW IMAGE" FRAME 1402, GET IMAGE ID, THE ID OF THE CASE FROM WHICH THE IMAGE IS DERIVED, AND THE ID OF THE CURRENT FEATURE. RETRIEVE THE IMAGE AND ALL OF ITS ASSOCIATED OVERLAYS FROM THE KNOWLEDGE BASE
1401
1403
INPUT THE IMAGE INTO THE "VIEW IMAGE" FRAME
1405
CREATE "IMAGE OVERLAY" LIST AND INPUT THE OVERLAY NAMES (WHICH ARE THE CASE FEATURE NAME + THE REPORTED VALUE) OF THE CURRENT IMAGE, SELECT THE FIRST OVERLAY CORRESPONDING TO THE CURRENT FEATURE AND DISPLAY THE OVERLAY
1407
CREATE "CASE" MESSAGE AND INPUT CASE ID
1409
CREATE "IMAGE" MESSAGE AND INPUT IMAGE ID
1411
GET THE Dx OF THE CASE, CREATE "CASE DIAGNOSIS" MESSAGE AND INPUT CASE DIAGNOSIS
1415
USER SELECTS 'EDIT CASE' ?
N
Y
GET ID OF CASE FROM WHICH THE CURRENT IMAGE IS DERIVED AND BRANCH TO EDIT CASE FIGS 20A-1, 20B-1
1417
1419
USER SELECTS A NEW OVERLAY NAME FROM THE "IMAGE OVERLAY" CHOICE ?
N
Y
1421
CLEAR PRESENT OVERLAY FROM THE IMAGE, GET THE OVERLAY CORRESPONDING TO THE SELECTED OVERLAY NAME AND DISPLAY THE RETRIEVED OVERLAY ON THE IMAGE
1413
WAIT
1423
USER SELECTS 'CANCEL' ?
N
Y
1425
DESTROY "VIEW IMAGE" FRAME

Nouvellement déposé

FIG.15

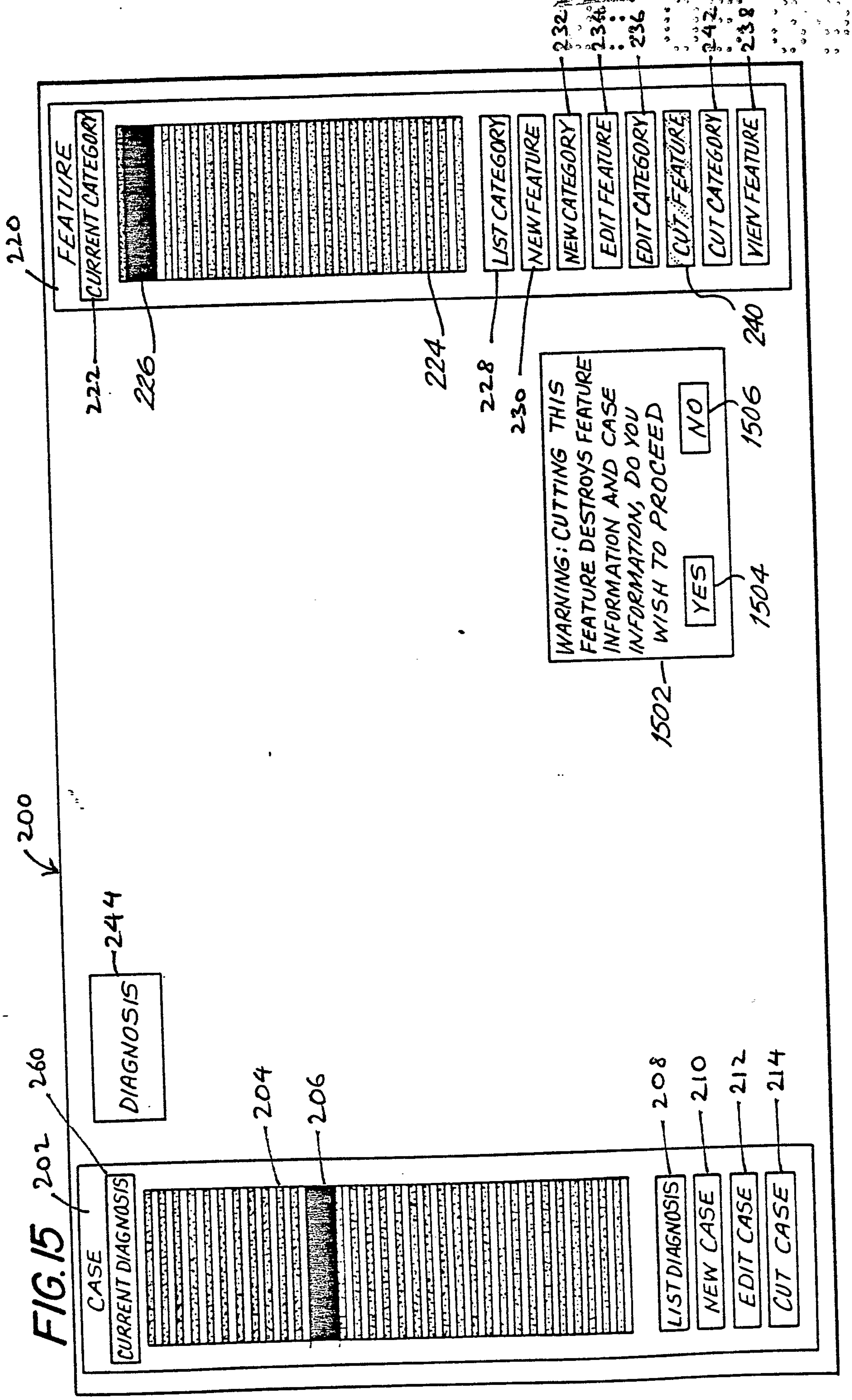

Nouvellement déposé

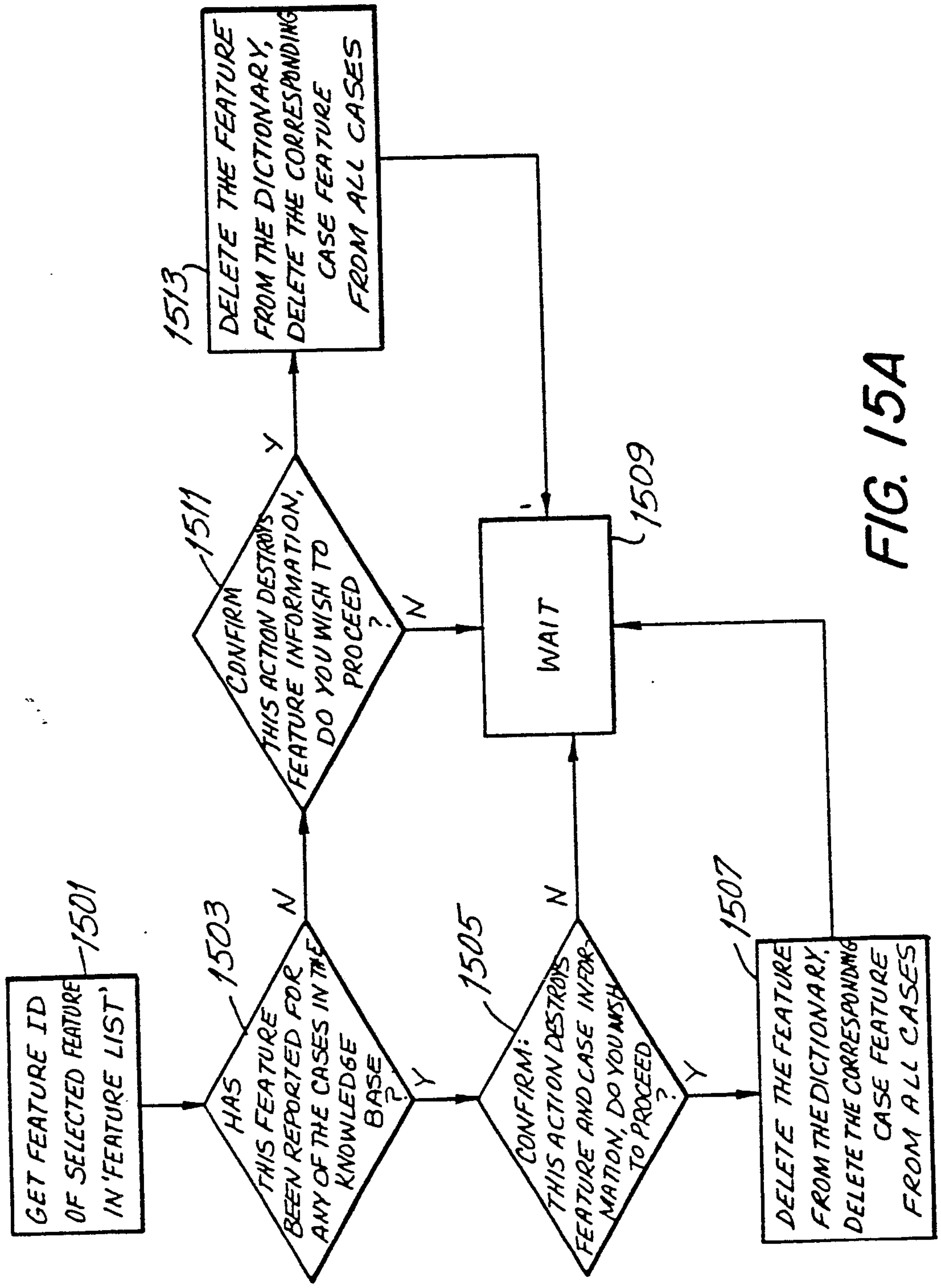


FIG. 15A

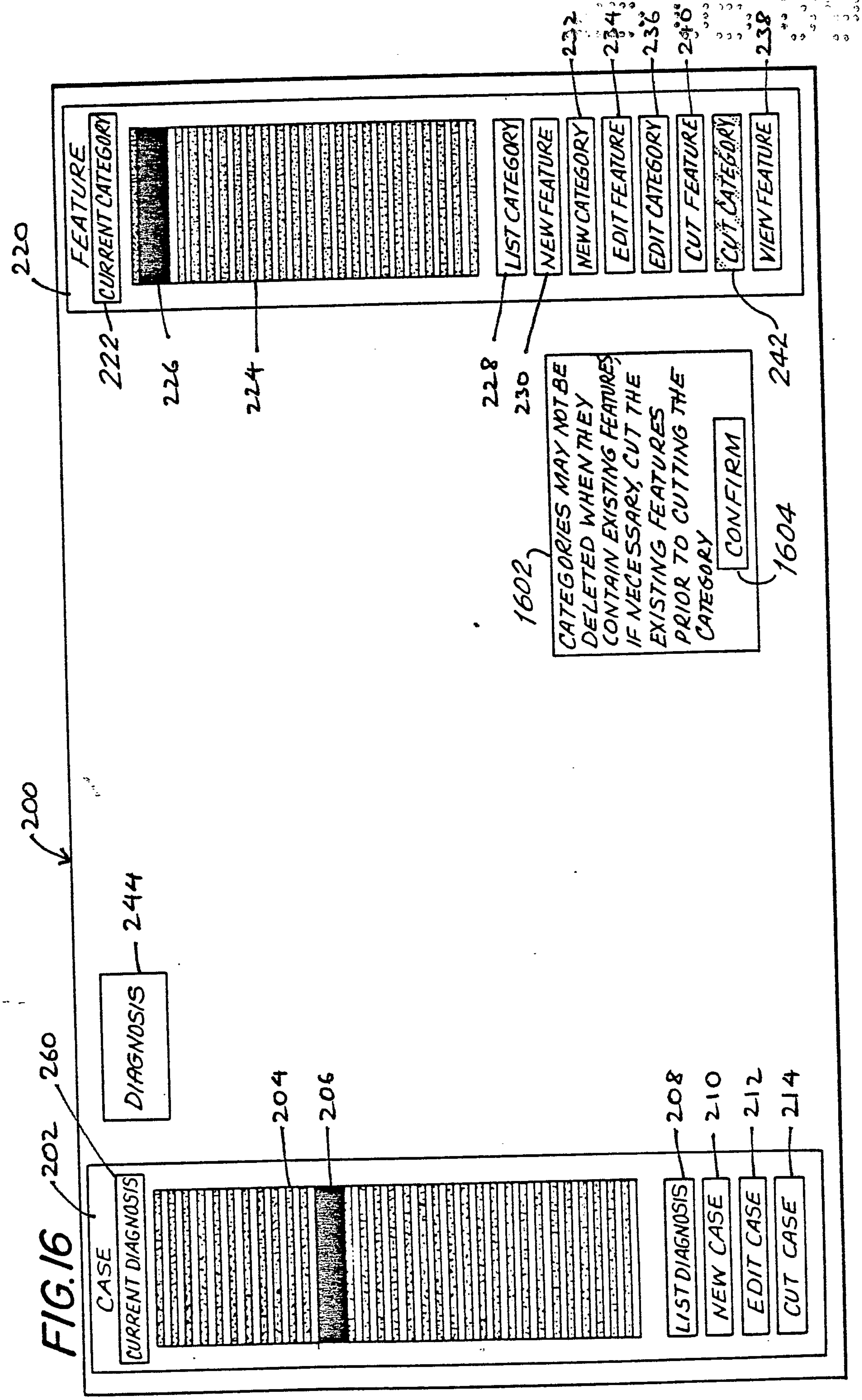
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
CATEGORIES MAY NOT BE DELETED WHEN THEY CONTAIN EXISTING FEATURES, IF NECESSARY, CUT THE EXISTING FEATURES PRIOR TO CUTTING THE CATEGORY
CONFIRM
DIAGNOSIS
CASE
CURRENT DIAGNOSIS
LIST DIAGNOSIS
NEW CASE
EDIT CASE
CUT CASE
200
202
204
206
208
210
212
214
220
222
224
226
228
230
232
234
236
238
240
242
244
260
1602
1604

FIG.16

Nouvellement déposé

FIG. 16A

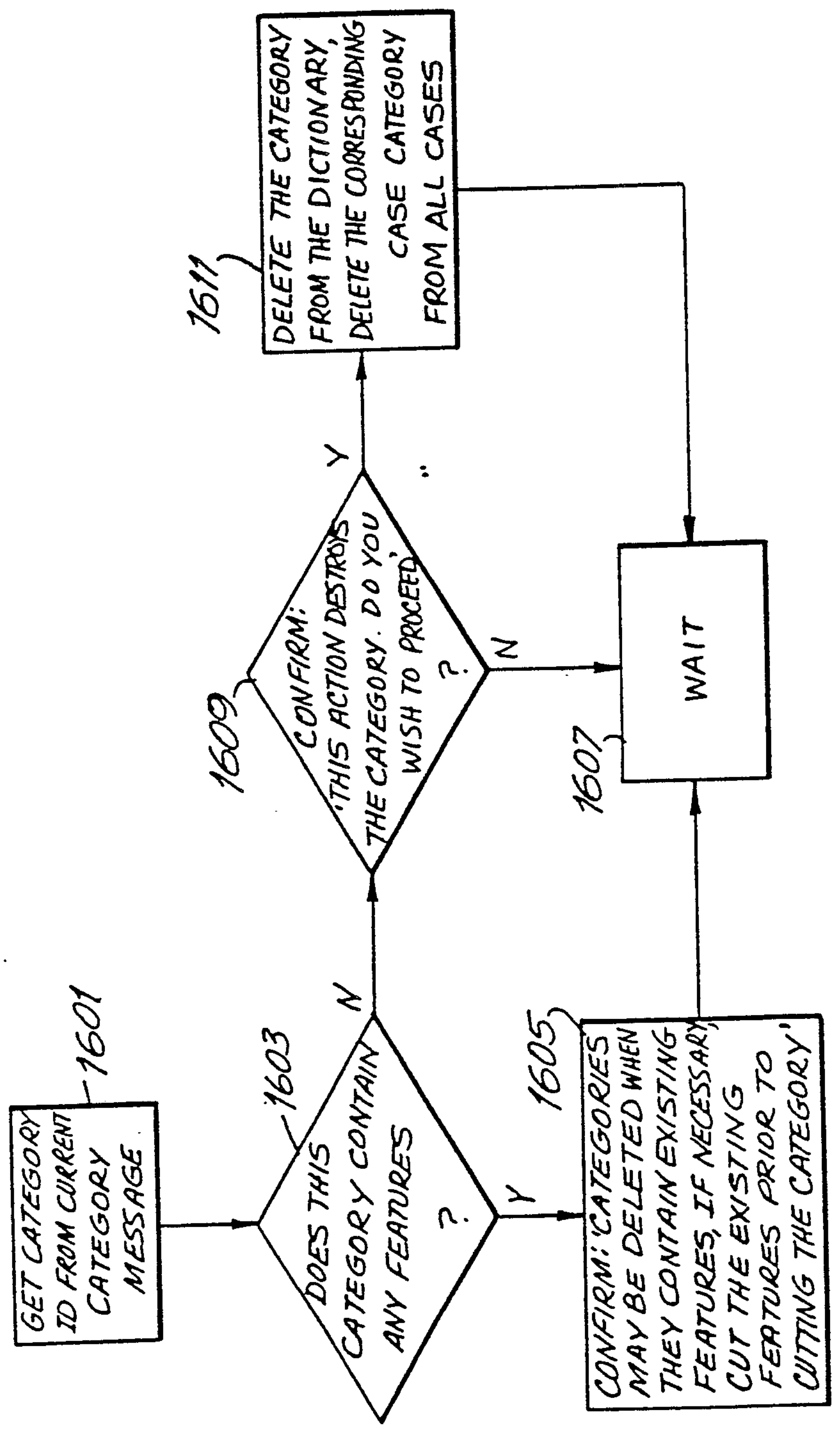

Nouvellement déposé

FIG. 17-1

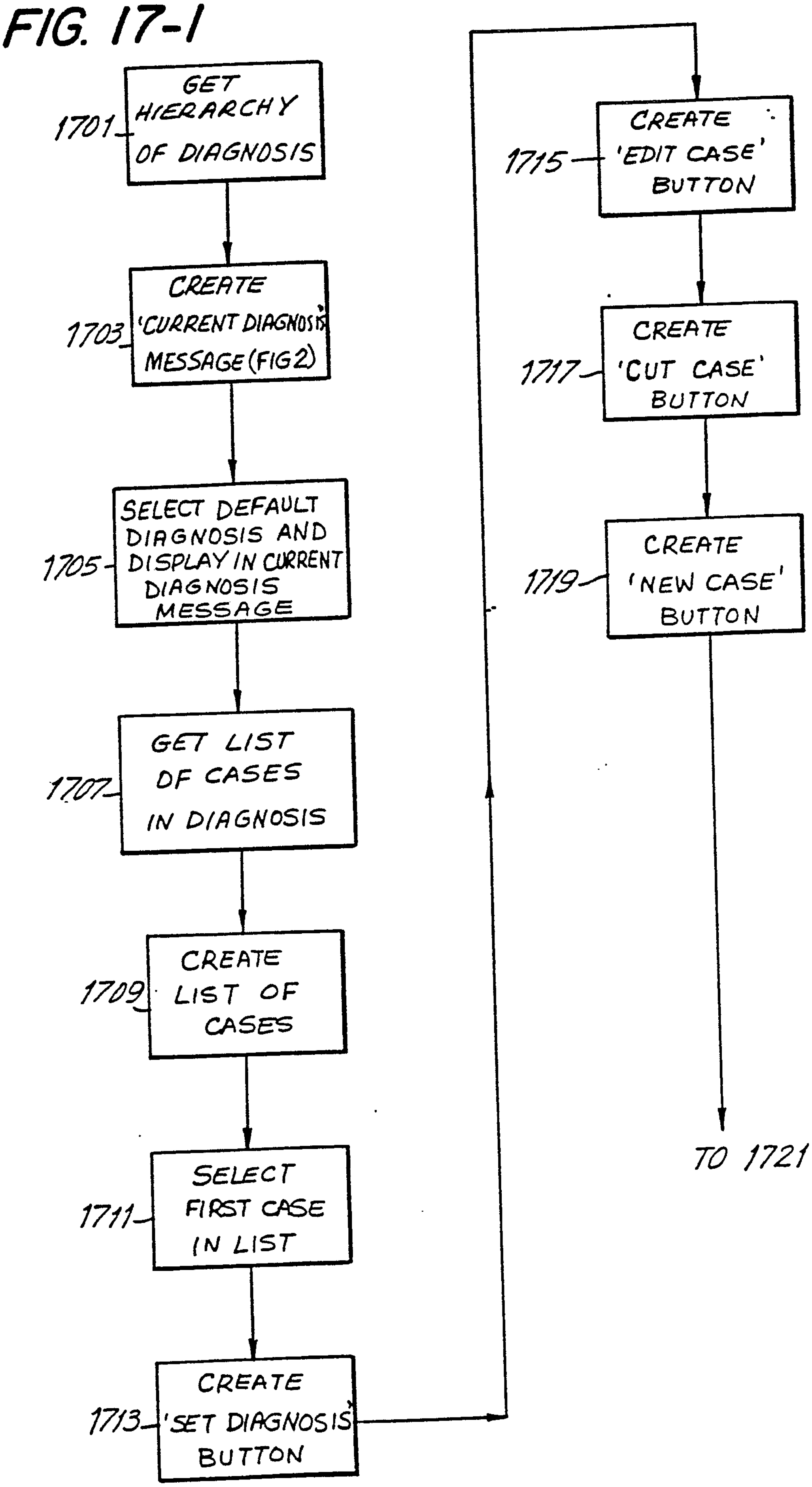

Nouvellement déposé

FIG. 17-2

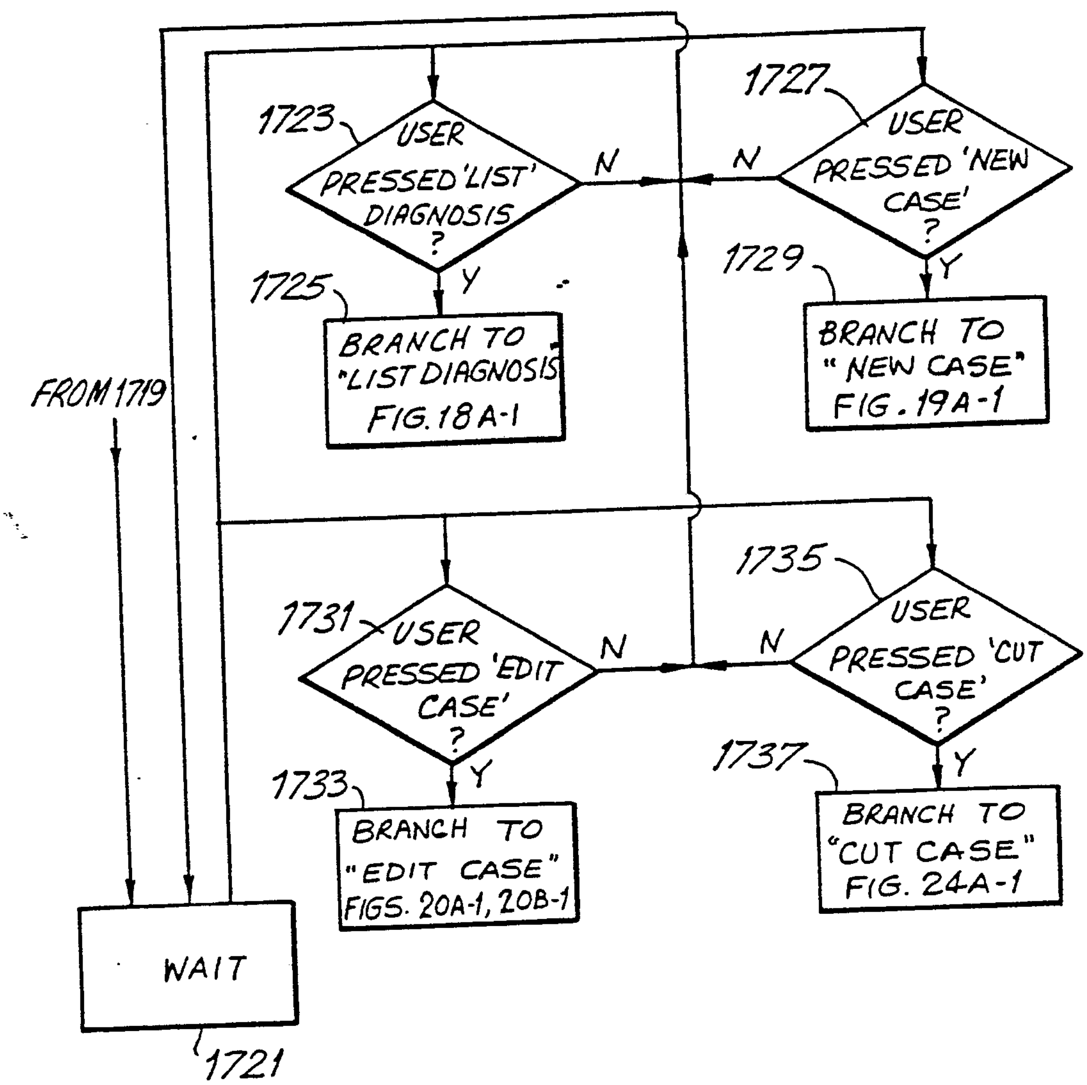

FIG. 18

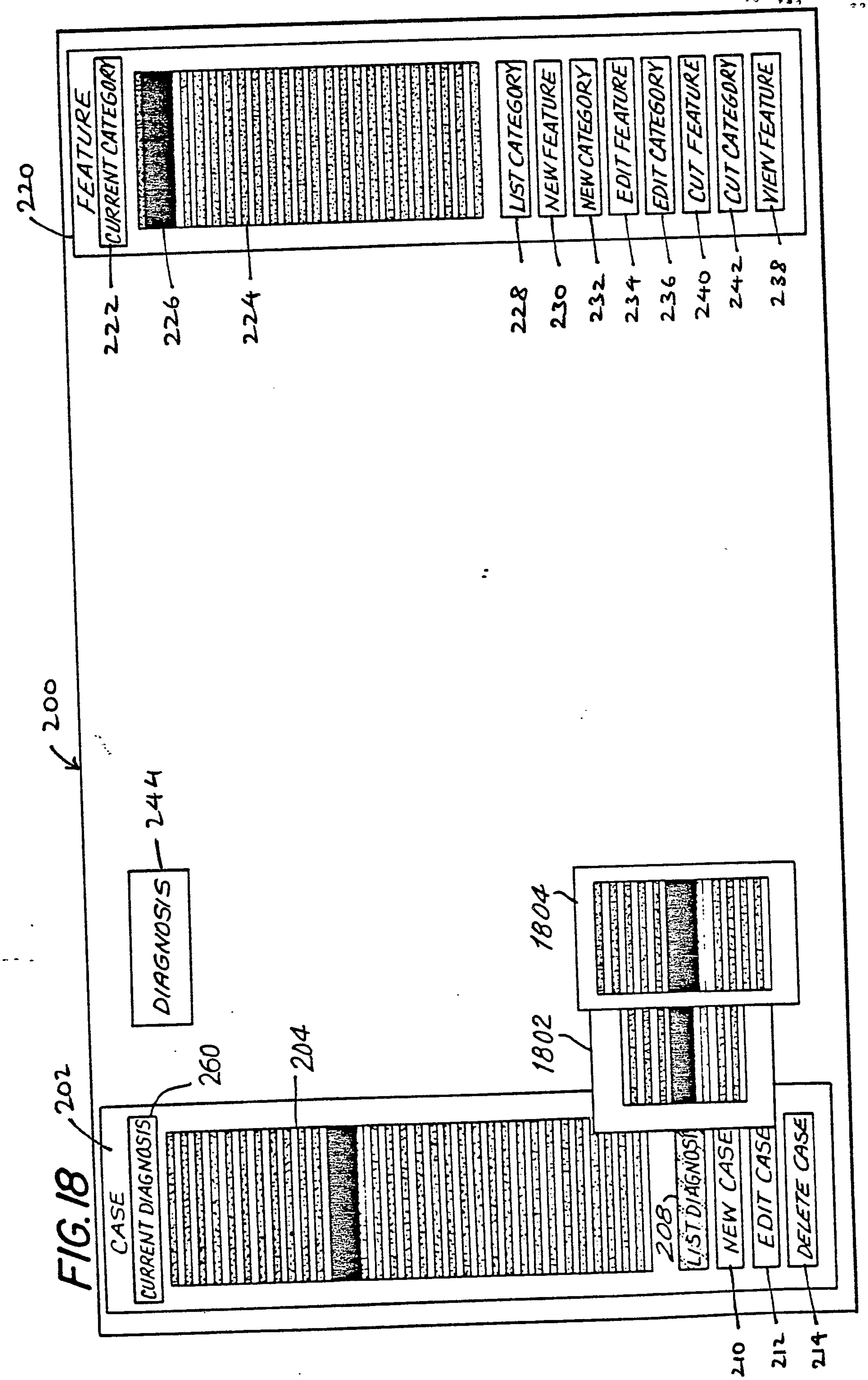

FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
220
222
226
224
228
230
232
234
236
240
242
238
200
DIAGNOSIS
244
1804
1802
CASE
CURRENT DIAGNOSIS
LIST DIAGNOSIS
NEW CASE
EDIT CASE
DELETE CASE
202
260
204
208
210
212
214

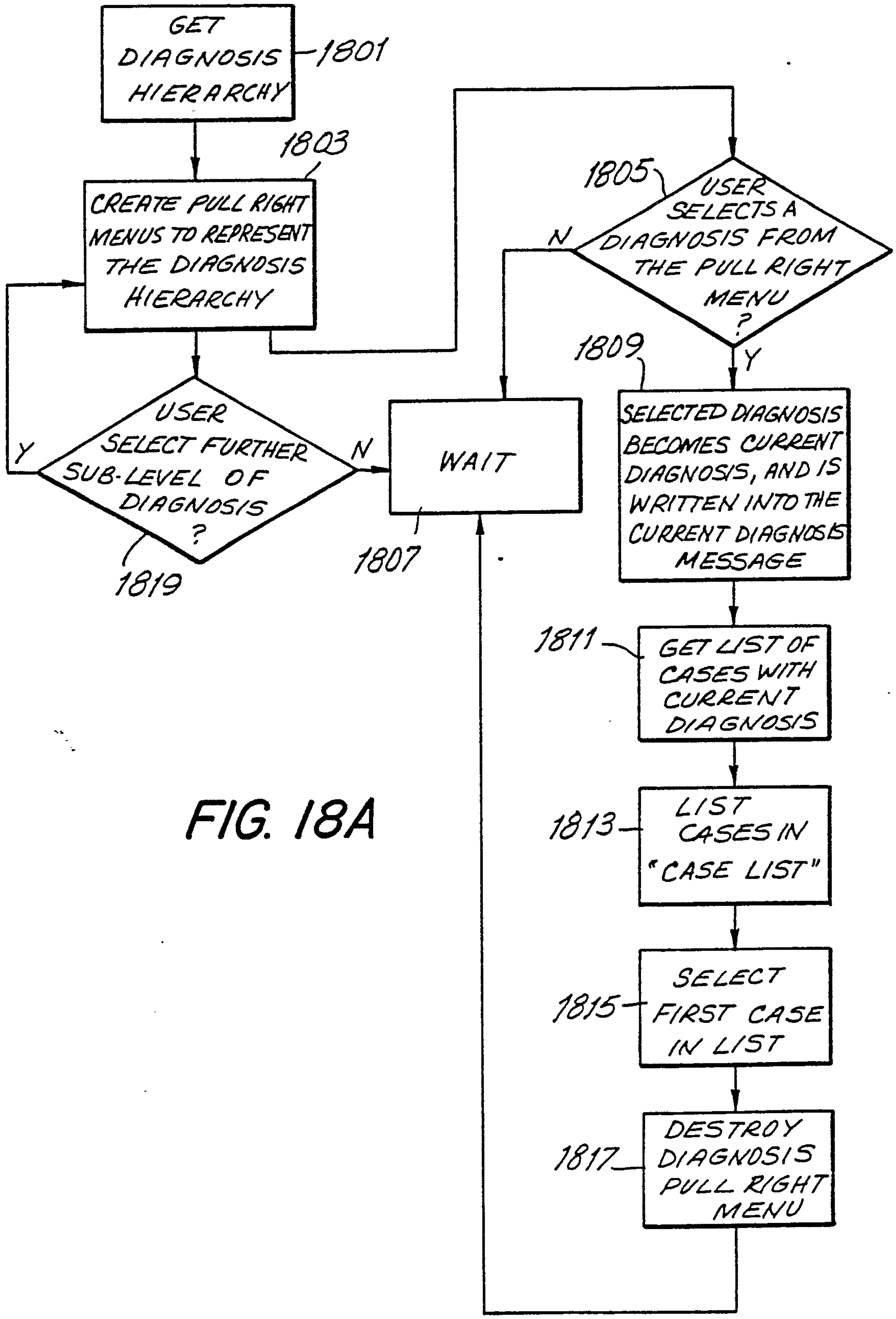
GET DIAGNOSIS HIERARCHY
1801
1803
CREATE PULL RIGHT MENUS TO REPRESENT THE DIAGNOSIS HIERARCHY
1805
USER SELECTS A DIAGNOSIS FROM THE PULL RIGHT MENU ?
N
Y
1809
USER SELECT FURTHER SUB-LEVEL OF DIAGNOSIS ?
Y
N
WAIT
SELECTED DIAGNOSIS BECOMES CURRENT DIAGNOSIS, AND IS WRITTEN INTO THE CURRENT DIAGNOSIS MESSAGE
1819
1807
1811
GET LIST OF CASES WITH CURRENT DIAGNOSIS
1813
LIST CASES IN "CASE LIST"
1815
SELECT FIRST CASE IN LIST
1817
DESTROY DIAGNOSIS PULL RIGHT MENU

FIG. 18A

Nouvellement déposé

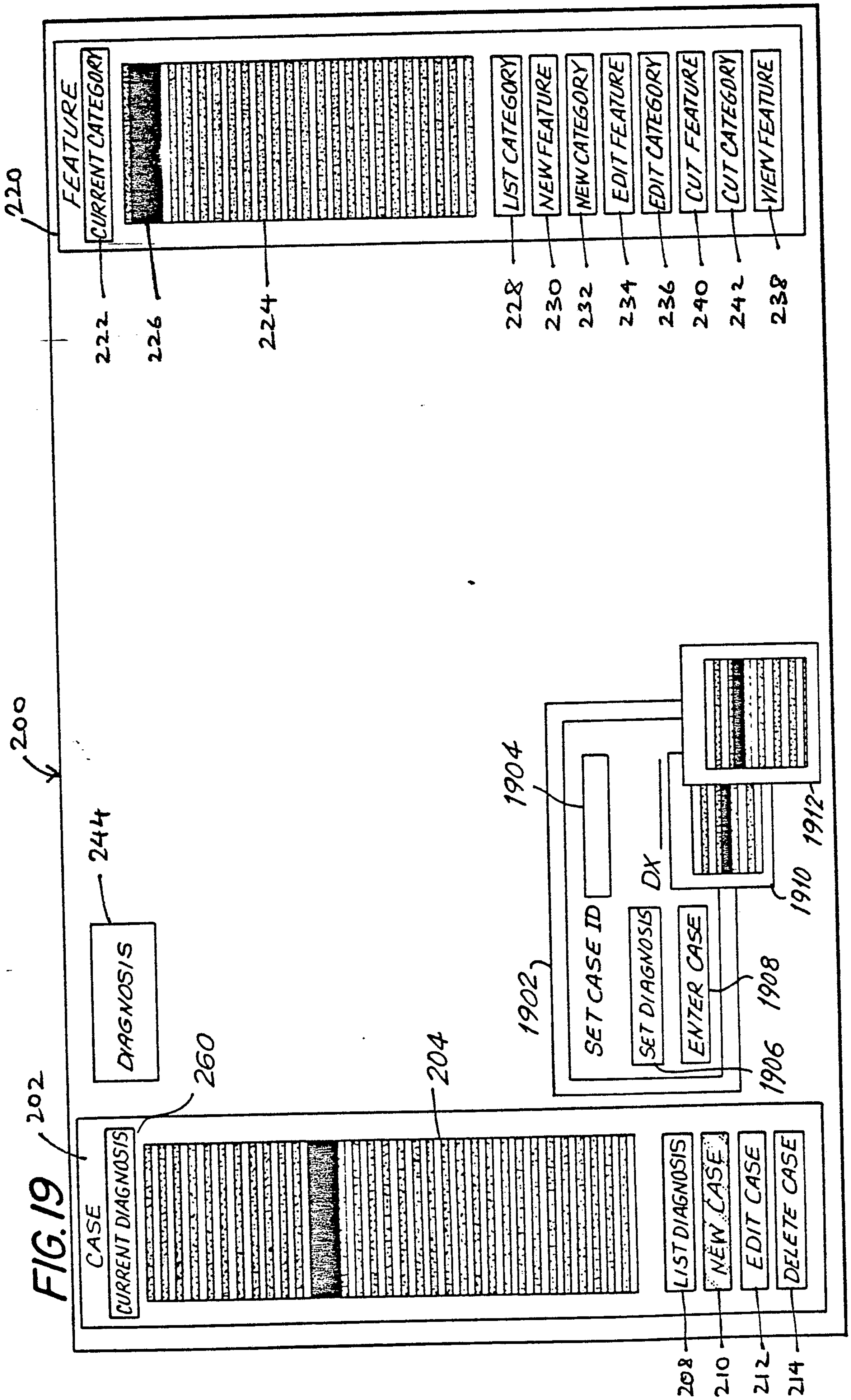


FIG.19

Nouvellement déposé

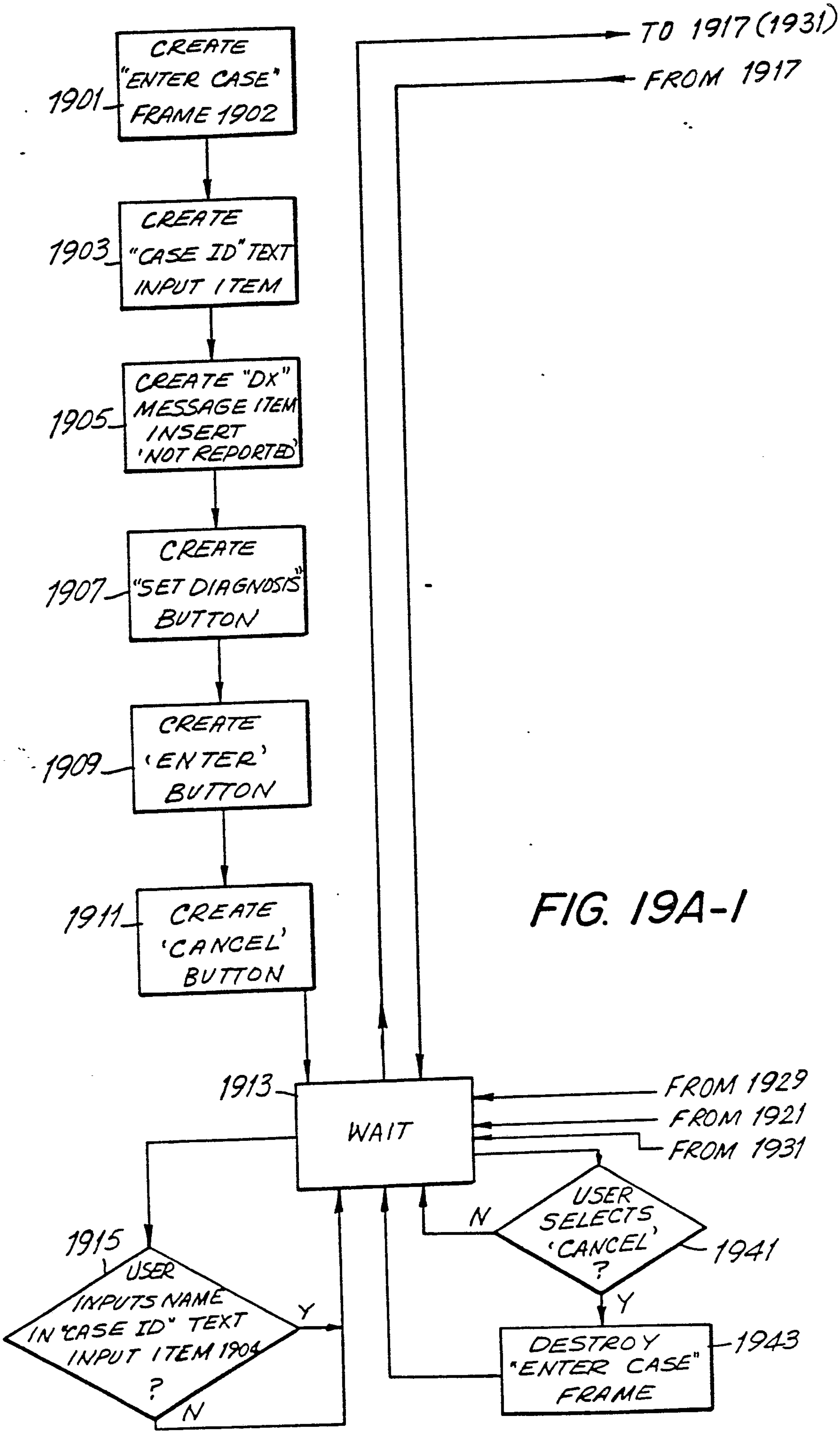


FIG. 19A-1

Nouvellement déposé

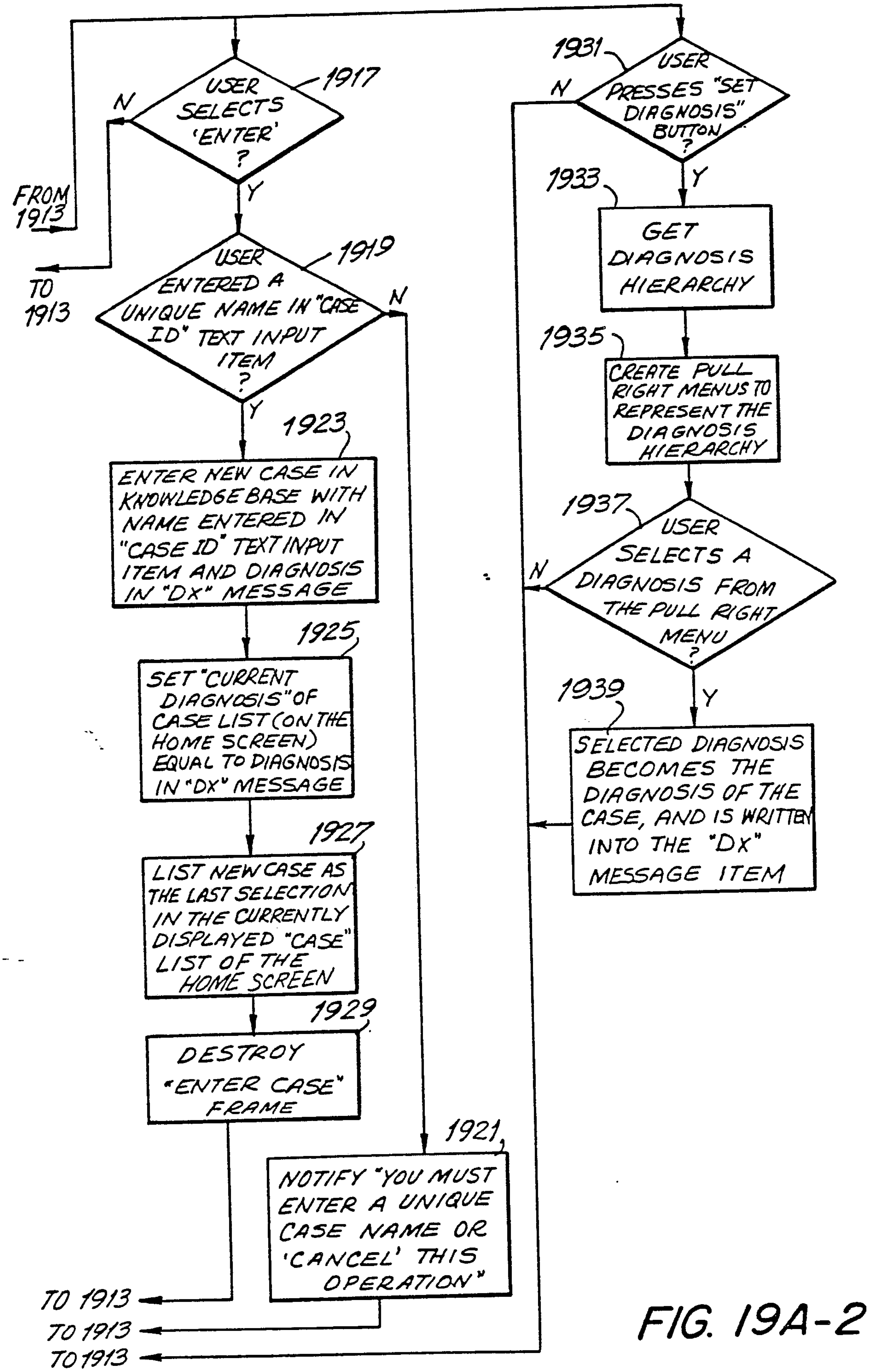


FIG. 19A-2

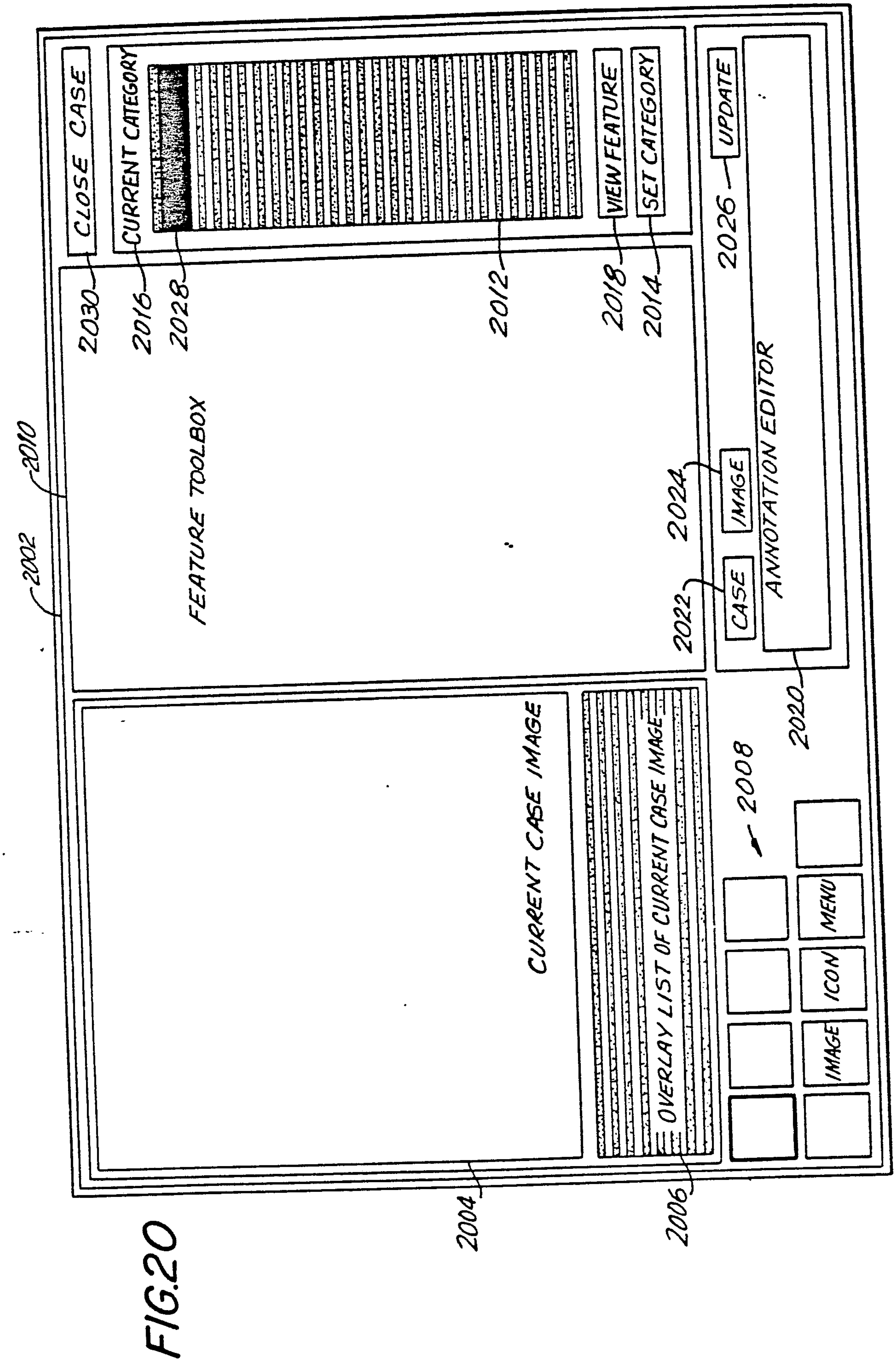
CLOSE CASE
CURRENT CATEGORY
VIEW FEATURE
SET CATEGORY
UPDATE
FEATURE TOOLBOX
CASE
IMAGE
ANNOTATION EDITOR
CURRENT CASE IMAGE
OVERLAY LIST OF CURRENT CASE IMAGE
IMAGE
ICON
MENU
2002
2004
2006
2008
2010
2012
2014
2016
2018
2020
2022
2024
2026
2028
2030

FIG.20

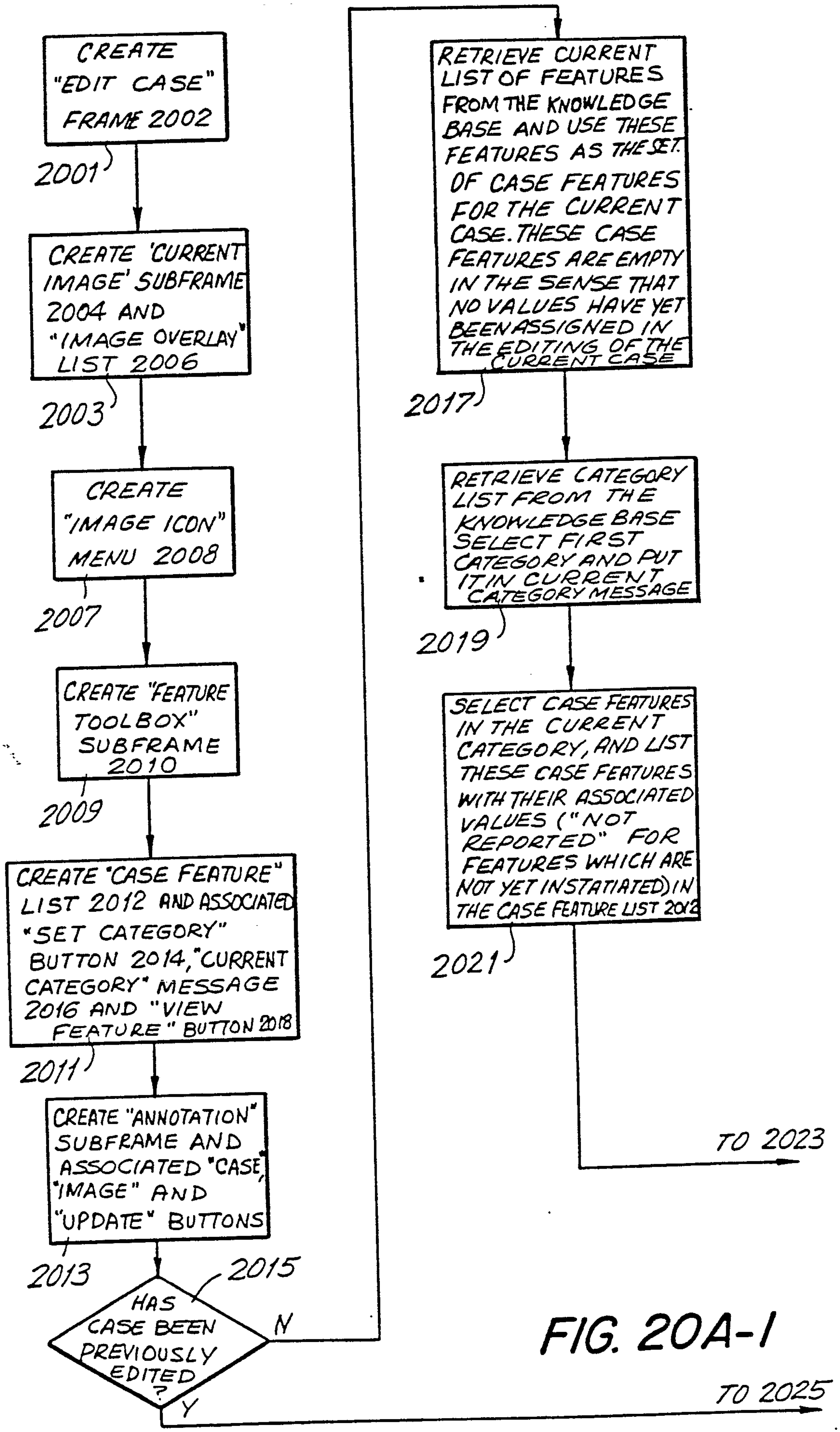
CREATE "EDIT CASE" FRAME 2002
2001
CREATE 'CURRENT IMAGE' SUBFRAME 2004 AND "IMAGE OVERLAY" LIST 2006
2003
CREATE "IMAGE ICON" MENU 2008
2007
CREATE "FEATURE TOOLBOX" SUBFRAME 2010
2009
CREATE "CASE FEATURE" LIST 2012 AND ASSOCIATED "SET CATEGORY" BUTTON 2014, "CURRENT CATEGORY" MESSAGE 2016 AND "VIEW FEATURE" BUTTON 2018
2011
CREATE "ANNOTATION" SUBFRAME AND ASSOCIATED "CASE", "IMAGE" AND "UPDATE" BUTTONS
2013
2015
HAS CASE BEEN PREVIOUSLY EDITED ?
N
Y
RETRIEVE CURRENT LIST OF FEATURES FROM THE KNOWLEDGE BASE AND USE THESE FEATURES AS THE SET OF CASE FEATURES FOR THE CURRENT CASE. THESE CASE FEATURES ARE EMPTY IN THE SENSE THAT NO VALUES HAVE YET BEEN ASSIGNED IN THE EDITING OF THE CURRENT CASE
2017
RETRIEVE CATEGORY LIST FROM THE KNOWLEDGE BASE SELECT FIRST CATEGORY AND PUT IT IN CURRENT CATEGORY MESSAGE
2019
SELECT CASE FEATURES IN THE CURRENT CATEGORY, AND LIST THESE CASE FEATURES WITH THEIR ASSOCIATED VALUES ("NOT REPORTED" FOR FEATURES WHICH ARE NOT YET INSTATIATED) IN THE CASE FEATURE LIST 2012
2021
TO 2023
TO 2025

FIG. 20A-1

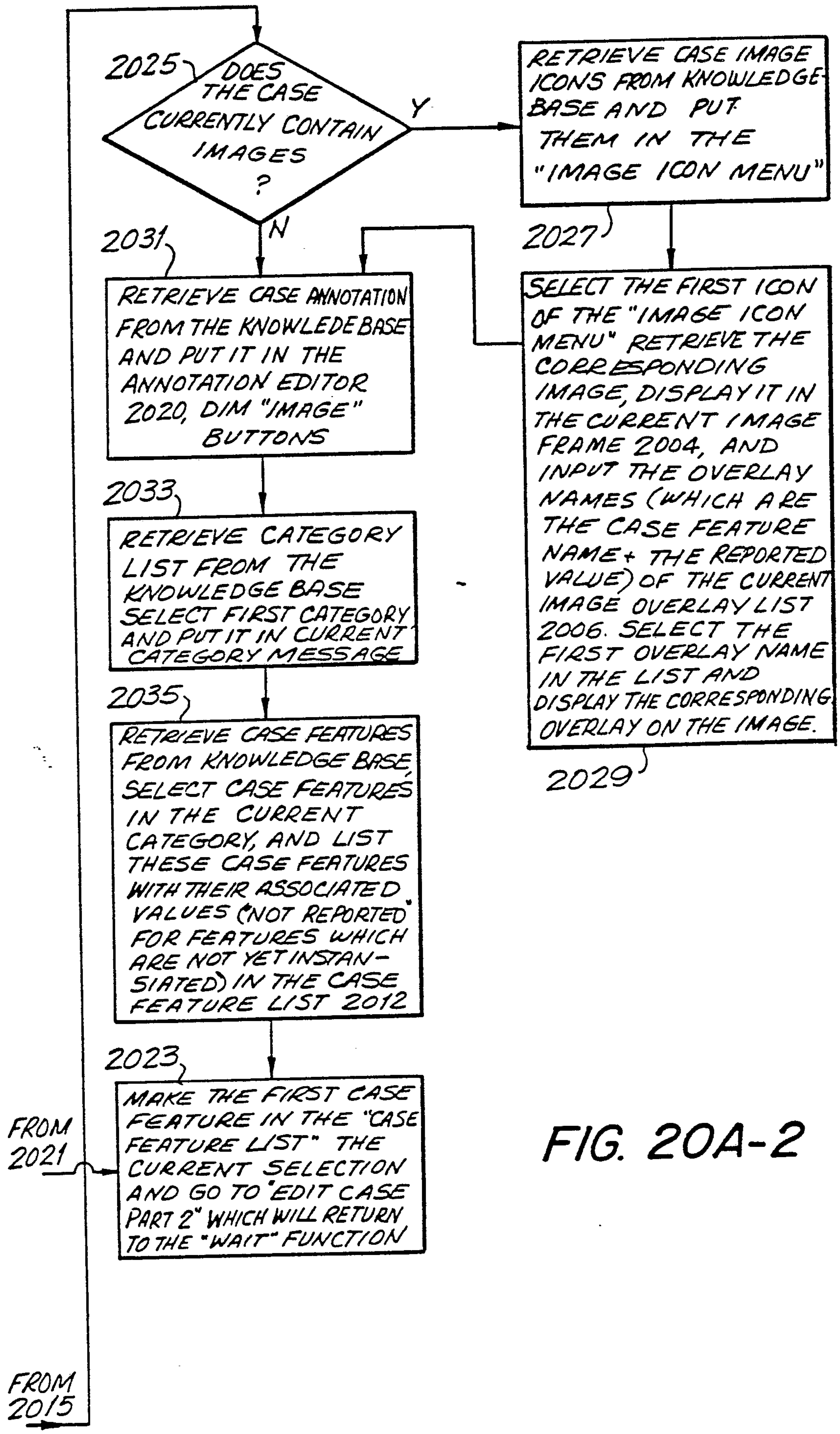
2025
DOES THE CASE CURRENTLY CONTAIN IMAGES ?
Y
N
RETRIEVE CASE IMAGE ICONS FROM KNOWLEDGE BASE AND PUT THEM IN THE "IMAGE ICON MENU"
2027
2031
RETRIEVE CASE ANNOTATION FROM THE KNOWLEDEBASE AND PUT IT IN THE ANNOTATION EDITOR 2020, DIM "IMAGE" BUTTONS
SELECT THE FIRST ICON OF THE "IMAGE ICON MENU" RETRIEVE THE CORRESPONDING IMAGE, DISPLAY IT IN THE CURRENT IMAGE FRAME 2004, AND INPUT THE OVERLAY NAMES (WHICH ARE THE CASE FEATURE NAME + THE REPORTED VALUE) OF THE CURRENT IMAGE OVERLAY LIST 2006. SELECT THE FIRST OVERLAY NAME IN THE LIST AND DISPLAY THE CORRESPONDING OVERLAY ON THE IMAGE.
2033
RETRIEVE CATEGORY LIST FROM THE KNOWLEDGE BASE SELECT FIRST CATEGORY AND PUT IT IN CURRENT CATEGORY MESSAGE
2035
RETRIEVE CASE FEATURES FROM KNOWLEDGE BASE, SELECT CASE FEATURES IN THE CURRENT CATEGORY, AND LIST THESE CASE FEATURES WITH THEIR ASSOCIATED VALUES ("NOT REPORTED" FOR FEATURES WHICH ARE NOT YET INSTANSIATED) IN THE CASE FEATURE LIST 2012
2029
2023
MAKE THE FIRST CASE FEATURE IN THE "CASE FEATURE LIST" THE CURRENT SELECTION AND GO TO "EDIT CASE PART 2" WHICH WILL RETURN TO THE "WAIT" FUNCTION
FROM 2021
FROM 2015

FIG. 20A-2

FIG. 20B-1

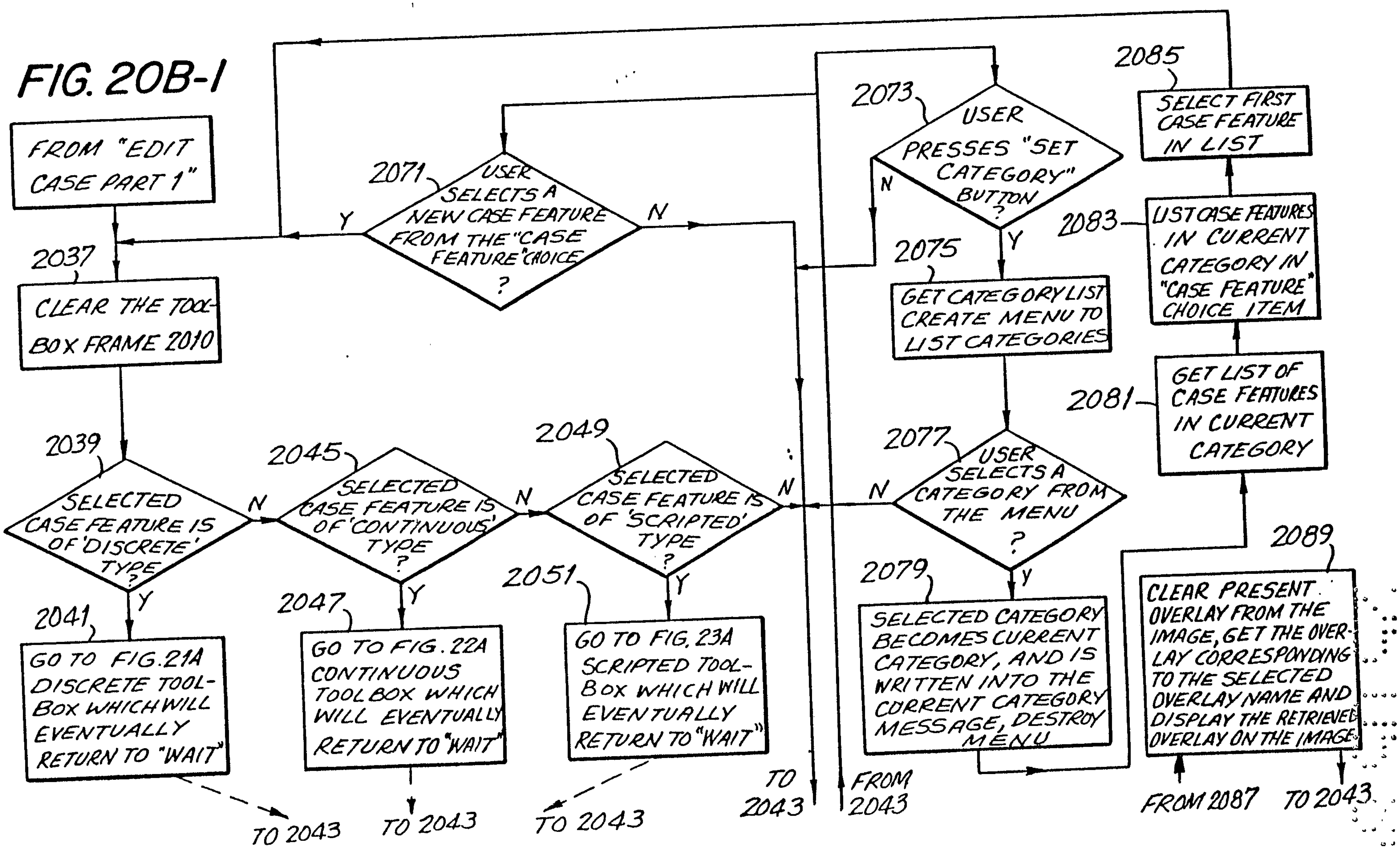
FROM "EDIT CASE PART 1"
2037
CLEAR THE TOOL-BOX FRAME 2010
2071
USER SELECTS A NEW CASE FEATURE FROM THE "CASE FEATURE" CHOICE ?
Y
N
2073
USER PRESSES "SET CATEGORY" BUTTON ?
N
Y
2075
GET CATEGORY LIST CREATE MENU TO LIST CATEGORIES
2077
USER SELECTS A CATEGORY FROM THE MENU ?
N
Y
2079
SELECTED CATEGORY BECOMES CURRENT CATEGORY, AND IS WRITTEN INTO THE CURRENT CATEGORY MESSAGE, DESTROY MENU
2081
GET LIST OF CASE FEATURES IN CURRENT CATEGORY
2083
LIST CASE FEATURES IN CURRENT CATEGORY IN "CASE FEATURE" CHOICE ITEM
2085
SELECT FIRST CASE FEATURE IN LIST
2089
CLEAR PRESENT OVERLAY FROM THE IMAGE, GET THE OVERLAY CORRESPONDING TO THE SELECTED OVERLAY NAME AND DISPLAY THE RETRIEVED OVERLAY ON THE IMAGE
FROM 2087
TO 2043
2039
SELECTED CASE FEATURE IS OF 'DISCRETE' TYPE ?
N
Y
2041
GO TO FIG.21A DISCRETE TOOL-BOX WHICH WILL EVENTUALLY RETURN TO "WAIT"
TO 2043
2045
SELECTED CASE FEATURE IS OF 'CONTINUOUS' TYPE ?
N
Y
2047
GO TO FIG.22A CONTINUOUS TOOLBOX WHICH WILL EVENTUALLY RETURN TO "WAIT"
TO 2043
2049
SELECTED CASE FEATURE IS OF 'SCRIPTED' TYPE ?
N
Y
2051
GO TO FIG. 23A SCRIPTED TOOL-BOX WHICH WILL EVENTUALLY RETURN TO "WAIT"
TO 2043
TO 2043
FROM 2043

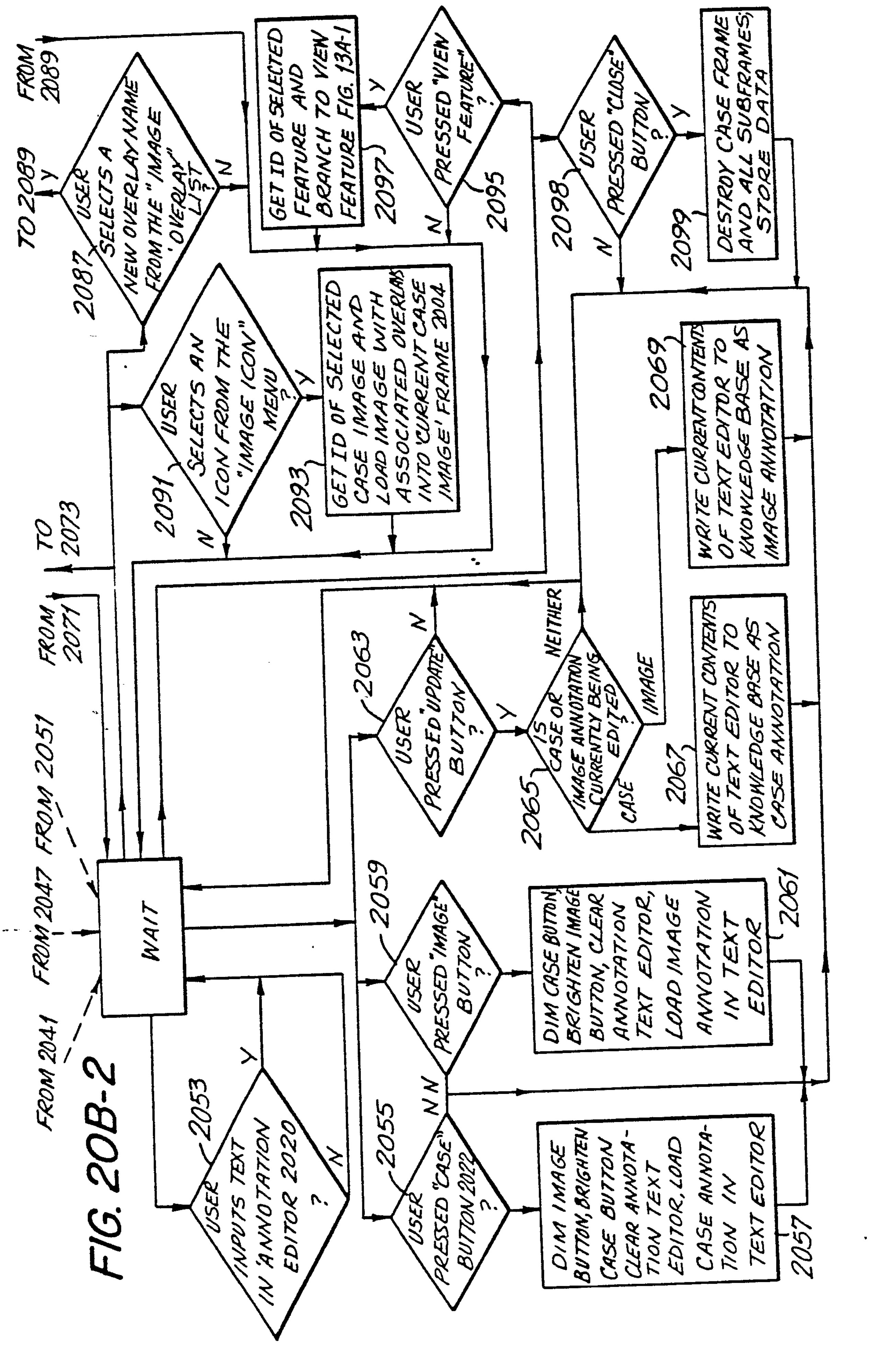
FROM 2041
FROM 2047
FROM 2051
FROM 2071
TO 2073
TO 2089
FROM 2089
WAIT
USER INPUTS TEXT IN 'ANNOTATION EDITOR 2020 ?
2053
Y
N
USER PRESSED "CASE" BUTTON 2022 ?
2055
N
DIM IMAGE BUTTON, BRIGHTEN CASE BUTTON CLEAR CASE ANNOTATION TEXT EDITOR, LOAD CASE ANNOTATION IN TEXT EDITOR
2057
USER PRESSED "IMAGE" BUTTON ?
2059
N
DIM CASE BUTTON, BRIGHTEN IMAGE BUTTON, CLEAR ANNOTATION TEXT EDITOR, LOAD IMAGE ANNOTATION IN TEXT EDITOR
2061
USER PRESSED "UPDATE" BUTTON ?
2063
Y
N
IS CASE OR IMAGE ANNOTATION CURRENTLY BEING EDITED ?
2065
CASE
IMAGE
NEITHER
WRITE CURRENT CONTENTS OF TEXT EDITOR TO KNOWLEDGE BASE AS CASE ANNOTATION
2067
WRITE CURRENT CONTENTS OF TEXT EDITOR TO KNOWLEDGE BASE AS IMAGE ANNOTATION
2069
USER SELECTS AN ICON FROM THE "IMAGE ICON" MENU ?
2091
Y
N
GET ID OF SELECTED CASE IMAGE AND LOAD IMAGE WITH ASSOCIATED OVERLAYS INTO 'CURRENT CASE IMAGE' FRAME 2004
2093
USER SELECTS A NEW OVERLAY NAME FROM THE "IMAGE 'OVERLAY' LIST ?
2087
Y
N
GET ID OF SELECTED FEATURE AND BRANCH TO VIEW FEATURE FIG. 13A-1
2097
USER PRESSED "VIEW FEATURE" ?
2095
Y
N
USER PRESSED "CLOSE" BUTTON ?
2098
Y
N
DESTROY CASE FRAME AND ALL SUBFRAMES; STORE DATA
2099

FIG. 20B-2

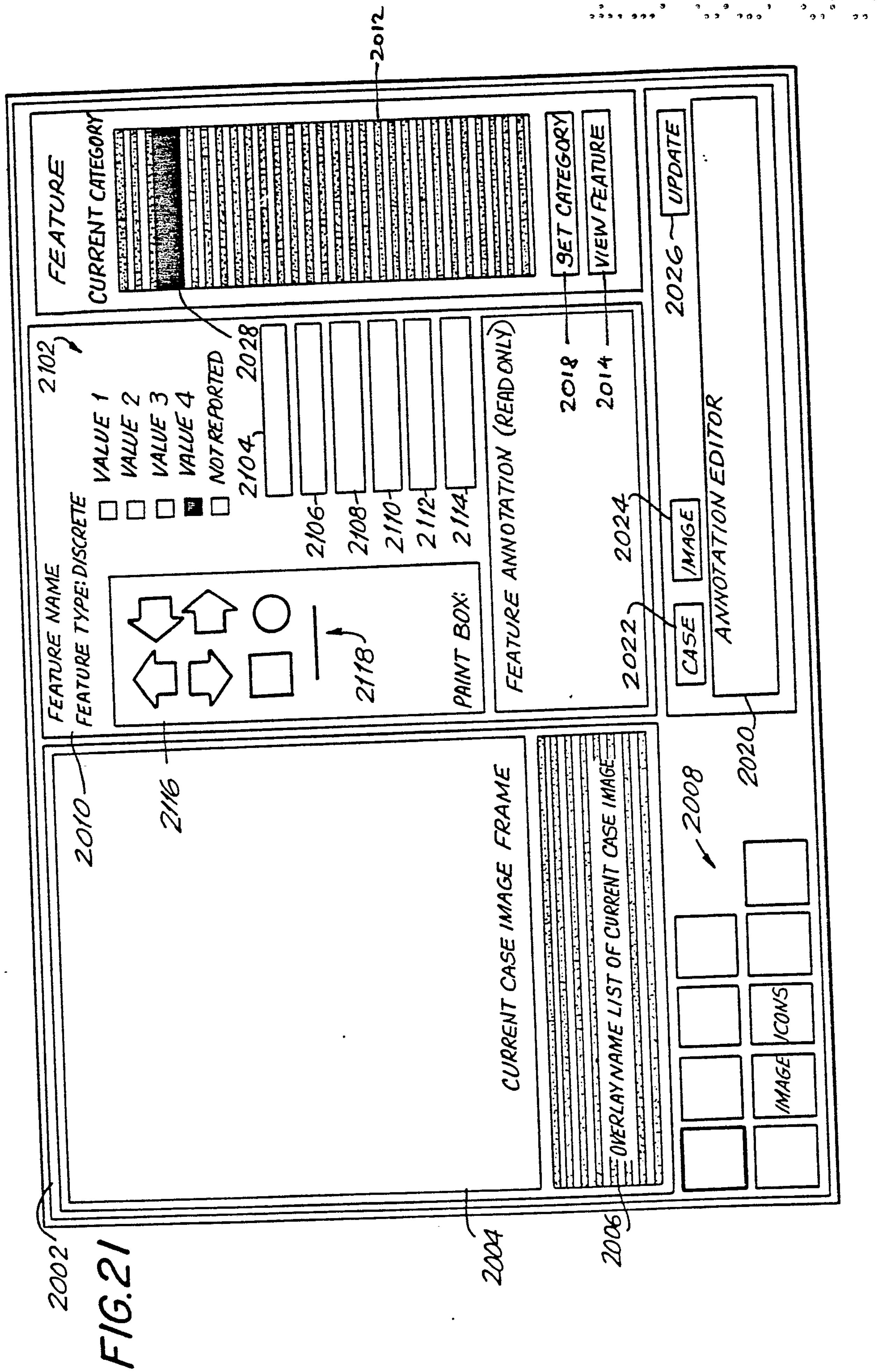
FEATURE
CURRENT CATEGORY
GET CATEGORY
VIEW FEATURE
UPDATE
FEATURE NAME
FEATURE TYPE: DISCRETE
VALUE 1
VALUE 2
VALUE 3
VALUE 4
NOT REPORTED
PAINT BOX:
FEATURE ANNOTATION (READ ONLY)
CASE
IMAGE
ANNOTATION EDITOR
CURRENT CASE IMAGE FRAME
OVERLAY NAME LIST OF CURRENT CASE IMAGE
IMAGE ICONS
2002
2004
2006
2008
2010
2012
2014
2018
2020
2022
2024
2026
2028
2102
2104
2106
2108
2110
2112
2114
2116
2118

FIG.21

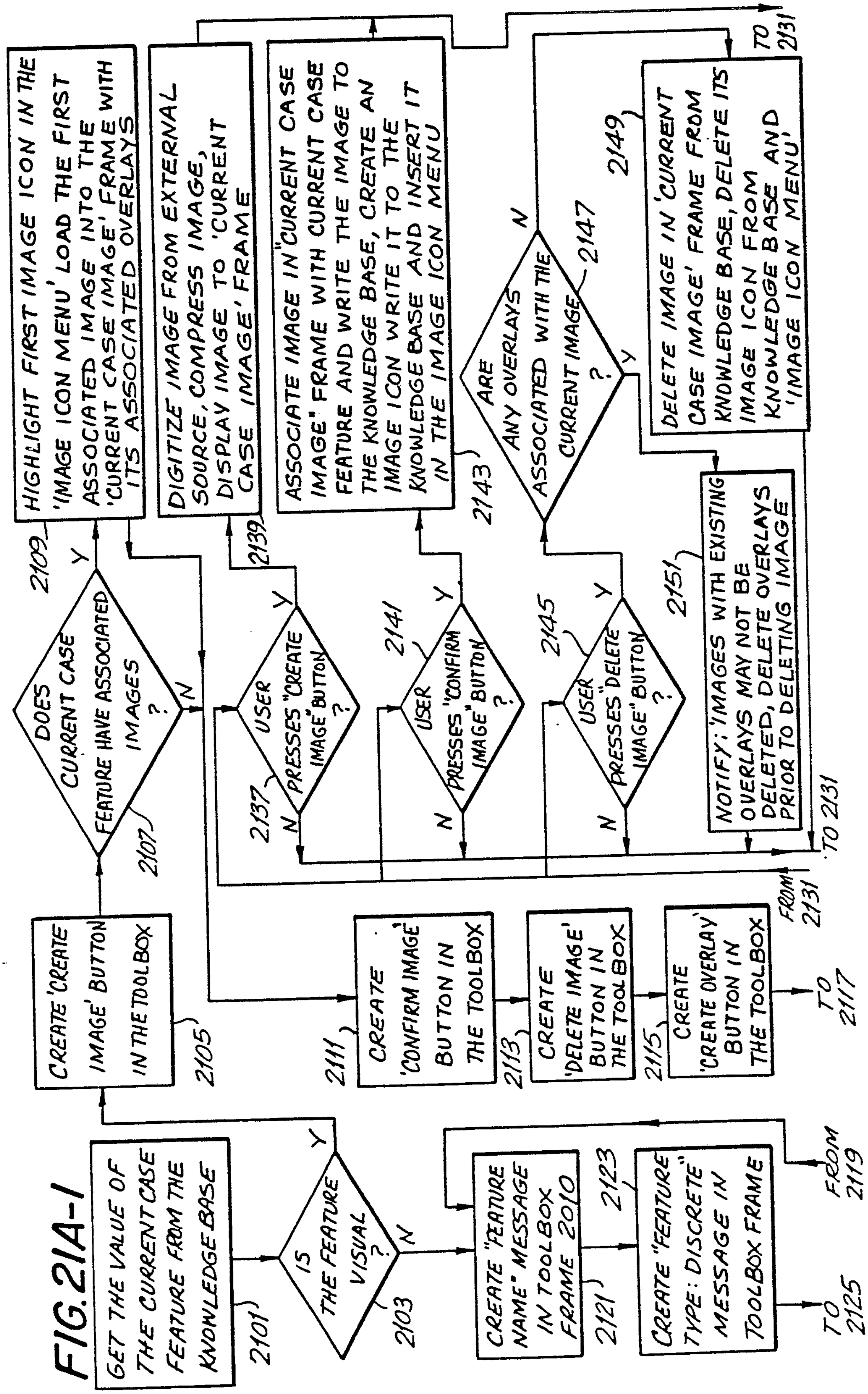
GET THE VALUE OF THE CURRENT CASE FEATURE FROM THE KNOWLEDGE BASE
2101
IS THE FEATURE VISUAL ?
2103
Y
N
CREATE 'CREATE IMAGE' BUTTON IN THE TOOLBOX
2105
DOES CURRENT CASE FEATURE HAVE ASSOCIATED IMAGES ?
2107
Y
N
HIGHLIGHT FIRST IMAGE ICON IN THE 'IMAGE ICON MENU' LOAD THE FIRST ASSOCIATED IMAGE INTO THE 'CURRENT CASE IMAGE' FRAME WITH ITS ASSOCIATED OVERLAYS
2109
CREATE 'CONFIRM IMAGE' BUTTON IN THE TOOLBOX
2111
CREATE 'DELETE IMAGE' BUTTON IN THE TOOLBOX
2113
CREATE 'CREATE OVERLAY' BUTTON IN THE TOOLBOX
2115
TO 2117
FROM 2131
CREATE "FEATURE NAME" MESSAGE IN TOOLBOX FRAME 2010
2121
CREATE "FEATURE TYPE: DISCRETE" MESSAGE IN TOOLBOX FRAME
2123
TO 2125
FROM 2119
USER PRESSES "CREATE IMAGE" BUTTON ?
2137
Y
N
DIGITIZE IMAGE FROM EXTERNAL SOURCE, COMPRESS IMAGE, DISPLAY IMAGE TO 'CURRENT CASE IMAGE' FRAME
2139
USER PRESSES "CONFIRM IMAGE" BUTTON ?
2141
Y
N
ASSOCIATE IMAGE IN "CURRENT CASE IMAGE" FRAME WITH CURRENT CASE FEATURE AND WRITE THE IMAGE TO THE KNOWLEDGE BASE, CREATE AN IMAGE ICON WRITE IT TO THE KNOWLEDGE BASE AND INSERT IT IN THE IMAGE ICON MENU
2143
USER PRESSES "DELETE IMAGE" BUTTON ?
2145
Y
N
ARE ANY OVERLAYS ASSOCIATED WITH THE CURRENT IMAGE ?
2147
Y
N
DELETE IMAGE IN 'CURRENT CASE IMAGE' FRAME FROM KNOWLEDGE BASE, DELETE ITS IMAGE ICON FROM KNOWLEDGE BASE AND 'IMAGE ICON MENU'
2149
NOTIFY: 'IMAGES WITH EXISTING OVERLAYS MAY NOT BE DELETED, DELETE OVERLAYS PRIOR TO DELETING IMAGE
2151
TO 2131
TO 2131

FIG.21A-1

Nouvellement déposé
25 05 89

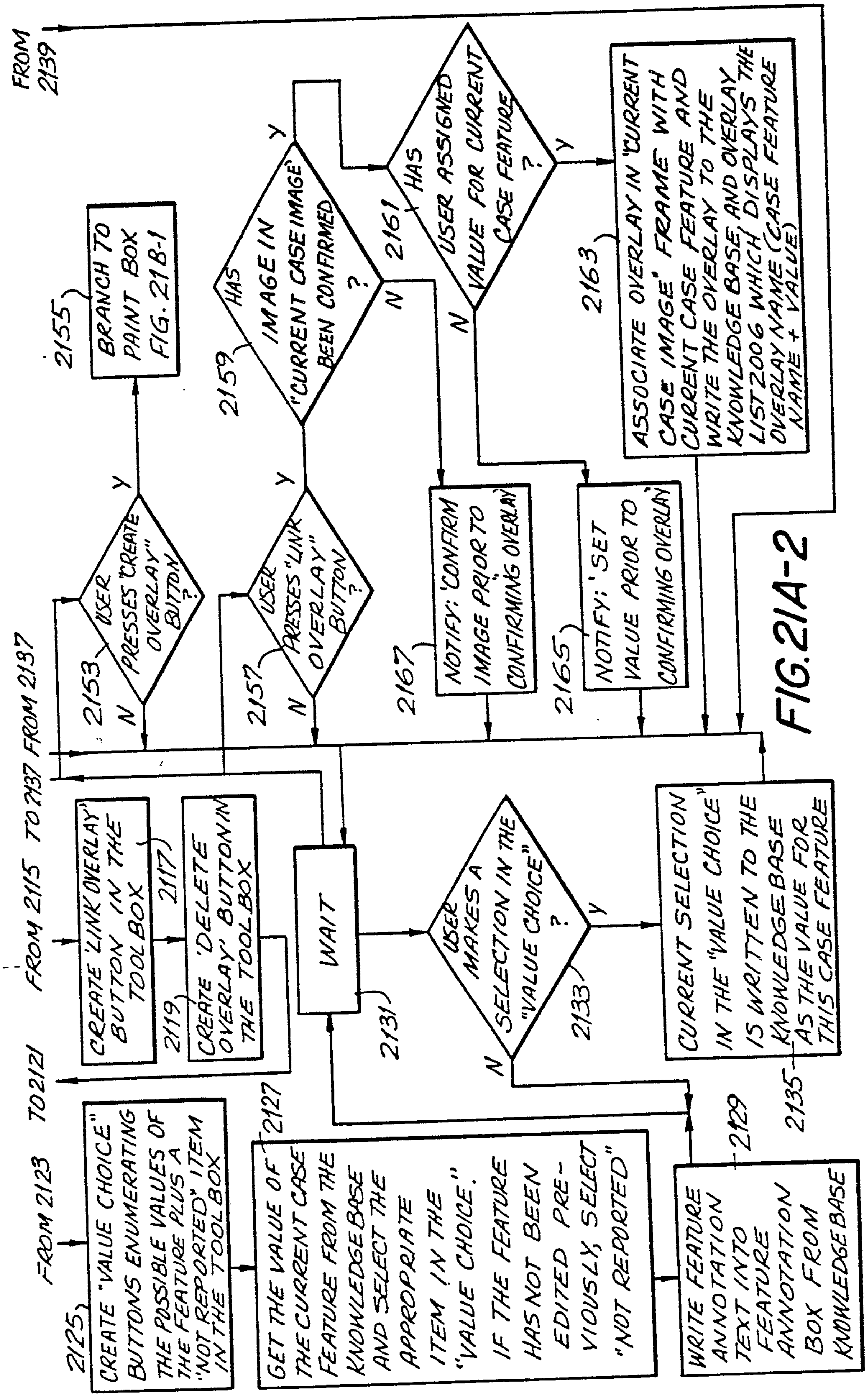


FIG.21A-2

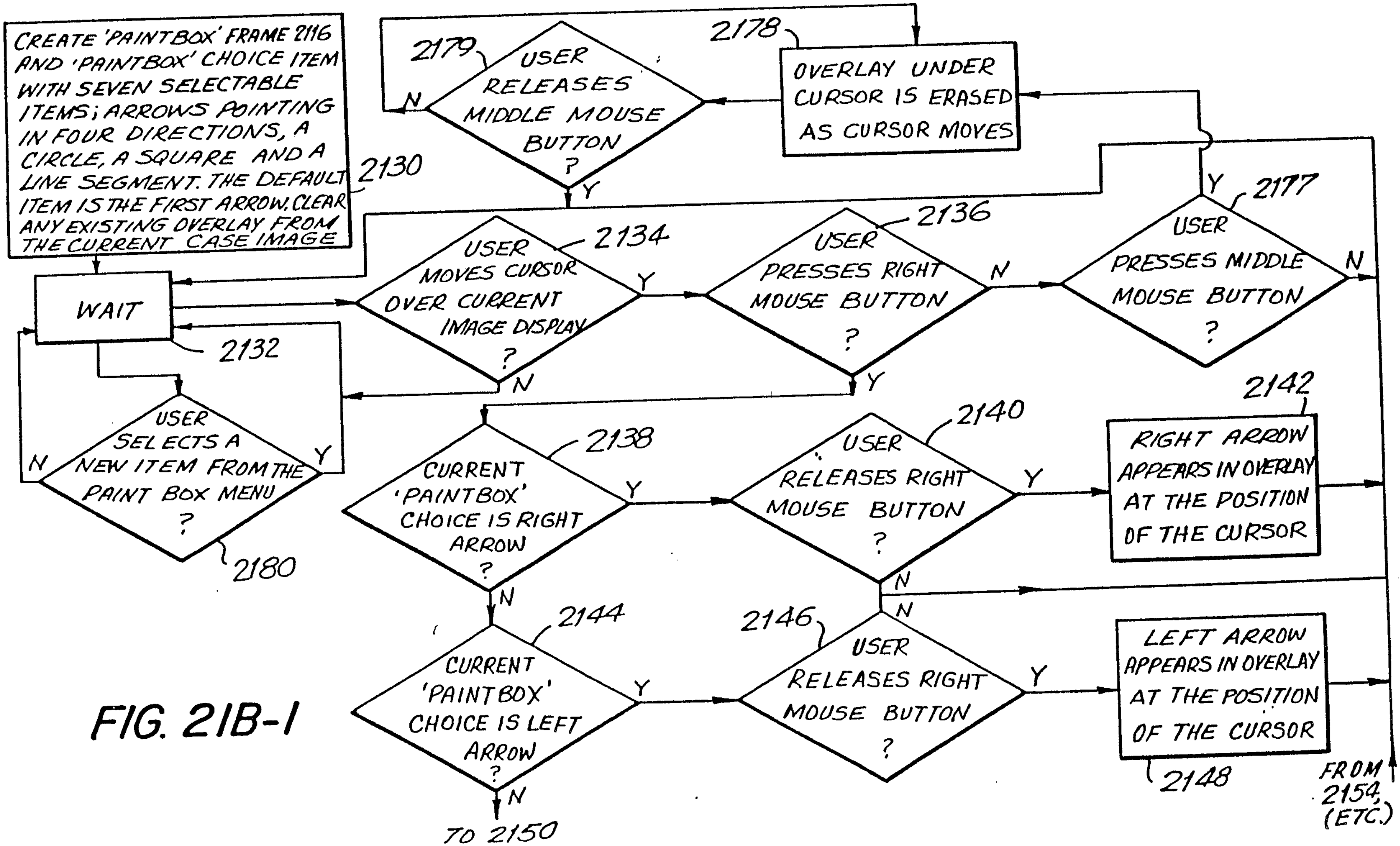
CREATE 'PAINTBOX' FRAME 2116 AND 'PAINTBOX' CHOICE ITEM WITH SEVEN SELECTABLE ITEMS; ARROWS POINTING IN FOUR DIRECTIONS, A CIRCLE, A SQUARE AND A LINE SEGMENT. THE DEFAULT ITEM IS THE FIRST ARROW. CLEAR ANY EXISTING OVERLAY FROM THE CURRENT CASE IMAGE
2130
WAIT
2132
USER SELECTS A NEW ITEM FROM THE PAINT BOX MENU ?
2180
USER RELEASES MIDDLE MOUSE BUTTON ?
2179
OVERLAY UNDER CURSOR IS ERASED AS CURSOR MOVES
2178
USER MOVES CURSOR OVER CURRENT IMAGE DISPLAY ?
2134
USER PRESSES RIGHT MOUSE BUTTON ?
2136
USER PRESSES MIDDLE MOUSE BUTTON ?
2177
CURRENT 'PAINTBOX' CHOICE IS RIGHT ARROW ?
2138
USER RELEASES RIGHT MOUSE BUTTON ?
2140
RIGHT ARROW APPEARS IN OVERLAY AT THE POSITION OF THE CURSOR
2142
CURRENT 'PAINTBOX' CHOICE IS LEFT ARROW ?
2144
USER RELEASES RIGHT MOUSE BUTTON ?
2146
LEFT ARROW APPEARS IN OVERLAY AT THE POSITION OF THE CURSOR
2148
TO 2150
FROM 2154, (ETC.)
Y
N

FIG. 21B-1

Nouvellement déposé

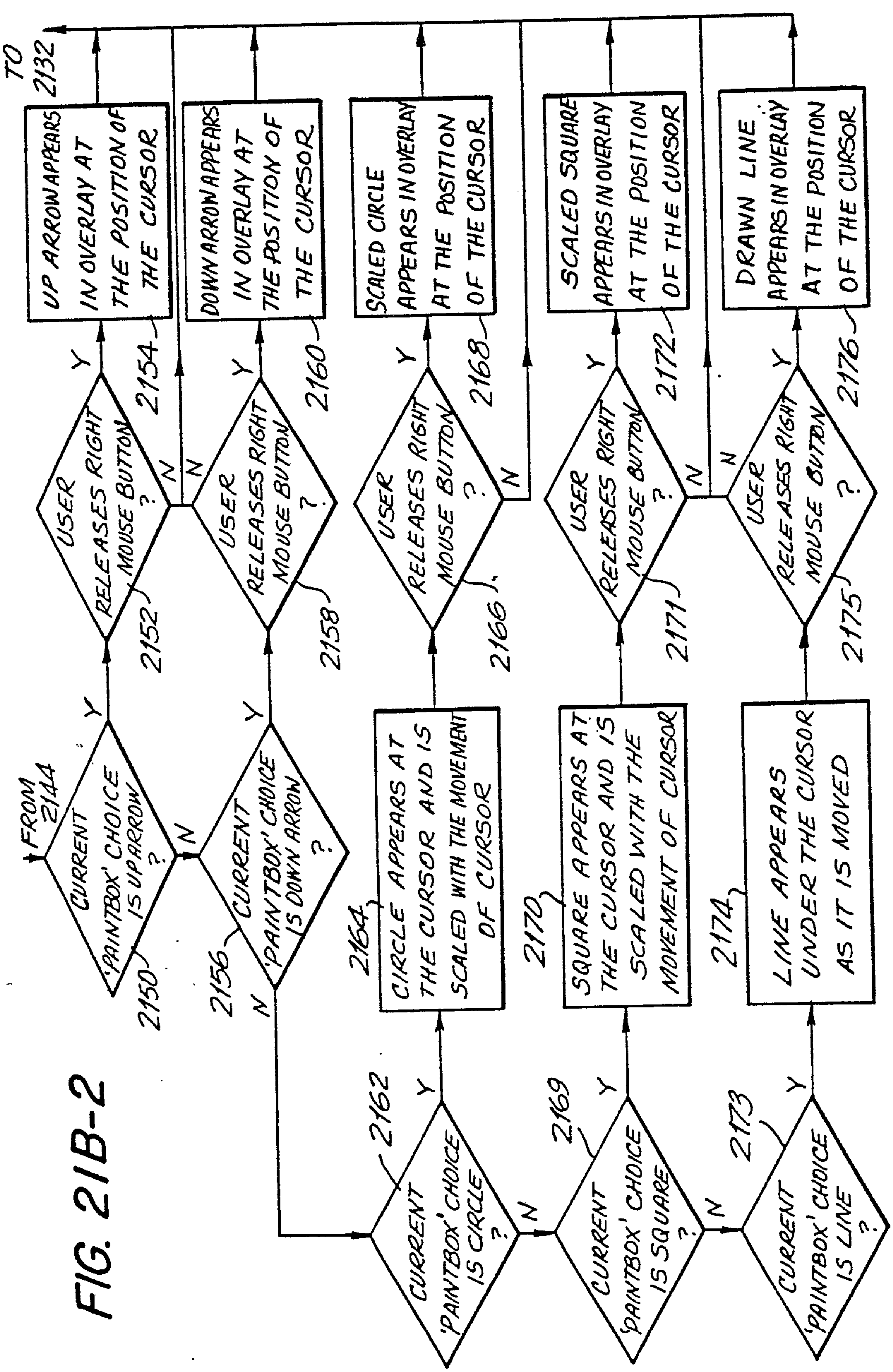


FIG. 21B-2

Nouvellement déposé

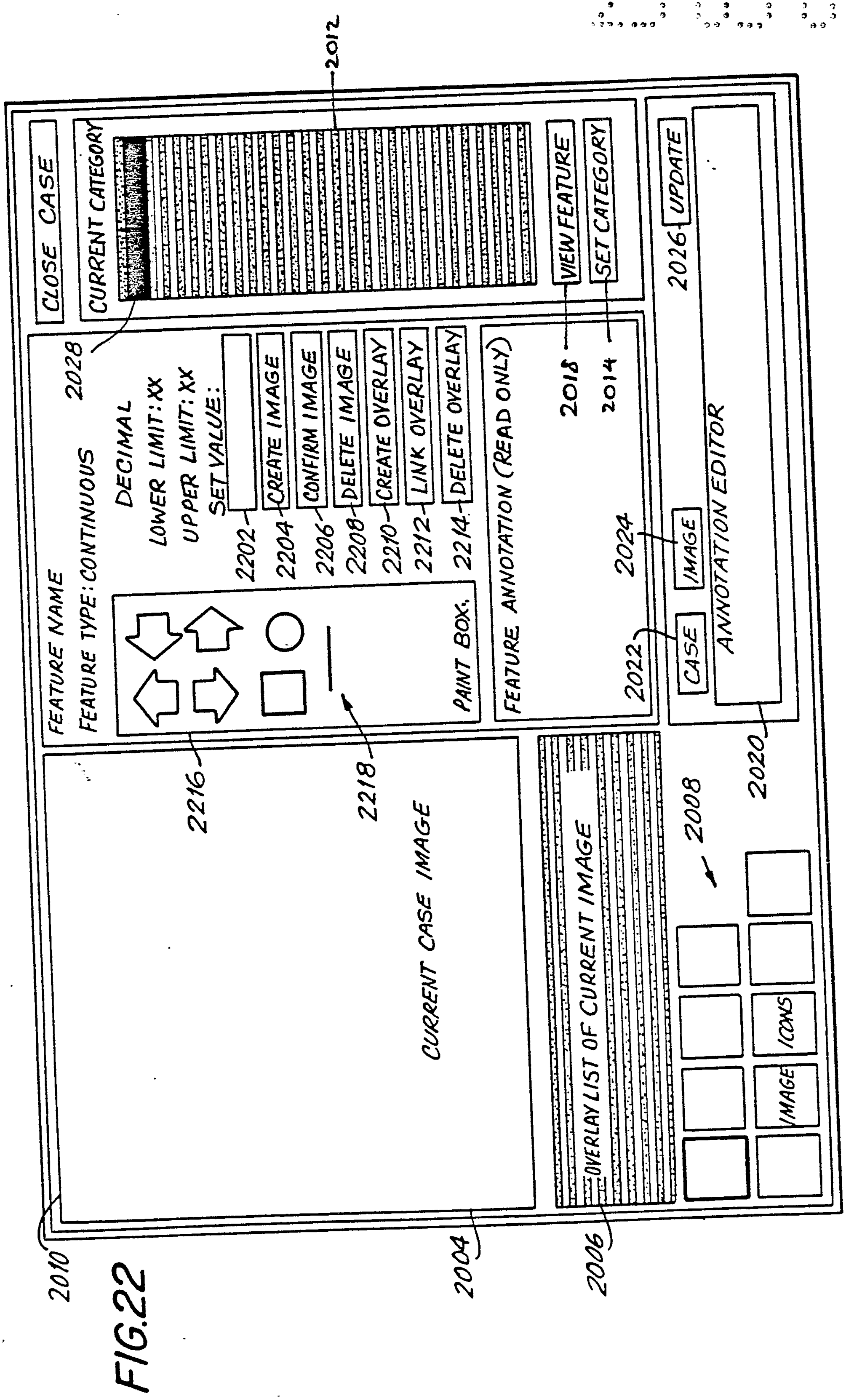


FIG.22

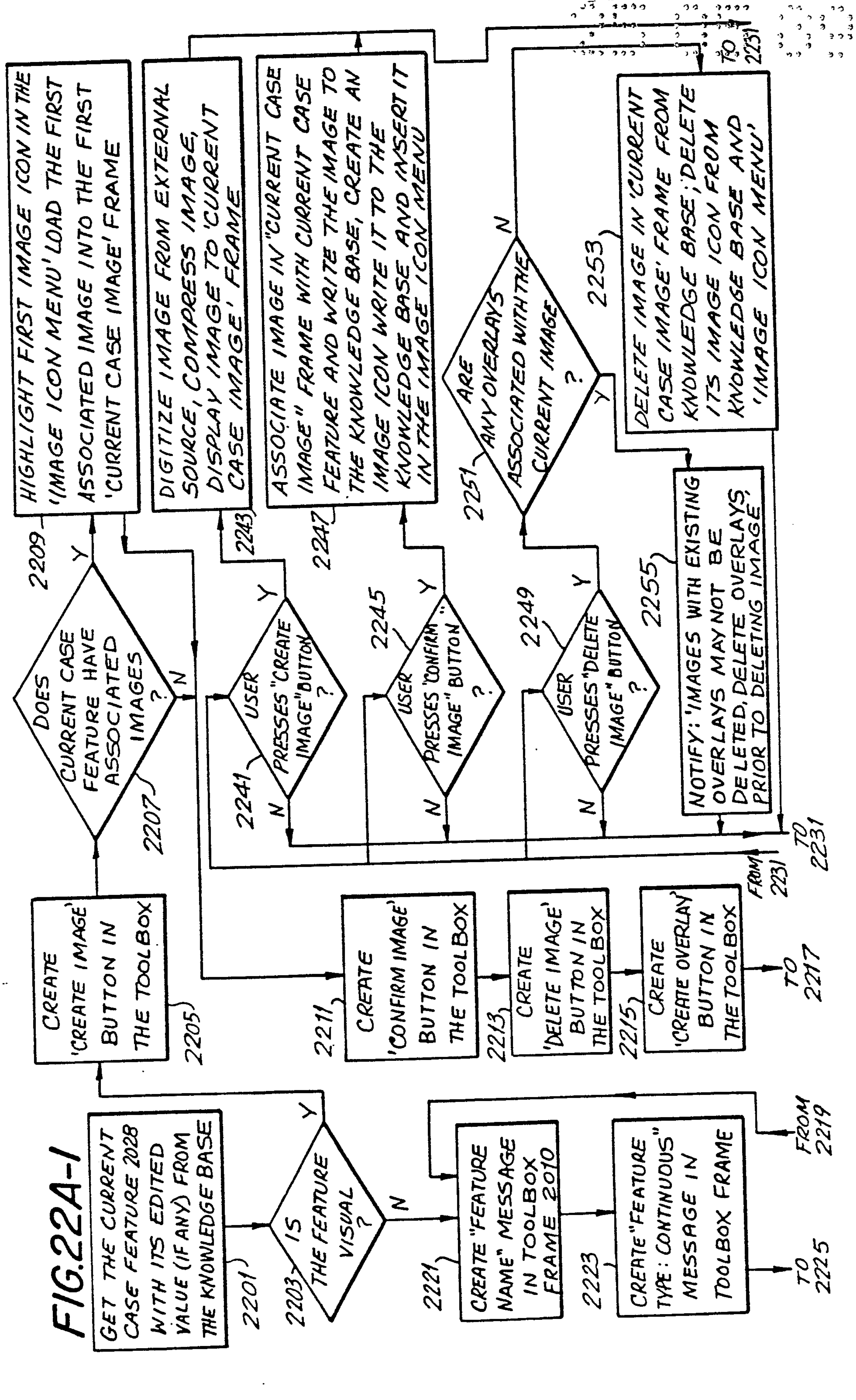
GET THE CURRENT CASE FEATURE 2028 WITH ITS EDITED VALUE (IF ANY) FROM THE KNOWLEDGE BASE
2201
IS THE FEATURE VISUAL ?
2203
Y
N
CREATE 'CREATE IMAGE' BUTTON IN THE TOOLBOX
2205
DOES CURRENT CASE FEATURE HAVE ASSOCIATED IMAGES ?
2207
Y
N
HIGHLIGHT FIRST IMAGE ICON IN THE 'IMAGE ICON MENU' LOAD THE FIRST ASSOCIATED IMAGE INTO THE FIRST 'CURRENT CASE IMAGE' FRAME
2209
CREATE 'CONFIRM IMAGE' BUTTON IN THE TOOLBOX
2211
CREATE 'DELETE IMAGE' BUTTON IN THE TOOLBOX
2213
CREATE 'CREATE OVERLAY' BUTTON IN THE TOOLBOX
2215
TO 2217
USER PRESSES "CREATE IMAGE" BUTTON ?
2241
Y
N
DIGITIZE IMAGE FROM EXTERNAL SOURCE, COMPRESS IMAGE, DISPLAY IMAGE TO 'CURRENT CASE IMAGE' FRAME
2243
USER PRESSES "CONFIRM IMAGE" BUTTON ?
2245
Y
N
ASSOCIATE IMAGE IN "CURRENT CASE IMAGE" FRAME WITH CURRENT CASE FEATURE AND WRITE THE IMAGE TO THE KNOWLEDGE BASE, CREATE AN IMAGE ICON WRITE IT TO THE KNOWLEDGE BASE AND INSERT IT IN THE IMAGE ICON MENU
2247
USER PRESSES "DELETE IMAGE" BUTTON ?
2249
Y
N
ARE ANY OVERLAYS ASSOCIATED WITH THE CURRENT IMAGE ?
2251
Y
N
DELETE IMAGE IN 'CURRENT CASE IMAGE' FRAME FROM KNOWLEDGE BASE; DELETE ITS IMAGE ICON FROM KNOWLEDGE BASE AND 'IMAGE ICON MENU'
2253
NOTIFY: 'IMAGES WITH EXISTING OVERLAYS MAY NOT BE DELETED, DELETE OVERLAYS PRIOR TO DELETING IMAGE'
2255
TO 2231
FROM 2231
CREATE "FEATURE NAME" MESSAGE IN TOOLBOX FRAME 2010
2221
CREATE "FEATURE TYPE: CONTINUOUS" MESSAGE IN TOOLBOX FRAME
2223
TO 2225
FROM 2219
FIG.22A-1

ouvellement déposé

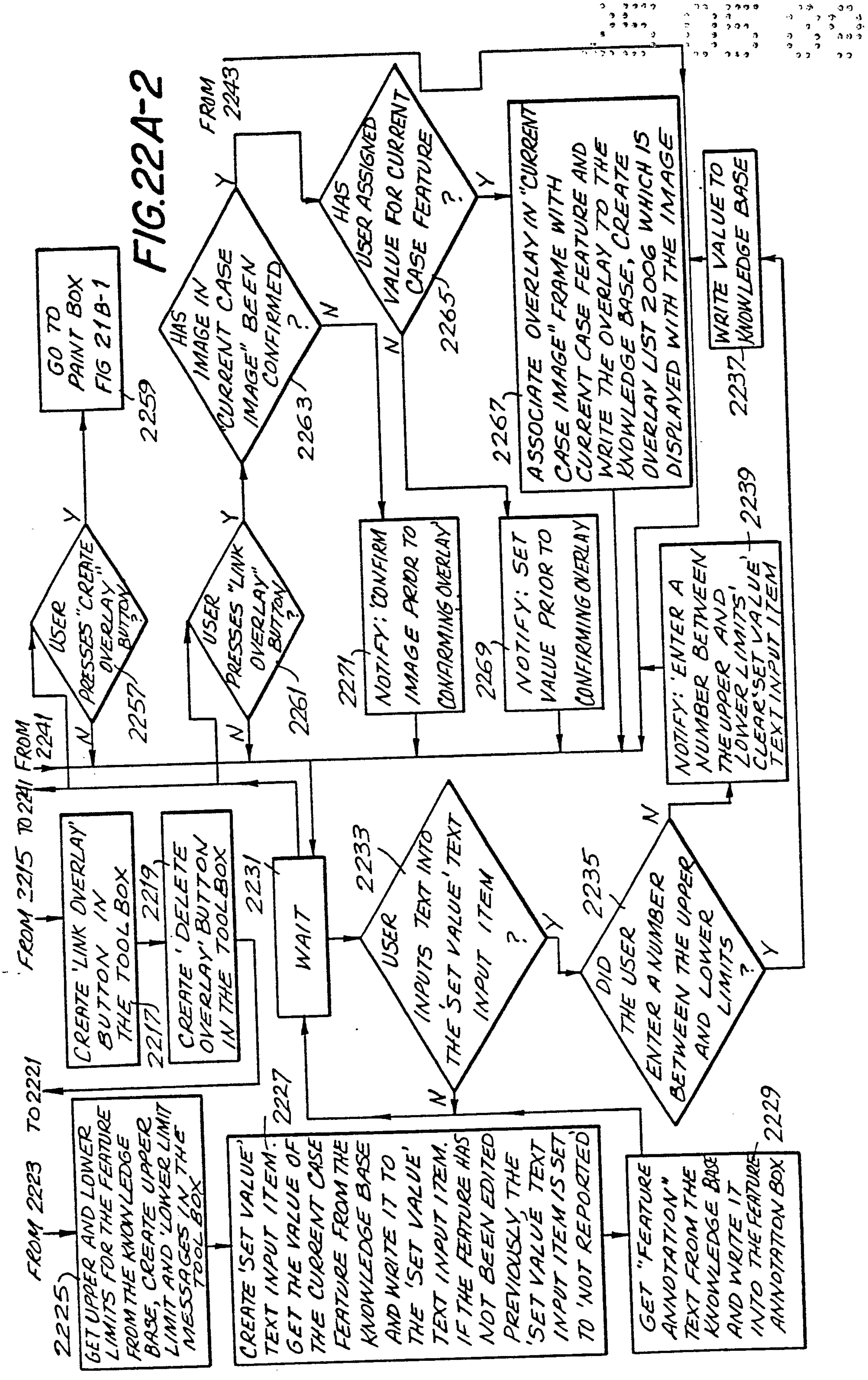


FIG.22A-2

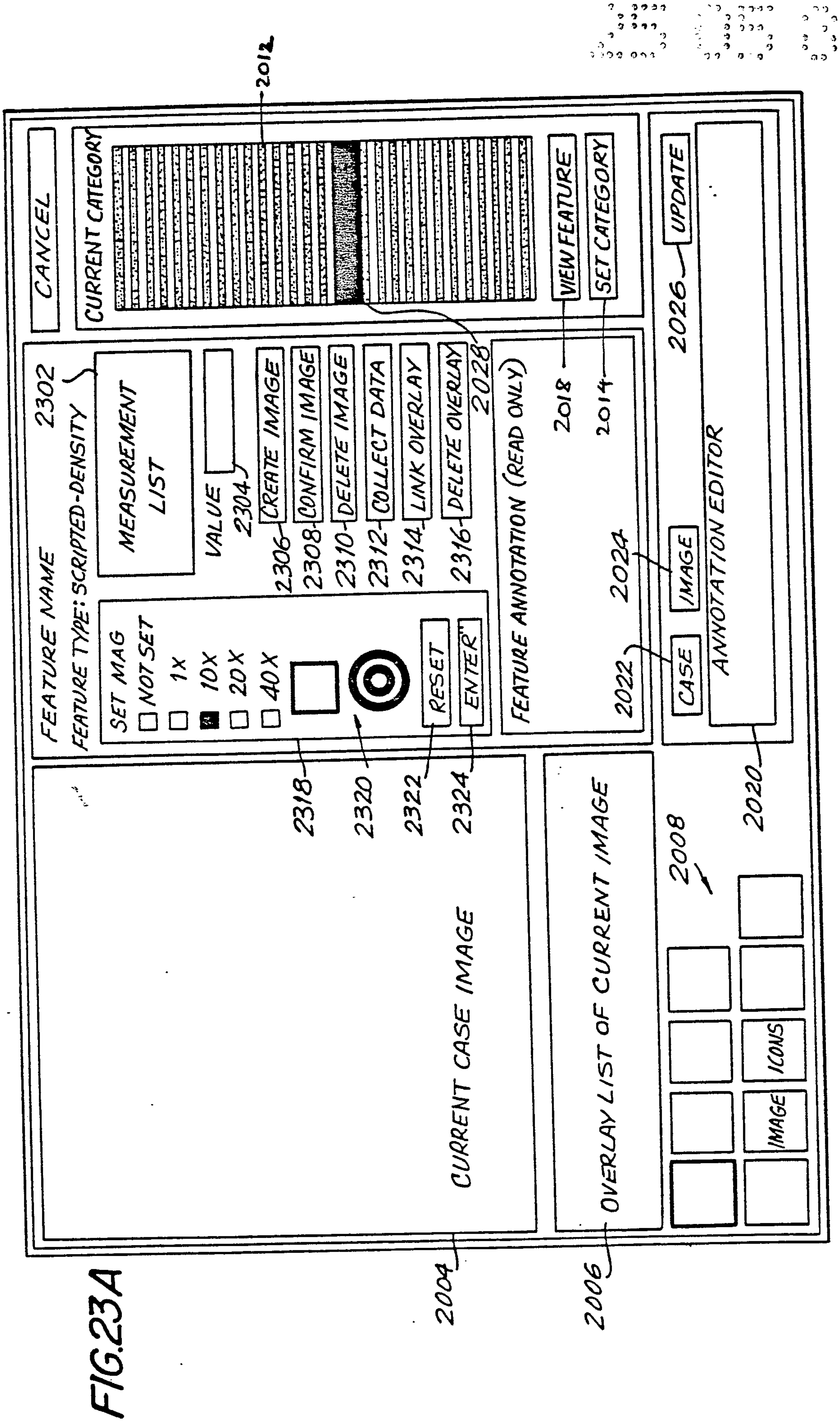
CANCEL
CURRENT CATEGORY
2012
VIEW FEATURE
SET CATEGORY
UPDATE
2026
FEATURE NAME
2302
FEATURE TYPE: SCRIPTED-DENSITY
MEASUREMENT LIST
VALUE
2304
CREATE IMAGE
CONFIRM IMAGE
DELETE IMAGE
COLLECT DATA
LINK OVERLAY
DELETE OVERLAY
2306
2308
2310
2312
2314
2316
2028
2018
2014
SET MAG
NOT SET
1x
10x
20x
40x
RESET
ENTER
2318
2320
2322
2324
FEATURE ANNOTATION (READ ONLY)
2022
2024
CASE
IMAGE
ANNOTATION EDITOR
2020
CURRENT CASE IMAGE
OVERLAY LIST OF CURRENT IMAGE
2008
IMAGE
ICONS
2004
2006

FIG.23A

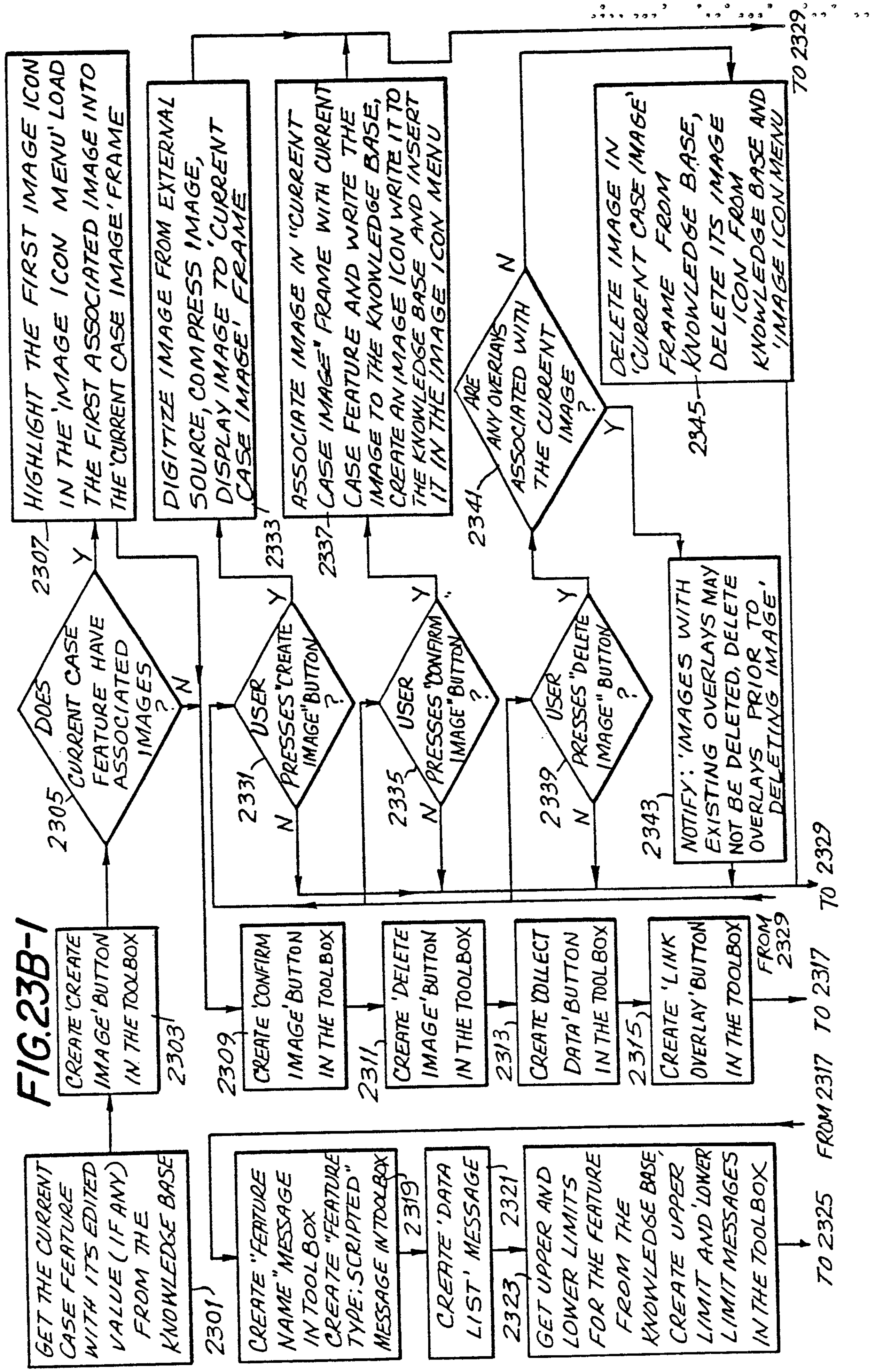
GET THE CURRENT CASE FEATURE WITH ITS EDITED VALUE (IF ANY) FROM THE KNOWLEDGE BASE
2301
CREATE 'CREATE IMAGE' BUTTON IN THE TOOLBOX
2303
DOES CURRENT CASE FEATURE HAVE ASSOCIATED IMAGES ?
2305
Y
N
2307
HIGHLIGHT THE FIRST IMAGE ICON IN THE 'IMAGE ICON MENU' LOAD THE FIRST ASSOCIATED IMAGE INTO THE 'CURRENT CASE IMAGE' FRAME
CREATE 'CONFIRM IMAGE' BUTTON IN THE TOOLBOX
2309
CREATE 'DELETE IMAGE' BUTTON IN THE TOOLBOX
2311
CREATE 'COLLECT DATA' BUTTON IN THE TOOLBOX
2313
CREATE 'LINK OVERLAY' BUTTON IN THE TOOLBOX
2315
FROM 2329
TO 2317
USER PRESSES "CREATE IMAGE" BUTTON ?
2331
Y
N
DIGITIZE IMAGE FROM EXTERNAL SOURCE, COMPRESS IMAGE, DISPLAY IMAGE TO 'CURRENT CASE IMAGE' FRAME
2333
USER PRESSES "CONFIRM IMAGE" BUTTON ?
2335
Y
N
ASSOCIATE IMAGE IN "CURRENT CASE IMAGE" FRAME WITH CURRENT CASE FEATURE AND WRITE THE IMAGE TO THE KNOWLEDGE BASE, CREATE AN IMAGE ICON WRITE IT TO THE KNOWLEDGE BASE AND INSERT IT IN THE IMAGE ICON MENU
2337
USER PRESSES "DELETE IMAGE" BUTTON ?
2339
Y
N
ARE ANY OVERLAYS ASSOCIATED WITH THE CURRENT IMAGE ?
2341
Y
N
NOTIFY: 'IMAGES WITH EXISTING OVERLAYS MAY NOT BE DELETED, DELETE OVERLAYS PRIOR TO DELETING IMAGE'
2343
DELETE IMAGE IN 'CURRENT CASE IMAGE' FRAME FROM KNOWLEDGE BASE, DELETE ITS IMAGE ICON FROM KNOWLEDGE BASE AND 'IMAGE ICON MENU
2345
TO 2329
TO 2329
CREATE "FEATURE NAME" MESSAGE IN TOOLBOX CREATE "FEATURE TYPE: SCRIPTED" MESSAGE IN TOOLBOX
2319
CREATE 'DATA LIST' MESSAGE
2321
GET UPPER AND LOWER LIMITS FOR THE FEATURE FROM THE KNOWLEDGE BASE, CREATE UPPER LIMIT AND 'LOWER LIMIT MESSAGES IN THE TOOLBOX
2323
FROM 2317
TO 2325

FIG.23B-1

FIG.23B-2

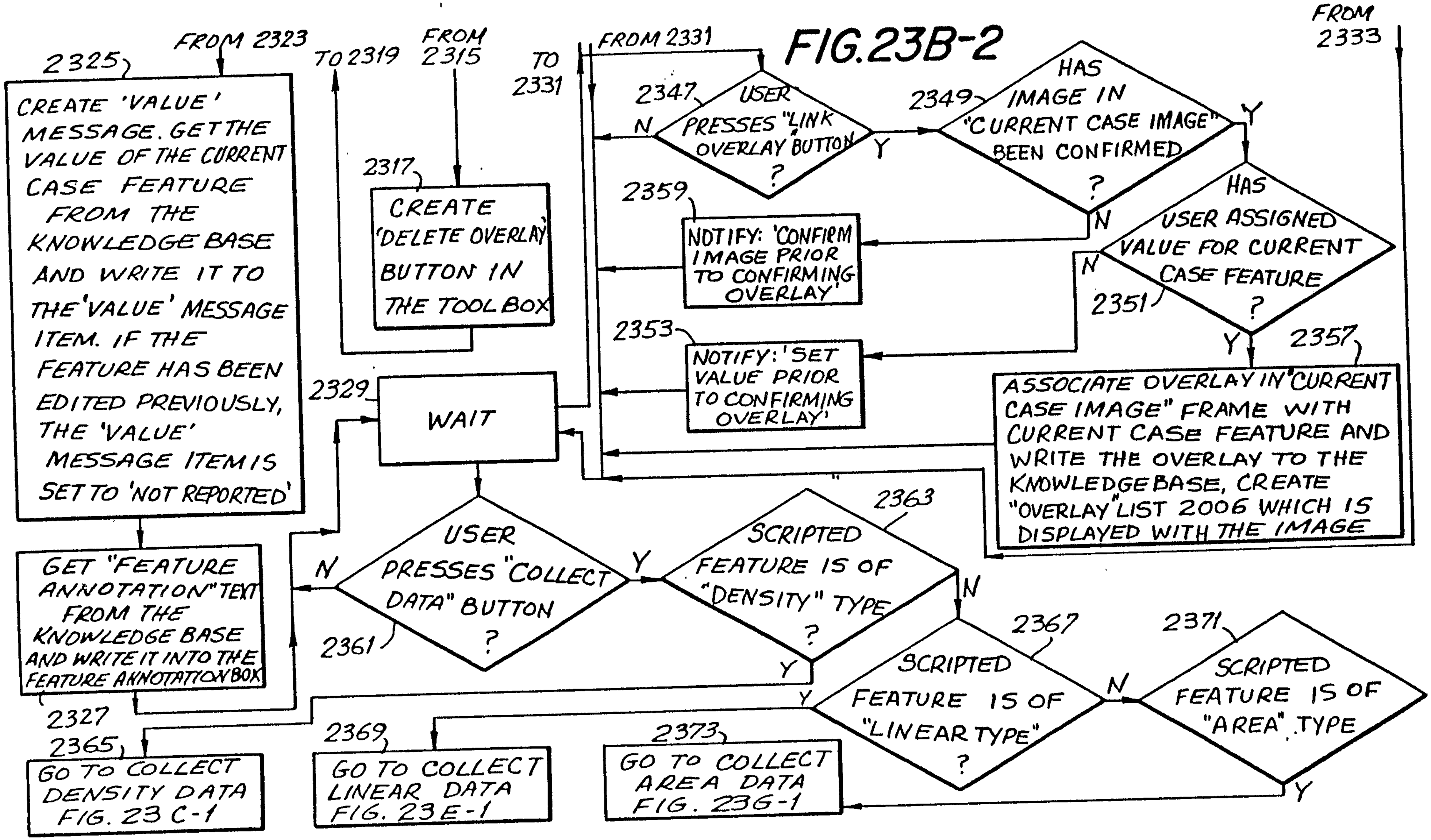
FROM 2323
2325
CREATE 'VALUE' MESSAGE. GET THE VALUE OF THE CURRENT CASE FEATURE FROM THE KNOWLEDGE BASE AND WRITE IT TO THE 'VALUE' MESSAGE ITEM. IF THE FEATURE HAS BEEN EDITED PREVIOUSLY, THE 'VALUE' MESSAGE ITEM IS SET TO 'NOT REPORTED'
TO 2319
FROM 2315
2317
CREATE 'DELETE OVERLAY' BUTTON IN THE TOOL BOX
TO 2331
FROM 2331
2347
USER PRESSES "LINK OVERLAY" BUTTON ?
N
Y
2349
HAS IMAGE IN "CURRENT CASE IMAGE" BEEN CONFIRMED ?
Y
N
FROM 2333
HAS USER ASSIGNED VALUE FOR CURRENT CASE FEATURE ?
2351
N
Y
2357
ASSOCIATE OVERLAY IN "CURRENT CASE IMAGE" FRAME WITH CURRENT CASE FEATURE AND WRITE THE OVERLAY TO THE KNOWLEDGE BASE, CREATE "OVERLAY" LIST 2006 WHICH IS DISPLAYED WITH THE IMAGE
2359
NOTIFY: 'CONFIRM IMAGE PRIOR TO CONFIRMING OVERLAY'
2353
NOTIFY: 'SET VALUE PRIOR TO CONFIRMING OVERLAY'
2329
WAIT
USER PRESSES "COLLECT DATA" BUTTON ?
2361
N
Y
2363
SCRIPTED FEATURE IS OF "DENSITY" TYPE ?
N
Y
2367
SCRIPTED FEATURE IS OF "LINEAR TYPE" ?
N
Y
2371
SCRIPTED FEATURE IS OF "AREA" TYPE
Y
GET "FEATURE ANNOTATION" TEXT FROM THE KNOWLEDGE BASE AND WRITE IT INTO THE FEATURE ANNOTATION BOX
2327
2365
GO TO COLLECT DENSITY DATA FIG. 23 C-1
2369
GO TO COLLECT LINEAR DATA FIG. 23 E-1
2373
GO TO COLLECT AREA DATA FIG. 23G-1

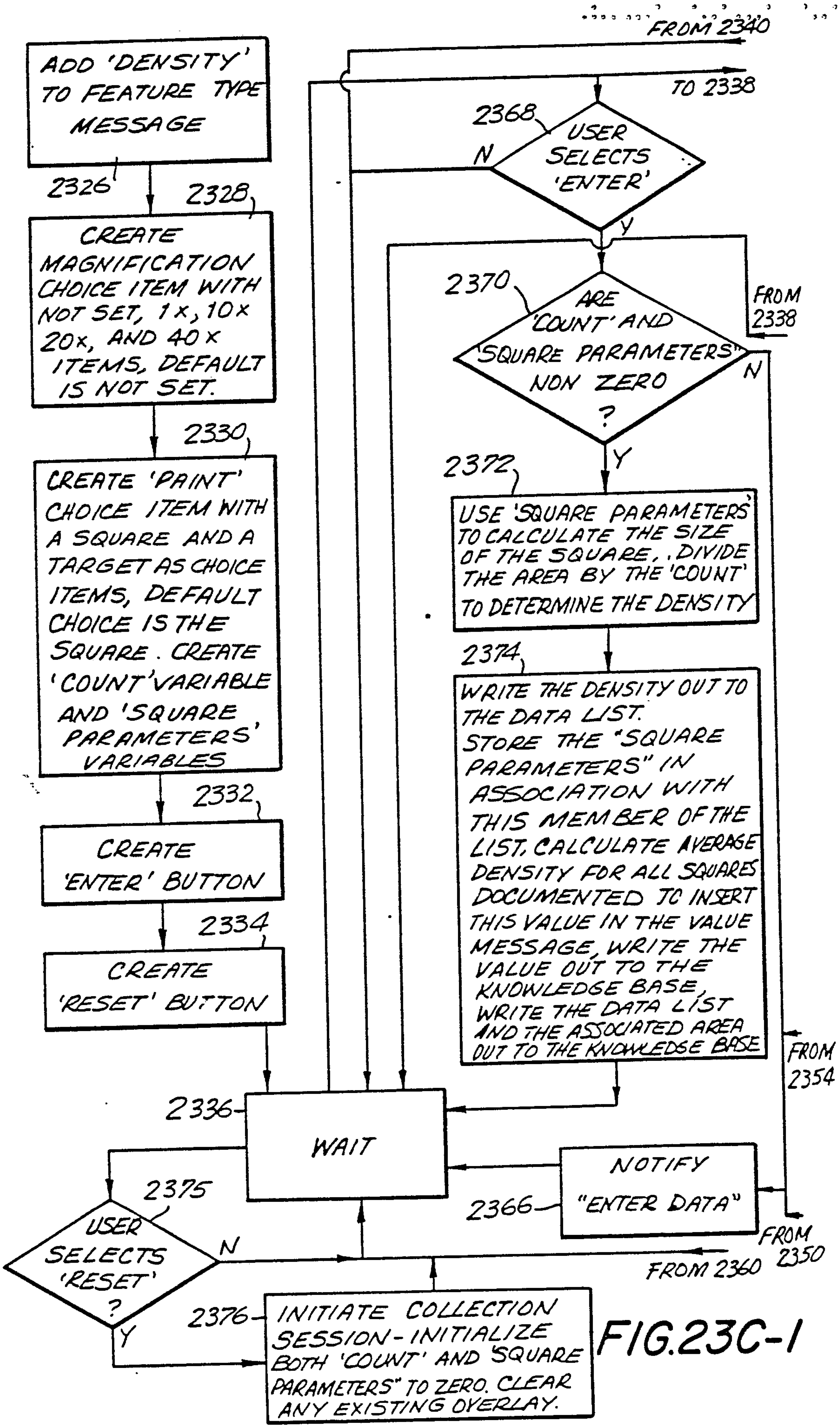
FROM 2340
TO 2338
ADD 'DENSITY' TO FEATURE TYPE MESSAGE
2326
2328
CREATE MAGNIFICATION CHOICE ITEM WITH NOT SET, 1x, 10x 20x, AND 40x ITEMS, DEFAULT IS NOT SET.
2330
CREATE 'PAINT' CHOICE ITEM WITH A SQUARE AND A TARGET AS CHOICE ITEMS, DEFAULT CHOICE IS THE SQUARE. CREATE 'COUNT' VARIABLE AND 'SQUARE PARAMETERS' VARIABLES
2332
CREATE 'ENTER' BUTTON
2334
CREATE 'RESET' BUTTON
2368
USER SELECTS 'ENTER'
N
Y
2370
ARE 'COUNT' AND 'SQUARE PARAMETERS' NON ZERO ?
FROM 2338
N
Y
2372
USE 'SQUARE PARAMETERS' TO CALCULATE THE SIZE OF THE SQUARE, DIVIDE THE AREA BY THE 'COUNT' TO DETERMINE THE DENSITY
2374
WRITE THE DENSITY OUT TO THE DATA LIST. STORE THE "SQUARE PARAMETERS" IN ASSOCIATION WITH THIS MEMBER OF THE LIST, CALCULATE AVERAGE DENSITY FOR ALL SQUARES DOCUMENTED TO INSERT THIS VALUE IN THE VALUE MESSAGE, WRITE THE VALUE OUT TO THE KNOWLEDGE BASE, WRITE THE DATA LIST AND THE ASSOCIATED AREA OUT TO THE KNOWLEDGE BASE
FROM 2354
2336
WAIT
NOTIFY "ENTER DATA"
2366
2375
USER SELECTS 'RESET' ?
N
Y
FROM 2350
FROM 2360
2376
INITIATE COLLECTION SESSION - INITIALIZE BOTH 'COUNT' AND 'SQUARE PARAMETERS' TO ZERO. CLEAR ANY EXISTING OVERLAY.

FIG.23C-1

Nouvellement déposé

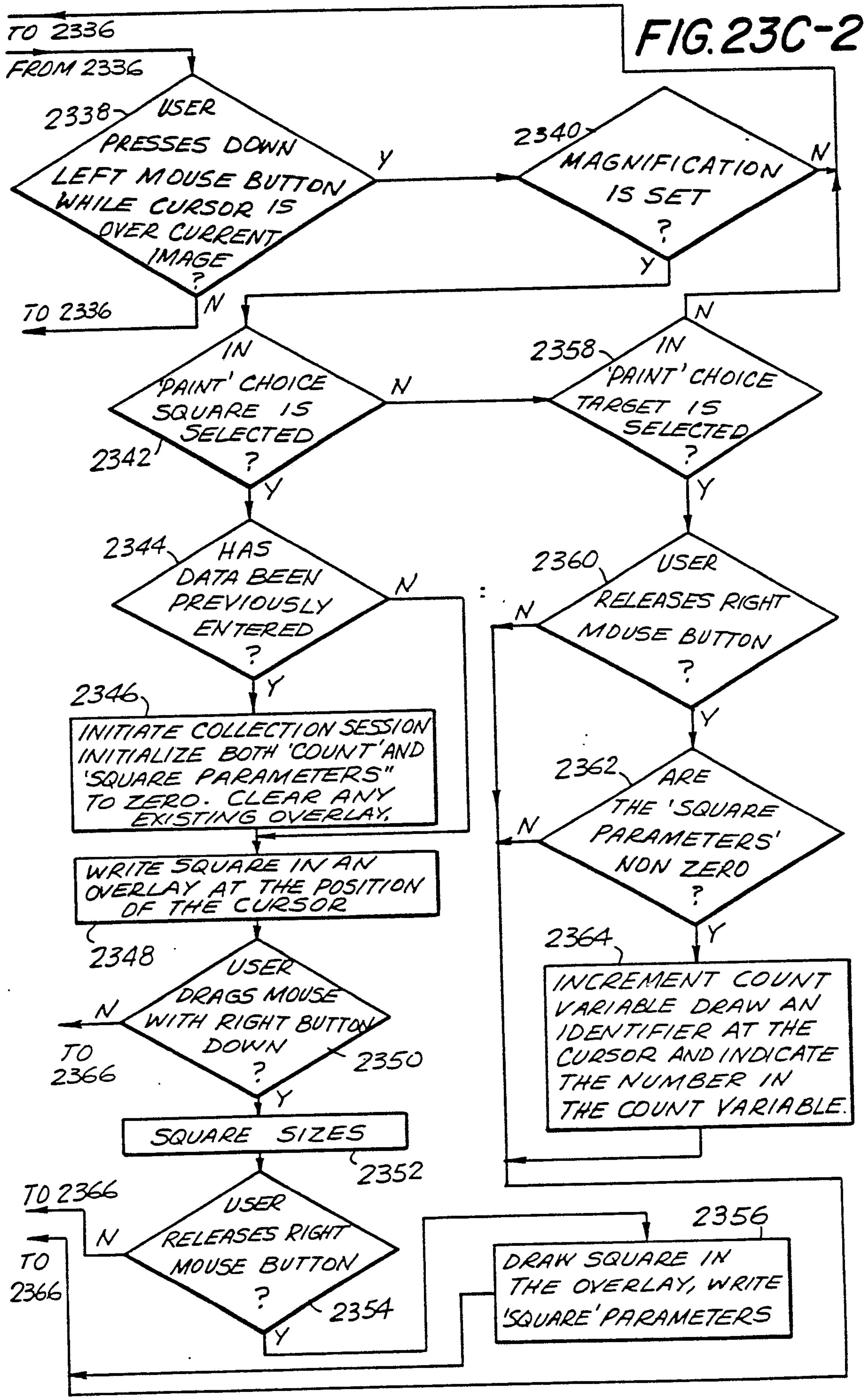


FIG.23C-2

Nouvellement déposé

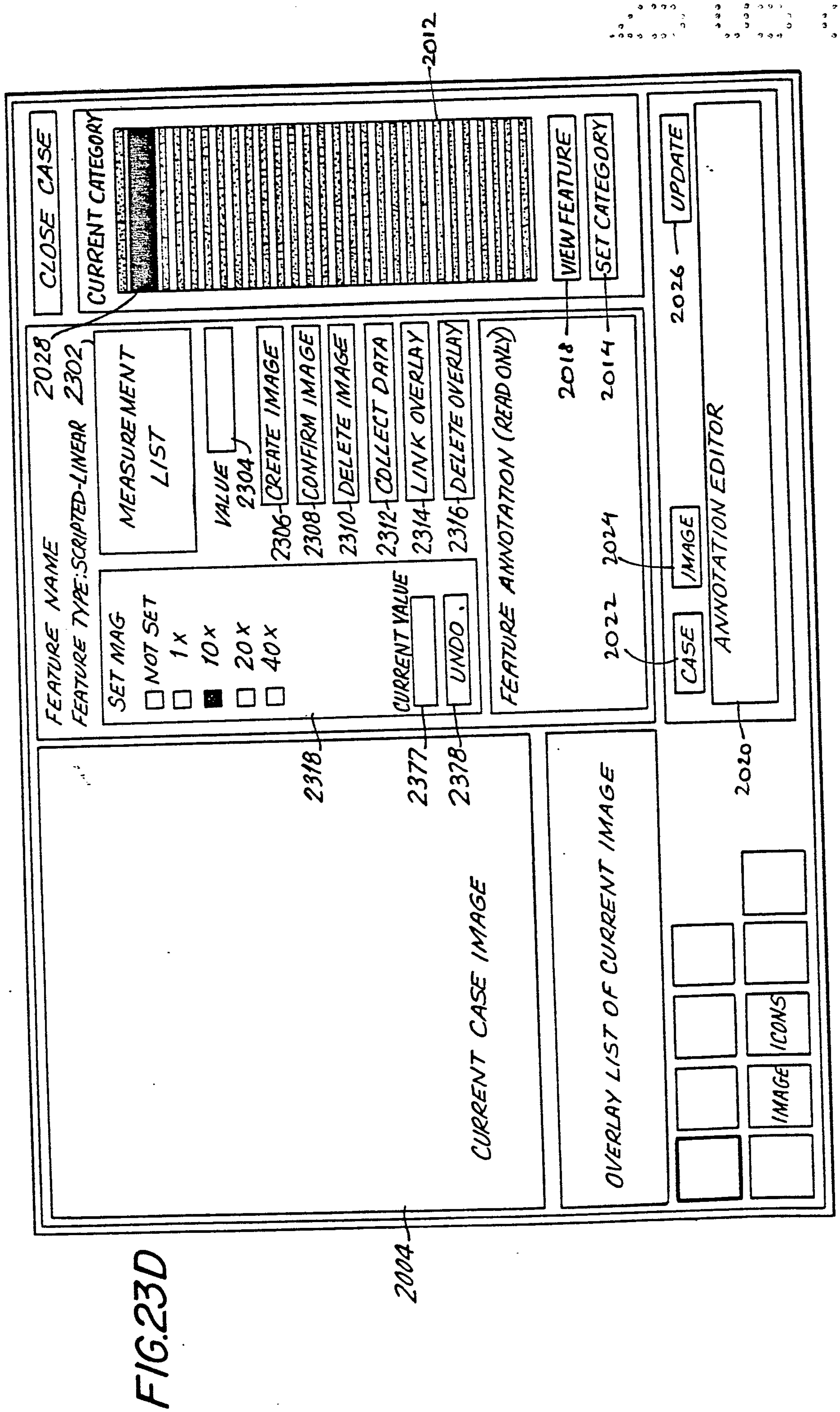


FIG.23D

document déposé 25 05 89

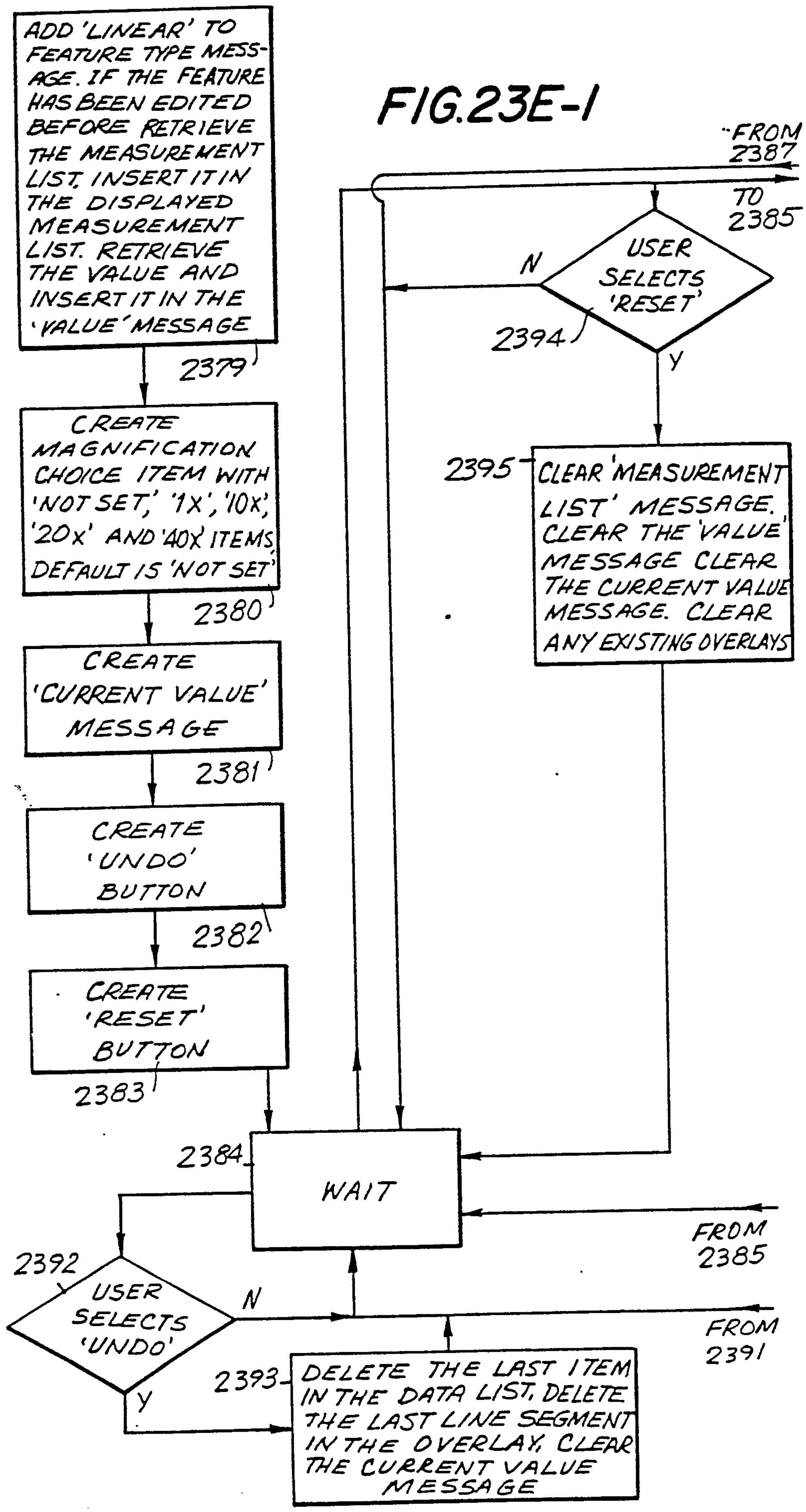

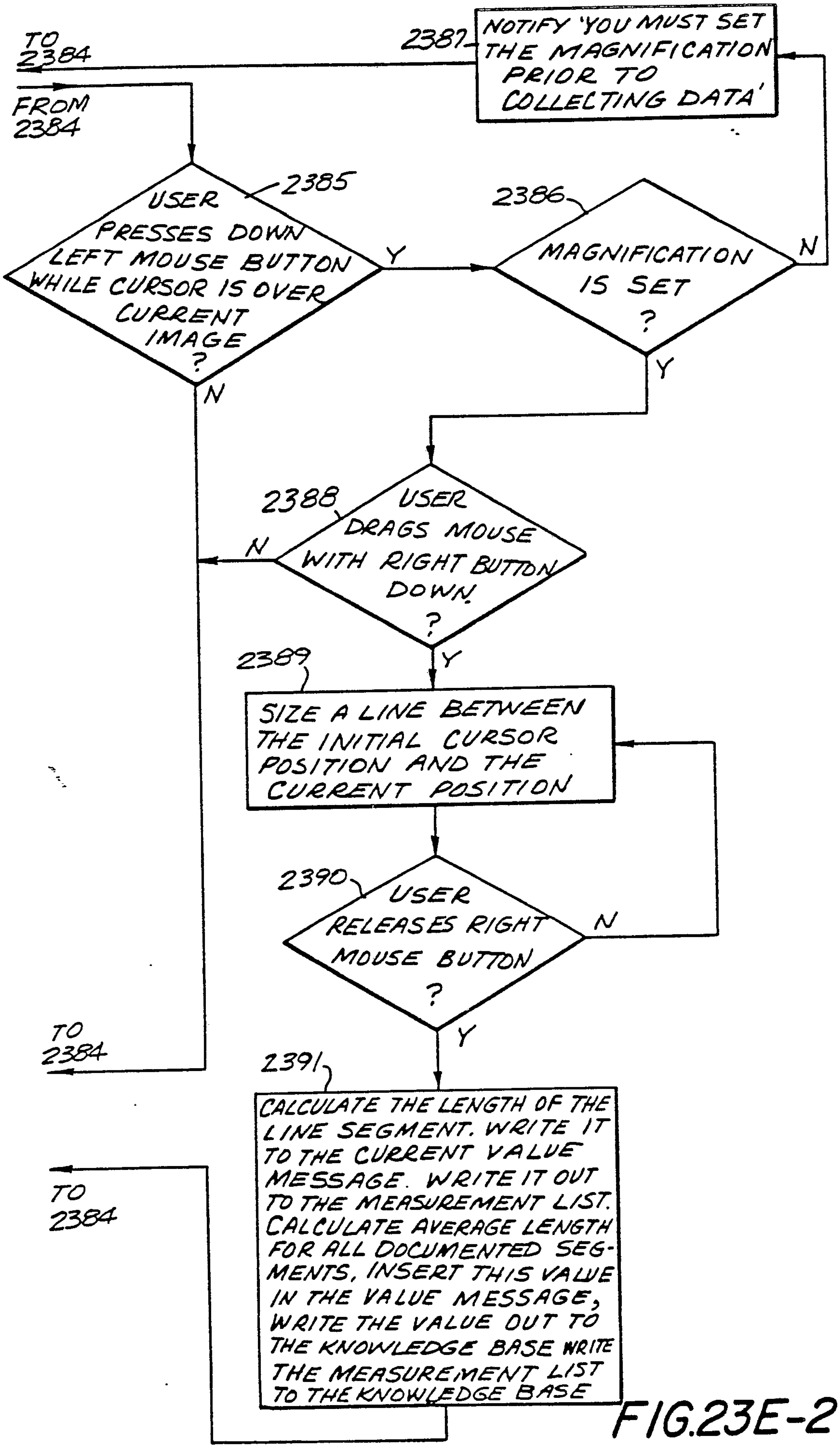

TO 2384
FROM 2384
2387
NOTIFY 'YOU MUST SET THE MAGNIFICATION PRIOR TO COLLECTING DATA'
2385
USER PRESSES DOWN LEFT MOUSE BUTTON WHILE CURSOR IS OVER CURRENT IMAGE ?
Y
N
2386
MAGNIFICATION IS SET ?
N
Y
2388
USER DRAGS MOUSE WITH RIGHT BUTTON DOWN ?
N
Y
2389
SIZE A LINE BETWEEN THE INITIAL CURSOR POSITION AND THE CURRENT POSITION
2390
USER RELEASES RIGHT MOUSE BUTTON ?
N
Y
TO 2384
2391
CALCULATE THE LENGTH OF THE LINE SEGMENT. WRITE IT TO THE CURRENT VALUE MESSAGE. WRITE IT OUT TO THE MEASUREMENT LIST. CALCULATE AVERAGE LENGTH FOR ALL DOCUMENTED SEGMENTS, INSERT THIS VALUE IN THE VALUE MESSAGE, WRITE THE VALUE OUT TO THE KNOWLEDGE BASE WRITE THE MEASUREMENT LIST TO THE KNOWLEDGE BASE
TO 2384


FIG.23E-2

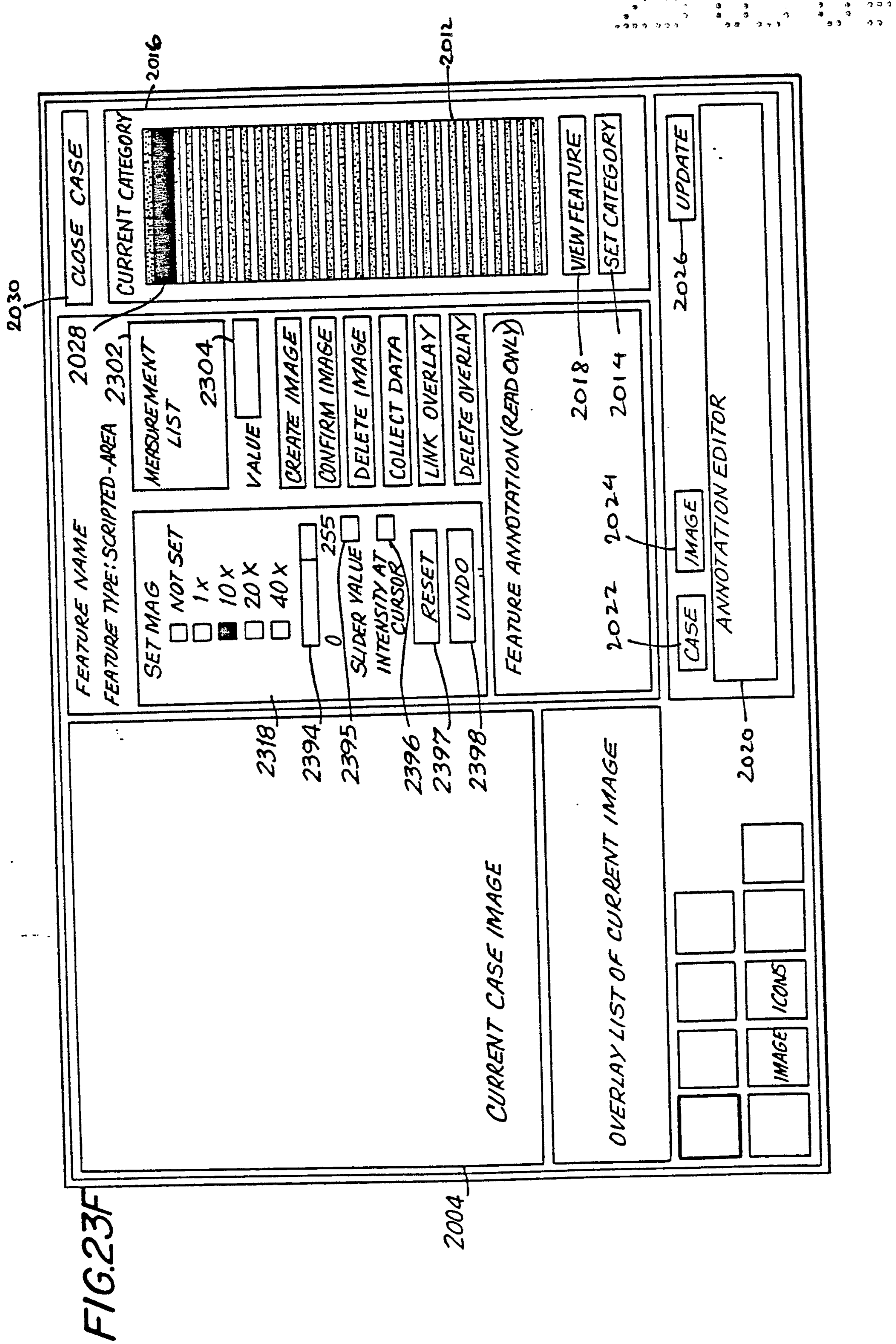
CLOSE CASE
CURRENT CATEGORY
VIEW FEATURE
SET CATEGORY
UPDATE
FEATURE NAME
FEATURE TYPE: SCRIPTED-AREA
MEASUREMENT LIST
VALUE
CREATE IMAGE
CONFIRM IMAGE
DELETE IMAGE
COLLECT DATA
LINK OVERLAY
DELETE OVERLAY
SET MAG
NOT SET
1x
10x
20x
40x
0
255
SLIDER VALUE
INTENSITY AT CURSOR
RESET
UNDO
FEATURE ANNOTATION (READ ONLY)
CASE
IMAGE
ANNOTATION EDITOR
CURRENT CASE IMAGE
OVERLAY LIST OF CURRENT IMAGE
IMAGE
ICONS
2016
2012
2030
2028
2302
2304
2018
2014
2026
2024
2022
2318
2394
2395
2396
2397
2398
2020
2004

FIG.23F

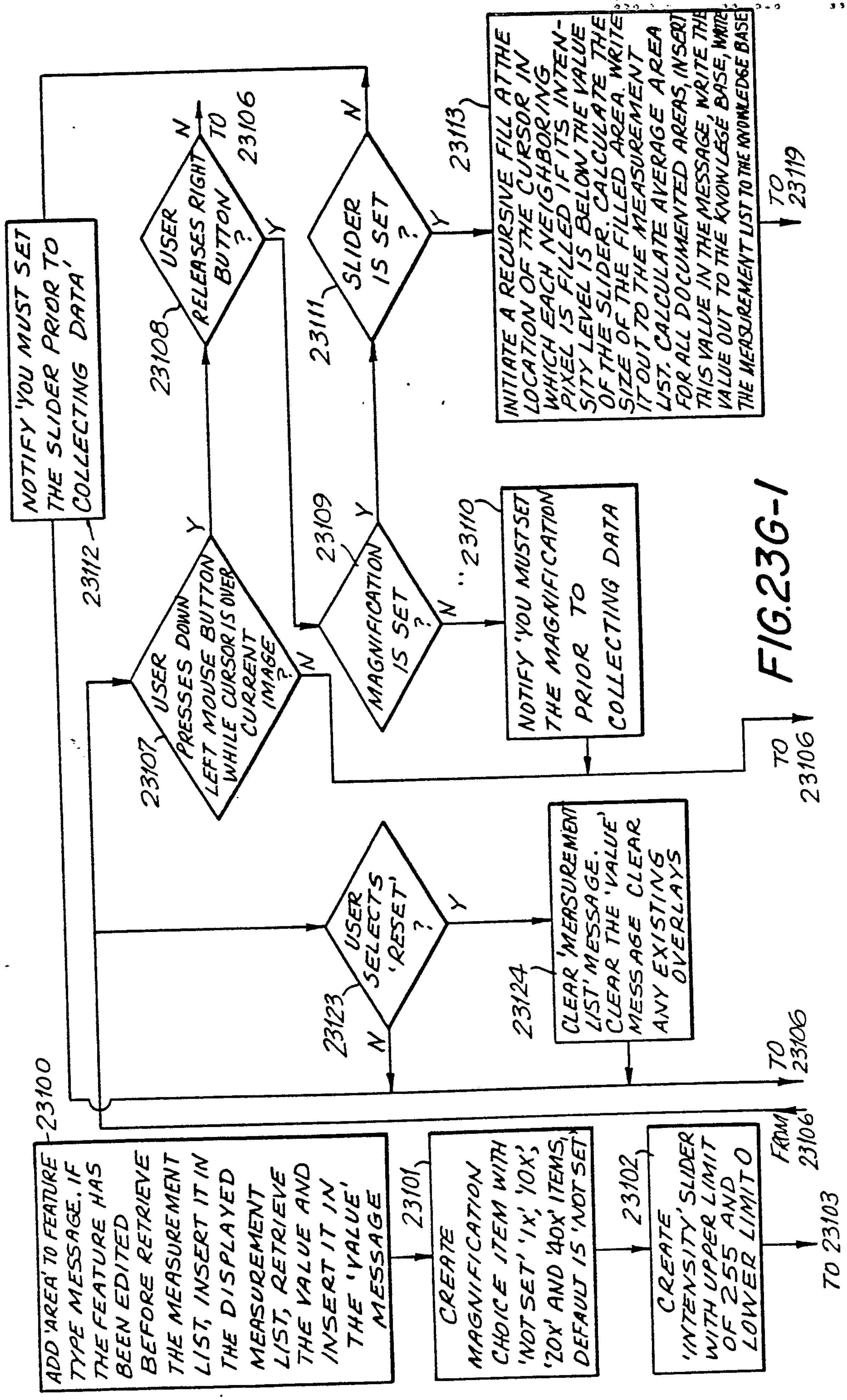
ADD 'AREA' TO FEATURE TYPE MESSAGE. IF THE FEATURE HAS BEEN EDITED BEFORE RETRIEVE THE MEASUREMENT LIST, INSERT IT IN THE DISPLAYED MEASUREMENT LIST, RETRIEVE THE VALUE AND INSERT IT IN THE 'VALUE' MESSAGE
23100
23101
CREATE MAGNIFICATION CHOICE ITEM WITH 'NOT SET' '1X', '10X', '20X' AND '40X' ITEMS, DEFAULT IS 'NOT SET'
23102
CREATE 'INTENSITY' SLIDER WITH UPPER LIMIT OF 255 AND LOWER LIMIT 0
TO 23103
FROM 23106
23123
USER SELECTS 'RESET' ?
Y
N
23124
CLEAR 'MEASUREMENT LIST' MESSAGE. CLEAR THE 'VALUE' MESSAGE CLEAR ANY EXISTING OVERLAYS
TO 23106
23107
USER PRESSES DOWN LEFT MOUSE BUTTON WHILE CURSOR IS OVER CURRENT IMAGE ?
Y
N
23109
MAGNIFICATION IS SET ?
Y
N
23110
NOTIFY 'YOU MUST SET THE MAGNIFICATION PRIOR TO COLLECTING DATA
TO 23106
23108
USER RELEASES RIGHT BUTTON ?
Y
N
TO 23106
23111
SLIDER IS SET ?
Y
N
23112
NOTIFY 'YOU MUST SET THE SLIDER PRIOR TO COLLECTING DATA'
23113
INITIATE A RECURSIVE FILL AT THE LOCATION OF THE CURSOR IN WHICH EACH NEIGHBORING PIXEL IS FILLED IF ITS INTENSITY LEVEL IS BELOW THE VALUE OF THE SLIDER. CALCULATE THE SIZE OF THE FILLED AREA. WRITE IT OUT TO THE MEASUREMENT LIST. CALCULATE AVERAGE AREA FOR ALL DOCUMENTED AREAS, INSERT THIS VALUE IN THE MESSAGE, WRITE THE VALUE OUT TO THE KNOWLEGE BASE, WRITE THE MEASUREMENT LIST TO THE KNOWLEDGE BASE
TO 23119

FIG.23G-1

Nouvellement déposé

FIG.23G-2

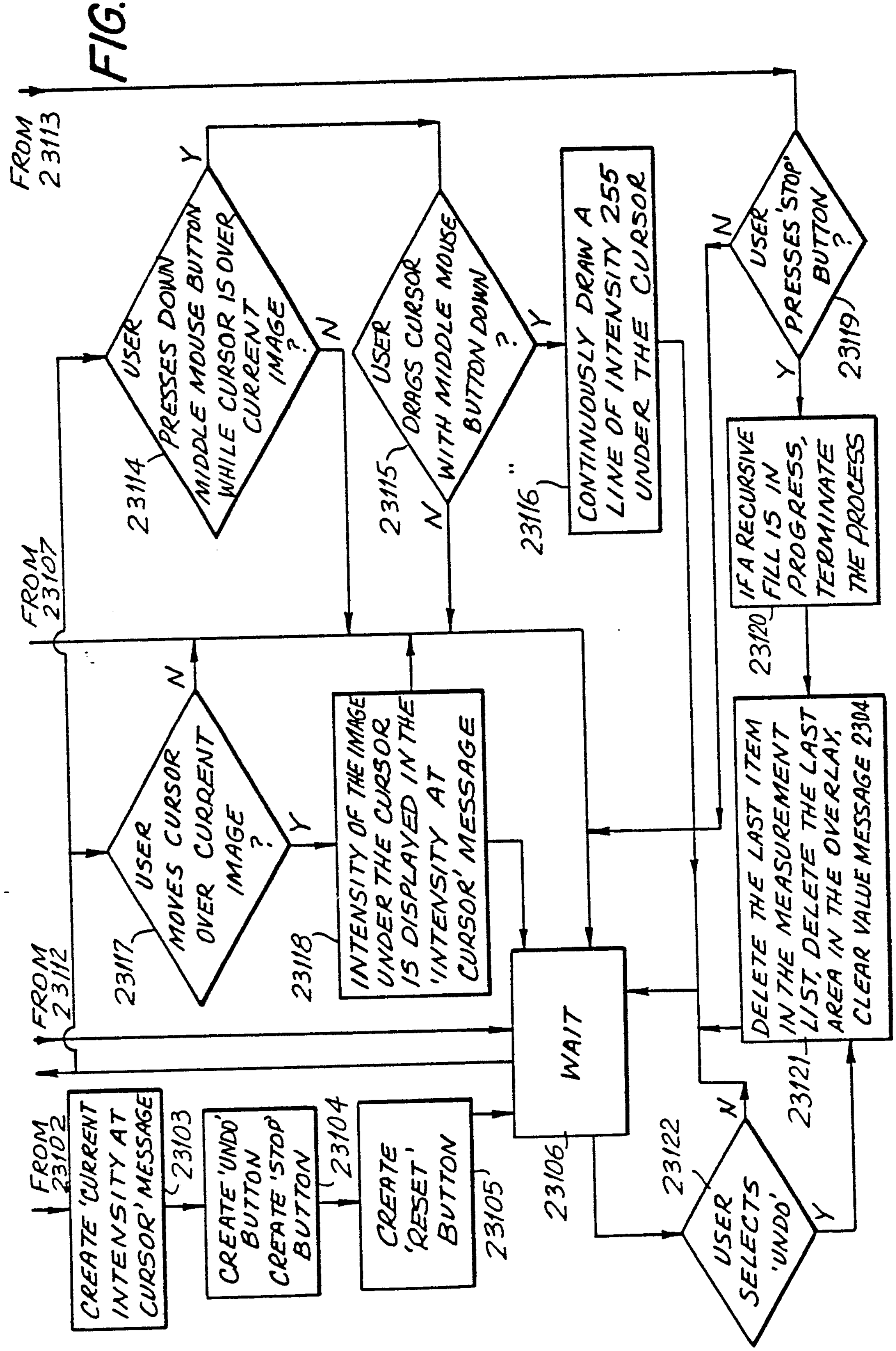

Nouvellement déposé

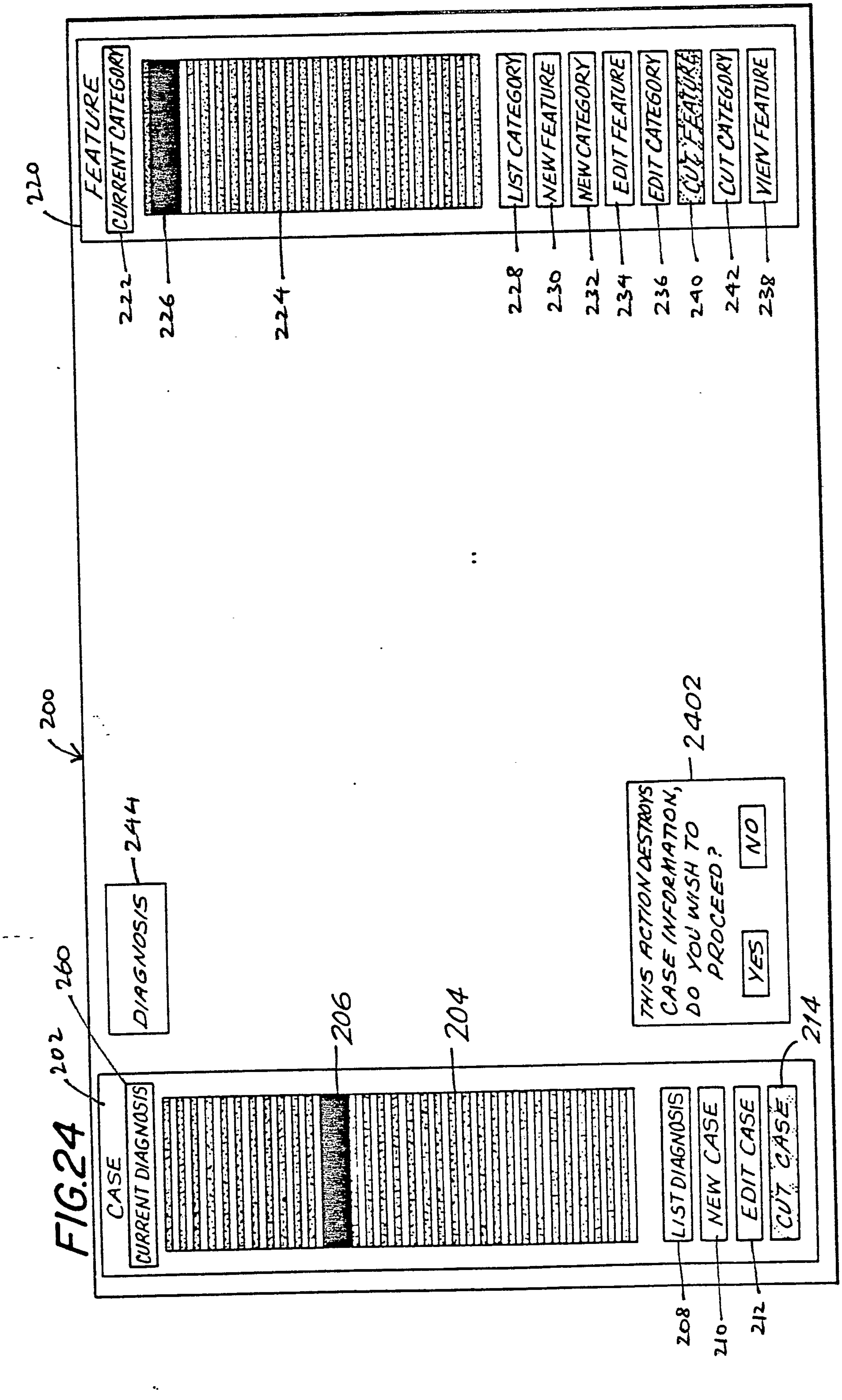


FIG.24

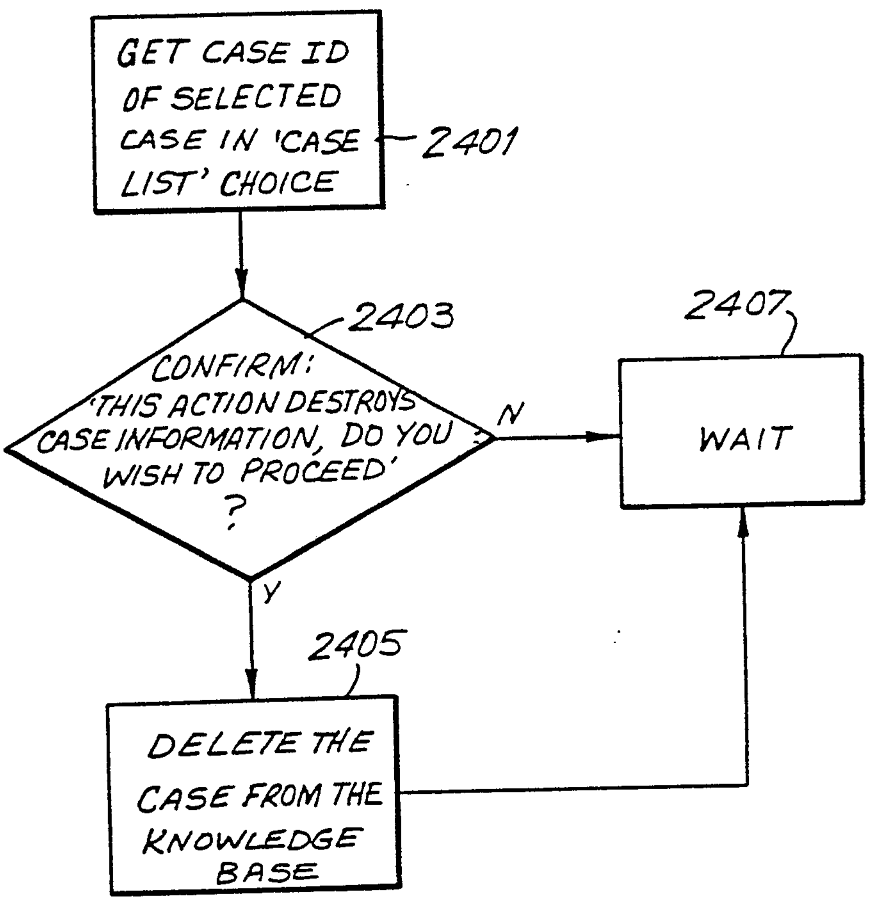
GET CASE ID OF SELECTED CASE IN 'CASE LIST' CHOICE
2401
2403
CONFIRM: 'THIS ACTION DESTROYS CASE INFORMATION, DO YOU WISH TO PROCEED' ?
N
2407
WAIT
Y
2405
DELETE THE CASE FROM THE KNOWLEDGE BASE

FIG. 24A

Neu eingereicht / Nouvellement déposé

FIG. 25

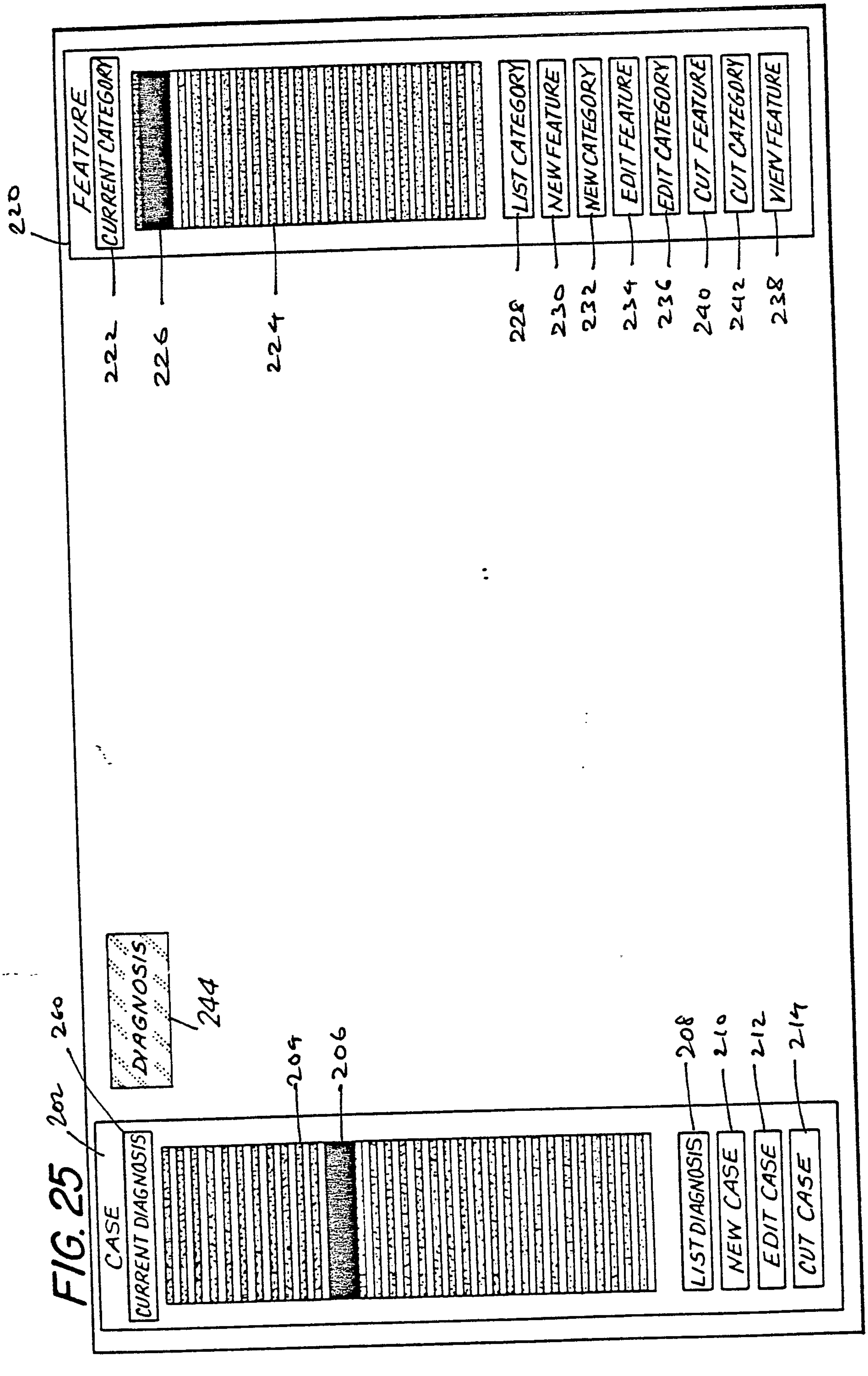

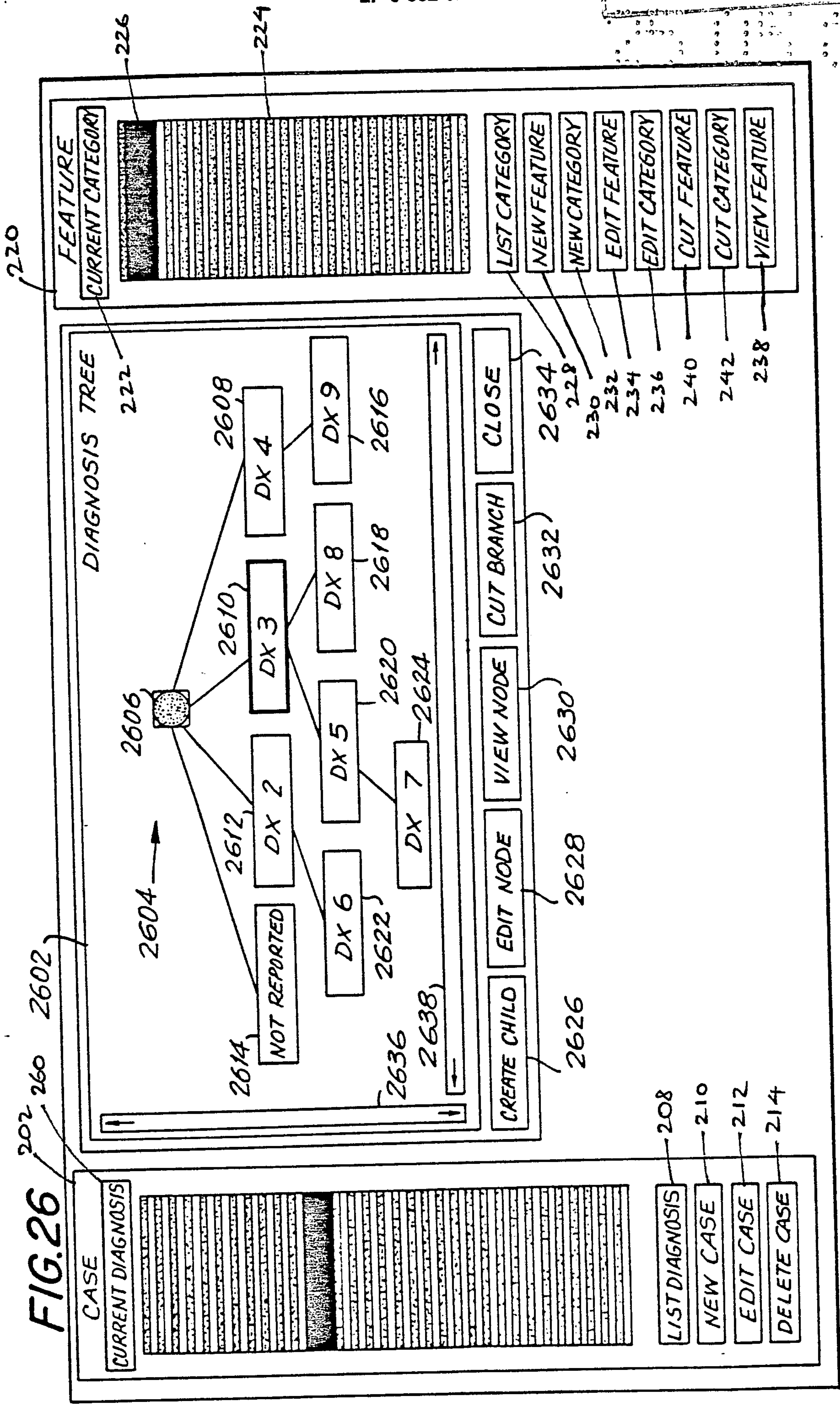
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
DIAGNOSIS TREE
DX 4
DX 9
DX 3
DX 8
DX 5
DX 2
DX 7
DX 6
NOT REPORTED
CLOSE
CUT BRANCH
VIEW NODE
EDIT NODE
CREATE CHILD
CASE
CURRENT DIAGNOSIS
LIST DIAGNOSIS
NEW CASE
EDIT CASE
DELETE CASE
220
222
224
226
228
230
232
234
236
238
240
242
2602
2604
2606
2608
2610
2612
2614
2616
2618
2620
2622
2624
2626
2628
2630
2632
2634
2636
2638
202
260
208
210
212
214

FIG. 26

Neu eingereicht / Newly filed
Nouvellement déposé

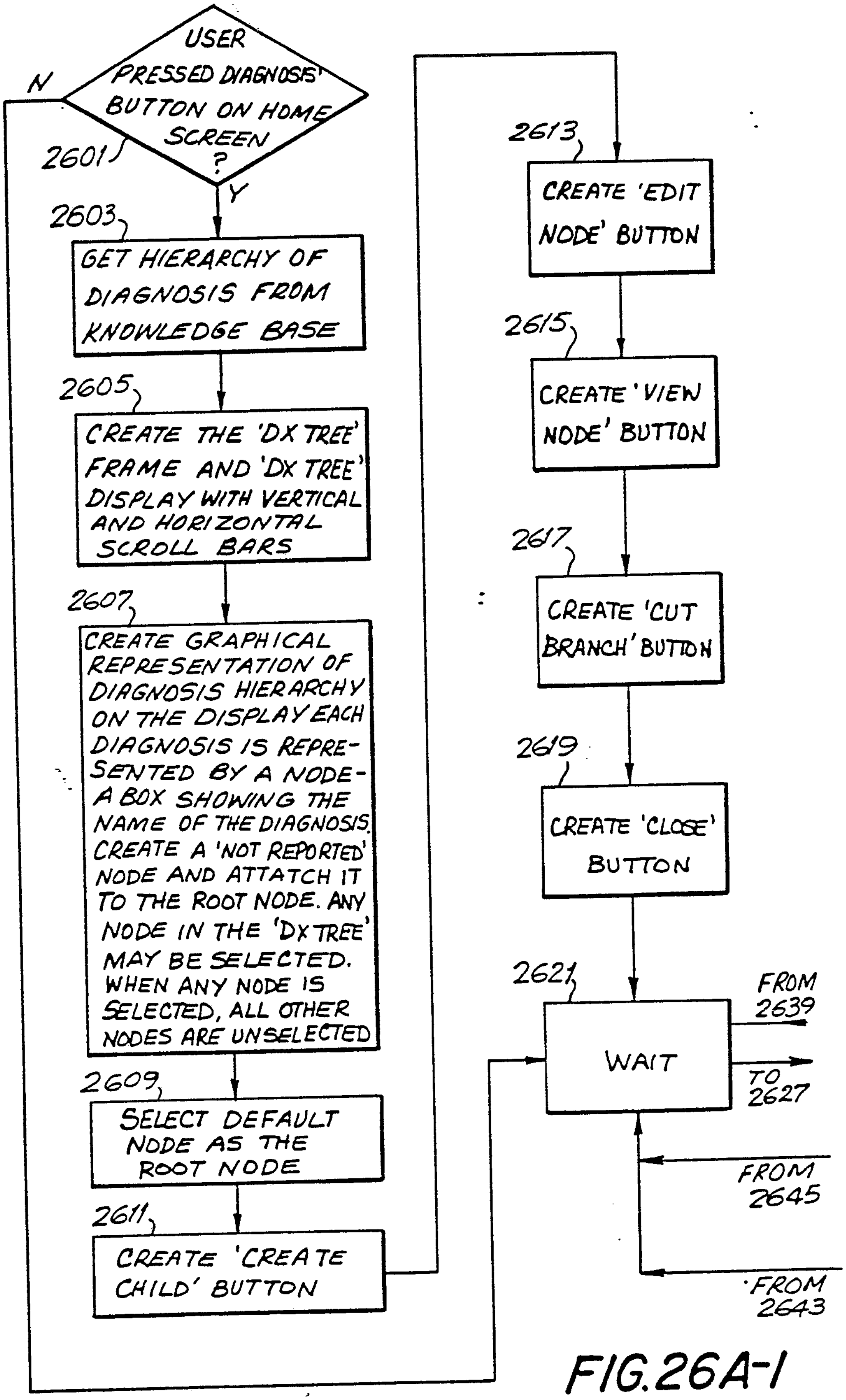


FIG.26A-1

Nouvellement déposé

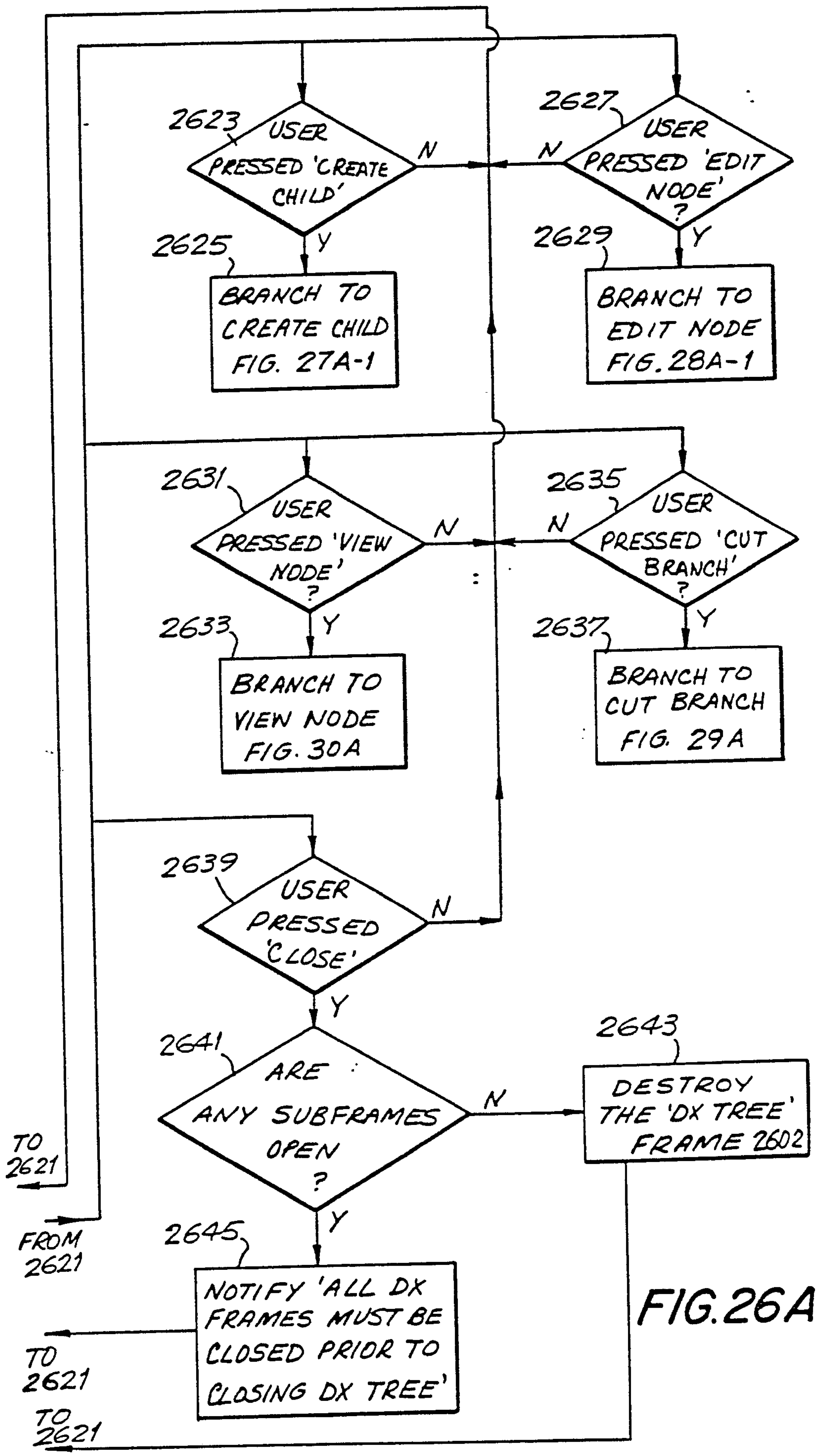


FIG.26A-2

Nouvellement déposé

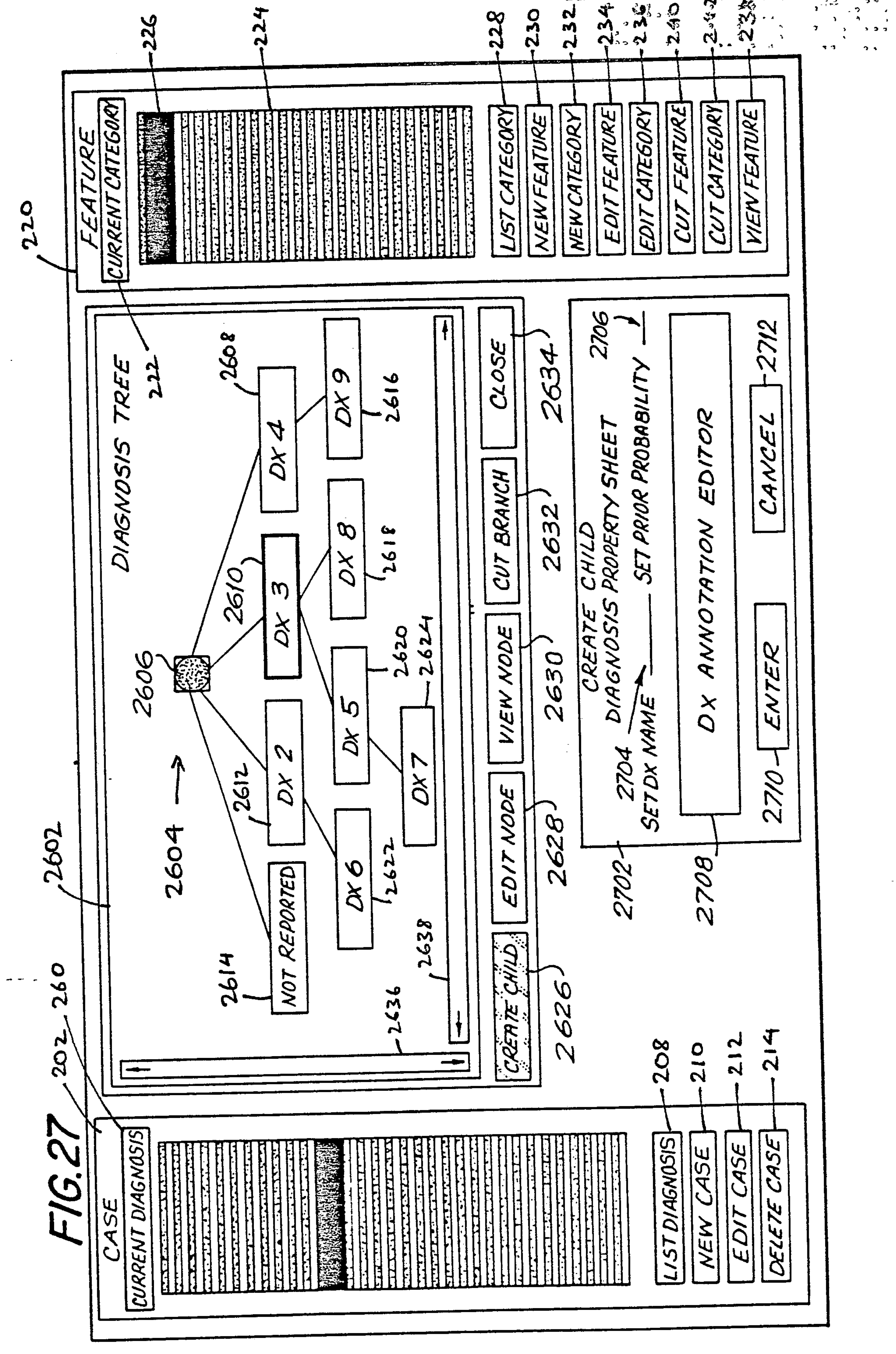


FIG.27

Nouvellement déposé

FIG.27A-1

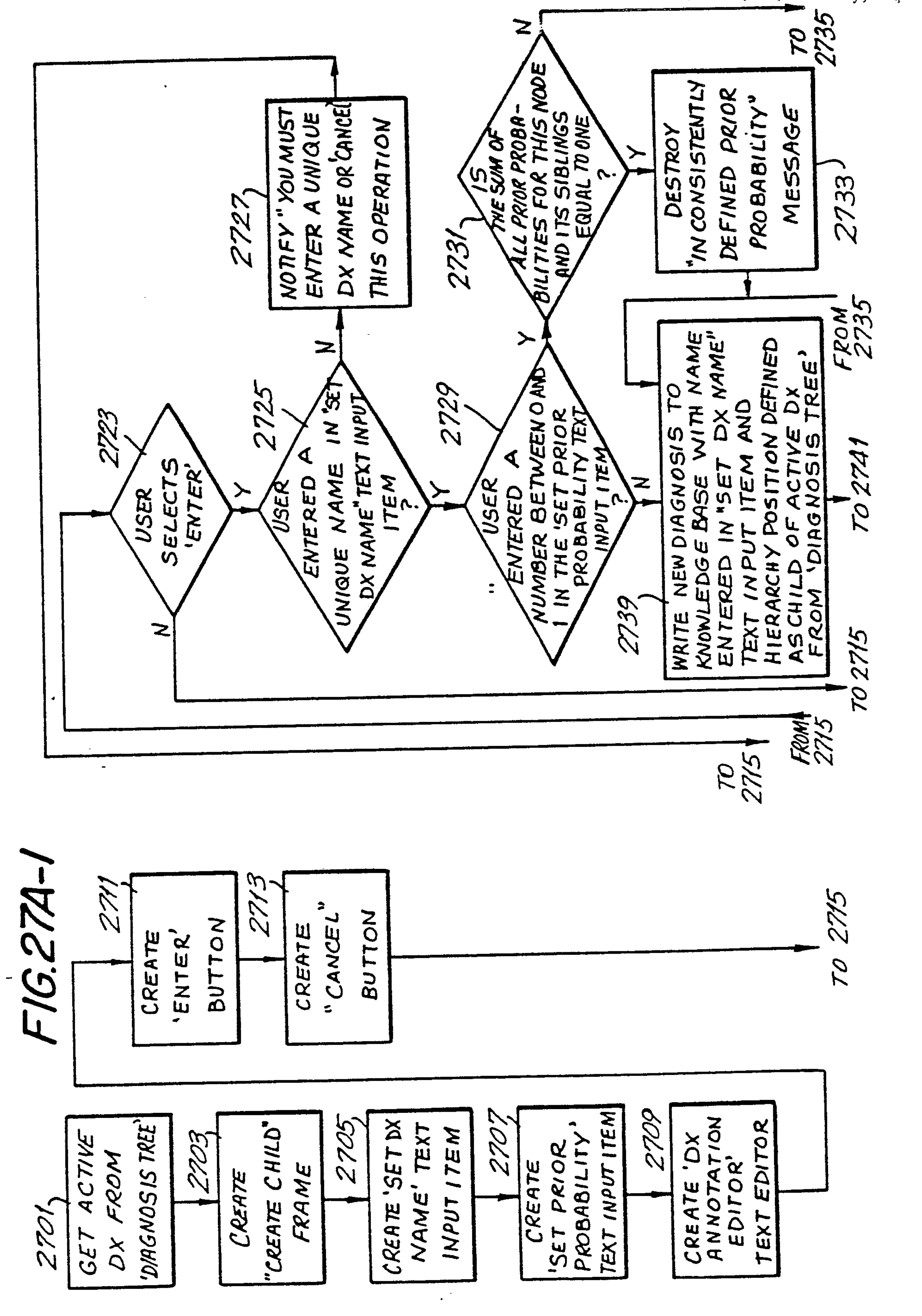

Nouvellement déposé

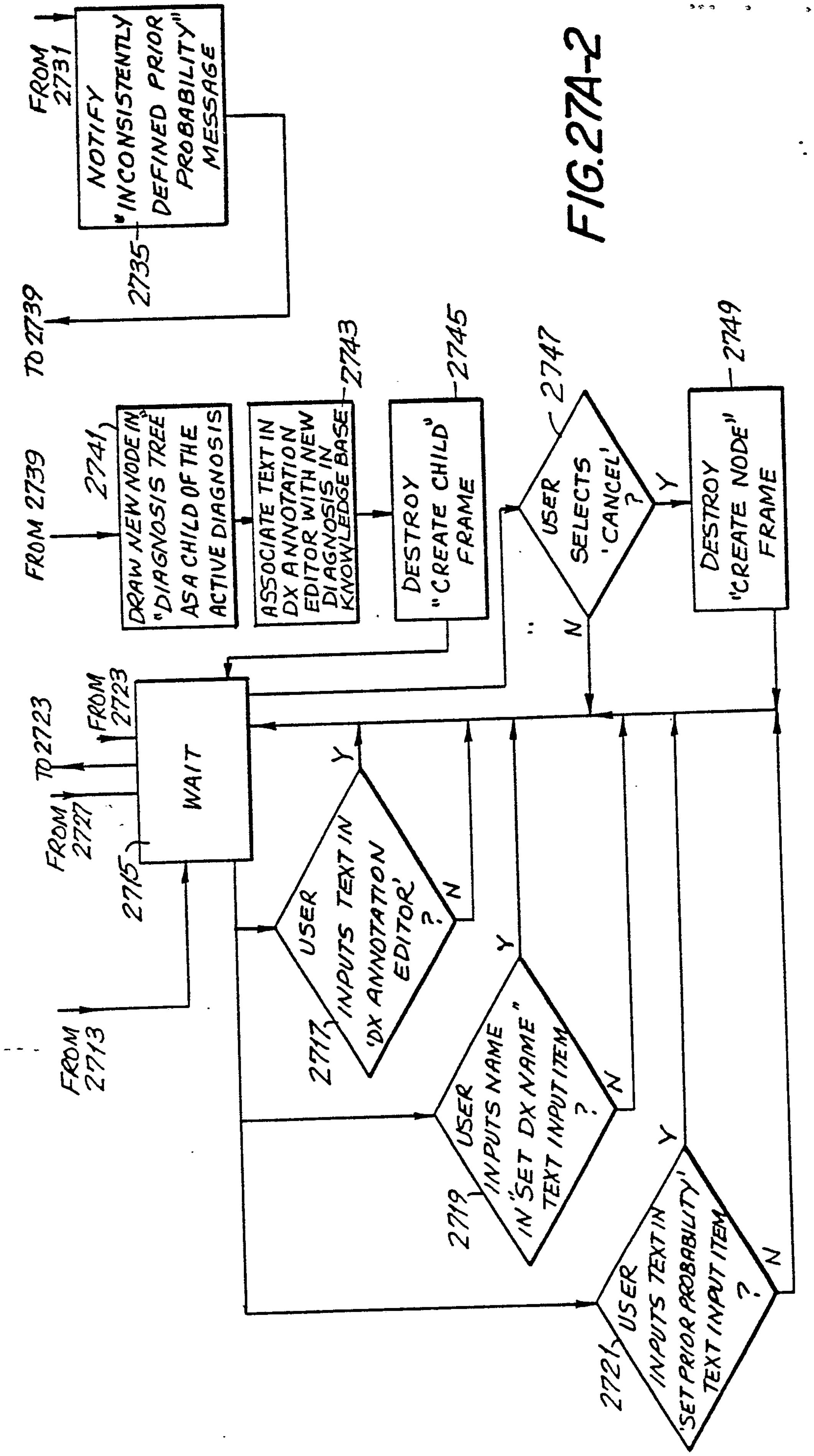


FIG.27A-2

FIG.28

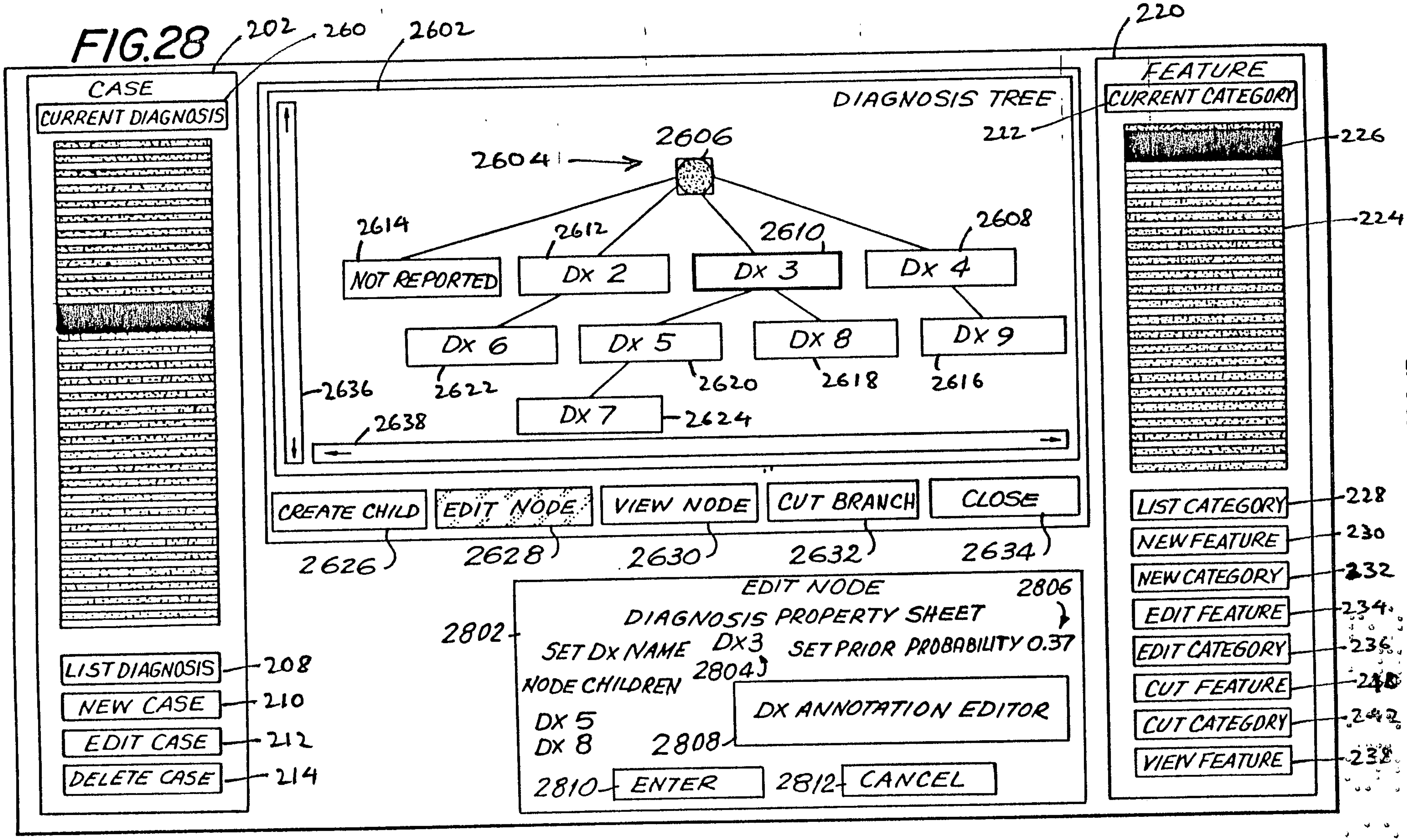
CASE
CURRENT DIAGNOSIS
LIST DIAGNOSIS
NEW CASE
EDIT CASE
DELETE CASE
DIAGNOSIS TREE
NOT REPORTED
Dx 2
Dx 3
Dx 4
Dx 6
Dx 5
Dx 8
Dx 9
Dx 7
CREATE CHILD
EDIT NODE
VIEW NODE
CUT BRANCH
CLOSE
EDIT NODE
DIAGNOSIS PROPERTY SHEET
SET Dx NAME Dx3
SET PRIOR PROBABILITY 0.37
NODE CHILDREN
Dx 5
Dx 8
Dx ANNOTATION EDITOR
ENTER
CANCEL
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
202
260
2602
220
222
226
224
2604
2606
2614
2612
2610
2608
2636
2622
2620
2618
2616
2638
2624
2626
2628
2630
2632
2634
228
230
232
234
236
242
2802
2806
2804
2808
2810
2812
208
210
212
214

FIG.28A-1

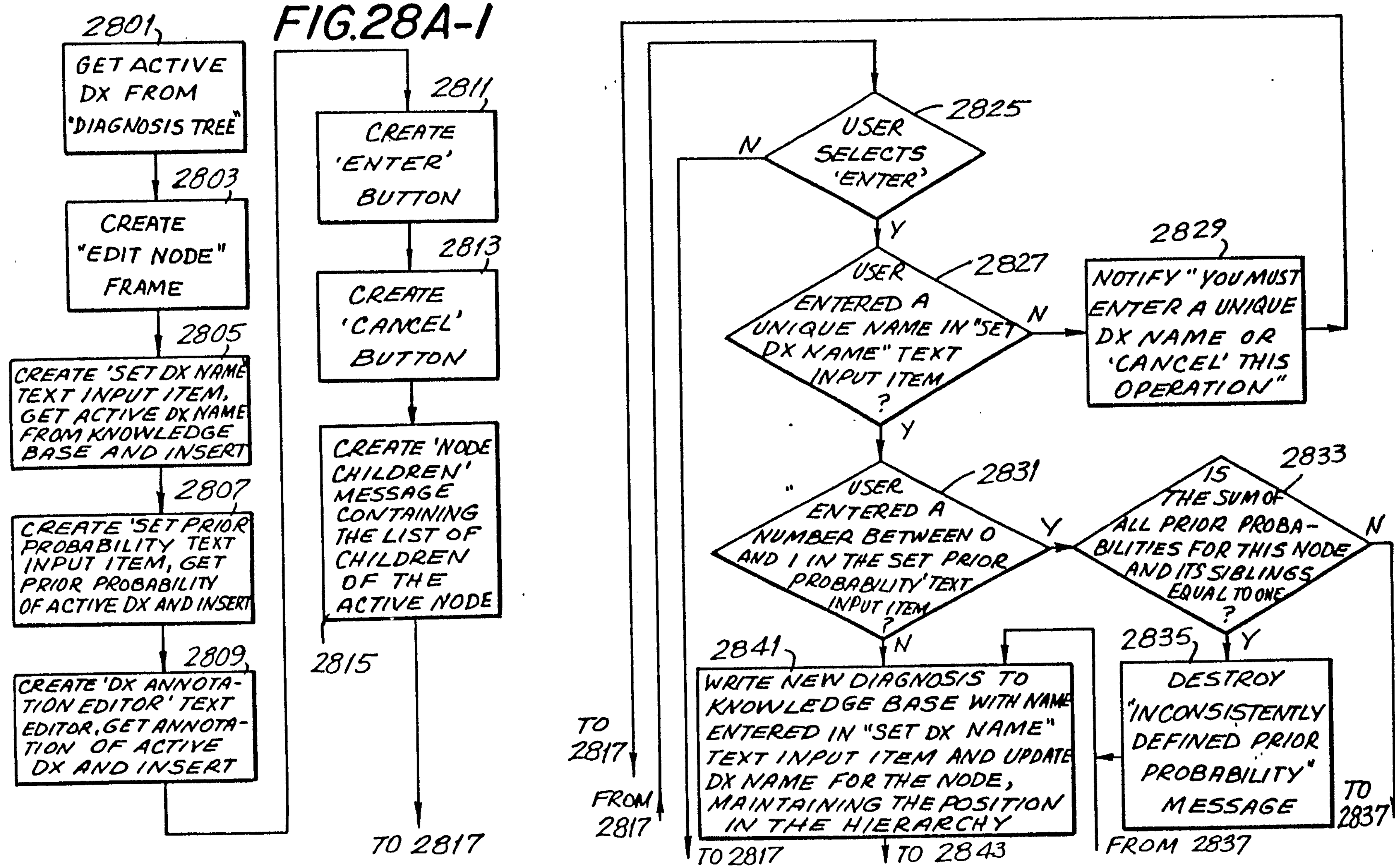
2801
GET ACTIVE DX FROM "DIAGNOSIS TREE"
2803
CREATE "EDIT NODE" FRAME
2805
CREATE 'SET DX NAME' TEXT INPUT ITEM, GET ACTIVE DX NAME FROM KNOWLEDGE BASE AND INSERT
2807
CREATE 'SET PRIOR PROBABILITY' TEXT INPUT ITEM, GET PRIOR PROBABILITY OF ACTIVE DX AND INSERT
2809
CREATE 'DX ANNOTATION EDITOR' TEXT EDITOR, GET ANNOTATION OF ACTIVE DX AND INSERT
2811
CREATE 'ENTER' BUTTON
2813
CREATE 'CANCEL' BUTTON
2815
CREATE 'NODE CHILDREN' MESSAGE CONTAINING THE LIST OF CHILDREN OF THE ACTIVE NODE
TO 2817
2825
USER SELECTS 'ENTER'
N
Y
2827
USER ENTERED A UNIQUE NAME IN "SET DX NAME" TEXT INPUT ITEM ?
N
Y
2829
NOTIFY "YOU MUST ENTER A UNIQUE DX NAME OR 'CANCEL' THIS OPERATION"
2831
USER ENTERED A NUMBER BETWEEN 0 AND 1 IN THE SET PRIOR PROBABILITY' TEXT INPUT ITEM ?
Y
N
2833
IS THE SUM OF ALL PRIOR PROBABILITIES FOR THIS NODE AND ITS SIBLINGS EQUAL TO ONE ?
N
Y
2835
DESTROY "INCONSISTENTLY DEFINED PRIOR PROBABILITY" MESSAGE
2841
WRITE NEW DIAGNOSIS TO KNOWLEDGE BASE WITH NAME ENTERED IN "SET DX NAME" TEXT INPUT ITEM AND UPDATE DX NAME FOR THE NODE, MAINTAINING THE POSITION IN THE HIERARCHY
TO 2817
FROM 2817
TO 2817
TO 2843
FROM 2837
TO 2837

Neu eingereicht / Newly filed
Nouvellement déposé

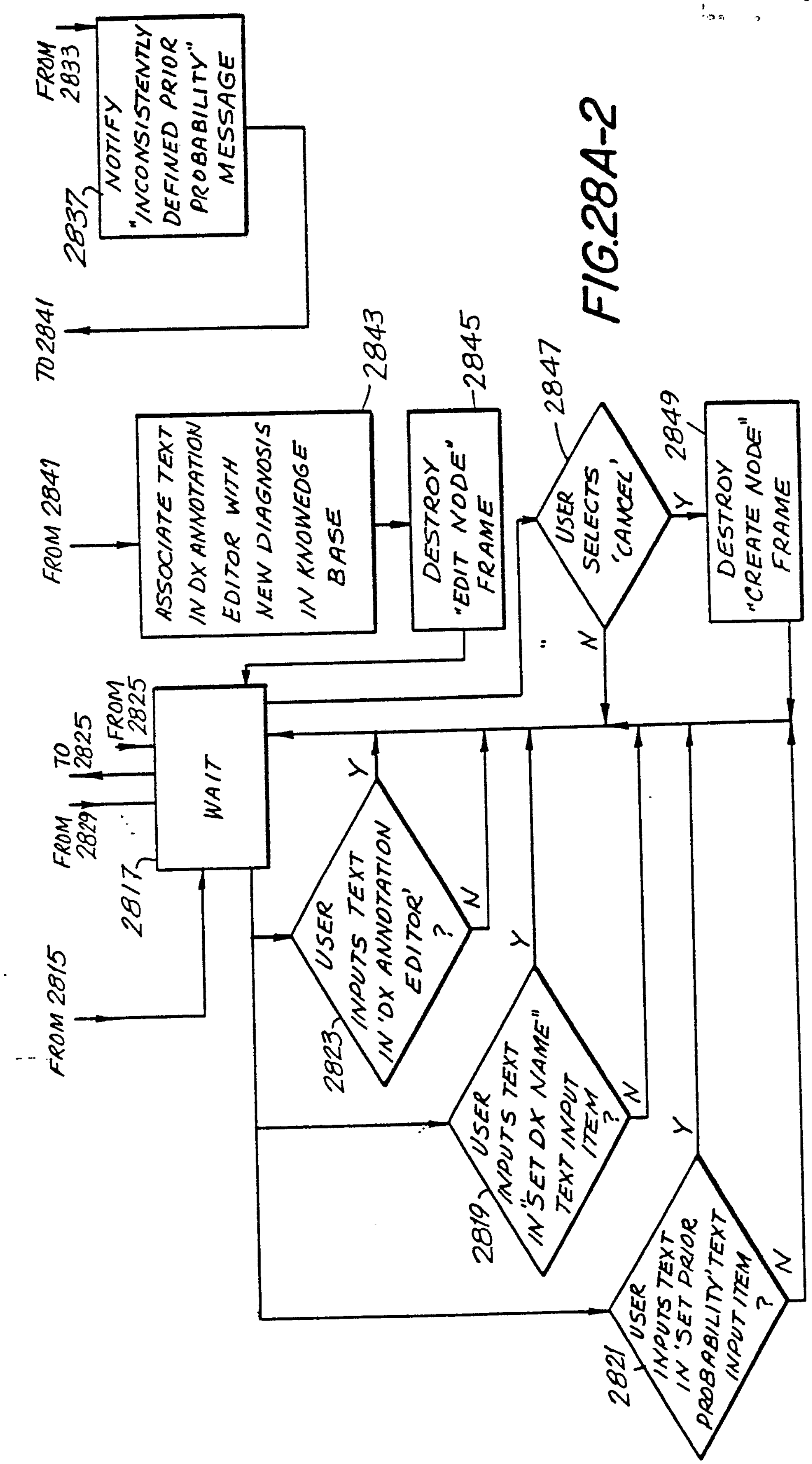


FIG.28A-2

Nouvellement déposé

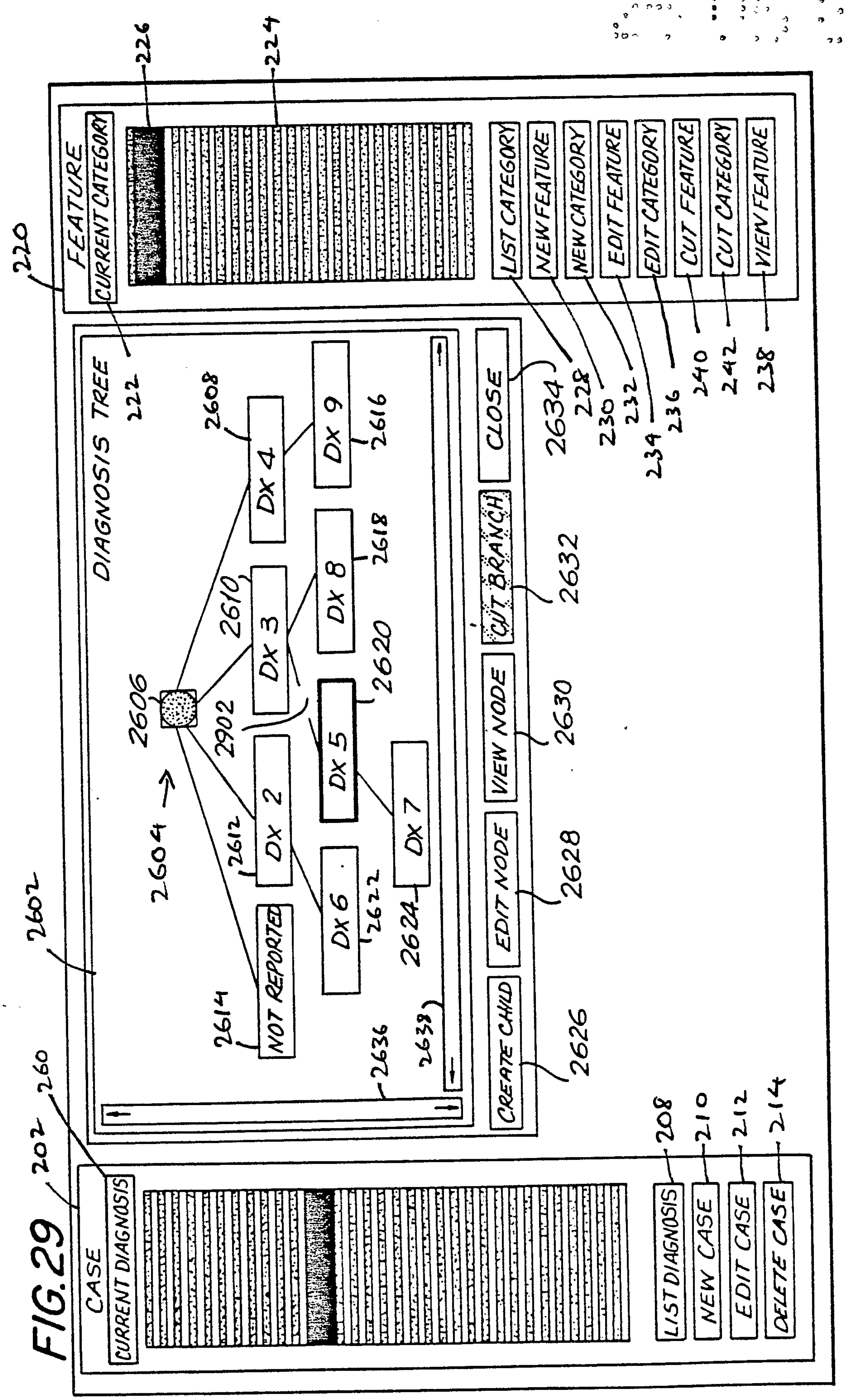


FIG. 29

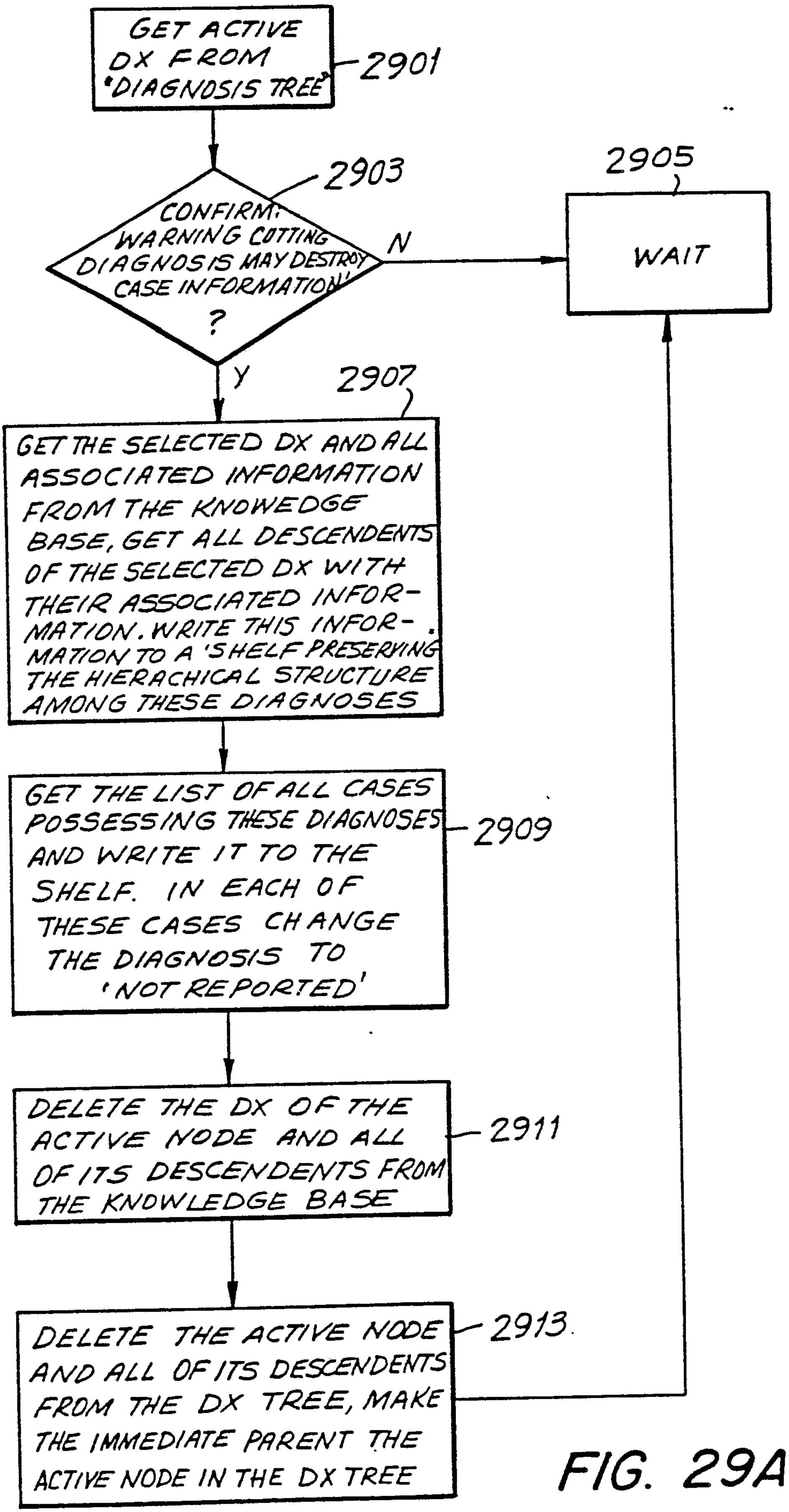
GET ACTIVE DX FROM "DIAGNOSIS TREE"
2901
2903
CONFIRM: "WARNING CUTTING DIAGNOSIS MAY DESTROY CASE INFORMATION" ?
N
2905
WAIT
Y
2907
GET THE SELECTED DX AND ALL ASSOCIATED INFORMATION FROM THE KNOWEDGE BASE, GET ALL DESCENDENTS OF THE SELECTED DX WITH THEIR ASSOCIATED INFORMATION. WRITE THIS INFORMATION TO A 'SHELF' PRESERVING THE HIERACHICAL STRUCTURE AMONG THESE DIAGNOSES
GET THE LIST OF ALL CASES POSSESSING THESE DIAGNOSES AND WRITE IT TO THE SHELF. IN EACH OF THESE CASES CHANGE THE DIAGNOSIS TO 'NOT REPORTED'
2909
DELETE THE DX OF THE ACTIVE NODE AND ALL OF ITS DESCENDENTS FROM THE KNOWLEDGE BASE
2911
DELETE THE ACTIVE NODE AND ALL OF ITS DESCENDENTS FROM THE DX TREE, MAKE THE IMMEDIATE PARENT THE ACTIVE NODE IN THE DX TREE
2913

FIG. 29A

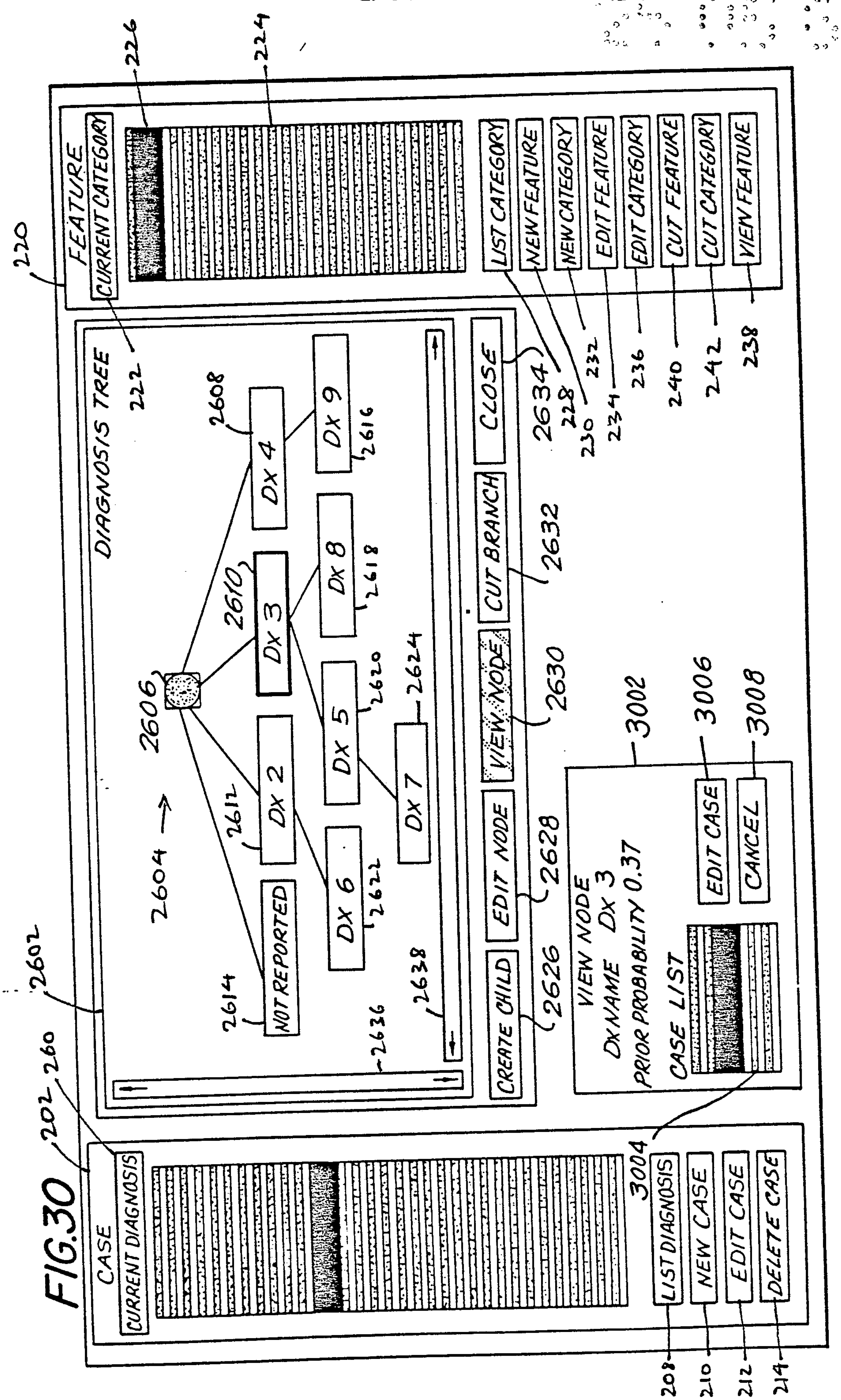
FEATURE
CURRENT CATEGORY
LIST CATEGORY
NEW FEATURE
NEW CATEGORY
EDIT FEATURE
EDIT CATEGORY
CUT FEATURE
CUT CATEGORY
VIEW FEATURE
DIAGNOSIS TREE
NOT REPORTED
DX 2
DX 3
DX 4
DX 5
DX 6
DX 7
DX 8
DX 9
CREATE CHILD
EDIT NODE
VIEW NODE
CUT BRANCH
CLOSE
VIEW NODE
DXNAME DX 3
PRIOR PROBABILITY 0.37
CASE LIST
EDIT CASE
CANCEL
CASE
CURRENT DIAGNOSIS
LIST DIAGNOSIS
NEW CASE
EDIT CASE
DELETE CASE
220
222
224
226
228
230
232
234
236
238
240
242
2602
2604
2606
2608
2610
2612
2614
2616
2618
2620
2622
2624
2626
2628
2630
2632
2634
2636
2638
3002
3004
3006
3008
202
260
208
210
212
214

FIG.30

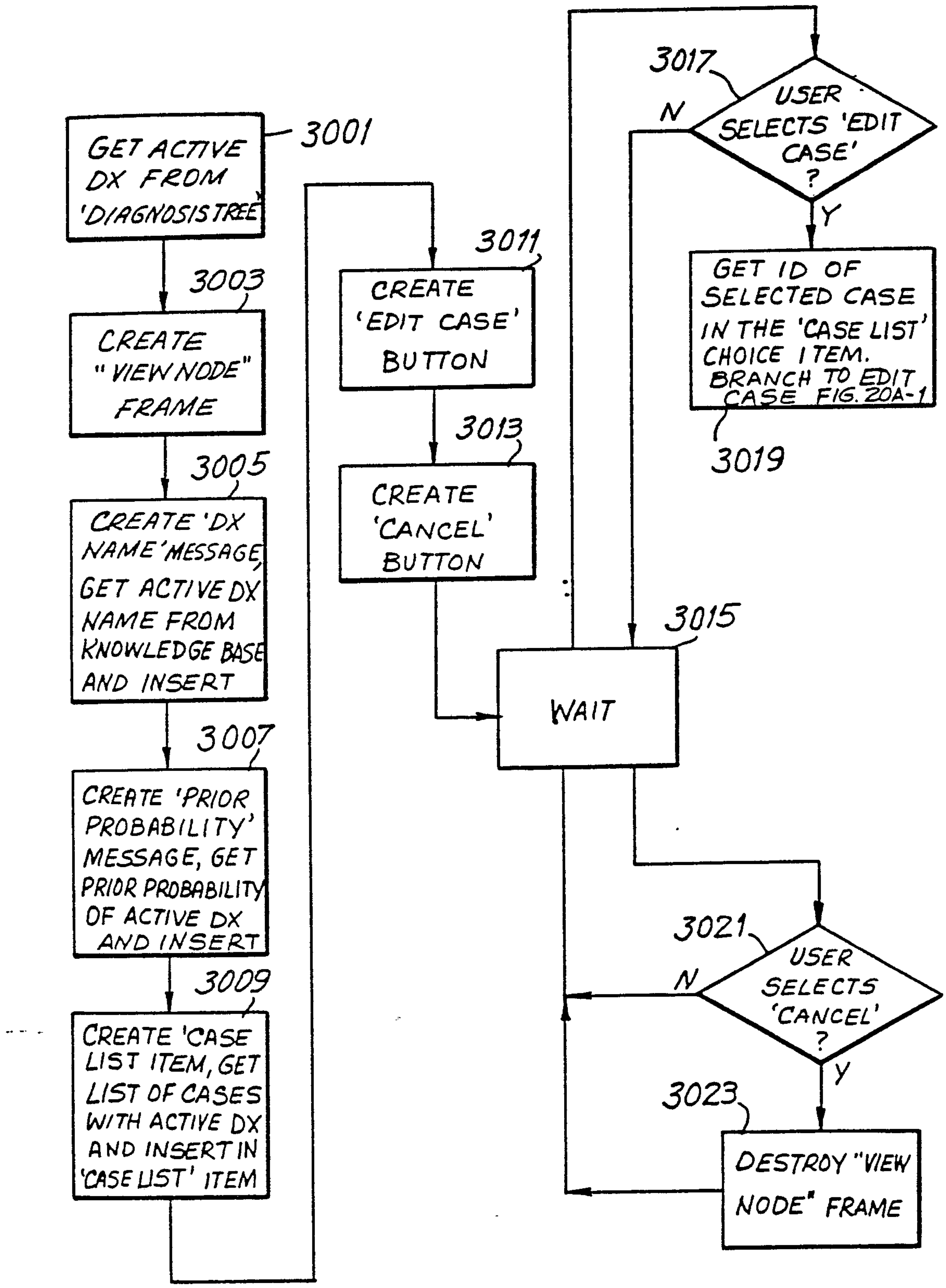
GET ACTIVE DX FROM 'DIAGNOSIS TREE'
3001
3003
CREATE "VIEW NODE" FRAME
3005
CREATE 'DX NAME' MESSAGE, GET ACTIVE DX NAME FROM KNOWLEDGE BASE AND INSERT
3007
CREATE 'PRIOR PROBABILITY' MESSAGE, GET PRIOR PROBABILITY OF ACTIVE DX AND INSERT
3009
CREATE 'CASE LIST ITEM, GET LIST OF CASES WITH ACTIVE DX AND INSERT IN 'CASE LIST' ITEM
3011
CREATE 'EDIT CASE' BUTTON
3013
CREATE 'CANCEL' BUTTON
3015
WAIT
3017
USER SELECTS 'EDIT CASE' ?
N
Y
GET ID OF SELECTED CASE IN THE 'CASE LIST' CHOICE ITEM. BRANCH TO EDIT CASE FIG. 20A-1
3019
3021
USER SELECTS 'CANCEL' ?
N
Y
3023
DESTROY "VIEW NODE" FRAME

FIG. 30A